# EUROPEAN PATENT APPLICATION

(11) **EP 4 506 343 A1**
(43) Date of publication of application: **12.02.2025**
(21) Application number: 23784997.1
(22) Date of filing: 05.04.2023
(51) Int. Cl.: C07D 401/12, A61K 31/517, A61K 31/5383, A61K 31/4709, A61K 31/519, A61P 35/00, C07D 239/94, C07D 471/04, C07D 401/14, C07D 498/04

(54) **HETEROARYL DERIVATIVE AND USE THEREOF**

(30) Priority: 05.04.2022 KR 20220042193
(71) Applicant: Voronoi Inc., Incheon 21984 (KR)
(72) Inventor: KO, Yikyung, Incheon 21984 (KR); LEE, Yeongdeok, Incheon 21984 (KR); IM, Hyerim, Incheon 21984 (KR); KIM, Kyuneun, Incheon 21984 (KR); HWANG, Dongkeun, Incheon 21984 (KR); HAN, Ahreum, Incheon 21984 (KR); NAM, Subeen, Incheon 21984 (KR); HEO, Myunghoe, Incheon 21984 (KR); CHO, Serin, Incheon 21984 (KR); LEE, Seungju, Incheon 21984 (KR); KO, Eunhwa, Incheon 21984 (KR); CHOI, Hwangeun, Incheon 21984 (KR); KIM, Sunghwan, Incheon 21984 (KR); KIM, Daekwon, Incheon 21984 (KR)
(74) Representative: Dehns
(86) International application number: PCT/KR2023/004601
(87) International publication number: WO 2023/195773

(57) **Abstract**

The present invention relates to a heteroaryl derivative and use thereof. The heteroaryl derivative represented by chemical formula (I) and a pharmaceutical composition including same effectively suppress HER2 activity, and thus are useful for the treatment or prevention of HER2-related diseases.

## Description

### Technical Field

The present invention relates to heteroaryl derivatives and pharmaceutical uses thereof. Specifically, the present invention relates to heteroaryl derivatives having HER2 inhibitory activity.

The research on the present invention was conducted by the support of the National Drug Development Project by Korea Drug Development Fund with the funding of the Ministry of Science and ICT, the Ministry of Trade, Industry and Energy, and the Ministry of Health and Welfare (Project ID: HN21C0826).

### Background Art

Human epidermal growth factor receptor 2 (HER2) is a human EGFR family member, and abnormal expression or activity of HER2 is known to be a major cause of various cancers such as breast cancer, lung cancer, gastric cancer, and ovarian cancer (J Control Release, 2010, 146(3), 264-275). In particular, the amplification of the HER2 gene or the overexpression of the HER2 protein is observed in about 15-25% of breast cancer patients, which is classified as HER2-positive. Cancer is caused by wild-type HER2 as well as various mutations of HER2, and for example, L755S, V777L, D769H/Y, T862A, and L755_T759del in the kinase domain are known.

Trastuzumab (Herceptin^{™}) and pertuzumab (Perjeta^{™}), which are representative HER2 monoclonal antibodies, inhibit HER2 signaling mechanisms and are thus used as therapeutic agents for breast cancer, and it has been reported that they improve clinical outcomes compared to chemotherapy alone (Journal of Clinical Oncology, 2002, 20(3), 719-726). An antibody-drug conjugate (ADC) made by attaching a cytotoxic drug to a HER2 selective antibody is also being developed as a related therapeutic agent, and drugs such as lapatinib (Tykerb^{™}) and tucatinib (Tukysa^{™}), which are low molecular compounds, have been developed and used as a breast cancer therapeutic agent.

It has been reported that patients with HER2 mutation in data of phase 1 and 2 clinical trials had partial responses to HER2 inhibitors, afatinib (Gilotrif^{™}), neratinib (Nerlynx^{™}), or dacomitinib (Vizimpro^{™}) (Annals of Oncology, 2015, 26(7), 1421-1427). Specifically, the SUMMIT clinical trial reported that the objective response rate (ORR) of HER2 mutant breast cancer patients administered neratinib was 24%, the ORR of HER2 mutant lung cancer patients was 3.8%, and other cancers having HER2 mutations were all 0% (Nature, 2018, 554 (7691), 189-194). However, different results of the ORR were found depending on the disease, suggesting that the HER2 inhibitory efficacy was cancer-specific, and there were also variant-specific differences in a single cancer (Nature, 2018, 554 (7691), 189-194). Studies on HER2 monoclonal antibodies and antibody-drug conjugates (ADCs) also found similar results as above, showing different ORRs depending on the type of mutations. This shows that there is still a need to prevent or treat various HER2 mutations.

### [Prior Art Document]

(Non-Patent Document 1) J Control Release, 2010, 146(3), 264-275
(Non-Patent Document 2) Journal of Clinical Oncology, 2002, 20(3), 719-726
(Non-Patent Document 3) Annals of Oncology, 2015, 26(7), 1421-1427
(Non-Patent Document 4) Nature, 2018, 554 (7691), 189-194

### Disclosure of Invention

### Technical Problem

As a result of intensive efforts to develop a new drug, the present inventors have found that compounds of the present invention effectively inhibit HER2 and thus have the therapeutic and prophylactic effects of HER2-related diseases, thereby completing the present invention.

Accordingly, an object of the present invention is to provide novel heteroaryl derivatives, or pharmaceutically acceptable salts or stereoisomers thereof, and a method for preparing the same.

Another object of the present invention is to provide a pharmaceutical use of the heteroaryl derivatives or pharmaceutically acceptable salts or stereoisomers thereof.

### Solution to Problem

According to the above objects, the present invention provides a compound represented by Formula (I) below, or a pharmaceutically acceptable salt or stereoisomer thereof. 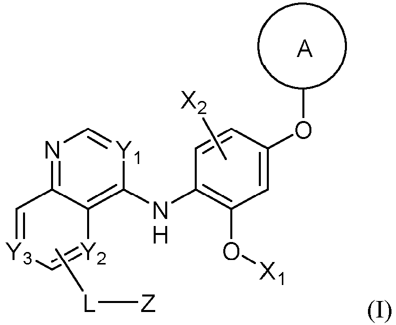 wherein ring A, X₁, X₂, Y₁, Y₂, Y₃, L, and Z are the same as defined in the present specification.

### Advantageous Effects of Invention

The compound represented by Formula (I), a stereoisomer thereof, or a pharmaceutically acceptable salt thereof may exhibit excellent inhibitory activity against HER2. Accordingly, the compound of the present invention can be used as an HER2 inhibitor, and a pharmaceutical composition including the same is useful as a therapeutic agent for HER2-related diseases, in particular, cancer.

### Best Mode for Carrying out the Invention

Hereinafter, the present invention will be described in more detail.

Embodiments of the present invention may be modified in different forms, and the scope of the present invention is not limited to the embodiments described below. The embodiments of the present invention are also provided to more fully explain the present invention to those skilled in the art. Furthermore, throughout the specification, a part "including" a component means that it may further include other components rather than exclude other components, unless otherwise stated.

In the present specification, the term "halo" or "halogen" refers to fluoro (F), chloro (Cl), bromo (Br), or iodo (I) unless otherwise stated.

The term "alkyl" refers to a radical in which one hydrogen is removed from a linear or branched saturated hydrocarbon group, unless otherwise specified. For example, "C₁₋₁₀ alkyl" refers to an alkyl having a skeleton formed of 1 to 10 carbon atoms. Specifically, C₁₋₁₀ alkyl may include methyl, ethyl, n-propyl, i-propyl, n-butyl, i-butyl, *tert*-butyl, n-pentyl, i-pentyl, *tert-*pentyl, *sec*-pentyl, neopentyl, hexyl, heptyl, octyl, nonyl, decyl, and the like.

The term "alkoxy" refers to an alkyl group linked by -O-, and for example, C₁₋₄ alkoxy may include methoxy, ethoxy, n-propoxy, i-propoxy, n-butoxy, i-butoxy, *sec*-butoxy, *tert*-butoxy, and the like, but is not limited thereto.

The term "alkylamino" refers to amino substituted with one alkyl or two alkyls. For example, C₁₋₆ alkylamino may refer to amino substituted with one or two C₁₋₆ alkyl groups, that is, -NH(C₁₋₆ alkyl) or -N(C₁₋₆ alkyl)₂, and may include dimethylamino, diethylamino, methylethylamino, methylpropylamino, and ethylpropylamino, but is not limited thereto.

The term "hydroxyalkyl" refers to a linear or branched alkyl having a carbon atom substituted with hydroxy (-OH), and includes, for example, methyl, ethyl, propyl, isopropyl, isobutyl, and n-butyl substituted with hydroxy, but is not limited thereto. Similarly, "hydroxyalkoxy" refers to a linear or branched alkoxy having a carbon atom substituted with hydroxy (-OH).

The term "haloalkyl" refers to a linear or branched alkyl having a carbon atom substituted with one or more halos (F, Cl, Br, and I). Examples of the haloalkyl include, but are not limited to, methyl, ethyl, propyl, isopropyl, isobutyl, and n-butyl substituted with one or more halos.

The term "deuterated" means that, for example, at least one hydrogen atom (H) present in a hydrocarbon group is substituted with a deuterium atom (D), and as a specific example, deuterated C₁₋₆ alkyl means that at least one hydrogen atom bonded to a carbon atom in C₁₋₆ alkyl is substituted with a deuterium atom.

The term "carbocycle" refers to an aromatic or non-aromatic hydrocarbon ring, which may be saturated or unsaturated, which may include a monocyclic ring or polycyclic ring. For example, the term "C₃₋₁₀ carbocycle" refers to a carbocycle having a skeleton formed of 3 to 10 carbon atoms, and may have one or more substituents. The carbocycle may include, but is not limited to, a C₃₋₁₅ carbocycle, a C₃₋₁₀ carbocycle, a C₃₋₇ carbocycle, a C₆₋₁₂ carbocycle, and the like depending on the number of carbon atoms constituting the ring. In a specific example, aromatic C₆₋₁₀ carbocycles include benzene and naphthalene.

The term "cycloalkyl" refers to a non-aromatic hydrocarbon ring radical, which may include a monocyclic ring or polycyclic ring. The cycloalkyl may include a saturated ring, and specific examples thereof may include cyclopropyl, cyclobutyl, cyclopentyl, cyclohexyl, adamantyl, and the like. Further, the cycloalkyl may include an unsaturated ring having one or more carbon-carbon double bonds in the ring as long as the ring is not rendered aromatic by the presence of the double bonds, and for example, cyclopentenyl or the like may also be included in the category. The cycloalkyl may include, but is not limited to C₃₋₁₄ cycloalkyl, C₃₋₁₂ cycloalkyl, C₃₋₁₀ cycloalkyl, C₃₋₆ cycloalkyl, C₆₋₁₂ cycloalkyl, and the like depending on the number of carbon atoms constituting the ring.

The term "aryl" refers to an aromatic hydrocarbon ring radical, which may include a monocyclic ring or polycyclic ring. Specific examples of the aryl may include, but are not limited to, phenyl, naphthalenyl, fluorenyl, anthracenyl, phenanthrenyl, biphenyl, terphenyl, and the like. Further, for the aryl, any hydrocarbon ring radical having at least one ring in which electrons are delocalized due to a single bond and a double bond arranged alternately, that is, a conjugated π bond may be included in the category. The aryl may include, but is not limited to, C₆₋₁₂ aryl, C₆₋₁₀ aryl, or the like depending on the number of carbon atoms constituting the ring.

The term "polycyclic ring" is at least a bicyclic ring, and includes a spiro ring, a fused or condensed ring, or a bridged ring.

The term "heterocycle" refers to an aromatic or non-aromatic ring having one or more heteroatoms, which may be saturated or unsaturated, and may include a monocyclic ring or polycyclic ring. For example, "5-12 membered heterocycle" refers to a heterocycle having a skeleton formed of a total of 5 to 12 atoms including a heteroatom and a carbon atom, and may have one or more substituents. The heterocycle may include, but is not limited to, a 5-12 membered heterocycle, a 5-10 membered heterocycle, a 5-7 membered heterocycle, and the like depending on the number of atoms constituting the ring.

The term "heteroaryl" refers to an aromatic heterocyclyl having one or more heteroatoms in the ring, which may include a monocyclic ring or polycyclic ring. The heteroaryl may be a radical in which one hydrogen atom is removed from an aromatic heterocycle, for example, thiophene, purine, pyrrole, pyrazole, imidazole, thiazole, oxazole, isothiazole, oxadiazole, triazole, pyridine, bipyridyl, triazine, acridyl, pyridazine, pyrazine, quinoline, quinazoline, quinoxaline, phenoxazine, phthalazine, pyrimidine, pyridopyrimidine, pyridopyrazine, pyrazinopyrazine, isoquinoline, indole, carbazole, imidazopyridazine, imidazopyridine, imidazopyrimidine, pyrazolopyrimidine, imidazopyrazine, pyrazolopyridine, triazolopyridine, triazolopyrimidine, *N*-arylcarbazole, *N*-heteroarylcarbazole, *N*-alkylcarbazole, benzoxazole, benzimidazole, benzothiazole, benzocarbazole, benzothiophene, dibenzothiophene, thienothiophene, benzofuran, phenanthroline, isoxazole, oxadiazole, thiadiazole, benzothiazole, tetrazole, phenothiazine, dibenzosilole, dibenzofuran, pyrazolopyridine, or the like, but is not limited thereto. Further, for the heteroaryl, any heterocyclic radical having at least one ring in which electrons are delocalized due to a single bond and a double bond arranged alternately, that is, a conjugated π bond may be included in the category. In addition, the heteroaryl may include, but is not limited to, 5-14 membered heteroaryl, 5-10 membered heteroaryl, 5-6 membered heteroaryl, 6-12 membered heteroaryl, 6-10 membered heteroaryl, and the like, depending on the number of atoms constituting the ring.

The term "heterocycloalkyl" refers to a non-aromatic heterocyclyl having one or more heteroatoms in the ring, which may include a monocyclic ring or polycyclic ring. The heterocycloalkyl may include a saturated ring. Further, the heterocycloalkyl may have one or more carbon-carbon double bonds or carbon-heteroatom double bonds in the ring as long as the ring is not rendered aromatic by the presence of the double bonds. The heterocycloalkyl may include, but is not limited to, 3-15 membered heterocycloalkyl, 3-12 membered heterocycloalkyl, 4-10 membered heterocycloalkyl, 4-8 membered heterocycloalkyl, 5-14 membered heterocycloalkyl, 5-12 membered heterocycloalkyl, or the like depending on the number of atoms constituting the ring. The heterocycloalkyl may include a radical in which one hydrogen atom is removed from a non-aromatic heterocycle, for example, aziridine, azetidin, pyrrolidine, piperidine, N-methylpiperidine, imidazolidine, pyrazolidine, butyrolactam, valerolactam, imidazolidinone, hydantoin, dioxolane, phthalimide, piperidine, pyrimidine-2,4(1*H*,3*H*)-dione, 1,4-dioxane, morpholine, thiomorpholine, thiomorpholine-*S*-oxide, thiomorpholine-*S*,*S*-oxide, piperazine, pyran, pyridone, 3-pyrroline, thiopyran, pyrone, tetrahydrofuran, tetrahydrothiophene, quinuclidine, tropane, isoxazolidine, benzo[d]isoxazolidine, oxazine, azabicyclo[2,2,1]heptane, 2-azaspiro[3.3]heptane, 5-oxa-6-azaspiro[2.4]heptane, (1*R*,5*S*)-3-azabicyclo[3.2.1]octane, (1s,4s)-2-azabicyclo[2.2.2]octane, diazaspiro[5.5]undecane, diazabicyclo[2.2.1]heptane, 2-azaspiro[3.3]heptane, (1*R*,5*S*)-3-azabicyclo[3.2.1]octane, (1s,4s)-2-azabicyclo[2.2.2]octane, (1*R*,4*R*)-2-oxa-5-azabicyclo[2.2.2]octane, or the like, but is not limited thereto.

The term "heteroatom" refers to an atom other than carbon (C), and may be, for example, O, N, P, Si, or S atom, more specifically, N, O, or S atom. The heterocycle, heteroaryl, and heterocycloalkyl as stated herein may include one or more heteroatoms, and, for example, 1, 1 to 2, 1 to 3, or 1 to 4 heteroatoms.

The term "substitution" refers to replacing a hydrogen atom in a molecular structure with a substituent, such that the valence on the designated atom is not exceeded, and such that a chemically stable compound results from the substitution. For example, "group Ais substituted with substituent B" or "group A has substituent B" means that a hydrogen atom bonded to an atom, such as carbon, which constitutes a skeleton of group A, is replaced with substituent B so that the group A and the substituent B form a covalent bond. Thus, it is substantially difficult or impossible for a group having no removable hydrogen atom to have a substituent. From this viewpoint, in a case where a range of combinations of various groups, which include groups that hardly have a substituent, with substituents are exemplified in the present specification, it should be interpreted that combinations of the groups, for which it is obvious that no substitution is possible, with the substituents are excluded from the range. Non-limiting examples of such substituents may include at least one selected from the group consisting of halo (F, Cl, Br, or I), C₁₋₁₀ alkyl, C₂₋₁₀ alkenyl, C₂₋₁₀ alkynyl, hydroxy, C₁₋₁₀ alkoxy, amino, nitro, thiol, thioether, imine, cyano, phosphine, carboxy, carbamoyl, acetal, thiocarbonyl, sulfonyl, sulfonamide, ketone, aldehyde, ester, acetyl, amide, oxo (=O), haloalkyl (*e*.*g*., trifluoromethyl), substituted aminoacyl and aminoalkyl, a monocyclic ring or fused or non-fused polycyclic ring as carbocyclic cycloalkyl (*e*.*g*., cyclopropyl, cyclobutyl, cyclopentyl, or cyclohexyl), or a monocyclic ring or fused or non-fused polycyclic ring as hetero cycloalkyl (*e*.*g*., pyrrolidinyl, piperidinyl, piperazinyl, morpholinyl, or thiazinyl), a carbocycle, a heterocyclic ring, a monocyclic ring, a fused polycyclic ring, a non-fused polycyclic ring, a fused polycyclic aryl or non-fused polycyclic aryl (*e.g*., phenyl, naphthyl, pyrrolyl, indolyl, furanyl, thienyl, imidazolyl, oxazolyl, isoxazolyl, thiazolyl, triazolyl, tetrazolyl, pyrazolyl, pyridinyl, quinolinyl, isoquinolinyl, acridinyl, pyrazinyl, pyridazinyl, pyrimidinyl, benzimidazolyl, benzothienyl, or benzofuranyl), amino (primary, secondary, or tertiary), aryl, aryloxy, and aryl-alkyl. In addition, each of the substituents exemplified above may be unsubstituted or substituted again with a substituent selected from the group of these substituents.

One or more substituents of the same or different kinds mentioned above may be substituted at the same position or different positions, and may be sequentially substituted. The term "sequential" means that one substituent is substituted in the formula and then another substituent is continuously substituted at the substituent, and for example, in the case where an alkyl group is substituted, a cycloalkyl group is then substituted at the alkyl group, and a carbonyl group is sequentially substituted at the cycloalkyl group, the nomenclature as carbonylcycloalkylalkyl may indicate that the group is formed by sequential substitution.

In the chemical structural formula shown in the present specification, the symbol (C) of carbon atom and the symbol (H) of hydrogen atom may be omitted according to the typical way to represent a chemical structural formula, and it should be understood that, for example, even though there is no sign of a hydrogen atomic symbol, hydrogen atoms having the number satisfying the valence of carbon forming a skeleton are actually bonded to carbon atoms. However, when deuterium (D) is indicated in the chemical structural formula, it should be understood that deuterium (D) is bonded to carbon (C).

In the present specification, the symbol "-" for connecting atoms and/or groups may mean a single bond, and the symbol "=" may mean a double bond. The symbol may be omitted, and may be indicated when necessary, such as the case in which a bonding atom or a bonding position is specified.

The bonding directions of linking radicals described herein are arbitrary unless otherwise specified. For example, the radical L linked in 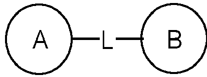 is -M-W-, where the -M-W- can link Ring A and Ring B in the same direction as the reading order from left to right to form 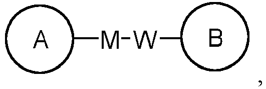 and can link Ring A and Ring B in the opposite direction to the reading order from left to right to form

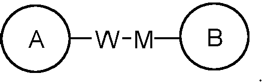

As used herein, the term "stereoisomer" refers to a compound having the same chemical formula or molecular formula, but being sterically different. In the present specification, the stereoisomer includes an enantiomer and a diastereomer, and the diastereomer also includes a conformational isomer, such as a rotamer, and a cis/trans isomer, wherein the respective isomers and mixtures thereof are included in the scope of the present invention. For example, Formula (I) of the present invention may include the stereoisomers of Formula (I) because the stereochemical structure is not specified. Specifically, the solid line bond ( 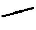 ) connected to the asymmetric carbon atom in the chemical structural formula may include a solid wedged bond ( 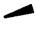 ) and a dashed wedged bond ( 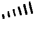 ) indicating an absolute arrangement of a stereocenter. It should be interpreted that stereoisomers of Example Compounds described in the present specification are also included within the scope of the present invention.

### Heteroaryl Derivatives

One aspect of the present invention provides a compound represented by Formula (I), or a pharmaceutically acceptable salt or stereoisomer thereof: 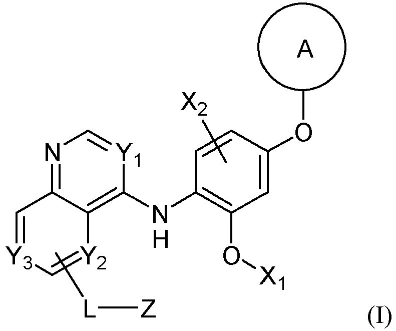 wherein:
ring A is an aromatic or non-aromatic C₆₋₁₂ carbocycle, or 5-12 membered heterocycle, wherein ring A has or does not have one or more substituents selected from the group consisting of C₁₋₆ alkyl, C₁₋₆ hydroxyalkyl, C₁₋₆ haloalkyl, -CN, -NR_{A1}R_{A2}, -NO₂, -OR_{A3}, -C(=O)-NR_{A1}R_{A2}, -C(=O)-OR_{A3}, -NH-C(=O)-(C₁₋₆ alkyl), =O, halo, and 5-6 membered heteroaryl, wherein the heteroaryl is unsubstituted or substituted with C₁₋₆ alkyl, C₁₋₆ haloalkyl, C₁₋₆ alkoxy, or halo;
R_{A1} to R_{A3} are each independently H or C₁₋₆ alkyl;
X₁ is C₁₋₆ alkyl, deuterated C₁₋₆ alkyl, deuterated C₁₋₆ haloalkyl, C₁₋₆ haloalkyl, C₃₋₁₂ cycloalkyl, or 3-12 membered heterocycloalkyl, wherein the cycloalkyl and heterocycloalkyl are unsubstituted or substituted with C₁₋₆ alkyl or C₁₋₆ haloalkyl;
X₂ is H, C₁₋₆ alkyl, C₃₋₆ cycloalkyl, C₁₋₆ hydroxyalkyl, C₁₋₆ haloalkyl, -CN, -NR_{B1}R_{B2}, - NO₂, -OR_{B3}, or halo;
R_{B1} to R_{B3} are each independently H or C₁₋₆ alkyl;
Y₁ is CR₁ or N;
Y₂ is CR₂, C(-L-Z), or N;
Y₃ is CR₃ or N;
R₁ is H_{,} C₁₋₆ alkyl, C₁₋₆ alkylamino, or -CN;
R₂ is H, C₁₋₆ alkyl, C₁₋₆ hydroxyalkyl, C₁₋₆ alkylamino, C₁₋₆ haloalkyl, -CN, -NH₂, or halo;
R₃ is H, halo, -OR₄, or -NR₅R₆, or R₃ is linked to L to form a 5-6 membered heterocycle;
R₄ is H, C₁₋₆ alkyl, -(CH₂)n-O-(C₁₋₆ alkyl), C₁₋₆ hydroxyalkyl, C₁₋₆ haloalkyl, -(CH₂)n-(C₃₋₆ cycloalkyl), 3-6 membered heterocycloalkyl, or -(CH₂)n-(3-6 membered heterocycloalkyl), wherein the cycloalkyl and heterocycloalkyl have or do not have one or more substituents selected from the group consisting of C₁₋₆ alkyl, C₁₋₆ hydroxyalkyl, C₁₋₆ haloalkyl, -CN, - NR_{C1}R_{C2}, -NO₂, -OR_{C3}, and halo;
n is each independently an integer of 1 to 4;
R_{C1} to R_{C3} are each independently H or C₁₋₆ alkyl;
R₅ and R₆ are each independently H or C₁₋₆ alkyl, or R₅ and R₆ are linked to each other to form a 3-12 membered heterocycle, wherein the heterocycle is unsubstituted or substituted with C₁₋₆ alkyl, C₁₋₆ hydroxyalkyl, C₁₋₆ haloalkyl, -CN, -NR_{D1}R_{D2}, -NO₂, -OR_{D3}, halo, or 3-6 membered heterocycloalkyl;
R_{D1} to R_{D3} are each independently H or C₁₋₆ alkyl;
L is -NH-, 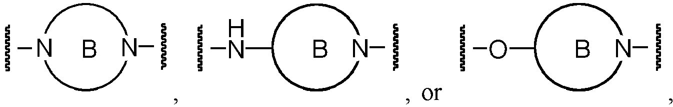 or L is linked to R₃ to form a 5-6 membered heterocycle, wherein ring B is a 3-8 membered heterocycle containing N atom in the ring, and ring B has or does not have one or more substituents selected from the group consisting of C₁₋₆ alkyl, C₁₋₆ hydroxyalkyl, C₁₋₆ alkylamino, C₁₋₆ haloalkyl, -OH, and halo;
Z is 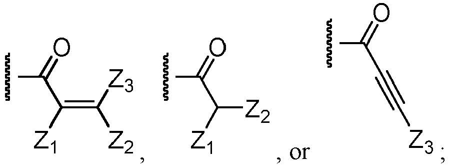
Z₁ is H, C₁₋₆ alkyl, C₁₋₆ haloalkyl, -CN, halo, or 5-6 membered heteroaryl;
Z₂ and Z₃ are each independently H, C₁₋₆ alkyl, C₁₋₆ hydroxyalkyl, C₁₋₆ alkylamino, C₁₋₆ haloalkyl, halo, C₃₋₁₂ cycloalkyl, 3-12 membered heterocycloalkyl, -(CH₂)m-(C₁₋₆ alkylamino), - (CH₂)m-(C₃₋₁₂ cycloalkyl), -(CH₂)m-(3-12 membered heterocycloalkyl), or -(CH₂)m-NH-(3-12 membered heterocycloalkyl), wherein -CH₂-, the cycloalkyl, and the heterocycloalkyl have or do not have one or more substituents selected from the group consisting of C₁₋₆ alkyl, deuterated C₁₋₆ alkyl, deuterated C₁₋₆ haloalkyl, -OH, =O, -C(=O)-(C₁₋₆ alkoxy), and 3-6 membered heterocycloalkyl;
m is each independently an integer of 1 to 4; and
the heterocycle, the heteroaryl, and the heterocycloalkyl each independently contain at least one heteroatom selected from the group consisting of N, S, and O.

In one embodiment of Formula (I) above,
ring A is an aromatic C₆₋₁₂ carbocycle, or an aromatic or non-aromatic 5-12 membered heterocycle, wherein ring A has or does not have one or more substituents selected from the group consisting of C₁₋₆ alkyl, -NR_{A1}R_{A2}, -OR_{A3}, -C(=O)-NR_{A1}R_{A2}, -C(=O)-OR_{A3}, -NH-C(=O)-(C₁₋₆ alkyl), =O, halo, and 5-6 membered heteroaryl, wherein the heteroaryl is unsubstituted or substituted with C₁₋₆ alkyl, C₁₋₆ alkoxy, or halo;
R_{A1} to R_{A3} are each independently H or C₁₋₆ alkyl;
the heterocycle and the heteroaryl each independently contain at least one heteroatom selected from the group consisting of N, S, and O; and
X₁, X₂, Y₁, Y₂, Y₃, L, and Z are the same as defined above.

In another embodiment of Formula (I) above,
Ring A is 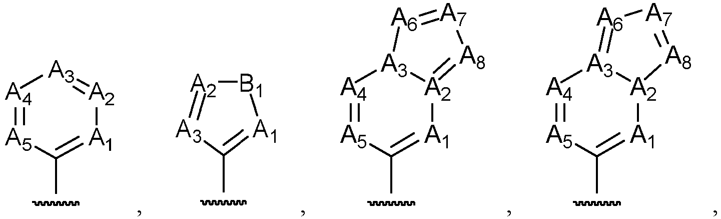 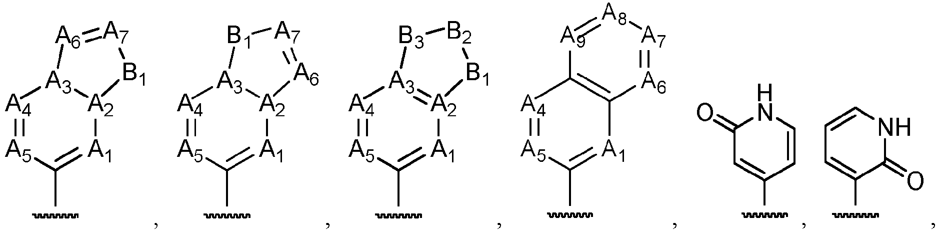 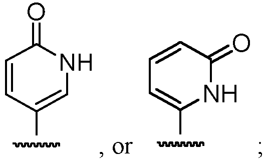
A₁ to A₉ are each independently CH or N;
B₁ to B₃ are each independently CH₂, NH, O, or S;
wherein ring A has or does not have one or more substituents selected from the group consisting of C₁₋₆ alkyl, -NR_{A1}R_{A2}, -OR_{A3}, -C(=O)-NR_{A1}R_{A2}, -C(=O)-OR_{A3}, -NH-C(=O)-(C₁₋₆ alkyl), =O, halo, and 5-6 membered heteroaryl, wherein the heteroaryl is unsubstituted or substituted with C₁₋₆ alkyl, C₁₋₆ alkoxy, or halo;
R_{A1} to R_{A3} are each independently H or C₁₋₆ alkyl;
the heteroaryl contains at least one heteroatom selected from the group consisting of N, S, and O; and
X₁, X₂, Y₁, Y₂, Y₃, L, and Z are the same as defined above.

In another embodiment of Formula (I) above,
X₁ is C₁₋₆ alkyl, deuterated C₁₋₆ alkyl, C₁₋₆ haloalkyl, C₃₋₁₂ cycloalkyl, or 3-12 membered heterocycloalkyl, wherein the cycloalkyl and heterocycloalkyl are unsubstituted or substituted with C₁₋₆ alkyl or C₁₋₆ haloalkyl;
X₂ is H, C₁₋₆ alkyl, C₃₋₆ cycloalkyl, C₁₋₆ haloalkyl, -NR_{B1}R_{B2}, or halo;
R_{B1} and R_{B2} are each independently C₁₋₆ alkyl;
the heterocycloalkyl each independently contains at least one heteroatom selected from the group consisting of N, S, and O; and
ring A, Y₁, Y₂, Y₃, L, and Z are the same as defined above.

In another embodiment of Formula (I) above,
ring A is an aromatic C₆₋₁₂ carbocycle, or an aromatic or non-aromatic 5-12 membered heterocycle, wherein ring A has or does not have one or more substituents selected from the group consisting of C₁₋₆ alkyl, -NR_{A1}R_{A2}, -OR_{A3}, -C(=O)-NR_{A1}R_{A2}, -C(=O)-OR_{A3}, -NH-C(=O)-(C₁₋₆ alkyl), =O, halo, and 5-6 membered heteroaryl, wherein the heteroaryl is unsubstituted or substituted with C₁₋₆ alkyl, C₁₋₆ alkoxy, or halo;
R_{A1} to R_{A3} are each independently H or C₁₋₆ alkyl;
X₁ is C₁₋₆ alkyl, deuterated C₁₋₆ alkyl, C₁₋₆ haloalkyl, C₃₋₁₂ cycloalkyl, or 3-12 membered heterocycloalkyl, wherein the cycloalkyl and heterocycloalkyl are unsubstituted or substituted with C₁₋₆ alkyl or C₁₋₆ haloalkyl;
X₂ is H, C₁₋₆ alkyl, C₃₋₆ cycloalkyl, C₁₋₆ haloalkyl, -NR_{B1}R_{B2}, or halo;
R_{B1} and R_{B2} are each independently C₁₋₆ alkyl;
the heterocycle, the heteroaryl, and the heterocycloalkyl each independently contain at least one heteroatom selected from the group consisting of N, S, and O; and
Y₁, Y₂, Y₃, L, and Z are the same as defined above.

In another embodiment of Formula (I) above,
Y₁ is CR₁ or N;
Y₂ is CR₂, C(-L-Z), or N;
Y₃ is CR₃ or N;
R₁ is H or -CN;
R₂ is H or halo;
R₃ is H, halo, -OR₄, or -NR₅R₆, or R₃ is linked to L to form a 5-6 membered heterocycle;
R₄ is C₁₋₆ alkyl, -(CH₂)n-O-(C₁₋₆ alkyl), C₁₋₆ haloalkyl, 3-6 membered heterocycloalkyl, or -(CH₂)n-(3-6 membered heterocycloalkyl), wherein the cycloalkyl and heterocycloalkyl have or do not have one or more substituents selected from the group consisting of C₁₋₆ alkyl, C₁₋₆ hydroxyalkyl, C₁₋₆ haloalkyl, -CN, -NR_{C1}R_{C2}, -NO₂, -OR_{C3}, and halo;
n is each independently an integer of 1 to 4;
R_{C1} to R_{C3} are each independently H or C₁₋₆ alkyl;
R₅ and R₆ are each independently H or C₁₋₆ alkyl, or R₅ and R₆ are linked to each other to form a 3-12 membered heterocycle, wherein the heterocycle is a 4-6 membered monocyclic ring or a 5-12 membered spiro ring, and the heterocycle is unsubstituted or substituted with halo, or 3-6 membered heterocycloalkyl;
the heterocycle and heterocycloalkyl each independently contain at least one heteroatom selected from the group consisting of N, S, and O; and
ring A, X₁, X₂, L, and Z are the same as defined above.

In another embodiment of Formula (I) above,
L is -NH-, 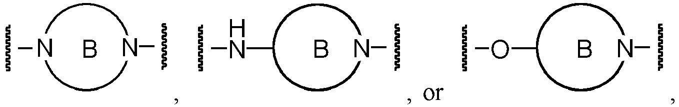 or L is linked to R₃ to form a 5-6 membered heterocycle, wherein ring B is a 3-8 membered heterocycle containing N atom in the ring, and ring B has or does not have one or more substituents selected from the group consisting of C₁₋₆ alkyl, C₁₋₆ haloalkyl, and halo;
Z is 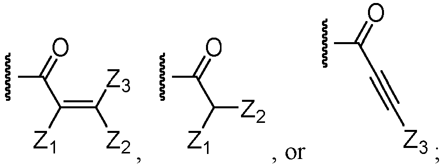
Z₁ is H, -CN, halo, or 5-6 membered heteroaryl;
Z₂ and Z₃ are each independently H, C₁₋₆ alkyl, C₁₋₆ alkylamino, halo, C₃₋₁₂ cycloalkyl, 3-12 membered heterocycloalkyl, -(CH₂)m-(C₁₋₆ alkylamino), -(CH₂)m-(C₃₋₁₂ cycloalkyl), - (CH₂)m-(3-12 membered heterocycloalkyl), or -(CH₂)m-NH-(3-12 membered heterocycloalkyl), wherein -CH₂-, the cycloalkyl, and the heterocycloalkyl have or do not have one or more substituents selected from the group consisting of C₁₋₆ alkyl, deuterated C₁₋₆ alkyl, -OH, =O, - C(=O)-(C₁₋₆ alkoxy), and 3-6 membered heterocycloalkyl;
m is each independently an integer of 1 to 4;
the heterocycle, the heteroaryl, and the heterocycloalkyl each independently contain at least one heteroatom selected from the group consisting of N, S, and O; and
ring A, X₁, X₂, Y₁, Y₂, and Y₃ are the same as defined above.

In another embodiment of Formula (I) above,
ring A is 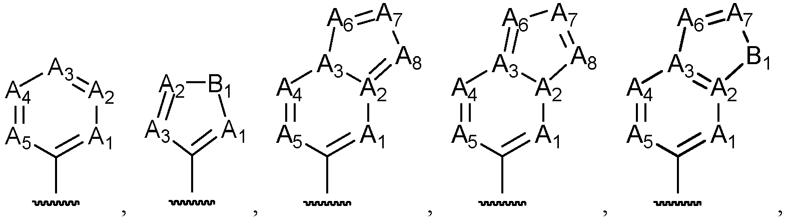 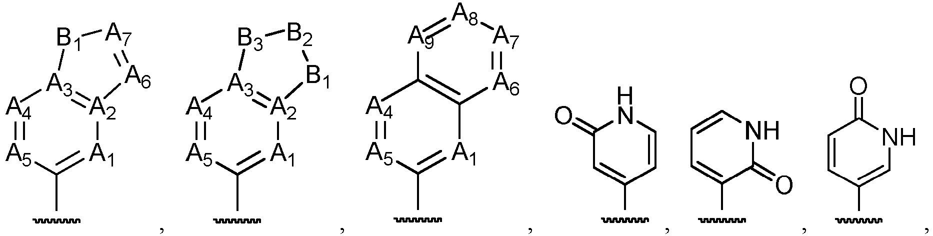 or 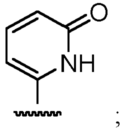
A₁ to A₉ are each independently CH or N;
B₁ to B₃ are each independently CH₂, NH, O, or S;
wherein ring A has or does not have one or more substituents selected from the group consisting of C₁₋₆ alkyl, -NR_{A1}R_{A2}, -OR_{A3}, -C(=O)-NR_{A1}R_{A2}, -C(=O)-OR_{A3}, -NH-C(=O)-(C₁₋₆ alkyl), =O, halo, and 5-6 membered heteroaryl, wherein the heteroaryl is unsubstituted or substituted with C₁₋₆ alkyl, C₁₋₆ alkoxy, or halo;
X₁ is C₁₋₆ alkyl, deuterated C₁₋₆ alkyl, C₁₋₆ haloalkyl, C₃₋₁₂ cycloalkyl, or 3-12 membered heterocycloalkyl, wherein the cycloalkyl and heterocycloalkyl are unsubstituted or substituted with C₁₋₆ alkyl or C₁₋₆ haloalkyl;
X₂ is H, C₁₋₆ alkyl, C₃₋₆ cycloalkyl, C₁₋₆ haloalkyl, -NR_{B1}R_{B2}, or halo;
R_{B1} and R_{B2} are each independently C₁₋₆ alkyl;
Y₁ is CR₁ or N;
Y₂ is CR₂, C(-L-Z), or N;
Y₃ is CR₃ or N;
R₁ is H or -CN;
R₂ is H or halo;
R₃ is H, halo, -OR₄, or -NR₅R₆, or R₃ is linked to L to form a 5-6 membered heterocycle;
R₄ is C₁₋₆ alkyl, -(CH₂)n-O-(C₁₋₆ alkyl), C₁₋₆ haloalkyl, 3-6 membered heterocycloalkyl, or -(CH₂)n-(3-6 membered heterocycloalkyl), wherein the cycloalkyl and heterocycloalkyl have or do not have one or more substituents selected from the group consisting of C₁₋₆ alkyl, C₁₋₆ hydroxyalkyl, C₁₋₆ haloalkyl, -CN, -NR_{C1}R_{C2}, -NO₂, -OR_{C3}, and halo;
n is each independently an integer of 1 to 4;
R_{C1} to R_{C3} are each independently H or C₁₋₆ alkyl;
R₅ and R₆ are each independently H or C₁₋₆ alkyl, or R₅ and R₆ are linked to each other to form a 3-12 membered heterocycle, wherein the heterocycle is a 4-6 membered monocyclic ring or a 5-12 membered spiro ring, and the heterocycle is unsubstituted or substituted with halo, or 3-6 membered heterocycloalkyl;
L is -NH-, 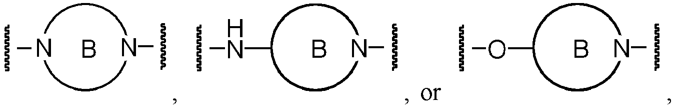 or L is linked to R₃ to form a 5-6 membered heterocycle, wherein ring B is a 3-8 membered heterocycle containing N atom in the ring, and ring B has or does not have one or more substituents selected from the group consisting of C₁₋₆ alkyl, C₁₋₆ haloalkyl, and halo;
Z is 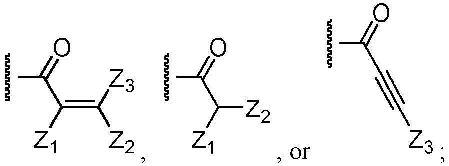
Z₁ is H, -CN, halo, or 5-6 membered heteroaryl;
Z₂ and Z₃ are each independently H, C₁₋₆ alkyl, C₁₋₆ alkylamino, halo, C₃₋₁₂ cycloalkyl, 3-12 membered heterocycloalkyl, -(CH₂)m-(C₁₋₆ alkylamino), -(CH₂)m-(C₃₋₁₂ cycloalkyl), - (CH₂)m-(3-12 membered heterocycloalkyl), or -(CH₂)m-NH-(3-12 membered heterocycloalkyl), wherein -CH₂-, the cycloalkyl, and the heterocycloalkyl have or do not have one or more substituents selected from the group consisting of C₁₋₆ alkyl, deuterated C₁₋₆ alkyl, -OH, =O, - C(=O)-(C₁₋₆ alkoxy), and 3-6 membered heterocycloalkyl;
m is each independently an integer of 1 to 4; and
the heterocycle, the heteroaryl, and the heterocycloalkyl each independently contain at least one heteroatom selected from the group consisting of N, S, and O.

In another embodiment of Formula (I) above,
L is -NH-, 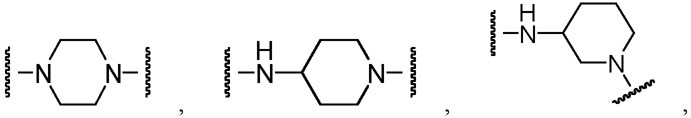 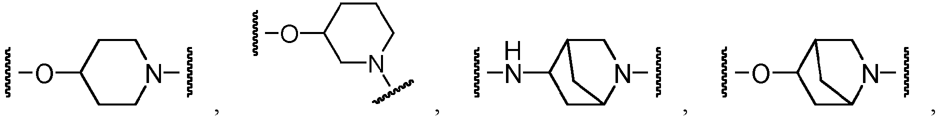 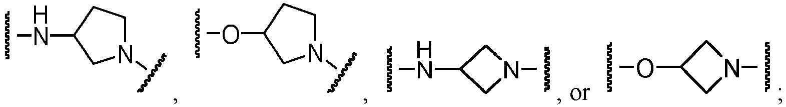
wherein the heterocycle containing N has or does not have one or more substituents selected from the group consisting of C₁₋₆ alkyl, C₁₋₆ haloalkyl, and halo, or
L is linked to R₃ to form a 5-6 membered heterocycle containing N;
the heterocycle containing N further contains or does not contain at least one heteroatom among S and O in addition to N; and
ring A, X₁, X₂, Y₁, Y₂, Y₃, and Z are the same as defined above.

In a specific example, the scope of the present invention includes a compound selected from the following group, or a pharmaceutically acceptable salt or stereoisomer thereof.
1> 1-(4-((4-((4-([1,2,4]triazolo[1,5-*a*]pyridin-7-yloxy)-2-methoxy-5-methylphenyl)amino)-7-methoxyquinazolin-6-yl)amino)piperidin-1-yl)prop-2-en-1-one;
2> 1-(4-((4-((5-chloro-4-(3-fluorophenoxy)-2-methoxyphenyl)amino)-7-methoxyquinazolin-6-yl)amino)piperidin-1-yl)prop-2-en-1-one;
3> 1-(4-((4-((5-chloro-2-methoxy-4-(pyridin-2-yloxy)phenyl)amino)-7-methoxyquinazolin-6-yl)amino)piperidin-1-yl)prop-2-en-1-one;
4> 1-(4-((4-((4-([1,2,4]triazolo[1,5-*a*]pyridin-7-yloxy)-5-methyl-2-((1-methylazetidin-3-yl)oxy)phenyl)amino)-7-methoxyquinazolin-6-yl)amino)piperidin-1-yl)prop-2-en-1-one;
5> 1-(4-((4-((4-([1,2,4]triazolo[1,5-*a*]pyridin-7-yloxy)-5-chloro-2-methoxyphenyl)amino)-7-methoxyquinazolin-6-yl)oxy)piperidin-1-yl)prop-2-en-1-one;
6> 1-(4-((4-((5-chloro-4-(3-fluorophenoxy)-2-methoxyphenyl)amino)-7-methoxyquinazolin-6-yl)oxy)-3-fluoropiperidin-1-yl)prop-2-en-1-one;
7> 1-(4-((4-((4-(3-fluorophenoxy)-2-methoxyphenyl)amino)-7-methoxyquinazolin-6-yl)oxy)piperidin-1-yl)prop-2-en-1-one;
8> 1-(4-((4-((5-chloro-4-(3-fluorophenoxy)-2-methoxyphenyl)amino)-7-methoxyquinazolin-6-yl)oxy)piperidin-1-yl)prop-2-en-1-one;
9> 1-(4-((4-((4-((1-(5-fluoro-6-methylpyridin-3-yl)-1*H*-pyrazol-3-yl)oxy)-2-methoxyphenyl)amino)-7-methoxyquinazolin-6-yl)oxy)piperidin-1-yl)prop-2-en-1-one;
10> 1-(4-((7-methoxy-4-((2-methoxy-4-((1-(2-methoxypyrimidin-5-yl)-1*H*-pyrazol-3-yl)oxy)phenyl)amino)quinazolin-6-yl)oxy)piperidin-1-yl)prop-2-en-1-one;
11> 1-(4-((4-((5-chloro-2-methoxy-4-(3-methoxyphenoxy)phenyl)amino)-7-methoxyquinazolin-6-yl)oxy)piperidin-1-yl)prop-2-en-1-one;
12> 4-((4-([1,2,4]triazolo[1,5-*a*]pyridin-7-yloxy)-2-methoxy-5-methylphenyl)amino)-6-((1-acryloylpiperidin-4-yl)oxy)-7-methoxyquinoline-3-carbonitrile;
13> methyl 4-(4-((6-((1-acryloylpiperidin-4-yl)oxy)-7-methoxyquinazolin-4-yl)amino)-5-methoxy-2-methylphenoxy)benzoate;
14> 1-(4-((4-((4-([1,2,4]triazolo[1,5-*a*]pyridin-7-yloxy)-2-methoxy-5-methylphenyl)amino)-7-methoxyquinazolin-6-yl)oxy)piperidin-1-yl)-2-chloro-2-fluoroethan-1-one;
15> 1-(4-((4-((4-([1,2,4]triazolo[1,5-*a*]pyridin-7-yloxy)-2-methoxy-5-methylphenyl)amino)-7-methoxyquinazolin-5-yl)oxy)-3,3-difluoropiperidin-1-yl)prop-2-en-1-one;
16> 1-(3-((4-((4-([1,2,4]triazolo[1,5-*a*]pyridin-7-yloxy)-2-methoxy-5-methylphenyl)amino)-7-methoxyquinazolin-5-yl)oxy)-4,4-difluoropiperidin-1-yl)prop-2-en-1-one;
17> 1-(4-((4-((4-([1,2,4]triazolo[1,5-*a*]pyridin-7-yloxy)-2-methoxy-5-methylphenyl)amino)pyrido[3,4-*d*]pyrimidin-6-yl)oxy)piperidin-1-yl)prop-2-en-1-one;
18> 1-(5-((4-((4-([1,2,4]triazolo[1,5-*a*]pyridin-7-yloxy)-2-methoxy-5-methylphenyl)amino)-7-methoxyquinazolin-6-yl)oxy)-2-azabicyclo[2.2.1]heptan-2-yl)prop-2-en-1-one;
19> 1-(3-((4-((4-([1,2,4]triazolo[1,5-*a*]pyridin-7-yloxy)-2-methoxy-5-methylphenyl)amino)-7-methoxyquinazolin-6-yl)oxy)azetidin-1-yl)prop-2-en-1-one;
20> 1-(3-((4-((4-([1,2,4]triazolo[1,5-*a*]pyridin-7-yloxy)-2-methoxy-5-methylphenyl)amino)-7-methoxyquinazolin-6-yl)oxy)pyrrolidin-1-yl)prop-2-en-1-one;
21> 1-(4-((4-([1,2,4]triazolo[1,5-*a*]pyridin-7-yloxy)-2-methoxy-5-methylphenyl)amino)-7,8-dihydro-6*H*-[1,4]oxazino[3,2-*g*]quinazolin-6-yl)prop-2-en-1-one;
22> *N*-(4-((4-([1,2,4]triazolo[1,5-*a*]pyridin-7-yloxy)-2-methoxy-5-methylphenyl)amino)-7-(4-morpholinopiperidin-1-yl)quinazolin-6-yl)acrylamide;
23> *N*-(4-((4-([1,2,4]triazolo[1,5-*a*]pyridin-7-yloxy)-2-methoxy-5-methylphenyl)amino)-7-morpholinoquinazolin-6-yl)acrylamide;
24> *N*-(4-((5-chloro-2-methoxy-4-(pyridin-2-yloxy)phenyl)amino)-7-methoxyquinazolin-6-yl)acrylamide;
25> *(E)-N*-(4-((5-chloro-4-(3-fluorophenoxy)-2-methoxyphenyl)amino)-7-methoxyquinazolin-6-yl)-4-(dimethylamino)but-2-enamide;
*26>* (*E*)-*N*-(4-((4-([1,2,4]triazolo[1,5-*a*]pyridin-7-yloxy)-2-methoxy-5-methylphenyl)amino)-5-chloroquinazolin-6-yl)-4-(dimethylamino)but-2-enamide;
27> 1-(4-(8-((4-([1,2,4]triazolo[1,5-*a*]pyridin-7-yloxy)-2-methoxy-5-methylphenyl)amino)pyrimido[5,4-*d*]pyrimidin-2-yl)piperazin-1-yl)prop-2-en-1-one;
28> *N*-(4-((4-([1,2,4]triazolo[1,5-*a*]pyridin-7-yloxy)-2-methoxy-5-methylphenyl)amino)-7-methoxyquinazolin-6-yl)but-2-ynamide;
*29>* (*R*,*E*)-*N*-(4-((4-([1,2,4]triazolo[1,5-*a*]pyridin-7-yloxy)-2-methoxy-5-methylphenyl)amino)-7-methoxyquinazolin-6-yl)-3-(1-methylpyrrolidin-2-yl)acrylamide;
30> *(R,E)-N-(4-((5-chloro-4-(3-fluorophenoxy)-2-methoxyphenyl)amino)-7-*methoxyquinazolin-6-yl)-3-(1-methylpyrrolidin-2-yl)acrylamide;
31> *(R,E)-N*-(4-((5-chloro-4-(3-fluorophenoxy)-2-methoxyphenyl)amino)-7-methoxyquinazolin-6-yl)-3-(pyrrolidin-2-yl)acrylamide;
32> (R,E)-N-(4-((4-([1,2,4]triazolo[1,5-*a*]pyridin-7-yloxy)-5-chloro-2-methoxyphenyl)amino)-7-methoxyquinazolin-6-yl)-3-(1-methylpyrrolidin-2-yl)acrylamide;
33> *(R,E)-N*-(4-((5-chloro-2-methoxy-4-(pyridin-2-yloxy)phenyl)amino)-7-methoxyquinazolin-6-yl)-3-(1-methylpyrrolidin-2-yl)acrylamide;
34> *(R,E)-N*-(8-((4-([1,2,4]triazolo[1,5-*a*]pyridin-7-yloxy)-2-methoxy-5-methylphenyl)amino)pyrimido[5,4-*d*]pyrimidin-2-yl)-3-(1-methylpyrrolidin-2-yl)acrylamide;
35> *(E)-N-(*4-((4-([1,2,4]triazolo[1,5-*a*]pyridin-7-yloxy)-2-methoxy-5-methylphenyl)amino)-7-methoxyquinazolin-6-yl)-3-(2-methyl-2-azabicyclo[3.1.0]hexan-3-yl)acrylamide;
36> (*E*)-*N*-(4-((4-([1,2,4]triazolo[1,5-*a*]pyridin-7-yloxy)-2-methoxy-5-methylphenyl)amino)-7-methoxyquinazolin-6-yl)-3-(1-methylpiperidin-2-yl)acrylamide;
37> (*S*,*E*)-*N*-(4-((4-([1,2,4]triazolo[1,5-*a*]pyridin-7-yloxy)-2-methoxy-5-methylphenyl)amino)-7-methoxyquinazolin-6-yl)-3-(1-methylpyrrolidin-2-yl)acrylamide;
38> (*E*)-*N*-(4-((4-([1,2,4]triazolo[1,5-*a*]pyridin-7-yloxy)-2-methoxy-5-methylphenyl)amino)-7-methoxyquinazolin-6-yl)-4-((1,1-dioxidotetrahydro-2H-thiopyran-4-yl)amino)but-2-enamide;
39> *(E)-N-(*4-((4-([1,2,4]triazolo[1,5-*a*]pyridin-7-yloxy)-2-methoxy-5-methylphenyl)amino)-7-methoxyquinazolin-6-yl)-4-(5-hydroxyhexahydrocyclopenta[c]pyrrol-2(1*H*)-yl)but-2-enamide;
40> *(E)-N-(*4-((4-([1,2,4]triazolo[1,5-*a*]pyridin-7-yloxy)-2-methoxy-5-methylphenyl)amino)-7-methoxyquinazolin-6-yl)-4-(2-oxa-6-azaspiro[3.3]heptan-6-yl)but-2-enamide;
41> *(R,E)-N-(*4-((4-([1,2,4]triazolo[1,5-*a*]pyridin-7-yloxy)-2-methoxy-5-methylphenyl)amino)-7-methoxyquinazolin-6-yl)-2-fluoro-3-(1-methylpyrrolidin-2-yl)acrylamide;
42> *(R,E)-N-(4-((5-chloro-4-(3-fluorophenoxy)-2-methoxyphenyl)amino)-7-*methoxyquinazolin-6-yl)-2-fluoro-3-(1-methylpyrrolidin-2-yl)acrylamide;
43> (*Z*)-*N*-(4-((4-([1,2,4]triazolo[1,5-*a*]pyridin-7-yloxy)-2-methoxy-5-methylphenyl)amino)-7-methoxyquinazolin-6-yl)-2-fluoro-3-(1-methylpyrrolidin-2-yl)acrylamide;
44> *(S,E)-N-(*4-((4-([1,2,4]triazolo[1,5-*a*]pyridin-7-yloxy)-2-methoxy-5-methylphenyl)amino)-7-methoxyquinazolin-6-yl)-2-fluoro-3-(1-methylpyrrolidin-2-yl)acrylamide;
45> *(R,E)-N-(*4-((4-([1,2,4]triazolo[1,5-*a*]pyridin-7-yloxy)-2-methoxy-5-methylphenyl)amino)-7-methoxyquinazolin-6-yl)-2-fluoro-3-(1-methyl-5-oxopyrrolidin-2-yl)acrylamide;
46> (*R*,*E*)-*N*-(4-((4-([1,2,4]triazolo[1,5-*a*]pyridin-7-yloxy)-2-methoxyphenyl)amino)-7-methoxyquinazolin-6-yl)-2-fluoro-3-(1-methylpyrrolidin-2-yl)acrylamide;
47> (*R*,*E*)-*N*-(4-((4-([1,2,4]triazolo[1,5-*c*]pyrimidin-7-yloxy)-2-methoxy-5-methylphenyl)amino)-7-methoxyquinazolin-6-yl)-2-fluoro-3-(1-methylpyrrolidin-2-yl)acrylamide;
48> (*R*,*Z*)*-N*-(4-((4-([1,2,4]triazolo[1,5-*c*]pyrimidin-7-yloxy)-2-methoxy-5-methylphenyl)amino)-7-methoxyquinazolin-6-yl)-2-fluoro-3-(1-methylpyrrolidin-2-yl)acrylamide;
49> (*R*,*E*)-*N*-(4-((4-([1,2,4]triazolo[1,5-*a*]pyridin-7-yloxy)-5-fluoro-2-methoxyphenyl)amino)-7-methoxyquinazolin-6-yl)-2-fluoro-3-(1-methylpyrrolidin-2-yl)acrylamide;
50> (*R*,*Z*)-*N*-(4-((4-([1,2,4]triazolo[1,5-*a*]pyridin-7-yloxy)-5-fluoro-2-methoxyphenyl)amino)-7-methoxyquinazolin-6-yl)-2-fluoro-3-(1-methylpyrrolidin-2-yl)acrylamide;
51> (*R*,*E*)-*N*-(4-((4-([1,2,4]triazolo[1,5-*a*]pyridin-7-yloxy)-5-chloro-2-methoxyphenyl)amino)-7-methoxyquinazolin-6-yl)-2-fluoro-3-(1-methylpyrrolidin-2-yl)acrylamide;
52> (*R*,*E*)-*N*-(4-((4-([1,2,4]triazolo[1,5-*a*]pyridin-7-yloxy)-2-methoxy-5-methylphenyl)amino)-7-methoxyquinazolin-6-yl)-3-(1-ethylpyrrolidin-2-yl)-2-fluoroacrylamide;
53> *(R,E)-N-(*4-((4-([1,2,4]triazolo[1,5-*a*]pyridin-7-yloxy)-2-methoxy-5-(trifluoromethyl)phenyl)amino)-7-methoxyquinazolin-6-yl)-2-fluoro-3-(1-methylpyrrolidin-2-yl)acrylamide;
54> (*R*,*Z*)-N-(4-((4-([1,2,4]triazolo[1,5-*a*]pyridin-7-yloxy)-2-methoxy-5-(trifluoromethyl)phenyl)amino)-7-methoxyquinazolin-6-yl)-2-fluoro-3-(1-methylpyrrolidin-2-yl)acrylamide;
55> (*R*,*E*)-2-fluoro-*N*-(7-methoxy-4-((2-methoxy-5-methyl-4-((1-methyl-1*H-*benzo[*d*]imidazol-6-yl)oxy)phenyl)amino)quinazolin-6-yl)-3-(1-methylpyrrolidin-2-yl)acrylamide;
56> (*R*,*Z*)-2-fluoro-*N*-(7-methoxy-4-((2-methoxy-5-methyl-4-((1-methyl-1*H-*benzo[*d*]imidazol-5-yl)oxy)phenyl)amino)quinazolin-6-yl)-3-(1-methylpyrrolidin-2-yl)acrylamide;
57> (*R*,*E*)-2-fluoro-*N*-(7-methoxy-4-((2-methoxy-5-methyl-4-((1-methyl-1*H-*benzo[d]imidazol-5-yl)oxy)phenyl)amino)quinazolin-6-yl)-3-(1-methylpyrrolidin-2-yl)acrylamide;
58> (*R*,*Z*)-*N*-(4-((5-chloro-2-methoxy-4-((1-methyl-1*H*-benzo[*d*]imidazol-5-yl)oxy)phenyl)amino)-7-methoxyquinazolin-6-yl)-2-fluoro-3-(1-methylpyrrolidin-2-yl)acrylamide;
59> *(R,E)-N*-(4-((5-chloro-2-methoxy-4-((1-methyl-1*H*-benzo[*d*]imidazol-5-yl)oxy)phenyl)amino)-7-methoxyquinazolin-6-yl)-2-fluoro-3-(1-methylpyrrolidin-2-yl)acrylamide;
60> (*R*,*E*)-2-fluoro-*N*-(4-((5-fluoro-2-methoxy-4-((1-methyl-1*H*-benzo[*d*]imidazol-5-yl)oxy)phenyl)amino)-7-methoxyquinazolin-6-yl)-3-(1-methylpyrrolidin-2-yl)acrylamide;
61> (*R*,*Z*)-2-fluoro-*N*-(4-((5-fluoro-2-methoxy-4-((1-methyl-1H-benzo[d]imidazol-5-yl)oxy)phenyl)amino)-7-methoxyquinazolin-6-yl)-3-(1-methylpyrrolidin-2-yl)acrylamide;
62> (*R*,*E*)-2-fluoro-*N*-(7-methoxy-4-((2-methoxy-5-methyl-4-(quinoxalin-6-yloxy)phenyl)amino)quinazolin-6-yl)-3-(1-methylpyrrolidin-2-yl)acrylamide;
63> (*R*,*Z*)-2-fluoro-*N*-(7-methoxy-4-((2-methoxy-5-methyl-4-(quinoxalin-6-yloxy)phenyl)amino)quinazolin-6-yl)-3-(1-methylpyrrolidin-2-yl)acrylamide;
64> (*R*,*Z*)-2-fluoro-*N*-(7-methoxy-4-((2-methoxy-5-methyl-4-(naphthalen-2-yloxy)phenyl)amino)quinazolin-6-yl)-3-(1-methylpyrrolidin-2-yl)acrylamide;
65> (*R,E*)-2-fluoro-*N*-(7-methoxy-4-((2-methoxy-5-methyl-4-(naphthalen-2-yloxy)phenyl)amino)quinazolin-6-yl)-3-(1-methylpyrrolidin-2-yl)acrylamide;
66> *(R,E)-N*-(4-((4-(benzo[*d*]*ox*azol-6-yloxy)-2-methoxy-5-methylphenyl)amino)-7-methoxyquinazolin-6-yl)-2-fluoro-3-(1-methylpyrrolidin-2-yl)acrylamide;
67> (*R*,*E*)-2-fluoro-*N*-(7-methoxy-4-((2-methoxy-5-methyl-4-((2-methylbenzo[*d*]oxazol-5-yl)oxy)phenyl)amino)quinazolin-6-yl)-3-(1-methylpyrrolidin-2-yl)acrylamide;
68> (*R*,*Z*)-2-fluoro-N-(7-methoxy-4-((2-methoxy-5-methyl-4-((2-methylbenzo[*d*]oxazol-5-yl)oxy)phenyl)amino)quinazolin-6-yl)-3-(1-methylpyrrolidin-2-yl)acrylamide;
69> (*E*)-2-fluoro-*N*-(4-((4-((2-hydroxy-2,3-dihydrobenzo[*d*]oxazol-5-yl)oxy)-2-methoxy-5-methylphenyl)amino)-7-methoxyquinazolin-6-yl)-3-((*R*)-1-methylpyrrolidin-2-yl)acrylamide;
70> (*Z*)-2-fluoro-*N*-(4-((4-((2-hydroxy-2,3-dihydrobenzo[d]oxazol-5-yl)oxy)-2-methoxy-5-methylphenyl)amino)-7-methoxyquinazolin-6-yl)-3-((*R*)-1-methylpyrrolidin-2-yl)acrylamide;
71> (*R*,*E*)-2-fluoro-*N*-(7-methoxy-4-((2-methoxy-5-methyl-4-((1-methyl-2-oxo-1,2-dihydropyridin-4-yl)oxy)phenyl)amino)quinazolin-6-yl)-3-(1-methylpyrrolidin-2-yl)acrylamide;
*72> (R,E)-N-(*4-((4-([1,2,4]triazolo[1,5-*a*]pyridin-6-yloxy)-2-methoxy-5-methylphenyl)amino)-7-methoxyquinazolin-6-yl)-2-fluoro-3-(1-methylpyrrolidin-2-yl)acrylamide;
73> (*R*,*Z*)*-N*-(4-((4-([1,2,4]triazolo[1,5-*a*]pyridin-6-yloxy)-2-methoxy-5-methylphenyl)amino)-7-methoxyquinazolin-6-yl)-2-fluoro-3-(1-methylpyrrolidin-2-yl)acrylamide;
74> (*R*,*E*)-2-fluoro-N-(7-methoxy-4-((2-methoxy-5-methyl-4-phenoxyphenyl)amino)quinazolin-6-yl)-3-(1-methylpyrrolidin-2-yl)acrylamide;
75> (*R*,*E*)-2-fluoro-N-(4-((4-(imidazo[1,2-*a*]pyridin-7-yloxy)-2-methoxy-5-methylphenyl)amino)-7-methoxyquinazolin-6-yl)-3-(1-methylpyrrolidin-2-yl)acrylamide;
76> (*R*,*Z*)-2-fluoro-N-(4-((4-(imidazo[1,2-*a*]pyridin-7-yloxy)-2-methoxy-5-methylphenyl)amino)-7-methoxyquinazolin-6-yl)-3-(1-methylpyrrolidin-2-yl)acrylamide;
77> (*R*,*E*)-2-fluoro-N-(4-((4-(imidazo[1,2-c]pyrimidin-7-yloxy)-2-methoxy-5-methylphenyl)amino)-7-methoxyquinazolin-6-yl)-3-(1-methylpyrrolidin-2-yl)acrylamide;
*78> (R,E)-N-(8-(*(4-([1,2,4]triazolo[1,5-*a*]pyridin-7-yloxy)-2-methoxy-5-methylphenyl)amino)pyrimido[5,4-*d*]pyrimidin-2-yl)-2-fluoro-3-(1-methylpyrrolidin-2-yl)acrylamide;
79> (*R*,*E*)-2-fluoro-*N*-(7-methoxy-4-((2-methoxy-5-methyl-4-(pyrazolo[1,5-*a*]pyrimidin-6-yloxy)phenyl)amino)quinazolin-6-yl)-3-(1-methylpyrrolidin-2-yl)acrylamide;
80> (*R*,*Z*)-2-fluoro-*N*-(7-methoxy-4-((2-methoxy-5-methyl-4-(pyrazolo[1,5-*a*]pyrimidin-6-yloxy)phenyl)amino)quinazolin-6-yl)-3-(1-methylpyrrolidin-2-yl)acrylamide;
81> *(R,E)-N*-(4-((4-(benzo[*d*]thiazol-6-yloxy)-2-methoxy-5-methylphenyl)amino)-7-methoxyquinazolin-6-yl)-2-fluoro-3-(1-methylpyrrolidin-2-yl)acrylamide;
82> (*R*,*Z*)-*N*-(4-((4-(benzo[d]thiazol-6-yloxy)-2-methoxy-5-methylphenyl)amino)-7-methoxyquinazolin-6-yl)-2-fluoro-3-(1-methylpyrrolidin-2-yl)acrylamide;
83> (*R*,*Z*)-*N*-(4-((4-(benzo[d]thiazol-5-yloxy)-2-methoxy-5-methylphenyl)amino)-7-methoxyquinazolin-6-yl)-2-fluoro-3-(1-methylpyrrolidin-2-yl)acrylamide;
84> *(R,E)-N*-(4-((4-(benzo[*d*]thiazol-5-yloxy)-2-methoxy-5-methylphenyl)amino)-7-methoxyquinazolin-6-yl)-2-fluoro-3-(1-methylpyrrolidin-2-yl)acrylamide;
85> *(R,E)-N*-(4-((4-(benzo[*d*][1,3]dioxol-5-yloxy)-2-methoxy-5-methylphenyl)amino)-7-methoxyquinazolin-6-yl)-2-fluoro-3-(1-methylpyrrolidin-2-yl)acrylamide;
86> (*R*,*Z*)-N-(4-((4-(benzo[*d*][1,3]dioxol-5-yloxy)-2-methoxy-5-methylphenyl)amino)-7-methoxyquinazolin-6-yl)-2-fluoro-3-(1-methylpyrrolidin-2-yl)acrylamide;
87> (*R*,*Z*)-2-fluoro-*N*-(7-methoxy-4-((2-methoxy-5-methyl-4-(quinolin-7-yloxy)phenyl)amino)quinazolin-6-yl)-3-(1-methylpyrrolidin-2-yl)acrylamide;
88> (*R*,*E*)-2-fluoro-*N*-(7-methoxy-4-((2-methoxy-5-methyl-4-(quinolin-7-yloxy)phenyl)amino)quinazolin-6-yl)-3-(1-methylpyrrolidin-2-yl)acrylamide;
89> (*R*,*E*)-2-fluoro-*N*-(7-methoxy-4-((2-methoxy-5-methyl-4-((1-methyl-1*H*-indazol-6-yl)oxy)phenyl)amino)quinazolin-6-yl)-3-(1-methylpyrrolidin-2-yl)acrylamide;
90> (*R*,*Z*)-2-fluoro-*N*-(7-methoxy-4-((2-methoxy-5-methyl-4-((1-methyl-1*H*-indazol-6-yl)oxy)phenyl)amino)quinazolin-6-yl)-3-(1-methylpyrrolidin-2-yl)acrylamide;
91> (*R*,*E*)-*N*-(4-((4-([1,2,4]triazolo[1,5-*a*]pyridin-7-yloxy)-2-methoxy-5-methylphenyl)amino)quinazolin-6-yl)-2-fluoro-3-(1-methylpyrrolidin-2-yl)acrylamide;
92> (*R*,*Z*)-*N*-(4-((4-([1,2,4]triazolo[1,5-*a*]pyridin-7-yloxy)-2-methoxy-5-methylphenyl)amino)quinazolin-6-yl)-2-fluoro-3-(1-methylpyrrolidin-2-yl)acrylamide;
93> (*R*,*E*)-*N*-(4-((4-([1,2,4]triazolo[1,5-*a*]pyridin-7-yloxy)-2-methoxy-5-methylphenyl)amino)-7-ethoxyquinazolin-6-yl)-2-fluoro-3-(1-methylpyrrolidin-2-yl)acrylamide;
94> (*R*,*E*)-*N*-(4-((4-([1,2,4]triazolo[1,5-*a*]pyridin-7-yloxy)-2-methoxy-5-methylphenyl)amino)-7-(2-methoxyethoxy)quinazolin-6-yl)-2-fluoro-3-(1-methylpyrrolidin-2-yl)acrylamide;
95> (*R*,*Z*)-*N*-(4-((4-([1,2,4]triazolo[1,5-*a*]pyridin-7-yloxy)-2-methoxy-5-methylphenyl)amino)-7-(2-methoxyethoxy)quinazolin-6-yl)-2-fluoro-3-(1-methylpyrrolidin-2-yl)acrylamide;
96> *(E)-N-(*4-((4-([1,2,4]triazolo[1,5-*a*]pyridin-7-yloxy)-2-methoxy-5-methylphenyl)amino)-7-((tetrahydrofuran-3-yl)oxy)quinazolin-6-yl)-2-fluoro-3-((*R*)-1-methylpyrrolidin-2-yl)acrylamide;
97> (*Z*)-*N*-(4-((4-([1,2,4]triazolo[1,5-*a*]pyridin-7-yloxy)-2-methoxy-5-methylphenyl)amino)-7-((tetrahydrofuran-3-yl)oxy)quinazolin-6-yl)-2-fluoro-3-((*R*)-1-methylpyrrolidin-2-yl)acrylamide;
98> (*R*,*Z*)*-N*-(4-((4-([1,2,4]triazolo[1,5-*a*]pyridin-7-yloxy)-2-methoxy-5-methylphenyl)amino)-7-(difluoromethoxy)quinazolin-6-yl)-2-fluoro-3-(1-methylpyrrolidin-2-yl)acrylamide;
99> *(R,E)-N-(*4-((4-([1,2,4]triazolo[1,5-*a*]pyridin-7-yloxy)-2-methoxy-5-methylphenyl)amino)-7-(difluoromethoxy)quinazolin-6-yl)-2-fluoro-3-(1-methylpyrrolidin-2-yl)acrylamide;
100> (*R*,*E*)-*N*-(4-((4-([1,2,4]triazolo[1,5-*a*]pyridin-7-yloxy)-2-methoxy-5-methylphenyl)amino)-7-(2-morpholinoethoxy)quinazolin-6-yl)-2-fluoro-3-(1-methylpyrrolidin-2-yl)acrylamide;
101> (*R*,*Z*)-*N*-(4-((4-([1,2,4]triazolo[1,5-*a*]pyridin-7-yloxy)-2-methoxy-5-methylphenyl)amino)-7-(2-morpholinoethoxy)quinazolin-6-yl)-2-fluoro-3-(1-methylpyrrolidin-2-yl)acrylamide;
102> (*R*,*E*)-*N*-(4-((4-([1,2,4]triazolo[1,5-*a*]pyridin-7-yloxy)-2-methoxy-5-methylphenyl)amino)-7-fluoroquinazolin-6-yl)-2-fluoro-3-(1-methylpyrrolidin-2-yl)acrylamide;
103> (*R*,*Z*)-*N*-(4-((4-([1,2,4]triazolo[1,5-*a*]pyridin-7-yloxy)-2-methoxy-5-methylphenyl)amino)-7-fluoroquinazolin-6-yl)-2-fluoro-3-(1-methylpyrrolidin-2-yl)acrylamide;
104> (*R*,*E*)-*N*-(4-((4-([1,2,4]triazolo[1,5-*a*]pyridin-7-yloxy)-2-ethoxy-5-methylphenyl)amino)-7-methoxyquinazolin-6-yl)-2-fluoro-3-(1-methylpyrrolidin-2-yl)acrylamide;
105> (*R*,*E*)-*N*-(4-((4-([1,2,4]triazolo[1,5-*a*]pyridin-7-yloxy)-5-methyl-2-(trifluoromethoxy)phenyl)amino)-7-methoxyquinazolin-6-yl)-2-fluoro-3-(1-methylpyrrolidin-2-yl)acrylamide;
106> (*R*,*E*)-*N*-(4-((4-([1,2,4]triazolo[1,5-*a*]pyridin-7-yloxy)-2-cyclopropoxy-5-methylphenyl)amino)-7-methoxyquinazolin-6-yl)-2-fluoro-3-(1-methylpyrrolidin-2-yl)acrylamide;
107> (*R*,*Z*)-*N*-(4-((4-([1,2,4]triazolo[1,5-*a*]pyridin-7-yloxy)-2-cyclopropoxy-5-methylphenyl)amino)-7-methoxyquinazolin-6-yl)-2-fluoro-3-(1-methylpyrrolidin-2-yl)acrylamide;
108> *(R,E)-N-(*4-((4-([1,2,4]triazolo[1,5-*a*]pyridin-7-yloxy)-5-methyl-2-(oxetan-3-yloxy)phenyl)amino)-7-methoxyquinazolin-6-yl)-2-fluoro-3-(1-methylpyrrolidin-2-yl)acrylamide;
109> *tert*-butyl (*R*,*E*)-2-(3-((4-((4-([1,2,4]triazolo[1,5-*a*]pyridin-7-yloxy)-2-(oxetan-3-yloxy)phenyl)amino)-7-methoxyquinazolin-6-yl)amino)-2-fluoro-3-oxoprop-1-en-1-yl)pyrrolidine-1-carboxylate;
110> *(E)-N-(*4-((4-([1,2,4]triazolo[1,5-*a*]pyridin-7-yloxy)-2-methoxy-5-methylphenyl)amino)-7-methoxyquinazolin-6-yl)-2-fluoro-3-((3*R*)-hexahydro-1*H*-pyrrolizin-3-yl)acrylamide;
111> (*R*,*Z*)-*N*-(4-((4-([1,2,4]triazolo[1,5-*a*]pyridin-7-yloxy)-2-methoxy-5-methylphenyl)amino)-7-methoxyquinazolin-6-yl)-2-fluoro-3-(1-methylpyrrolidin-2-yl)acrylamide;
112> *(S,Z)-N-(4-((4-([*1,2,4]triazolo[1,5-*a*]pyridin-7-yloxy)-2-methoxy-5-methylphenyl)amino)-7-methoxyquinazolin-6-yl)-2-fluoro-3-(1-methylpyrrolidin-2-yl)acrylamide;
113> (*R*,*E*)-*N*-(4-((4-([1,2,4]triazolo[1,5-*a*]pyridin-7-yloxy)-2-methoxy-5-methylphenyl)amino)-7-(4,4-difluoropiperidin-1-yl)quinazolin-6-yl)-2-fluoro-3-(1-methylpyrrolidin-2-yl)acrylamide;
114> (*R*,*Z*)-*N*-(4-((4-([1,2,4]triazolo[1,5-*a*]pyridin-7-yloxy)-2-methoxy-5-methylphenyl)amino)-7-(3,3-difluoroazetidin-1-yl)quinazolin-6-yl)-2-fluoro-3-(1-methylpyrrolidin-2-yl)acrylamide;
115> (*R*,*E*)-*N*-(4-((4-([1,2,4]triazolo[1,5-*a*]pyridin-7-yloxy)-2-methoxy-5-methylphenyl)amino)-7-(4-morpholinopiperidin-1-yl)quinazolin-6-yl)-2-fluoro-3-(1-methylpyrrolidin-2-yl)acrylamide;
116> (*R*,*E*)-*N*-(4-((4-([1,2,4]triazolo[1,5-*a*]pyridin-7-yloxy)-2-methoxy-5-methylphenyl)amino)-7-(2-oxa-6-azaspiro[3.3]heptan-6-yl)quinazolin-6-yl)-2-fluoro-3-(1-methylpyrrolidin-2-yl)acrylamide;
117> *N*-(4-((4-([1,2,4]triazolo[1,5-*a*]pyridin-7-yloxy)-2-methoxy-5-methylphenyl)amino)-7-(2-oxa-6-azaspiro[3.3]heptan-6-yl)quinazolin-6-yl)acrylamide;
118> (*R*,*E*)-*N*-(4-((4-([1,2,4]triazolo[1,5-*a*]pyridin-7-yloxy)-2-methoxy-5-methylphenyl)amino)-7-(methylamino)quinazolin-6-yl)-2-fluoro-3-(1-methylpyrrolidin-2-yl)acrylamide;
119> *(R,E)-N-(*4-((4-([1,2,4]triazolo[1,5-*a*]pyridin-7-yloxy)-2-methoxy-6-methylphenyl)amino)-7-methoxyquinazolin-6-yl)-2-fluoro-3-(1-methylpyrrolidin-2-yl)acrylamide;
120> (*R*,*Z*)-N-(4-((4-([1,2,4]triazolo[1,5-*a*]pyridin-7-yloxy)-2-methoxy-5-methylphenyl)amino)-7-ethoxyquinazolin-6-yl)-2-fluoro-3-(1-methylpyrrolidin-2-yl)acrylamide;
121> *(R,E)-N-(*4-((4-([1,2,4]triazolo[1,5-*a*]pyridin-7-yloxy)-2-methoxy-5-methylphenyl)amino)-7-ethoxyquinazolin-6-yl)-2-fluoro-3-(pyrrolidin-2-yl)acrylamide;
*122> (S,E)-N-(*4-((4-([1,2,4]triazolo[1,5-*a*]pyridin-7-yloxy)-2-methoxy-5-methylphenyl)amino)-7-ethoxyquinazolin-6-yl)-2-fluoro-3-(1-methylpyrrolidin-2-yl)acrylamide;
123> *(R,E)-N*-(4-((4-(4-(dimethylamino)phenoxy)-2-methoxy-5-methylphenyl)amino)-7-methoxyquinazolin-6-yl)-2-fluoro-3-(1-methylpyrrolidin-2-yl)acrylamide;
124> methyl (*R*,*E*)-4-(4-((6-(2-fluoro-3-(1-methylpyrrolidin-2-yl)acrylamido)-7-methoxyquinazolin-4-yl)amino)-5-methoxy-2-methylphenoxy)benzoate;
125> (*R*,*Z*)-4-(4-((6-(2-fluoro-3-(1-methylpyrrolidin-2-yl)acrylamido)-7-methoxyquinazolin-4-yl)amino)-5-methoxy-2-methylphenoxy)-*N*,*N*-dimethylbenzamide;
126> (*R*,*E*)-4-(4-((6-(2-fluoro-3-(1-methylpyrrolidin-2-yl)acrylamido)-7-methoxyquinazolin-4-yl)amino)-5-methoxy-2-methylphenoxy)-*N*,*N*-dimethylbenzamide;
127> *(R,E)-N*-(4-((4-([1,2,4]triazolo[1,5-*a*]pyridin-7-yloxy)-5-(dimethylamino)-2-methoxyphenyl)amino)-7-methoxyquinazolin-6-yl)-2-fluoro-3-(1-methylpyrrolidin-2-yl)acrylamide;
128> (*R*,*Z*)-*N*-(4-((4-([1,2,4]triazolo[1,5-*a*]pyridin-7-yloxy)-5-chloro-2-methoxyphenyl)amino)-7-methoxyquinazolin-6-yl)-2-fluoro-3-(1-methylpyrrolidin-2-yl)acrylamide;
129> (*R*,*Z*)-*N*-(4-((4-([1,2,4]triazolo[1,5-*a*]pyridin-7-yloxy)-5-chloro-2-methoxyphenyl)amino)-7-ethoxyquinazolin-6-yl)-2-fluoro-3-(1-methylpyrrolidin-2-yl)acrylamide;
130> (*R*,*E*)-*N*-(4-((4-([1,2,4]triazolo[1,5-*a*]pyridin-7-yloxy)-5-chloro-2-methoxyphenyl)amino)-7-morpholinoquinazolin-6-yl)-2-fluoro-3-(1-methylpyrrolidin-2-yl)acrylamide;
131> (*R*,*Z*)-*N*-(4-((4-([1,2,4]triazolo[1,5-*a*]pyridin-7-yloxy)-2-methoxy-5-methylphenyl)amino)-7-methoxyquinazolin-6-yl)-2-fluoro-3-(1-methylpiperidin-2-yl)acrylamide;
132> *(R,E)-N-(*4-((4-([1,2,4]triazolo[1,5-*a*]pyridin-7-yloxy)-2-methoxy-5-methylphenyl)amino)-3-cyano-7-ethoxyquinolin-6-yl)-2-fluoro-3-(1-methylpyrrolidin-2-yl)acrylamide;
133> (*R*,*Z*)-*N*-(4-((4-(3-acetamidophenoxy)-2-methoxy-5-methylphenyl)amino)-7-methoxyquinazolin-6-yl)-2-fluoro-3-(1-methylpyrrolidin-2-yl)acrylamide;
134> *(R,E)-N-*(4-((4-([1,2,4]triazolo[1,5-*a*]pyridin-7-yloxy)-2-methoxy-5-methylphenyl)amino)-7-methoxyquinazolin-6-yl)-2-fluoro-3-(pyrrolidin-2-yl)acrylamide;
135> (*R*,*Z*)-*N*-(4-((4-([1,2,4]triazolo[1,5-*a*]pyridin-7-yloxy)-2-methoxy-5-methylphenyl)amino)-7-methoxyquinazolin-6-yl)-2-fluoro-3-(pyrrolidin-2-yl)acrylamide;
136> *(R,E)-N-(*4-((4-([1,2,4]triazolo[1,5-*a*]pyridin-7-yloxy)-2-methoxy-5-methylphenyl)amino)-7-methoxyquinazolin-6-yl)-2-chloro-3-(1-methylpyrrolidin-2-yl)acrylamide;
137> (*R*,*Z*)-N-(4-((4-([1,2,4]triazolo[1,5-*a*]pyridin-7-yloxy)-5-cyclopropyl-2-methoxyphenyl)amino)-7-methoxyquinazolin-6-yl)-2-fluoro-3-(1-methylpyrrolidin-2-yl)acrylamide;
138> (*R*,*E*)-*N*-(4-((4-([1,2,4]triazolo[1,5-*a*]pyridin-7-yloxy)-5-cyclopropyl-2-methoxyphenyl)amino)-7-methoxyquinazolin-6-yl)-2-fluoro-3-(1-methylpyrrolidin-2-yl)acrylamide;
139> (*R*,*Z*)-*N*-(4-((4-([1,2,4]triazolo[1,5-*a*]pyridin-7-yloxy)-5-bromo-2-methoxyphenyl)amino)-7-methoxyquinazolin-6-yl)-2-fluoro-3-(1-methylpyrrolidin-2-yl)acrylamide;
140> (*R*,*E*)-*N*-(4-((4-([1,2,4]triazolo[1,5-*a*]pyridin-7-yloxy)-5-bromo-2-methoxyphenyl)amino)-7-methoxyquinazolin-6-yl)-2-fluoro-3-(1-methylpyrrolidin-2-yl)acrylamide;
141> (*R*,*Z*)-*N*-(4-((4-([1,2,4]triazolo[1,5-*a*]pyridin-7-yloxy)-2-methoxy-5-methylphenyl)amino)-5-chloroquinazolin-6-yl)-2-fluoro-3-(1-methylpyrrolidin-2-yl)acrylamide;
142> (*R*,*E*)-*N*-(4-((4-([1,2,4]triazolo[1,5-*a*]pyridin-7-yloxy)-2-methoxy-5-methylphenyl)amino)-5-chloroquinazolin-6-yl)-2-fluoro-3-(1-methylpyrrolidin-2-yl)acrylamide;
143> (*R*,*Z*)-2-fluoro-N-(7-methoxy-4-((2-methoxy-5-methyl-4-((5-methyl-[1,2,4]triazolo[1,5-*a*]pyridin-7-yl)oxy)phenyl)amino)quinazolin-6-yl)-3-(1-methylpyrrolidin-2-yl)acrylamide;
144> (*Z*)-*N*-(4-((4-([1,2,4]triazolo[1,5-*a*]pyridin-7-yloxy)-2-methoxy-5-methylphenyl)amino)-7-methoxyquinazolin-6-yl)-2-cyano-4-methyl-4-(4-(oxetan-3-yl)piperazin-1-yl)pent-2-enamide;
145> *(E)-N-(*4-((4-([1,2,4]triazolo[1,5-*a*]pyridin-7-yloxy)-2-methoxy-5-methylphenyl)amino)-7-methoxyquinazolin-6-yl)-2-fluoro-4-methyl-4-(4-(oxetan-3-yl)piperazin-1-yl)pent-2-enamide;
146> (*E*)-*N*-(4-((4-([1,2,4]triazolo[1,5-*a*]pyridin-7-yloxy)-2-methoxy-5-methylphenyl)amino)-7-methoxyquinazolin-6-yl)-2-fluoro-4,4-dimethylpent-2-enamide;
147> *N*-(4-((4-([1,2,4]triazolo[1,5-*a*]pyridin-7-yloxy)-2-methoxy-5-methylphenyl)amino)-7-methoxyquinazolin-6-yl)-2-(1*H*-pyrazol-1-yl)acrylamide;
148> *(R,E)-N-(*4-((4-([1,2,4]triazolo[1,5-*a*]pyridin-7-yloxy)-2-(methoxy-*d*₃)-5-methylphenyl)amino)-7-methoxyquinazolin-6-yl)-3-(1-methylpyrrolidin-2-yl)acrylamide;
149> (*R*,*Z*)-*N*-(4-((4-([1,2,4]triazolo[1,5-*a*]pyridin-7-yloxy)-2-(methoxy-*d*₃)-5-methylphenyl)amino)-7-methoxyquinazolin-6-yl)-2-fluoro-3-(1-methylpyrrolidin-2-yl)acrylamide;
150> *(R,E)-N-(*4-((4-([1,2,4]triazolo[1,5-*a*]pyridin-7-yloxy)-2-(methoxy-*d*₃)-5-methylphenyl)amino)-7-methoxyquinazolin-6-yl)-2-fluoro-3-(1-methylpyrrolidin-2-yl)acrylamide;
151> (*R*,*Z*)-N-(4-((4-([1,2,4]triazolo[1,5-*a*]pyridin-7-yloxy)-5-fluoro-2-(methoxy-*d*₃)phenyl)amino)-7-methoxyquinazolin-6-yl)-2-fluoro-3-(1-methylpyrrolidin-2-yl)acrylamide;
152> *(R,E)-N-*(4-((4-([1,2,4]triazolo[1,5-*a*]pyridin-7-yloxy)-5-fluoro-2-(methoxy-*d*₃)phenyl)amino)-7-methoxyquinazolin-6-yl)-2-fluoro-3-(1-methylpyrrolidin-2-yl)acrylamide;
153> *(R,E)-N-(*4-((4-([1,2,4]triazolo[1,5-*a*]pyridin-7-yloxy)-2-methoxy-5-methylphenyl)amino)-7-methoxyquinazolin-6-yl)-3-(1-(methyl-*d*₃)pyrrolidin-2-yl)acrylamide;
154> (*R*,*Z*)-*N*-(4-((4-([1,2,4]triazolo[1,5-*a*]pyridin-7-yloxy)-2-methoxy-5-methylphenyl)amino)-7-methoxyquinazolin-6-yl)-2-fluoro-3-(1-(methyl-*d*₃)pyrrolidin-2-yl)acrylamide;
155> (*R*,*Z*)-*N*-(4-((4-([1,2,4]triazolo[1,5-*a*]pyridin-7-yloxy)-5-fluoro-2-methoxyphenyl)amino)-7-methoxyquinazolin-6-yl)-2-fluoro-3-(1-(methyl-*d*₃)pyrrolidin-2-yl)acrylamide;
156> (*R,Z*)-*N*-(4-((4-([1,2,4]triazolo[1,5-*a*]pyridin-7-yloxy)-5-chloro-2-methoxyphenyl)amino)-7-methoxyquinazolin-6-yl)-2-fluoro-3-(1-(methyl-*d*₃)pyrrolidin-2-yl)acrylamide; and
157> (*R*,*E*)-*N*-(4-((4-([1,2,4]triazolo[1,5-*a*]pyridin-7-yloxy)-5-fluoro-2-methoxyphenyl)amino)-7-methoxyquinazolin-6-yl)-2-fluoro-3-(1-(methyl-*d*₅)pyrrolidin-2-yl)acrylamide.

The compound of Formula (I) of the present invention may exist in the form of a pharmaceutically acceptable salt. Accordingly, the scope of the compound of the present invention includes pharmaceutically acceptable salts of the compound represented by Formula (I) above. As used herein, the term "pharmaceutically acceptable salt" refers to any organic acid or inorganic acid addition salt of the compounds, which has a concentration for exhibiting an effective action, being relatively non-toxic and unharmful to patients, and in which adverse effects resulting from the salt do not deteriorate the beneficial efficacy of the compounds represented by Formula (I).

In particular, the pharmaceutically acceptable salt may be an acid addition salt formed by a free acid. Here, the acid addition salt may be obtained from inorganic acids such as hydrochloric acid, nitric acid, phosphoric acid, sulfuric acid, hydrobromic acid, hydroiodic acid, nitrous acid, and phosphorous acid, non-toxic organic acids such as aliphatic mono- and dicarboxylate, phenyl-substituted alkanoate, hydroxy alkanoate, alkanedioate, aromatic acids, and aliphatic and aromatic sulfonic acids, and organic acids such as trifluoroacetic acid, acetate, benzoic acid, citric acid, lactic acid, maleic acid, gluconic acid, methanesulfonic acid, 4-toluenesulfonic acid, tartaric acid, and fumaric acid.

Types of such pharmaceutically acceptable salts may include sulfate, sulfite, nitrate, phosphate, pyrophosphate, chloride, bromide, iodide, fluoride, acetate, propionate, decanoate, caprylate, acrylate, formate, isobutyrate, caprate, heptanoate, propiolate, oxalate, malonate, succinate, suberate, sebacate, fumarate, maleate, benzoate, phthalate, benzenesulfonate, toluenesulfonate, phenylacetate, phenylpropionate, phenylbutyrate, citrate, lactate, glycolate, malate, tartrate, mandelate, and the like.

The acid addition salt may be prepared by a conventional method, for example, by dissolving a derivative of Formula (I) in an organic solvent such as methanol, ethanol, acetone, methylene chloride, or acetonitrile, and filtering and drying a precipitate produced by adding an organic acid or an inorganic acid, or may be prepared by distilling a solvent and an excess acid under reduced pressure and then drying and crystallizing in an organic solvent.

In addition, the pharmaceutically acceptable salt may be a salt or a metal salt obtained using a base. As an example of the metal salt, an alkali metal or alkaline earth metal salt may be obtained by dissolving the compound in an excess alkali metal hydroxide or alkaline earth metal hydroxide solution, filtering the undissolved compound salt, and evaporating and drying the filtrate. As the alkali metal salt, sodium, potassium, or calcium salts may be pharmaceutically suitable. In addition, the corresponding salt may be obtained by reacting an alkali metal or alkaline earth metal salt with an appropriate silver salt (for example, silver nitrate).

In addition, the compounds of the present invention may have a chiral carbon center, and thus may be present in the form of R or S isomers or mixtures, individual enantiomers or mixtures, or individual diastereomers or mixtures, and all such stereoisomers and mixtures thereof (*e.g*., racemic mixtures) may fall within the scope of the present invention.

In addition, the compounds of the present invention may include hydrates and solvates of the compounds of Formula (I). The hydrates and solvates may be prepared using known methods and are preferably non-toxic and water-soluble. In particular, the hydrates and the solvates may preferably be those obtained by binding, to the compound, 1 to 5 molecules of water and alcoholic solvent (in particular, ethanol or the like), respectively.

In addition, metabolites may be formed in vivo upon the administration of the compounds of the present invention, and specifically, such metabolites can be produced from oxidation, reduction, hydrolysis, amidation, deamidation, esterification, enzymatic cleavage, and the like of the administered compounds. For example, a compound produced by a method including contacting the compound of the present invention with a mammal for a period of time sufficient to yield a metabolite of the compound of the invention may also fall within the scope of the present invention.

In addition, the compound of the present invention has little or no pharmacological activity by itself, but when administered into the body, it can be prepared in the form of a prodrug which may be converted into a compound having desired activity by, for example, hydrolysis. Such a prodrug may be derivatives of a compound including a biologically reactive functional group, and the biologically reactive functional group can be cleaved from the compound or react in other ways to provide the compound under biological conditions (in vivo or in vitro). Typically, the prodrug is inactive or at least has lower activity than the compound, which makes the compound exhibit its activity after cleaved from the biologically reactive functional group. The biologically reactive functional group can be hydrolyzed or oxidized under biological conditions to form a compound. For example, the prodrug may contain a biologically hydrolysable group. Examples of the biologically hydrolysable group include, but are not limited to, biologically hydrolysable phosphates, biologically hydrolysable esters, biologically hydrolysable amides, biologically hydrolysable carbonic esters, biologically hydrolysable carbamates, and biologically hydrolysable ureides.

### Use of Heteroaryl Derivatives

Another aspect of the present invention provides a pharmaceutical use of the compound represented by Formula (I) or a pharmaceutically acceptable salt or stereoisomer thereof.

The compound of Formula (I) according to the present invention or a pharmaceutically acceptable salt or stereoisomer thereof may inhibit the activity of Human epidermal growth factor receptor 2 (HER2). Specifically, the compound of Formula (I) according to the present invention or a pharmaceutically acceptable salt or stereoisomer thereof may inhibit binding between the HER2 and a ligand.

Accordingly, the present invention provides a HER2 inhibitor including the compound of Formula (I) or a pharmaceutically acceptable salt or stereoisomer thereof as an active ingredient.

In addition, the present invention provides a method for inhibiting the activity of HER2, the method including treating a specimen or cells with the compounds of Formula (I), or pharmaceutically acceptable salts or stereoisomers thereof.

As such, the compound of Formula (I) or a pharmaceutically acceptable salt or stereoisomer thereof may be used to treat or prevent HER2-related diseases, in particular, cancer.

In particular, the compound of Formula (I), or a pharmaceutically acceptable salt or stereoisomer thereof exhibits excellent inhibitory activity against HER2 mutation (*e*.*g*., HER2 T798I, HER2 L755S, HER2 T862A, *etc*.) and is useful for treating or preventing cancer types induced by HER2 or HER2 mutation.

Here, the "prevention" refers to any act of inhibiting or delaying the occurrence, spread, and recurrence of the disease, and the "treatment" refers to any act of ameliorating or beneficially altering symptoms of the disease by the administration of the compound.

Accordingly, the present invention provides a use of the compound represented by Formula (I) or a pharmaceutically acceptable salt or stereoisomer thereof for treating or preventing HER2-related diseases.

The present invention also provides a use of the compound represented by Formula (I) or a pharmaceutically acceptable salt or stereoisomer thereof for preparing a medicament for treating or preventing HER2-related diseases.

The present invention also provides a method for treating or preventing HER2-related diseases, the method including administering to a subject in need thereof a therapeutically effective amount of the compound represented by Formula (I) or a pharmaceutically acceptable salt or stereoisomer thereof.

Specifically, the present invention provides a method for treating or preventing at least one disease selected from the group consisting of pseudomyxoma, intrahepatic cholangiocarcinoma, hepatoblastoma, liver cancer, thyroid cancer, colon cancer, testicular cancer, myelodysplastic syndrome, glioblastoma, oral cancer, lip cancer, mycosis fungoides, acute myeloid leukemia, acute lymphoblastic leukemia, basal cell carcinoma, ovarian epithelial cancer, ovarian germ cell carcinoma, male breast cancer, brain cancer, pituitary adenoma, multiple myeloma, gallbladder cancer, biliary tract cancer, colorectal cancer, chronic myelogenous leukemia, chronic lymphocytic leukemia, retinoblastoma, choroidal melanoma, ampulla of Vater cancer, bladder cancer, peritoneal cancer, parathyroid cancer, adrenal cancer, sinonasal cancer, non-small cell lung cancer, tongue cancer, astrocytoma, small cell lung cancer, pediatric brain cancer, pediatric lymphoma, pediatric leukemia, small bowel cancer, meningioma, esophageal cancer, glioma, renal pelvis cancer, renal cancer, heart cancer, duodenal cancer, malignant soft tissue tumor, malignant bone cancer, malignant lymphoma, malignant mesothelioma, malignant melanoma, eye cancer, vulvar cancer, ureteral cancer, urethral cancer, cancer of unknown primary site, gastric lymphoma, gastric cancer, gastric carcinoid, gastrointestinal stromal cancer, Wilms' tumor, breast cancer, sarcoma, penile cancer, pharyngeal cancer, gestational choriocarcinoma, cervical cancer, endometrial cancer, uterine sarcoma, prostate cancer, metastatic bone cancer, metastatic brain cancer, mediastinal cancer, rectal cancer, rectal carcinoid, vaginal cancer, spinal cord cancer, vestibular schwannoma, pancreatic cancer, salivary gland cancer, Kaposi's sarcoma, Paget's disease, tonsil cancer, squamous cell carcinoma, lung adenocarcinoma, lung cancer, squamous cell carcinoma of lung, skin cancer, anal cancer, rhabdomyosarcoma, laryngeal cancer, pleura cancer, hematological cancer, and thymus cancer, the method including administering to a subject in need thereof, a therapeutically effective amount of the compound represented by Formula (I), or a pharmaceutically acceptable salt or stereoisomer thereof.

Here, the term "subject in need thereof" refers to any animal, specifically a mammal, such as a monkey, a cow, a horse, a sheep, a pig, a chicken, a turkey, a quail, a cat, a dog, a mouse, a rat, a rabbit, and a guinea pig, including a human (patient), who has or is likely to develop the disease, and specifically, may refer to a mammal including a human. In addition, the subject in need thereof may refer to a biological sample.

In addition, the "administration" means providing a predetermined substance to a subject in need thereof by any appropriate method, and administration of the compound of the present invention may be achieved via any common route, such as an oral or parenteral route, as long as the route allows the substance to reach a target tissue.

The present invention also provides a pharmaceutical composition containing the compound of Formula (I), or a pharmaceutically acceptable salt or stereoisomer thereof as an active ingredient.

The present invention also provides a pharmaceutical composition for treating or preventing HER2-related diseases, the composition containing the compound of Formula (I), or a pharmaceutically acceptable salt or stereoisomer thereof as an active ingredient.

The HER2-related diseases may be, for example, cancer. The cancer includes all cancers in which the pharmaceutical composition of the present invention may exhibit therapeutic and prophylactic efficacy due to the inhibition ofHER2 activity, and may be solid cancer or blood cancer. Also, the cancer includes not only primary cancer but also metastatic cancer. As a specific example, the HER2-related disease may be at least one selected from the group consisting of pseudomyxoma, intrahepatic cholangiocarcinoma, hepatoblastoma, liver cancer, thyroid cancer, colon cancer, testicular cancer, myelodysplastic syndrome, glioblastoma, oral cancer, lip cancer, mycosis fungoides, acute myeloid leukemia, acute lymphoblastic leukemia, basal cell carcinoma, ovarian epithelial cancer, ovarian germ cell carcinoma, male breast cancer, brain cancer, pituitary adenoma, multiple myeloma, gallbladder cancer, biliary tract cancer, colorectal cancer, chronic myelogenous leukemia, chronic lymphocytic leukemia, retinoblastoma, choroidal melanoma, ampulla of Vater cancer, bladder cancer, peritoneal cancer, parathyroid cancer, adrenal cancer, sinonasal cancer, non-small cell lung cancer, tongue cancer, astrocytoma, small cell lung cancer, pediatric brain cancer, pediatric lymphoma, pediatric leukemia, small bowel cancer, meningioma, esophageal cancer, glioma, renal pelvis cancer, renal cancer, heart cancer, duodenal cancer, malignant soft tissue tumor, malignant bone cancer, malignant lymphoma, malignant mesothelioma, malignant melanoma, eye cancer, vulvar cancer, ureteral cancer, urethral cancer, cancer of unknown primary site, gastric lymphoma, gastric cancer, gastric carcinoid, gastrointestinal stromal cancer, Wilms' tumor, breast cancer, sarcoma, penile cancer, pharyngeal cancer, gestational choriocarcinoma, cervical cancer, endometrial cancer, uterine sarcoma, prostate cancer, metastatic bone cancer, metastatic brain cancer, mediastinal cancer, rectal cancer, rectal carcinoid, vaginal cancer, spinal cord cancer, vestibular schwannoma, pancreatic cancer, salivary gland cancer, Kaposi's sarcoma, Paget's disease, tonsil cancer, squamous cell carcinoma, lung adenocarcinoma, lung cancer, squamous cell carcinoma of lung, skin cancer, anal cancer, rhabdomyosarcoma, laryngeal cancer, pleura cancer, hematological cancer, and thymus cancer, but is not limited thereto.

The pharmaceutical composition may include conventional and non-toxic pharmaceutically acceptable additives which are blended into a preparation according to a conventional method. Accordingly, the present invention provides a pharmaceutical composition containing the compound of Formula (I), or a pharmaceutically acceptable salt or stereoisomer thereof, as an active ingredient, and containing a pharmaceutically acceptable additive. For example, the pharmaceutical composition may further contain a pharmaceutically acceptable carrier, diluent, or excipient. In an embodiment, the present invention provides a pharmaceutical composition containing a compound of Formula (I), or a pharmaceutically acceptable salt or stereoisomer thereof, and a pharmaceutically acceptable carrier. As the pharmaceutically acceptable carrier, at least one from among a saline solution, sterilized water, a Ringer's solution, a buffered saline solution, a dextrose solution, a maltodextrin solution, glycerol, ethanol, and the like may be used, and other conventional additives such as an antioxidant, a buffer solution, and a bacteriostatic agent may be added, if necessary.

In addition, the pharmaceutical composition may be formulated into an injection formulation such as an aqueous solution, a suspension, or an emulsion, a pill, a capsule, a granule, or a tablet by additionally adding a diluent, a dispersant, a surfactant, a binder, and a lubricant. Accordingly, the composition of the present invention may be a patch agent, a liquid agent, a pill, a capsule, a granule, a tablet, a suppository, or the like.

These formulations may be prepared by a conventional method used in the art for formulation or by a method disclosed in Remington's Pharmaceutical Science, Mack Publishing Company, Easton PA and may be formulated in a variety of formulations depending on the disease or ingredients.

In addition, the pharmaceutical composition of the present invention may further contain one or more active ingredients exhibiting the same or similar medicinal effects, in addition to the compound represented by Formula (I) or the pharmaceutically acceptable salt or stereoisomer thereof.

The pharmaceutical composition may be made into various preparation forms for oral administration (for example, tablets, pills, powders, capsules, syrups, or emulsions) or parenteral administration (for example, intramuscular, intravenous, or subcutaneous injection).

For example, the pharmaceutical composition may be made into a preparation for oral administration, in which additives used may include cellulose, calcium silicate, corn starch, lactose, sucrose, dextrose, calcium phosphate, stearic acid, magnesium stearate, calcium stearate, gelatin, talc, surfactants, suspending agents, emulsifying agents, diluents, and the like. Specifically, solid preparations for oral administration include tablets, pills, powders, granules, capsules and the like. Such solid preparations may be formulated by mixing at least one excipients, for example, starch, calcium carbonate, sucrose, lactose, gelatin, etc., into the composition. In addition to simple excipients, lubricants such as magnesium stearate and talc may also be used. Further, as liquid preparations for oral administration, suspensions, emulsions, syrups, and the like may be exemplified, and the liquid preparations may contain various excipients such as wetting agents, sweeteners, fragrances, and preservatives in addition to water and liquid paraffin which are commonly used as simple diluents.

In addition, examples of preparations for parenteral administration may include sterilized aqueous solutions, non-aqueous solutions, suspensions, emulsions, freeze-dried preparations, and suppositories. For the non-aqueous solutions and the suspensions, propylene glycol, polyethylene glycol, vegetable oil such as olive oil, injectable ester such as ethyl oleate, or the like may be used. As the suppository base, Witepsol^{™}, macrogol, Tween^{™} 61, cacao butter, laurin fat, glycerogelatin, or the like may be used. Injections may contain conventional additives such as solubilizers, isotonic agents, suspending agents, emulsifiers, stabilizers, and preservatives.

The compound or composition of the present invention may be administered to a patient in a therapeutically effective amount or in a pharmaceutically effective amount.

As used herein, the term "therapeutically effective amount" refers to an amount of the compound represented by Formula (I) that is effective in treating or preventing HER2-related diseases. Specifically, the "therapeutically effective amount" refers to an amount sufficient to treat a disease at a reasonable benefit/risk ratio applicable to any medical treatment, and the effective dose level of the compound or composition may be determined according to the factors including a type, an age, and a sex of individual, severity, a type of disease, an activity of drug, a sensitivity to drug, an administration time, an administration route and an excretion rate, duration of treatment, drugs used in combination with the composition, and other factors well known in the medical field.

The compound or composition of the present invention may be administered as an individual therapeutic agent or in combination with other therapeutic agents, may be administered sequentially or simultaneously with a therapeutic agent on the market, and may be administered in single or multiple doses. Taking all of the above factors into consideration, it is important to administer an amount that is a minimum amount and allows the maximum effect to be obtained without side effects, and such an amount may be easily determined by those skilled in the art.

The dosage of the pharmaceutical composition of the present invention may be determined by an expert according to various factors such as a patient's condition, age, sex, and complications. The active ingredient of the pharmaceutical composition of the present invention has excellent safety, and thus may be used in an amount equal to or greater than a determined dosage.

In addition, for the preparation of the medicament using the compound represented by Formula (I), an adjuvant, a diluent, a carrier, and the like may be mixed, and the compound may be prepared as a combination formulation together with other active agents so as to exhibit synergistic effects of active ingredients.

The matters mentioned in the application, composition, and treatment method of the present invention are applied in the same manner, unless they contradict each other.

### Mode for the Invention

Hereinafter, the present invention will be described in detail through Examples and Experimental Examples. However, the following Examples and Experimental Examples merely illustrate the present invention, and the scope of the present invention is not limited thereto.

### Analysis and Purification Conditions

### 1. HPLC Analysis Conditions

Instrument Name: e2695 from Waters Corporation
Column: YMC-Pack ODS-AM, 150 x 4.6 mm, 3 µm, 12 nm, 40 °C
Mobile phase: 10% → 90% acetonitrile/H₂O + 0.1% TFA
Analysis time: 20 minutes, flow rate: 1 mL/min
UV detector: 254 nm

### 2. LC-MS Analysis Conditions

Instrument Name: AQUITY UPLC from Waters Corporation
Column: AQUITY UPLC^{®} BEH C18, 50 x 2.1 mm, 5 µm, 40 °C
Mobile phase: Acetonitrile/H₂O + 0.1% TFA
Flow rate: 0.6 mL/min
UV detector: 254 nm

### 3. MPLC Purification Conditions

Instrument Name: CombiFlash^{®} Rf+ from Teledyne ISCO, Inc.
UV detector: 254 nm

### 4. Prep-HPLC Purification Conditions (A)

Instrument Name: ACCQPrep^{®} HP125 from Teledyne ISCO, Inc.
Column: XBridge^{®} Prep Shield RP18, 250 x 19 mm, 10 µm
Mobile phase: Acetonitrile/0.1% TFA H₂O
Flow rate: 25 mL/min
UV detector: 254 nm

### 5. Prep-HPLC Purification Conditions (B)

Instrument Name: ACCQPrep^{®} ACCQPrep HP125 from Teledyne ISCO, Inc.
Column: XBridge^{®} Prep Shield RP18, 250 x 19 mm, 10 µm
Mobile phase: Acetonitrile/0.1% FA H₂O
Flow rate: 25 mL/min
UV detector: 254 nm

### 6. ¹H NMR

Instrument Name: Bruker Ascend^{™} 400 (400 MHz)

The commercially available reagent was used without further purification. In the specification, room temperature (r.t.) or ambient temperature refers to a temperature of 5 °C to 40 °C, as an example, 10 °C to 30 °C, and as another example, 20 °C to 27 °C, and is not strictly limited within the above range. A rotary evaporator was used for concentration under reduced pressure or solvent distillation removal.

### Preparation Example 1: Preparation of 1-(4-((4-((4-([1,2,4]triazolo[1,5-a]pyridin-7-yloxy)-2-methoxy-5-methylphenyl)amino)-7-methoxyquinazolin-6-yl)amino)piperidin-1-yl)prop-2-en-1-one (Example 1)

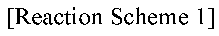

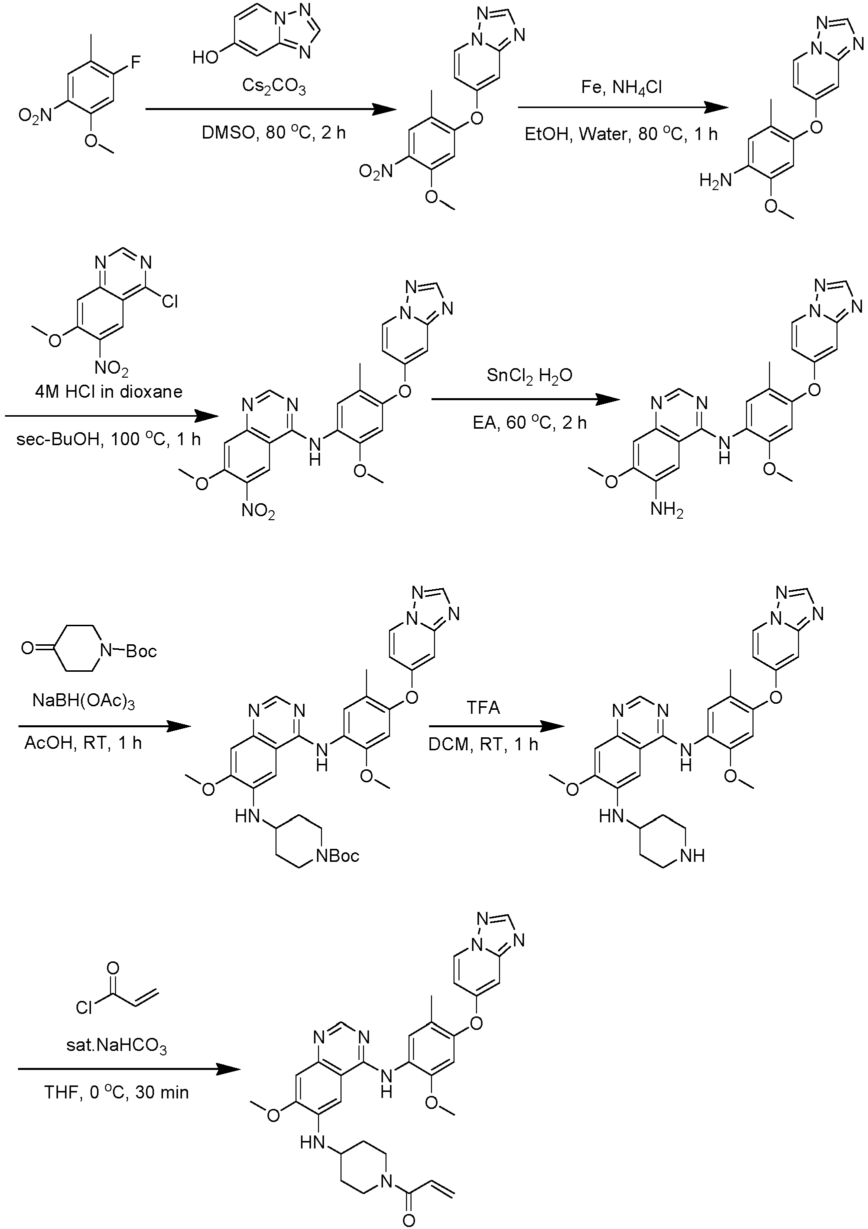

### [Step 1] Preparation of 7-(5-methoxy-2-methyl-4-nitrophenoxy)-[1,2,4]triazolo[1,5-a]pyridine

1-Fluoro-5-methoxy-2-methyl-4-nitrobenzene (1.0 eq), [1,2,4]triazolo[1,5-*a*]pyridin-7-ol (1.2 eq), and Cs₂CO₃ (2.4 eq) were dissolved in DMSO (0.2 M), followed by stirring at 80 °C for 2 hours. A solid was formed by pouring ice water into the reaction mixture and then filtered to obtain the desired compound as a yellow solid (yield: 99%, MS (ESI): m/z 301 [M+H]⁺).

### [Step 2] Preparation of 4-([1,2,4]triazolo[1,5-a]pyridin-7-yloxy)-2-methoxy-5-methylaniline

7-(5-Methoxy-2-methyl-4-nitrophenoxy)-[1,2,4]triazolo[1,5-*a*]pyridine (1.0 eq) prepared in step 1, ammonium chloride (0.45 eq), and iron (4.5 eq) were dissolved in an ethanol-water solution (3:1), followed by stirring at 80 °C for 1 hour. The mixture was filtered through a celite filter and then concentrated. The reaction mixture was purified using MPLC (dichloromethane:methanol) to obtain the desired compound as a brown solid (yield: 98%, MS (ESI): m/z 271 [M+H]⁺).

### [Step 3] Preparation of N-(4-([1,2,4]triazolo[1,5-a]pyridin-7-yloxy)-2-methoxy-5-methylphenyl)-7-methoxy-6-nitroquinazolin-4-amine

4-([1,2,4]Triazolo[1,5-*a*]pyridin-7-yloxy)-2-methoxy-5-methylaniline (1.0 eq) obtained in step 2 and 4-chloro-7-methoxy-6-nitroquinazoline (1.0 eq) were dissolved in *sec*-BuOH (0.05 M), and then 4 M HCl (0.2 eq) dissolved in 1,4-dioxane was added thereto, followed by stirring at 100 °C for 1 hour. Ether was added to the reaction mixture, and then the desired compound as a yellow solid was obtained through a filter (yield: 95%, MS (ESI): m/z 474 [M+H]⁺).

### [Step 4] Preparation of N⁴-(4-([1,2,4]triazolo[1,5-a]pyridin-7-yloxy)-2-methoxy-5-methylphenyl)-7-methoxyquinazoline-4,6-diamine

*N*-(4-([1,2,4]Triazolo[1,5-*a*]pyridin-7-yloxy)-2-methoxy-5-methylphenyl)-7-methoxy-6-nitroquinazolin-4-amine (1.0 eq) obtained in step 3 and SnCl₂.2H₂O (5.0 eq) were dissolved in ethyl acetate (0.1 M), and the mixture was stirred at 60 °C for 2 hours. An aqueous ammonia solution was added to the reaction mixture and then concentrated. The reaction mixture was purified using MPLC (dichloromethane:methanol) to obtain the desired compound as a pale yellow solid (yield: 65%, MS (ESI): m/z 444 [M+H]⁺).

### [Step 5] Preparation of tert-butyl 4-((4-((4-([1.2.4]triazolo[1,5-a]pyridin-7-yloxy)-2-methoxy-5-methylphenyl)amino)-7-methoxyquinazolin-6-yl)amino)piperidine-1-carboxylate

*N*⁴-(4-([1,2,4]Triazolo[1,5-*a*]pyridin-7-yloxy)-2-methoxy-5-methylphenyl)-7-methoxyquinazoline-4,6-diamine (1.0 eq) prepared in step 4 and *tert-butyl* 4-oxopiperidine-1-carboxylate (3.0 eq) were dissolved in acetic acid (0.1 M), followed by stirring at room temperature for 1 hour. Sodium triacetoxyborohydride (4.0 eq) was added to the mixture, followed by stirring at room temperature for 1 hour. Organic materials were extracted with a saturated aqueous NaHCO₃ solution and dichloromethane, and then remaining water in collected organic layer was removed using MgSO₄, the collected organic layer was concentrated. The obtained desired compound as a yellow solid was used in the following reaction without further purification (yield: 100%, MS (ESI): m/z 627 [M+H]⁺).

### [Step 6] Preparation of N⁴-(4-([1,2,4]triazolo[1,5-a]pyridin-7-yloxy)-2-methoxy-5-methylphenyl)-7-methoxy-N⁶-(piperidin-4-yl)quinazoline-4,6-diamine

The compound obtained in step 5, *tert*-butyl 4-((4-((4-([1,2,4]triazolo[1,5-*a*]pyridin-7-yloxy)-2-methoxy-5-methylphenyl)amino)-7-methoxyquinazolin-6-yl)amino)piperidine-1-carboxylate (1.0 eq) was dissolved in dichloromethane (0.1 M), and then trifluoroacetic acid (TFA, 60.0 eq) was added thereto, followed by stirring at room temperature for 1 hour and then concentrating. The reaction mixture was purified using MPLC (dichloromethane:methanol) to obtain the desired compound as a yellow solid (yield: 59%, MS (ESI): m/z 527 [M+H]⁺).

### [Step 7] Preparation of 1-(4-((4-((4-([1,2,4]triazolo[1,5-a]pyridin-7-yloxy)-2-methoxy-5-methylphenyl)amino)-7-methoxyquinazolin-6-yl)amino)piperidin-1-yl)prop-2-en-1-one

*N*⁴-(4-([1,2,4]Triazolo[1,5-*a*]pyridin-7-yloxy)-2-methoxy-5-methylphenyl)-7-methoxy-*N*⁶-(piperidin-4-yl)quinazoline-4,6-diamine (1.0 eq) obtained in step 6 was dissolved in tetrahydrofuran (THF, 0.1 M), a 1 M aqueous NaHCO₃ solution (5.0 eq) was added thereto at 0 °C, and acryloyl chloride (1.0 eq) was added thereto, followed by stirring at 0 °C for 30 minutes. The reaction mixture was concentrated and then purified by prep-HPLC to obtain the desired compound as an ivory solid (yield: 34%, MS (ESI): m/z 581 [M+H]⁺).

### Preparation of Compounds of Examples 2 to 4

The compounds of Examples 2 to 4 according to the present invention were prepared in a similar manner to Preparation Example 1.

The chemical structural formulas, compound names, ¹H NMR, MS, yields, HPLC data, and purities of the compounds of Examples 1 to 4 are summarized in Table 1 below.

**[Table 1]**

| Ex. | Structure | Compound name | ¹H NMR, MS | Yield (%) | HPLC (min), Purity (%) |
|---|---|---|---|---|---|
| 1 | 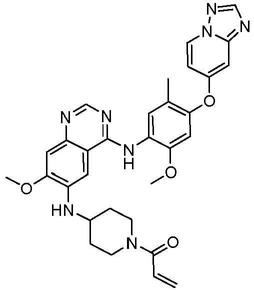 | 1-(4-((4-((4-([1,2,4]triazolo[1,5 -*a*]pyridin-7-yloxy)-2-methoxy-5-methylphenyl)amin o)-7-methoxyquinazolin -6-yl)amino)piperidin-1-yl)prop-2-en-1-one | ¹H NMR (400 MHz, free, Methanol-d₄) δ8.81 (d, J = 7.6 Hz, 1H), 8.50 (s, 1H), 8.34 (s, 1H), 7.54 (s, 1H), 7.46 (s, 1H), 7.16-7.14 (m, 2H), 7.05 (s, 1H), 6.89-6.82 (m, 2H), 6.27-6.22 (m, 1H), 5.81-5.78 (m, 1H), 4.66-4.62 (m, 1H), 4.26-4.23 (m, 1H), 4.13 (s, 3H), 3.88-3.83 (m, 4H), 3.37-3.31 (m, 1H), 3.04-2.98 (m, 1H), 2.25-2.20 (m, 5H), 1.61-1.55 (m, 2H); MS (ESI): m/z 581 [M+H]⁺ | 34 | 8.87, 100 |
| 2 | 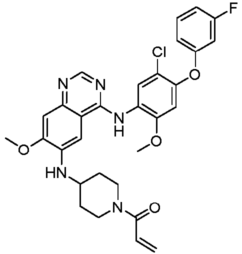 | 1-(4-((4-((5-chloro-4-(3-fluorophenoxy)-2-methoxyphenyl)am ino)-7-methoxyquinazolin -6-yl)amino)piperidin-1-yl)prop-2-en-1-one | ¹H NMR (400 MHz, FA salt, Methanol-d₄) δ 8.28 (s, 1H), 7.85 (s, 1H), 7.38-7.32 (m, 1H), 7.23 (s, 1H), 7.09 (s, 1H), 6.97 (s, 1H), 6.85-6.78 (m, 3H), 6.77-6.73 (m, 1H), 6.23-6.19 (m, 1H), 5.77-5.74 (m, 1H), 4.58 (d, J = 12.8 Hz, 1H), 4.19 (d, J = 13.2 Hz, 1H), 4.05 (s, 3H), 3.88-3.82 (m, 4H), 3.40-3.35 (m, 1H), 3.03 (tr, J = 11.6 Hz, 1H), 2.25-2.21 (m, 2H), 1.59-1.51 (m, 2H); MS (ESI): m/z 578 [M]⁺ | 12 | 11.29, 99 |
| 3 | 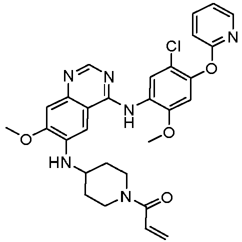 | 1-(4-((4-((5-chloro-2-methoxy-4-(pyridin-2-yloxy)phenyl)amin o)-7-methoxyquinazolin -6-yl)amino)piperidin-1-yl)prop-2-en-1-one | ¹H NMR (400 MHz, free, Methanol-d₄) δ 8.53 (s, 1H), 8.15-8.13 (m, 1H), 7.93-7.88 (m, 1H), 7.72 (s, 1H), 7.46 (s, 1H), 7.19-7.15 (m, 3H), 7.11 (d, J = 8.4 Hz, 1H), 6.89-6.82 (m, 1H), 6.27-6.22 (m, 1H), 5.81-5.77 (m, 1H), 4.65-4.62 (m, 1H), 4.23-4.21 (m, 1H), 4.13 (s, 3H), 3.88-3.86 (m, 4H), 3.34-3.32 (m, 1H), 3.01-2.99 (m, 1H), 2.25-2.22 (m, 2H), 1.69-1.58 (m, 2H); MS (ESI): m/z 562 [M+H]⁺ | 28 | 10.10, 100 |
| 4 | 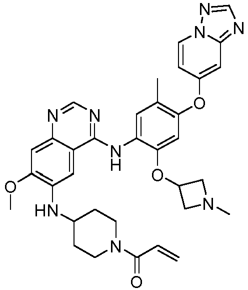 | 1-(4-((4-((4-([1,2,4]triazolo[1,5 -*a*]pyridin-7-yloxy)-5-methyl-2-((1-methylazetidin-3-yl)oxy)phenyl)ami no)-7-methoxyquinazolin -6-yl)amino)piperidin-1-yl)prop-2-en-1-one | ¹H NMR (400MHz, TFA salt, Methanol-d₄) δ 8.95 (s, 1H), 8.79 (d, J = 7.3 Hz, 1H), 8.34 (s, 1H), 7.34 (s, 1H), 7.26 (s, 1H), 7.15 (d, J = 8.9 Hz, 1H), 7.08 (s, 2H), 7.01 (s, 1H), 6.80 (d, J = 8.9, 1H), 6.77 - 6.58 (m, 1H), 6.19-6.13 (m, 1H), 5.72 (s, 1H), 5.01 (s, 1H), 4.51 - 4.48 (m, 2H), 4.09 (s, 3H), 3.98 - 3.72 (m, 4H), 3.63 - 3.42 (m, 4H), 2.84 (s, 3H), 2.03 - 1.56 (m, 4H) ; MS (ESI): m/z 636 [M+H]⁺ | 5 | 7.60, 86 |

### Preparation Example 2: Preparation of 1-(4-((4-((4-([1,2,4]triazolo[1,5-a]pyridin-7-yloxy)-5-chloro-2-methoxyphenyl)amino)-7-methoxyquinazolin-6-yl)oxy)piperidin-1-yl)prop-2-en-1-one (Example 5)

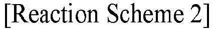

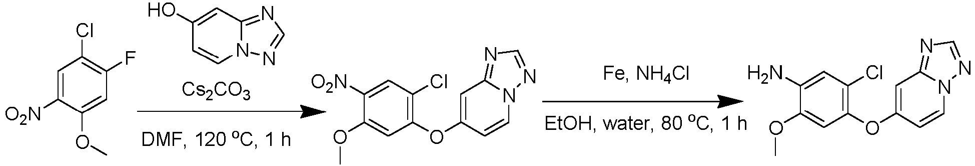

### [Step A1] Preparation of 7-(2-chloro-5-methoxy-4-nitrophenoxy)-[1,2,4]triazolo[1,5-a]pyridine

1-Chloro-2-fluoro-4-methoxy-5-nitrobenzene (1.0 eq), [1,2,4]triazolo[1,5-*a*]pyridin-7-ol (1.0 eq) were dissolved in DMF (0.1 M) and Cs₂CO₃ (1.2 eq) was added thereto, followed by stirring at 120 °C for 1 hour. The reaction mixture was put into ice water and stirred to form a solid, and the resulting solid was filtered using a filter, and the obtained desired compound as a yellow solid was used in the following reaction without further purification (yield: 100%, MS (ESI): m/z 321 [M+H]⁺).

### [Step A2] Preparation of 4-([1,2,4]triazolo[1,5-a]pyridin-7-yloxy)-5-chloro-2-methoxyaniline

7-(2-Chloro-5-methoxy-4-nitrophenoxy)-[1,2,4]triazolo[1,5-*a*]pyridine (1.0 eq) obtained in step A1 and iron (4.5 eq) were dissolved in an ethanol-water solution (3:1, 0.566 M), and ammonium chloride (0.45 eq) was added thereto, followed by stirring at 80 °C for 1 hour. The reaction mixture was filtered by a filter, organic materials were extracted with water and dichloromethane, and then remaining water in collected organic layer was removed using MgSO₄, the collected organic layer was concentrated. The reaction mixture was purified using MPLC (dichloromethane:methanol) to obtain the desired compound as a yellow solid (yield: 36%, MS (ESI): m/z 291 [M+H]⁺).

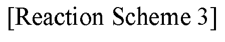

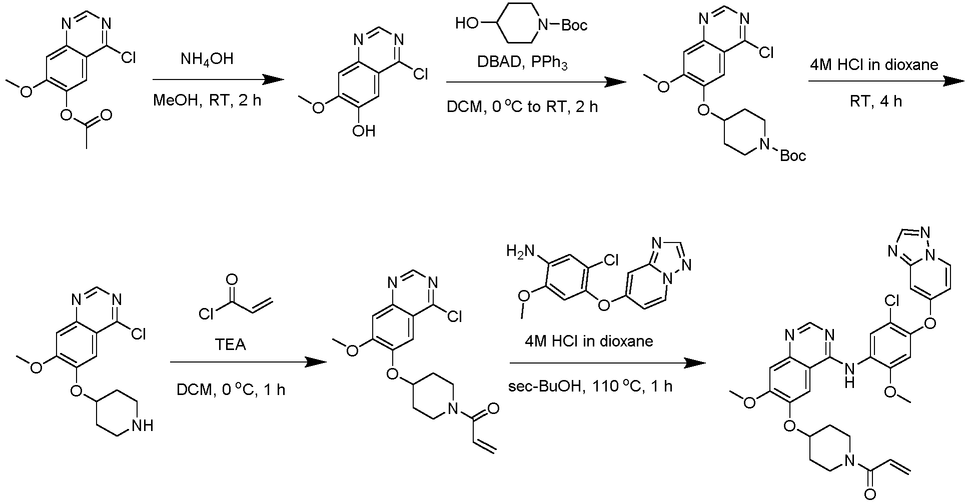

### [Step B1] Preparation of 4-chloro-7-methoxyquinazolin-6-ol

After 4-chloro-7-methoxyquinazolin-6-yl acetate (1.0 eq) was dissolved in methanol (0.7 M), ammonia water (20.0 eq) was added, and the reaction mixture was stirred at room temperature for 2 hours. After ether was added to the reaction mixture to form a solid, the resulting solid was filtered to obtain the desired compound as a yellow solid (yield: 100%, MS (ESI): m/z 211 [M+H]⁺).

### [Step B2] Preparation of tert-butyl 4-((4-chloro-7-methoxyquinazolin-6-yl)oxy)piperidine-1-carboxylate

4-Chloro-7-methoxyquinazolin-6-ol (1.0 eq) obtained in step B1, *tert*-butyl *4-*hydroxypiperidine-1-carboxylate (1.4 eq), and triphenylphosphine (1.3 eq) were dissolved in dichloromethane, and then di-*tert*-butyl azodicarboxylate (DTBAD; 1.5 eq) was slowly added thereto at 0 °C and stirred at room temperature for 2 hours. The reaction mixture was concentrated and purified using MPLC (dichloromethane:methanol) to obtain the desired compound as a white solid (yield: 52%, MS (ESI): m/z 394 [M+H]⁺).

### [Step B3] Preparation of 4-chloro-7-methoxy-6-(piperidin-4-yloxy)quinazoline

To *tert*-butyl 4-((4-chloro-7-methoxyquinazolin-6-yl)oxy)piperidine-1-carboxylate (1.0 eq) obtained in step B2, 4.0 M HCl (20.0 eq) dissolved in 1,4-dioxane was added, followed by stirring at room temperature for 4 hours. Ether was added to the reaction mixture to form a solid, and then the resulting solid was filtered to obtain the desired compound as a pale yellow solid (yield: 100%, MS (ESI): m/z 294 [M+H]⁺).

### [Step B4] Preparation of 1-(4-((4-chloro-7-methoxyquinazolin-6-yl)oxy)piperidin-1-yl)prop-2-en-1-one

After 4-chloro-7-methoxy-6-(piperidin-4-yloxy)quinazoline (1.0 eq) obtained in step B3 was dissolved in dichloromethane (0.2 M), triethylamine (3.5 eq) and acryloyl chloride (1.05 eq) were added thereto at 0 ° C and stirred for 1 hour. Organic materials were extracted by adding an aqueous NaHCO₃ solution and dichloromethane to the reaction mixture, and remaining water in collected organic layer was removed using MgSO₄, the collected organic layer was concentrated. The concentrated reaction mixture was purified using MPLC to obtain the desired compound as a white solid (yield: 100%, MS (ESI): m/z 348 [M+H]⁺).

### [Step B5] Preparation of 1-(4-((4-((4-([1,2,4]triazolo[1,5-a]pyridin-7-yloxy)-5-chloro-2-methoxyphenyl)amino)-7-methoxyquinazolin-6-yl)oxy)piperidin-1-yl)prop-2-en-1-one

1-(4-((4-Chloro-7-methoxyquinazolin-6-yl)oxy)piperidin-1-yl)prop-2-en-1-one (1.0 eq) obtained in step B4 and 4-([1,2,4]triazolo[1,5-*a*]pyridin-7-yloxy)-5-chloro-2-methoxyaniline (1.0 eq) obtained in step A2 of Reaction Scheme 2 were dissolved in *sec*-BuOH (0.1 M), and then 4.0 M HCl (0.3 eq) dissolved in 1,4-dioxane was added thereto, and stirred at 110 °C for 1 hour. The reaction mixture was concentrated and then purified using prep-HPLC to obtain the desired compound as a brown oil (yield: 10%, MS (ESI): m/z 603 [M+H]⁺).

### Preparation of Compounds of Examples 6 to 14

The compounds of Examples 6 to 14 according to the present invention were prepared in a similar manner to Preparation Example 2.

The chemical structural formulas, compound names, ¹H NMR, MS, yields, HPLC data, and purities of the compounds of Examples 5 to 14 are summarized in Table 2 below.

**[Table 2]**

| Ex. | Structure | Compound name | ¹H NMR, MS | Yield (%) | HPLC (min), Purity (%) |
|---|---|---|---|---|---|
| 5 | 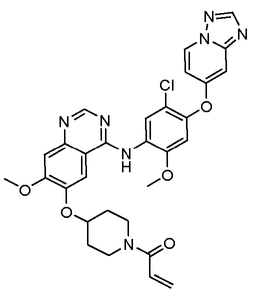 | 1-(4-((4-((4-([1,2,4]triazolo[1, 5-*a*]pyridin-7-yloxy)-5-chloro-2-methoxyphenyl)a mino)-7-methoxyquinazoli n-6-yl)oxy)piperidin-1-yl)prop-2-en-1-one | ¹H NMR (400 MHz, TFA salt, Methanol-d₄) δ 8.81 (d, J = 7.1 Hz, 1H), 8.67 (s, 1H), 8.34 (s, 1H), 8.03 (s, 1H), 7.83 (s, 1H), 7.27 (d, J = 4.9 Hz, 2H), 7.14 (dd, J = 7.5, 2.6 Hz, 1H), 6.89 (s, 1H), 6.83 (dd, J = 16.8, 10.6 Hz, 1H), 6.23 (dd, J = 16.8, 2.0 Hz, 1H), 5.78 (dd, J = 10.7, 1.9 Hz, 1H), 4.10 (s, 3H), 3.99-3.91 (m, 2H), 3.87 (s, 3H), 3.74-3.64 (m, 2H), 3.36-3.32 (m, 1H), 2.18-2.08 (m, 2H), 1.97-1.87 (m, 2H); MS (ESI): m/z 602 [M+H]⁺ | 10 | 8.89, 95 |
| 6 | 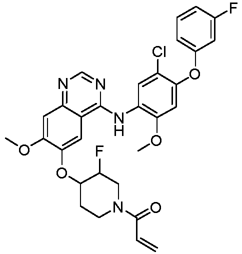 | 1-(4-((4-((5-chloro-4-(3-fluorophenoxy)-2-methoxyphenyl)a mino)-7-methoxyquinazoli n-6-yl)oxy)-3-fluoropiperidin-1-yl)prop-2-en-1-one | ¹H NMR (400 MHz, HCl salt, DMSO-d₆) δ 11.06 (brs, 1H), 8.79 (s, 1H), 8.38 (s, 1H), 7.75 (s, 1H), 7.48-7.43 (m, 1H), 7.33 (s, 1H), 7.16 (s, 1H), 7.02-6.98 (m, 1H), 6.90-6.78 (m, 3H), 6.17-6.12 (m, 1H), 5.75-5.71 (m, 1H), 4.96-4.74 (m, 2H), 4.09-4.02 (m, 4H), 3.83-3.77 (m, 4H), 3.68 (brs, 2H), 2.20-2.12 (m, 1H), 1.77-1.72 (m, 1H); MS (ESI): m/z 597 [M]⁺ | 58 | 11.29, 98 |
| 7 | 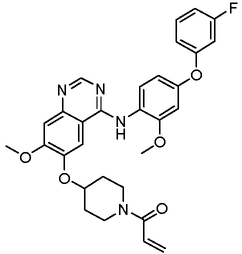 | 1-(4-((4-((4-(3-fluorophenoxy)-2-methoxyphenyl)a mino)-7-methoxyquinazoli n-6-yl)oxy)piperidin-1-yl)prop-2-en-1-one | ¹H NMR (400 MHz, HCl salt, Methanol-d₆) δ 8.55 (s, 1H), 8.01 (s, 1H), 7.48-7.46 (d, J = 8.58 Hz, 1H), 7.43-7.37 (m, 1H), 7.23 (s, 1H), 6.92-6.88 (m, 3H), 6.86-6.79 (m, 2H), 6.71-6.68 (dd, J = 8.56 Hz, 1H), 6.25-6.20 (dd, J = 16.80 Hz, 1H), 5.78-5.75 (dd, J = 10.65 Hz, 1H), 4.86-4.80 (m, 1H) 4.08 (s, 3H), 3.98-3.91 (m, 2H), 3.81 (s, 3H), 3.71-3.63 (m, 2H), 2.16-2.08 (m, 2H), 1.97-1.85 (m, 2H); MS (ESI): m/z 545 [M+H]⁺ | 55 | 10.61, 99 |
| 8 | 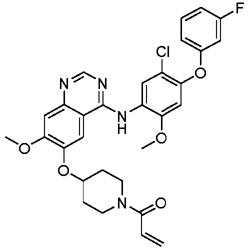 | 1-(4-((4-((5-chloro-4-(3-fluorophenoxy)-2-methoxyphenyl)a mino)-7-methoxyquinazoli n-6-yl)oxy)piperidin-1-yl)prop-2-en-1-one | ¹H NMR (400 MHz, HCl salt, Methanol-d₆) δ 8.64 (s, 1H), 8.04 (s, 1H), 7.72 (s, 1H), 7.41-7.35 (m, 1H), 7.26 (s, 1H), 7.00 (s, 1H), 6.90-6.86 (m, 1H), 6.83-6.79 (m, 2H), 6.75-6.71 (m, 1H), 6.25-6.20 (dd, J = 16.80 Hz, 1H), 5.79-5.76 (dd, J = 10.64 Hz, 1H), 4.95-4.90 (m, 1H), 4.09 (s, 3H), 3.98-3.89 (m, 2H), 3.80 (s, 3H), 3.72-3.64 (m, 2H), 2.16-2.09 (m, 2H), 1.98-1.86 (m, 2H); MS (ESI): m/z 579 [M+H]⁺ | 29 | 11.10, 99 |
| 9 | 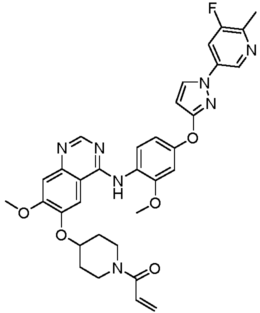 | 1-(4-((4-((4-((1-(5-fluoro-6-methylpyridin-3-yl)-1*H*-pyrazol-3-yl)oxy)-2-methoxyphenyl)a mino)-7-methoxyquinazoli n-6-yl)oxy)piperidin-1-yl)prop-2-en-1-one | ¹H NMR (400 MHz, HCl salt, Methanol-d₄) δ 8.75 (d, J = 2.0 Hz, 1H), 8.74 (s, 1H), 8.56 (s, 1H), 8.35 (d, J = 2.8 Hz, 1H), 8.01-7.96 (m, 2H), 7.51 (d, J = 8.4 Hz, 1H), 7.24 (s, 1H), 7.10 (d, J = 2.4 Hz, 1H), 6.94-6.91 (m, 1H), 6.67-6.22 (m, 2H), 5.80-5.77 (m, 1H), 4.10 (s, 3H), 3.99-3.91 (m, 3H), 3.70 (s, 3H), 3.69-3.62 (m, 2H), 2.55 (s, 3H), 2.21-2.12 (m, 2H), 1.99-1.87 (m, 2H); MS (ESI): m/z 626 [M+H]⁺ | 18 | 9.87, 100 |
| 10 | 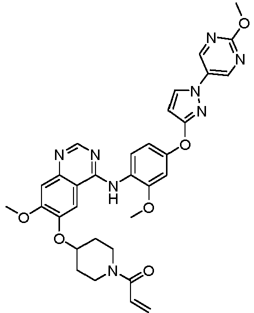 | 1-(4-((7-methoxy-4-((2-methoxy-4-((1-(2-methoxypyrimidi n-5-yl)-1*H-*pyrazol-3-yl)oxy)phenyl)am ino)quinazolin-6-yl)oxy)piperidin-1-yl)prop-2-en-1-one | ¹H NMR (400 MHz, HCl salt, Methanol-d₄) δ 8.96 (s, 2H), 8.55 (s, 1H), 8.26 (d, J = 2.8 Hz, 1H), 8.02 (s, 1H), 7.50 (d, J = 8.8 Hz, 1H), 7.24 (s, 1H), 7.08 (s, 1H), 6.93-6.80 (m, 2H), 6.27-6.22 (m, 2H), 5.80-5.77 (m, 1H), 4.09 (s, 3H), 4.08 (s, 3H), 3.97-3.91 (m, 3H), 3.86 (s, 3H), 3.69-2.97 (m, 2H), 2.12-2.11 (m, 2H), 2.00-1.85 (m, 2H) ; MS (ESI): m/z 625 [M+H]⁺ | 35 | 9.12, 100 |
| 11 | 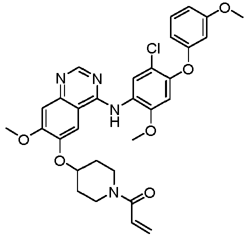 | 1-(4-((4-((5-chloro-2-methoxy-4-(3-methoxyphenoxy )phenyl)amino)-7-methoxyquinazoli n-6-yl)oxy)piperidin-1-yl)prop-2-en-1-one | ¹H NMR (400 MHz, HCl salt, Methanol-d₆) δ 8.62 (s, 1H), 8.01 (s, 1H), 7.69 (s, 1H), 7.28 (t, J = 8.19 Hz, 1H), 7.25 (s, 1H), 6.88 (s, 1H), 6.83 (dd, J = 16.8, 10.7 Hz 1H), 6.75-6.72 (m, 1H), 6.59-6.55 (m, 1H), 6.23 (dd, J = 16.8, 1.94 Hz 1H), 5.77 (dd, J = 10.7, 1.93 Hz 1H), 4.93-4.89 (m, 1H), 4.08 (s, 3H), 3.98-3.92 (m, 2H), 3.80 (s, 3H), 3.75 (s, 3H), 3.72-3.63 (m, 2H), 2.17-2.07 (m, 2H), 1.97-1.87 (m, 2H); MS (ESI): m/z 591 [M+H]⁺ | 92 | 11.45, 94 |
| 12 | 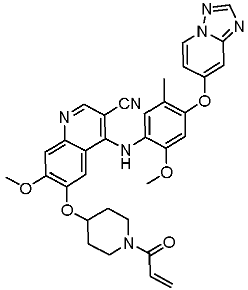 | 4-((4-([1,2,4]triazolo[1, 5-*a*]pyridin-7-yloxy)-2-methoxy-5-methylphenyl)am ino)-6-((1-acryloylpiperidin-4-yl)oxy)-7-methoxyquinolin e-3-carbonitrile | MS (ESI): m/z 606 [M+H]⁺ | | |
| 13 | 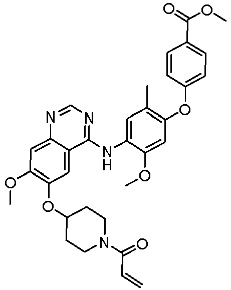 | methyl 4-(4-((6-((1-acryloylpiperidin-4-yl)oxy)-7-methoxyquinazoli n-4-yl)amino)-5-methoxy-2-methylphenoxy)b enzoate | ¹H NMR (400 MHz, FA salt, Methanol-d₄) δ 8.33 (s, 1H), 8.22 (s, 1H), 8.01 (d, J = 8.9 Hz, 2H), 7.85 (s, 1H), 7.49 (s, 1H), 7.21 (s, 1H), 7.04 (d, J = 8.9 Hz, 2H), 6.89-6.78 (m, 2H), 6.22 (dd, J = 16.8, 1.9 Hz, 1H), 5.76 (dd, J = 10.7, 1.9 Hz, 1H), 4.59 (s, 1H), 4.02 (s, 3H), 4.00-3.92 (m, 2H), 3.89 (s, 3H), 3.76 (s, 3H), 3.71-3.60 (m, 2H), 2.17-2.03 (m, 5H), 1.99-1.85 (m, 2H) ; MS (ESI) : m/z 599 [M+H]⁺ | | |
| 14 | 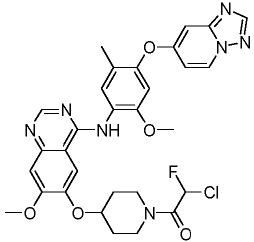 | 1-(4-((4-((4-([1,2,4]triazolo[1, 5-*a*]pyridin-7-yloxy)-2-methoxy-5-methylphenyl)am ino)-7-methoxyquinazoli n-6-yl)oxy)piperidin-1-yl)-2-chloro-2-fluoroethan-1-one | ¹H NMR (400 MHz, TFA salt, Methanol-d₄) δ 8.75 (d, J = 7.5 Hz, 1H), 8.33 (s, 1H), 8.29 (s, 1H), 7.84 (s, 1H), 7.59 (s, 1H), 7.22 (s, 1H), 7.14-6.92 (m, 4H), 4.58 (s, 1H), 4.02 (s, 3H), 3.96-3.81 (m, 2H), 3.80 (s, 3H), 3.77-3.65 (m, 1H), 3.64-3.56 (m, 1H), 2.19-2.08 (m, 5H), 2.06-1.93 (m, 2H); MS (ESI) : m/z 622 [M+H]⁺ | 31 | 9.29, 100 |

### Preparation Example 3: Preparation of 1-(4-((4-((4-([1,2,4]triazolo[1,5-a]pyridin-7-yloxy)-2-methoxy-5-methylphenyl)amino)-7-methoxyquinazolin-5-yl)oxy)-3,3-difluoropiperidin-1-yl)prop-2-en-1-one (Example 15)

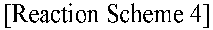

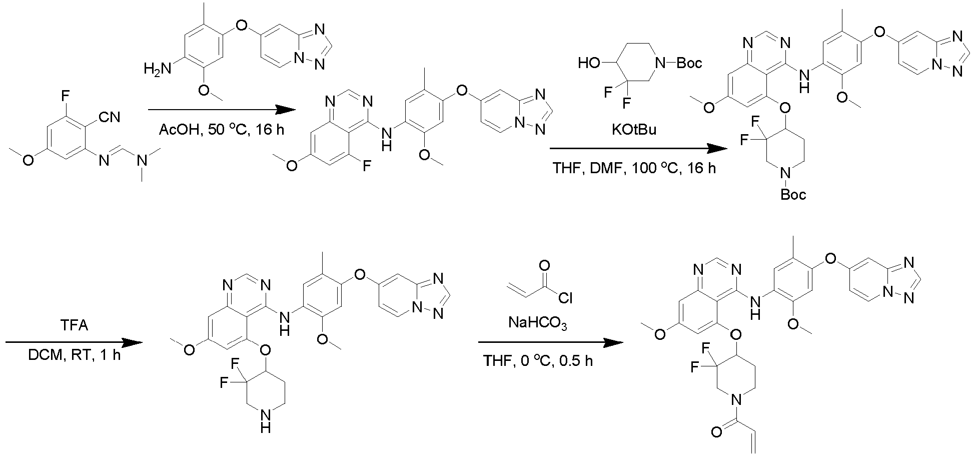

### [Step 1] Preparation of N-(4-([1,2,4]triazolo[1,5-a]pyridin-7-yloxy)-2-methoxy-5-methylphenyl)-5-fluoro-7-methoxyquinazolin-4-amine

(*E*)-*N*'-(2-Cyano-3-fluoro-5-methoxyphenyl)-*N*,*N*-dimethylformimidamide (1.0 eq) and 4-([1,2,4]triazolo[1,5-*a*]pyridin-7-yloxy)-2-methoxy-5-methylaniline (1.5 eq) obtained in step 2 of Preparation Example 1 were dissolved in acetic acid (52.0 eq), followed by stirring at 50 °C for 16 hours. After the reaction, organic materials were extracted with a saturated aqueous NaHCO₃ solution and ethyl acetate, the organic layer was washed with brine, and remaining water was removed with Na₂SO₄, followed by filtering and concentration. The reaction mixture was purified through MPLC (dichloromethane:methanol) to obtain the desired compound as a yellow solid (yield: 72%, MS (ESI): m/z 447 [M+H]⁺).

### [Step 2] Preparation of tert-butyl 4-((4-((4-([1,2,4]triazolo[1,5-a]pyridin-7-yloxy)-2-methoxy-5-methylphenyl)amino)-7-methoxyquinazolin-5-yl)oxy)-3,3-difluoropiperidine-1-carboxylate

*N*-(4-([1,2,4]Triazolo[1,5-*a*]pyridin-7-yloxy)-2-methoxy-5-methylphenyl)-5-fluoro-7-methoxyquinazolin-4-amine (1.0 eq) obtained in step 1, *tert-butyl* 3,3-difluoro-4-hydroxypiperidine-1-carboxylate (2.0 eq), and KOtBu (3.0 eq) were dissolved in THF:DMF=1:1 (0.02 M) and stirred at 100 °C for 16 hours in nitrogen. The reaction mixture was concentrated and purified through MPLC (dichloromethane:methanol) to obtain the desired compound as a yellow solid (yield: 51%, MS (ESI): m/z 664 [M+H]⁺).

### [Step 3] Preparation of N-(4-([1,2,4]triazolo[1,5-a]pyridin-7-yloxy)-2-methoxy-5-methylphenyl)-5-((3,3-difluoropiperidin-4-yl)oxy)-7-methoxyquinazolin-4-amine

*tert-Butyl* 4-((4-((4-([1,2,4]triazolo[1,5-*a*]pyridin-7-yloxy)-2-methoxy-5-methylphenyl)amino)-7-methoxyquinazolin-5-yl)oxy)-3,3-difluoropiperidine-1-carboxylate (1.0 eq) obtained in step 2 and TFA (10.0 eq) were dissolved in dichloromethane (0.1M) and stirred at room temperature for 1 hour. The reaction mixture was concentrated and purified through MPLC (dichloromethane:methanol) to obtain the desired compound as an orange solid (yield: 84%, MS (ESI): m/z 564 [M+H]⁺).

### [Step 4] Preparation of 1-(4-((4-((4-([1,2,4]triazolo[1,5-a]pyridin-7-yloxy)-2-methoxy-5-methylphenyl)amino)-7-methoxyquinazolin-5-yl)oxy)-3,3-difluoropiperidin-1-yl)prop-2-en-1-one

*N*-(4-([1,2,4]Triazolo[1,5-*a*]pyridin-7-yloxy)-2-methoxy-5-methylphenyl)-5-((3,3-difluoropiperidin-4-yl)oxy)-7-methoxyquinazolin-4-amine (1.0 eq) obtained in step 3 and a 1 M aqueous NaHCO₃ solution (5.0 eq) were dissolved in tetrahydrofuran (0.1 M), and then acryloyl chloride (1.0 eq) was added thereto at 0 °C. The mixture was stirred at room temperature for 30 minutes, and the reaction mixture was concentrated and purified through prep-HPLC to obtain the desired compound as a pale yellow solid (yield: 34%, MS (ESI): m/z 618 [M+H]⁺).

### Preparation of Compounds of Examples 16 to 17

The compounds of Examples 16 to 17 according to the present invention were prepared in a similar manner to Preparation Example 3.

The chemical structural formulas, compound names, ¹H NMR, MS, yields, HPLC data, and purities of Examples 15 to 17 are summarized in Table 3 below.

**[Table 3]**

| Ex. | Structure | Compound name | ¹H NMR, MS | Yield (%) | HPLC (min), Purity (%) |
|---|---|---|---|---|---|
| 15 | 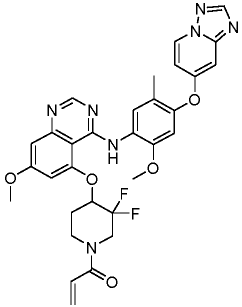 | 1-(4-((4-((4-([1,2,4]triazolo[1,5 -*a*]pyridin-7-yloxy)-2-methoxy-5-methylphenyl)amin o)-7-methoxyquinazolin -5-yl)oxy)-3,3-difluoropiperidin-1-yl)prop-2-en-1-one | ¹H NMR (400 MHz, TFA salt, DMSO-d₆) δ 10.49 (s, 1H), 8.94-8.89 (m, 2H), 8.39 (s, 1H), 8.30 (s, 1H), 7.29 (s, 1H), 7.06 (s, 1H), 7.04-7.02 (m, 1H), 6.94-6.88 (m, 3H), 6.20-6.15 (m, 1H), 5.77-5.69 (m, 2H), 4.78-4.48 (m, 3H), 3.97 (s, 3H), 3.76 (s, 3H), 3.45-3.43 (m, 2H), 2.14 (s, 3H), 1.97-1.93 (m, 1H); MS (ESI): m/z 618 [M+H]⁺ | 34 | 9.49, 99 |
| 16 | 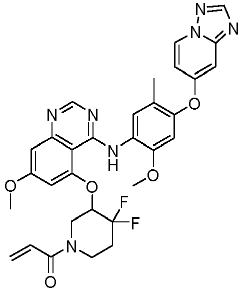 | 1-(3-((4-((4-([1,2,4]triazolo[1,5 -*a*]pyridin-7-yloxy)-2-methoxy-5-methylphenyl)amin o)-7-methoxyquinazolin -5-yl)oxy)-4,4-difluoropiperidin-1-yl)prop-2-en-1-one | ¹H NMR (400 MHz, TFA salt, DMSO-d₆) δ 8.87 (d, J = 7.6 Hz, 1H), 8.83 (s, 1H), 8.34 (s, 1H), 8.06 (s, 1H), 7.29 (d, J = 2 Hz, 1H), 7.04-7.02 (m, 2H), 6.93 (d, J = 2 Hz, 1H), 6.83 (d, J = 2 Hz, 1H), 6.72 (brs, 1H), 6.03 (d, J = 1 Hz, 1H), 5.65-5.52 (m, 2H), 4.25-4.18 (m, 1H), 3.95 (s, 3H), 3.91-3.64 (m, 7H), 2.29-2.25 (m, 2H), 2.13 (s, 3H); MS (ESI): m/z 618 [M+H]⁺ | 33 | 9.55, 99 |
| 17 | 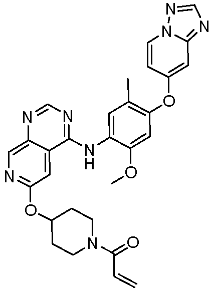 | 1-(4-((4-((4-([1,2,4]triazolo[1,5 -*a*]pyridin-7-yloxy)-2-methoxy-5-methylphenyl)amin o)pyrido[3,4-*d*]pyrimidin-6-yl)oxy)piperidin-1-yl)prop-2-en-1-one | MS (ESI): m/z 553 [M+H]⁺ | | |

### Preparation Example 4: Preparation of 1-(5-((4-((4-([1,2,4]triazolo[1,5-a]pyridin-7-yloxy)-2-methoxy-5-methylphenyl)amino)-7-methoxyquinazolin-6-yl)oxy)-2-azabicyclo[2.2.1]heptan-2-yl)prop-2-en-1-one (Example 18)

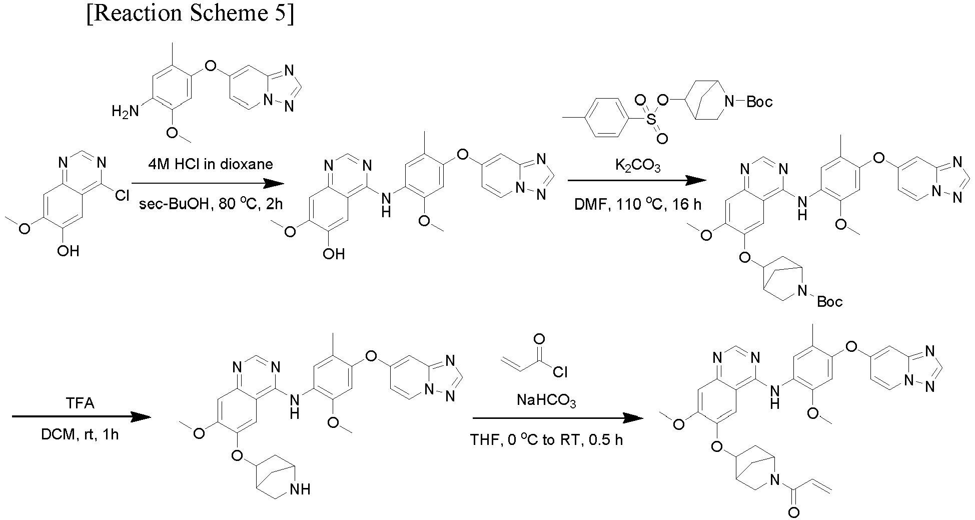

### [Step 1] Preparation of 4-((4-([1,2,4]triazolo[1,5-a]pyridin-7-yloxy)-2-methoxy-5-methylphenyl)amino)-7-methoxyquinazolin-6-ol

4-Chloro-7-methoxyquinazolin-6-ol (1.0 eq), 4-([1,2,4]triazolo[1,5-*a*]pyridin-7-yloxy)-2-methoxy-5-methylaniline (1.0 eq) obtained in step 2 of Preparation Example 1, and 4 M HCl (0.2 eq) dissolved in 1,4-dioxane were dissolved in *sec*-BuOH (0.1 M), followed by stirring at 80 °C for 2 hours. The reaction mixture was concentrated and purified through MPLC (dichloromethane:methanol) to obtain the desired compound as a white solid (yield: 75%, MS (ESI): m/z 445 [M+H]⁺).

### [Step 2] Preparation of tert-butyl 5-((4-((4-([1,2,4]triazolo[1,5-a]pyridin-7-yloxy)-2-methoxy-5-methylphenyl)amino)-7-methoxyquinazolin-6-yl)oxy)-2-azabicyclo[2.2.1]heptane-2-carboxylate

4-((4-([1,2,4]Triazolo[1,5-*a*]pyridin-7-yloxy)-2-methoxy-5-methylphenyl)amino)-7-methoxyquinazolin-6-ol (1.0 eq) obtained in step 1, *tert-butyl* 5-(tosyloxy)-2-azabicyclo[2.2.1]heptane-2-carboxylate (1.2 eq), and K₂CO₃ (2.0 eq) were dissolved in DMF (0.1 M) and stirred at 110 °C for 16 hours. After the reaction, organic materials were extracted with distilled water and ethyl acetate, and the organic layer was washed with brine, and then remaining water was removed with Na₂SO₄, followed by filtering and concentration. The reaction mixture was purified through MPLC (dichloromethane:methanol) to obtain the desired compound as a yellow solid (yield: 79%, MS (ESI): m/z 640 [M+H]⁺).

### [Step 3] Preparation of 6-((2-azabicyclo[2.2.1]heptan-5-yl)oxy)-N-(4-([1,2,4]triazolo[1,5-a]pyridin-7-yloxy)-2-methoxy-5-methylphenyl)-7-methoxyquinazolin-4-amine

*tert*-Butyl 5-((4-((4-([1,2,4]triazolo[1,5-*a*]pyridin-7-yloxy)-2-methoxy-5-methylphenyl)amino)-7-methoxyquinazolin-6-yl)oxy)-2-azabicyclo[2.2.1]heptane-2-carboxylate (1.0 eq) obtained in step 2 and TFA (20.0 eq) were dissolved in DCM (0.5M) and then stirred at room temperature for 1 hour. The reaction mixture was concentrated and then purified through MPLC (dichloromethane:methanol) to obtain the desired compound as a yellow solid (yield: 89%, MS (ESI): m/z 540 [M+H]⁺).

### [Step 4] Preparation of 1-(5-((4-((4-([1,2,4]triazolo[1,5-a]pyridin-7-yloxy)-2-methoxy-5-methylphenyl)amino)-7-methoxyquinazolin-6-yl)oxy)-2-azabicyclo[2.2.1]heptan-2-yl)prop-2-en-1-one

6-((2-Azabicyclo[2.2.1]heptan-5-yl)oxy)-*N*-(4-([1,2,4]triazolo[1,5-*a*]pyridin-7-yloxy)-2-methoxy-5-methylphenyl)-7-methoxyquinazolin-4-amine (1.0 eq) obtained in step 3 and a 1 M aqueous NaHCO₃ solution (5.0 eq) were dissolved in tetrahydrofuran (0.1 M), and then acryloyl chloride (1.0 eq) was added thereto at 0 °C. The mixture was stirred at room temperature for 30 minutes, and then the reaction mixture was concentrated and purified through prep-HPLC to obtain the desired compound as a white solid (yield: 22%, MS (ESI): m/z 594 [M+H]⁺).

### Preparation of Compounds of Examples 19 to 20

The compounds of Examples 19 to 20 according to the present invention were prepared in a similar manner to Preparation Example 4.

The chemical structural formulas, compound names, ¹H NMR, MS, yields, HPLC data, and purities of Examples 18 to 20 are summarized in Table 4 below.

**[Table 4]**

| Ex. | Structure | Compound name | ¹H NMR, MS | Yield (%) | HPLC (min), Purity (%) |
|---|---|---|---|---|---|
| 18 | 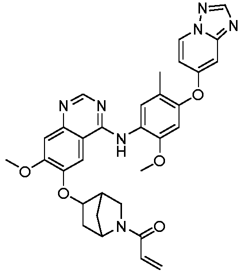 | 1-(5-((4-((4-([1,2,4]triazolo[ 1,5-*a*]pyridin-7-yloxy)-2-methoxy-5-methylphenyl)a mino)-7-methoxyquinaz olin-6-yl)oxy)-2-azabicyclo[2.2. 1]heptan-2-yl)prop-2-en-1-one | ¹H NMR (400 MHz, FA salt, Methanol-d₄) δ 8.79 (d, J = 7.6 Hz, 8.72 (d, J = 11.6 Hz, 1H), 8.39-8.32 (m, 4H), 7.13-7.09 (m, 3H), 7.04 (d, J = 2 Hz, 1H), 6.98 (s, 1H), 6.96 (s, 1H), 6.87-6.81 (m, 4H), 6.67-6.60 (m, 1H), 6.27-6.20 (m, 1H), 6.07-6.03 (m, 1H), 5.90-5.86 (m, 1H), 5.90-5.86 (m, 1H), 5.67-5.64 (m, 1H), 5.55-5.46 (m, 1H), 5.43-5.40 (m, 1H), 4.66 (d, J = 13.2 Hz, 2H), 4.05 (s, 3H), 4.04 (s, 3H), 3.90 (s, 3H), 3.89 (s, 3H), 3.65-3.62 (m, 2H), 3.52-3.48 (m, 1H), 3.35-3.33 (m, 2H), 2.65-2.50 (m, 2H), 2.20 (s, 3H), 2.19 (s, 3H), 2.12-2.08 (m, 1H), 2.03-1.94 (m, 6H); MS (ESI): m/z 594 [M+H]⁺ | 22 | 9.06, 97 |
| 19 | 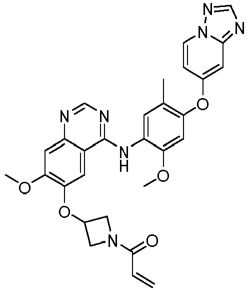 | 1-(3-((4-((4-([1,2,4]triazolo[ 1,5-*a*]pyridin-7-yloxy)-2-methoxy-5-methylphenyl)a mino)-7-methoxyquinaz olin-6-yl)oxy)azetidin-1-yl)prop-2-en-1-one | ¹H NMR (400 MHz, FA salt, Methanol-d₄) δ 8.75 (d, J = 7.6 Hz, 1H), 8.37 (s, 1H), 8.29 (s, 1H), 7.61 (s, 1H), 7.49 (s, 1H), 7.24 (s, 1H), 7.12-7.10 (m, 1H), 6.96 (s, 1H), 6.95 (d, J = 2.4 Hz, 1H), 6.44-6.37 (m, 1H), 6.31-6.26 (m, 1H), 5.80-5.76 (m, 1H), 5.29-5.24 (m, 1H), 4.83 (s, 1H), 4.64-4.59 (m, 1H), 4.46-4.43 (m, 1H), 4.18-4.15 (m, 1H), 4.04 (s, 3H), 3.81 (s, 3H), 2.17 (s, 3H); MS (ESI): m/z 554 [M+H]⁺ | 28 | 8.31, 99 |
| 20 | 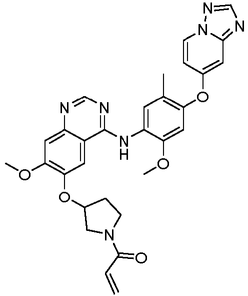 | 1-(3-((4-((4-([1,2,4]triazolo[ 1,5-*a*]pyridin-7-yloxy)-2-methoxy-5-methylphenyl)a mino)-7-methoxyquinaz olin-6-yl)oxy)pyrrolidi n-1-yl)prop-2-en-1-one | ¹H NMR (400 MHz, TFA salt, Methanol-d₆) δ 8.83 (d, J = 7.52 Hz, 1H), 8.64 (d, J = 2.36 Hz, 1H), 8.44 (d, J = 2.02 Hz, 1H), 8,01 (d, J = 7.28 Hz, 1H), 7.53 (s, 1H), 7.26 (d, J = 2.08 Hz, 1H), 7.18 (dd, J = 7.54, 2.08 Hz 1H), 7.05 (s, 1H), 6.91 (s, 1H), 6.72-6.65 (m, 1H), 6.32 (dd, J = 16.8, 1.84 Hz 1H), 5.48 (dd, J = 10.7, 1.87 Hz 1H), 5.32 (d, J = 23.2 Hz 1H), 4.08 (s, 3H), 4.03-3.93 (m, 2H), 3.89-3.80 (m, 5H), 2.50-2.29 (m, 2H), 2.19 (s, 3H); MS (ESI): m/z 568 [M+H]⁺ | 22 | 8.44, 97 |

### Preparation Example 5: Preparation of 1-(4-((4-([1,2,4]triazolo[1,5-a]pyridin-7-yloxy)-2-methoxy-5-methylphenyl)amino)-7,8-dihydro-6H-[1,4]oxazino[3,2-g]quinazolin-6-yl)prop-2-en-1-one (Example 21)

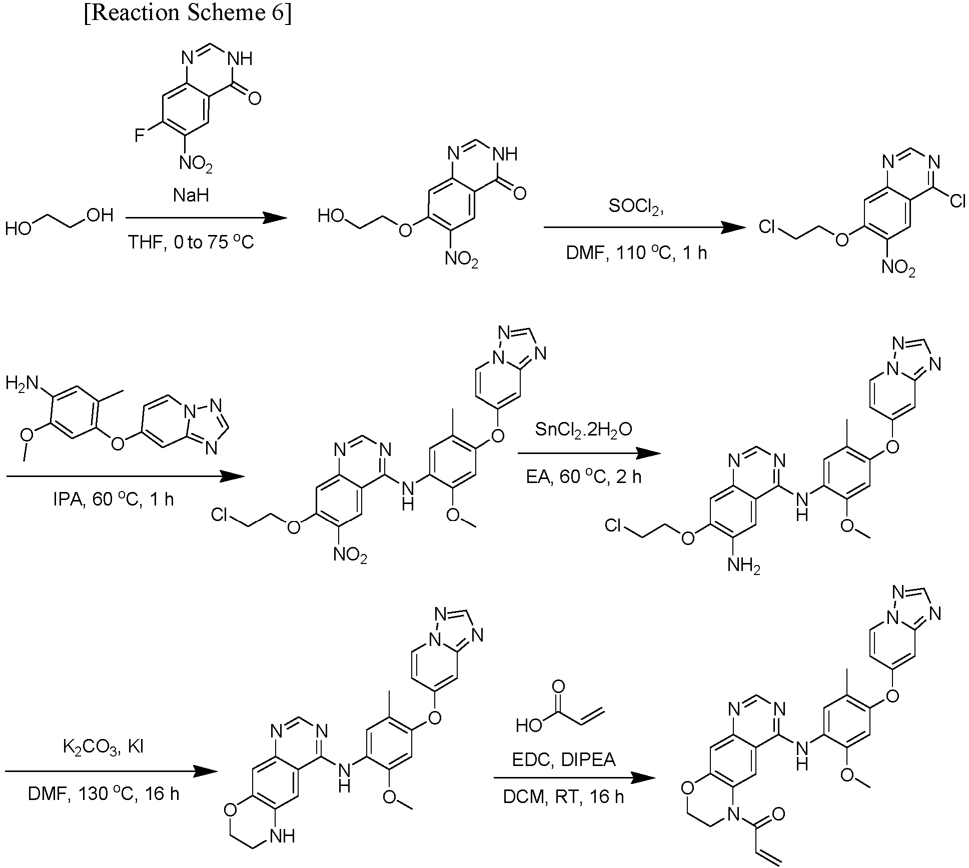

### [Step 1] Preparation of 7-(2-hydroxyethoxy)-6-nitroquinazolin-4(3H)-one

Ethylene glycol (1.0 eq) was dissolved in anhydrous tetrahydrofuran (1.5 M), NaH (0.7 eq) was slowly added to the solution at 0 °C and after the addition was completed, the temperature was raised to room temperature, followed by stirring for 1 hour. Then, 7-fluoro-6-nitroquinazolin-4(3H)-one (0.3 eq) was added to the reaction mixture and stirred at 75 °C for 16 hours. After acetic acid was slowly added to the reaction mixture at 0 °C to adjust the pH to 7, organic materials were extracted with water and chloroform:isopropanol=4:1 (v/v), and then after remaining water in collected organic layer was removed using Na₂SO₄, the collected organic layer was filtered and concentrated under reduced pressure. The concentrated mixture was purified using MPLC (dichloromethane:methanol) to obtain the desired compound as a pale yellow solid (yield: 12%, MS (ESI): m/z 252 [M+H]⁺).

### [Step 2] Preparation of 4-chloro-7-(2-chloroethoxy)-6-nitroquinazoline

7-(2-Hydroxyethoxy)-6-nitroquinazolin-4(3H)-one (1.0 eq) obtained in step 1 and SOCl₂ (0.1 M) were added thereto, DMF (0.1 eq) was added thereto, and then the mixture was stirred at 110 °C for 1 hour. The reaction mixture was concentrated under reduced pressure to obtain the desired compound as a white solid, which was used in the following reaction without further purification (yield: 100%, MS (ESI): m/z 287 [M+H]⁺).

### [Step 3] Preparation of N-(4-([1,2,4]triazolo[1,5-a]pyridin-7-yloxy)-2-methoxy-5-methylphenyl)-7-(2-chloroethoxy)-6-nitroquinazolin-4-amine

4-Chloro-7-(2-chloroethoxy)-6-nitroquinazoline (1.0 eq) obtained in step 2 was dissolved in isopropanol (0.1 M), and then 4-([1,2,4]triazolo[1,5-*a*]pyridin-7-yloxy)-2-methoxy-5-methylaniline (1.1 eq) prepared in step 2 of Preparation Example 1 was added thereto, followed by stirring at 60 °C for 1 hour. The reaction mixture was cooled and filtered to obtain a solid, and the solid was washed with isopropanol. The obtained solid was dried under vacuum to obtain the desired compound as a yellow solid (yield: 65%, MS (ESI): m/z 522 [M+H]⁺).

### [Step 4] Preparation of N⁴-(4-([1,2,4]triazolo[1,5-a]pyridin-7-yloxy)-2-methoxy-5-methylphenyl)-7-(2-chloroethoxy)quinazoline-4,6-diamine

*N*-(4-([1,2,4]Triazolo[1,5-*a*]pyridin-7-yloxy)-2-methoxy-5-methylphenyl)-7-(2-chloroethoxy)-6-nitroquinazolin-4-amine (1.0 eq) obtained in step 3 was dissolved in ethyl acetate (0.05 M), and then SnCl_{2.}2H₂O (5.0 eq) was added thereto, followed by stirring at 60 °C for 2 hours. The reaction mixture was cooled to 0 °C, and then ammonia water was slowly added thereto. Organic materials were extracted by adding a saturated aqueous NaHCO₃ solution and ethyl acetate to the reaction mixture, and then after remaining water in collected organic layer was removed using Na₂SO₄, the collected organic layer was filtered and concentrated under reduced pressure. The reaction mixture was purified through MPLC (dichloromethane:methanol) to obtain the desired compound as a white solid (yield: 83%, MS (ESI): m/z 492 [M+H]⁺).

### [Step 5] Preparation of N-(4-([1,2,4]triazolo[1,5-a]pyridin-7-yloxy)-2-methoxy-5-methylphenyl)-7,8-dihydro-6H-[1,4]oxazino[3 2-g]quinazolin-4-amine

*N*⁴-(4-([1,2,4]Triazolo[1,5-*a*]pyridin-7-yloxy)-2-methoxy-5-methylphenyl)-7-(2-chloroethoxy)quinazoline-4,6-diamine (1.0 eq) obtained in step 4 was dissolved in DMF (0.1 M), and then K₂CO₃ (1.02 eq) and KI (1.0 eq) were added thereto, and the mixture was stirred at 130 °C for 16 hours. To the reaction mixture, organic materials were extracted by adding ethyl acetate and brine, and then after remaining water in collected organic layer was removed using Na₂SO₄, the collected organic layer was filtered and concentrated under reduced pressure. The concentrated reaction mixture was purified using MPLC (dichloromethane:methanol) to obtain the desired compound as a yellow solid (yield: 60%, MS (ESI): m/z 456 [M+H]⁺).

### [Step 6] Preparation of 1-(4-((4-([1,2,4]triazolo[1,5-a]pyridin-7-yloxy)-2-methoxy-5-methylphenyl)amino)-7,8-dihydro-6H-[1,4]oxazino[3,2-gjquinazolin-6-yl)prop-2-en-1-one

*N*-(4-([1,2,4]Triazolo[1,5-*a*]pyridin-7-yloxy)-2-methoxy-5-methylphenyl)-7,8-dihydro-6H-[1,4]oxazino[3,2-g]quinazolin-4-amine (1.0 eq) obtained in step 5 was dissolved in dichloromethane (0.05 M), and then acrylic acid (3.5 eq), 1-ethyl-3-(3-dimethylaminopropyl)carbodiimide (EDC, 3.5 eq), and *N,N*-diisopropylethylamine (DIPEA, 2.5 eq) were added thereto, followed by stirring at room temperature for 16 hours. The reaction mixture was concentrated and then purified using MPLC (dichloromethane:methanol) to obtain the desired compound as a yellow solid (yield: 5%, MS (ESI): m/z 510 [M+H]⁺).

**[Table 5]**

| Ex. | Structure | Compound name | ¹H NMR, MS | Yield (%) | HPLC (min), Purity (%) |
|---|---|---|---|---|---|
| 21 | 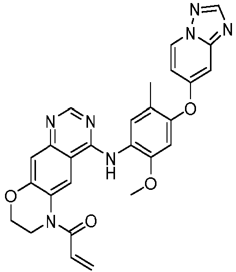 | 1-(4-((4-([1,2,4]triazolo[1,5 -*a*]pyridin-7-yloxy)-2-methoxy-5-methylphenyl)amin o)-7,8-dihydro-6*H-*[1,4]oxazino[3,2-g]quinazolin-6-yl)prop-2-en-1-one | ¹H NMR (400 MHz, TFA salt, MeOD-d₄) δ 8.76 (d, J = 7.6 Hz, 1H), 8.55 (s, 1H), 8.47 (s, 1H), 8.29 (s, 1H), 7.74 (s, 1H), 7.25 (s, 1H), 7.11-7.08 (m, 1H), 6.96-6.89 (m, 3H), 6.52-6.47 (m, 1H), 5.97-5.94 (m, 1H), 4.52 (t, J = 4.8 Hz, 2H), 4.18 (t, J = 4.8 Hz, 2H), 3.82 (s, 3H), 3.31 (s, 3H); MS (ESI): m/z 510 [M+H]⁺ | 5 | 8.52, 100 |

### Preparation Example 6: Preparation of N-(4-((4-([1,2,4]triazolo[1,5-a]pyridin-7-yloxy)-2-methoxy-5-methylphenyl)amino)-7-(4-morpholinopiperidin-1-yl)quinazolin-6-yl)acrylamide (Example 22)

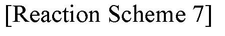

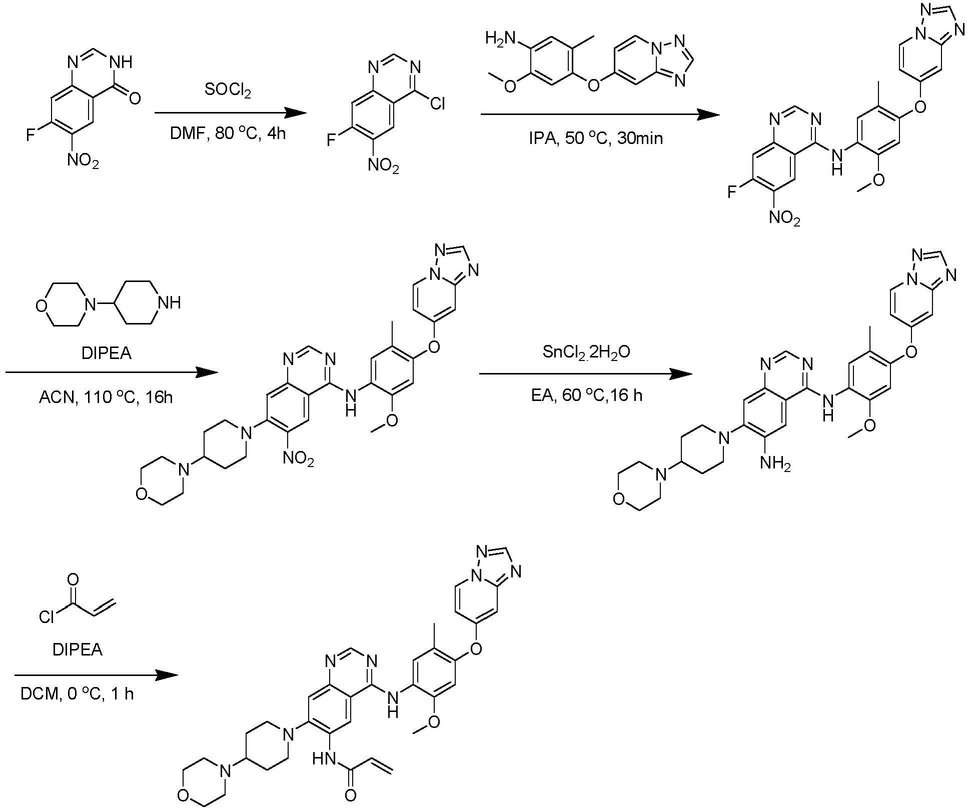

### [Step 1] Preparation of 4-chloro-7-fluoro-6-nitroquinazoline

7-Fluoro-6-nitroquinazolin-4(3*H*)-one (1.0 eq) and DMF (0.1 eq) were dissolved in SOCl₂ (20.0 eq), followed by stirring at 80 °C for 4 hours. The reaction mixture was concentrated and dissolved in dichloromethane and ether was added thereto. The resulting solid was filtered and then dried in vacuum to obtain a yellow solid. The obtained yellow solid was used in the following reaction without further purification (yield: 36%, MS (ESI): m/z 228 [M+H]⁺).

### [Step 2] Preparation of N-(4-([1,2,4]triazolo[1,5-a]pyridin-7-yloxy)-2-methoxy-5-methylphenyl)-7-fluoro-6-nitroquinazolin-4-amine

4-Chloro-7-fluoro-6-nitroquinazoline (1.0 eq) obtained in step 1 and 4-([1,2,4]triazolo[1,5-*a*]pyridin-7-yloxy)-2-methoxy-5-methylaniline (1.0 eq) prepared in step 2 of Preparation Example 1 were dissolved in isopropanol (0.2 M), and then stirred at 50 °C for 30 minutes. The reaction mixture was filtered to obtain a solid, and the obtained solid was washed with ether to obtain the desired compound as an ivory solid (yield: 72%, MS (ESI): m/z 462 [M+H]⁺).

### [Step 3] Preparation of N-(4-([1,2,4]triazolo[1,5-a]pyridin-7-yloxy)-2-methoxy-5-methylphenyl)-7-(4-morpholinopiperidin-1-yl)-6-nitroquinazolin-4-amine

*N*-(4-([1,2,4]Triazolo[1,5-*a*]pyridin-7-yloxy)-2-methoxy-5-methylphenyl)-7-fluoro-6-nitroquinazolin-4-amine (1.0 eq) obtained in step 2, 4-(piperidin-4-yl)morpholine (2.2 eq), and DIPEA (4.0 eq) were dissolved in 1,4-dioxane (0.3 M) and then stirred at 110 °C for 16 hours. The reaction mixture was concentrated and then purified through MPLC to obtain the desired compound as a yellow solid (yield: 79%, MS (ESI): m/z 612 [M+H]⁺).

### [Step 4] Preparation of N⁴-(4-([1,2,4]tiiazolo[1,5-a]pyridin-7-yloxy)-2-methoxy-5-methylphenyl)-7-(4-morpholinopiperidin-1-yl)quinazoline-4,6-diamine

*N*-(4-([1,2,4]Triazolo[1,5-*a*]pyridin-7-yloxy)-2-methoxy-5-methylphenyl)-7-(4-morpholinopiperidin-1-yl)-6-nitroquinazolin-4-amine (1.0 eq) obtained in step 3 and SnCl_{2.}2H₂O (5.0 eq) were dissolved in ethyl acetate (0.1 M), and the mixture was stirred at 60 °C for 16 hours. An aqueous ammonia solution was added to the reaction mixture, and then concentrated. The reaction mixture was purified using MPLC to obtain the desired compound as a pale yellow solid (yield: 33%, MS (ESI): m/z 582 [M+H]⁺).

### [Step 5] Preparation of N-(4-((4-([1,2,4]triazolo[1,5-a]pyridin-7-yloxy)-2-methoxy-5-methylphenyl)amino)-7-(4-morpholinopiperidin-1-yl)quinazolin-6-yl)acrylamide

*N*⁴-(4-([1,2,4]Triazolo[1,5-*a*]pyridin-7-yloxy)-2-methoxy-5-methylphenyl)-7-(4-morpholinopiperidin-1-yl)quinazoline-4,6-diamine (1.0 eq) obtained in step 4 was dissolved in dichloromethane (0.1 M), and then DIPEA (3.0 eq) was added thereto, followed by stirring at 0 °C. Then, acryloyl chloride (1.05 eq) was added thereto and stirred at 0 °C for 1 hour. The reaction mixture was concentrated and purified using prep-HPLC to obtain the desired compound as a yellow solid (yield: 3%, MS (ESI): m/z 636 [M+H]⁺).

### Preparation of Compounds of Examples 23 to 25

The compounds of Examples 23 to 25 according to the present invention were prepared in a similar manner to Preparation Example 6.

The chemical structural formulas, compound names, ¹H NMR, MS, yields, HPLC data, and purities of Examples 22 to 25 are summarized in Table 6 below.

**[Table 6]**

| Ex. | Structure | Compound name | ¹H NMR, MS | Yield (%) | HPLC (min), Purity (%) |
|---|---|---|---|---|---|
| 22 | 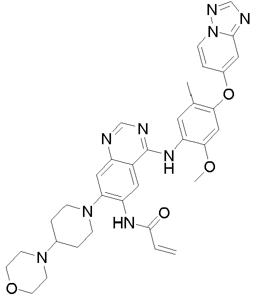 | N-(4-((4-([1,2,4]triazolo[ 1,5-*a*]pyridin-7-yloxy)-2-methoxy-5-methylphenyl)a mino)-7-(4-morpholinopipe ridin-1-yl)quinazolin-6-yl)acrylamide | ¹H NMR (400MHz, FA salt, Methanol-d₄) δ 8.77 - 8.72 (m, 1H), 8.41 (s, 1H), 8.28 (s, 1H), 7.88 (s, 1H), 7.41 (s, 1H), 7.08 (d, J = 5.9 Hz, 1H), 6.93 (s, 2H), 6.68 - 6.61 (m, 1H), 6.47 (d, J = 16.6 Hz, 1H), 5.88 (d, J = 10.2 Hz, 1H), 3.80 (m, 7H), 2.81 (t, J = 12.1 Hz, 2H), 2.68 (s, 4H), 2.43-2.38 (m, 1H), 2.16 - 2.09 (m, 5H), 1.80-1.77 (m, , 2H) ; MS (ESI): m/z 636 [M+H]⁺ | 3 | 7.5, 98 |
| 23 | 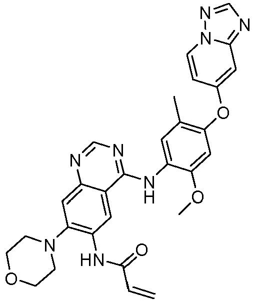 | N-(4-((4-([1,2,4]triazolo[ 1,5-*a*]pyridin-7-yloxy)-2-methoxy-5-methylphenyl)a mino)-7-morpholinoquin azolin-6-yl)acrylamide | ¹H NMR (400 MHz, TFA salt, Methanol-d₄) δ 8.80-8.77 (m, 2H), 8.35 (s, 1H), 7.42 (s, 1H), 7.32 (s, 1H), 7.19 (s, 1H), 7.10 (d, J = 6.4 Hz, 1H), 6.99 (s, 1H), 6.90 (s, 1H), 6.49-6.45 (m, 1H), 6.39-6.32 (m, 1H), 5.83-5.80 (m, 1H), 3.95 (t, J = 4.0 Hz, 4H), 3.76 (s, 3H), 3.19 (t, J = 4.4 Hz, 4H), 2.10 (s, 3H); MS (ESI): m/z 553 [M+H]⁺ | 1 | 10.16, 92 |
| 24 | 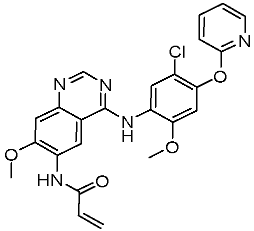 | *N-*(4-((5-chloro-2-methoxy-4-(pyridin-2-yloxy)phenyl)a mino)-7-methoxyquinaz olin-6-yl)acrylamide | ¹H NMR (400 MHz, HCl salt, Methanol-ds) δ 9.24 (s, 1H), 8.67 (s, 1H), 8.13-8.11 (dd, J = 5.02 Hz, 1H), 7.90-7.86 (m, 2H), 7.30 (s, 1H), 7.16-7.12 (m, 2H), 7.08-7.06 (d, J = 8.35 Hz, 1H), 6.74-6.67 (m, 1H), 6.52-6.48 (dd, J = 16.94 Hz, 1H), 5.90-5.87 (dd, J = 10.23 Hz, 1H), 4.17 (s, 3H), 3.87 (s, 3H); MS (ESI): m/z 478 [M+H]⁺ | 11 | 10.10, 98 |
| 25 | 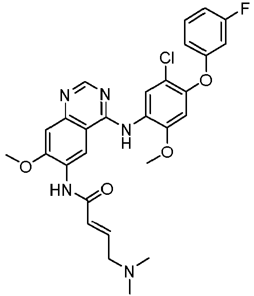 | (*E)-N*-(4-((5-chloro-4-(3-fluorophenoxy) -2-methoxyphenyl )amino)-7-methoxyquinaz olin-6-yl)-4-(dimethylamino )but-2-enamide | ¹H NMR (400 MHz, FA salt, Methanol-d₄) δ 9.04 (s, 1H), 8.49 (s, 1H), 8.24 (s, 1H), 7.38-7.32 (m, 1H), 7.27 (s, 1H), 7.02-6.94 (m, 2H), 6.85-6.71 (m, 4H), 4.11 (s, 3H), 3.88 (s, 3H), 3.77 (d, J = 6.8 Hz, 2H), 2.76 (s, 6H); MS (ESI): m/z 552 [M+H]⁺ | 64 | 9.13, 99 |

### Preparation Example 7: (E)-N-(4-((4-([1,2,4]triazolo[1,5-a]pyridin-7-yloxy)-2-methoxy-5-methylphenyl)amino)-5-chloroquinazolin-6-yl)-4-(dimethylamino)but-2-enamide (Example 26)

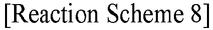

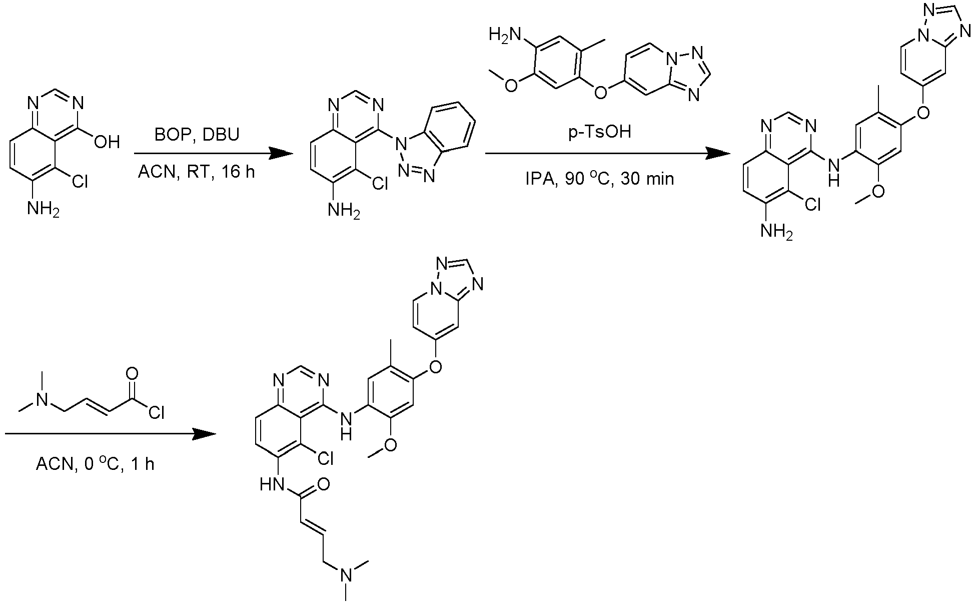

### [Step 1] Preparation of 4-(1H-benzo[d][1,2,3]triazol-1-yl)-5-chloroquinazolin-6-amine

6-Amino-5-chloroquinazolin-4-ol (1.0 eq) was dissolved in acetonitrile (0.1 M), and then benzotriazol-1yloxytris(dimethylamino)phosphonium hexafluorophosphate (BOP, 3.0 eq), 1,8-diazabicyclo[5,4,0]undec-7-ene (DBU, 4.0 eq) were added thereto, followed by stirring at room temperature for 16 hours. The reaction mixture was concentrated under reduced pressure to obtain the desired compound as a brow oil, and the obtained desired compound was used in the following reaction without further purification (yield: 15%, MS (ESI): m/z 297 [M+H]⁺).

### [Step 2] Preparation of N⁴-(4-([1,2,4]triazolo[1,5-a]pyridin-7-yloxy)-2-methoxy-5-methylphenyl)-5-chloroquinazoline-4,6-diamine

4-(1*H*-Benzo[*d*][1,2,3]triazol-1-yl)-5-chloroquinazolin-6-amine (1.0 eq) obtained in step 1 and 4-([1,2,4]triazolo[1,5-*a*]pyridin-7-yloxy)-2-methoxy-5-methylaniline (1.0 eq) obtained in step 2 of Preparation Example 1 were dissolved in isopropanol (0.1 M), refluxed at 90 °C for 10 minutes, and then p-TsOH (0.1 eq) was added to the reaction mixture and refluxed at 90 °C for 30 minutes. To the reaction mixture, dichloromethane (0.05 M), brine, and a saturated aqueous NaHCO₃ solution were added to extract organic materials, and then after remaining water was removed using Na₂SO₄, the collected organic layer was filtered and concentrated under reduced pressure. The concentrated mixture was purified using MPLC (dichloromethane:methanol), and then purified using prep-HPLC. The compound obtained through prep-HPLC was collected and concentrated, and brine was added thereto and stirred at room temperature for 1 hour. After the reaction, the resulting solid was filtered and then dried to obtain the desired compound as a yellow solid (yield: 15%, MS (ESI): m/z 448 [M+H]⁺).

### [Step 3] Preparation of (E)-N-(4-((4-([1,2,4]triazolo[1,5-a]pyridin-7-yloxy)-2-methoxy-5-methylphenyl)amino)-5-chloroquinazolin-6-yl)-4-(dimethylamino)but-2-enamide

*N*⁴-(4-([1,2,4]Triazolo[1,5-*a*]pyridin-7-yloxy)-2-methoxy-5-methylphenyl)-5-chloroquinazoline-4,6-diamine (1.0 eq) obtained in step 2 was dissolved in acetonitrile (0.1 M), then cooled to 0 °C, and (*E*)-4-(dimethylamino)but-2-enoyl chloride (1.0 eq) was added thereto, followed by stirring at 0 °C for 1 hour. To the reaction mixture, dichloromethane (0.05 M) and a saturated aqueous NaHCO₃ solution were added to extract organic materials, and then after remaining water in collected organic layer was removed using Na₂SO₄, the collected organic layer was filtered and concentrated under reduced pressure. The concentrated mixture was purified using prep-HPLC to obtain the desired compound as a pale yellow solid (yield: 26%, MS (ESI): m/z 559 [M+H]⁺).

**[Table 7]**

| Ex. | Structure | Compound name | ¹H NMR, MS | Yield (%) | HPLC (min), Purity (%) |
|---|---|---|---|---|---|
| 26 | 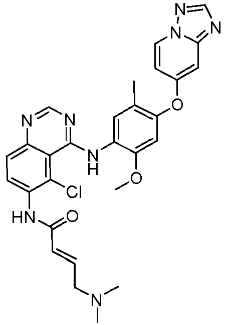 | (*E*)-*N*-(4-((4-([1,2,4]triazolo[1, 5-*a*]pyridin-7-yloxy)-2-methoxy-5-methylphenyl)am ino)-5-chloroquinazolin-6-yl)-4-(dimethylamino)b ut-2-enamide | ¹H NMR (400 MHz, freebase, Methanol-d₄) δ8.74-8.73 (m, 2H), 8.60 (s, 1H), 8.29 (s, 1H), 8.18 (d, J = 8.8 Hz, 1H), 7.78 (d, J = 8.4 Hz, 1H), 7.06-6.97 (m, 3H), 6.80 (s, 1H), 6.67 (d, J = 14.8 Hz, 1H), 3.92 (s, 3H), 3.77 (d, J = 5.2 Hz, 2H), 2.74-2.65 (m, 6H), 2.17 (s, 3H) ; MS (ESI): m/z 559 [M+H]⁺ | 17 | 7.74, 100 |

### Preparation Example 8: Preparation of 1-(4-(8-((4-([1,2,4]triazolo[1,5-a]pyridin-7-yloxy)-2-methoxy-5-methylphenyl)amino)pyrimido[5,4-d]pyrimidin-2-yl)piperazin-1-yl)prop-2-en-1-one (Example 27)

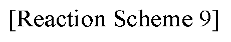

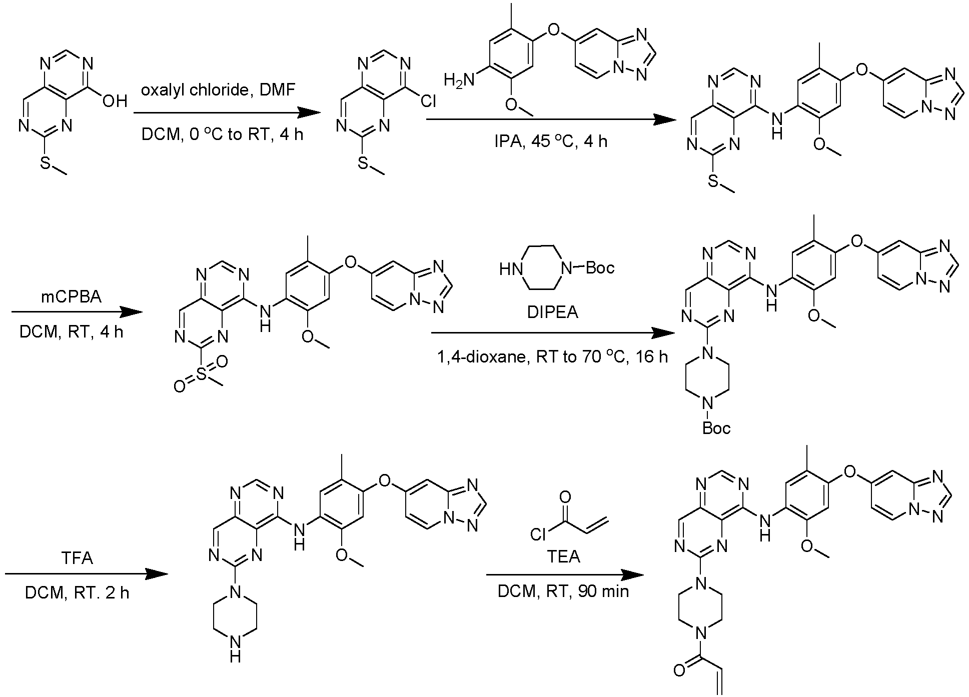

### [Step 1] Preparation of 8-chloro-2-(methylthio)pyrimido[5,4-d]pyrimidine

6-(Methylthio)pyrimido[5,4-*d*]pyrimidin-4-ol (1.0 eq) and DMF (1.6 eq) were dissolved in dichloromethane (0.5 M), and then oxalyl chloride (2.0 eq) was slowly added thereto at 0 °C. The reaction mixture was stirred at room temperature for 4 hours. The reaction mixture was concentrated to obtain the desired compound as a white solid, and then the obtained compound was used in the following reaction without further purification (yield: 100%, MS (ESI): m/z 213 [M+H]⁺).

### [Step 2] Preparation of N-(4-([1,2,4]triazolo[1,5-a]pyridin-7-yloxy)-2-methoxy-5-methylphenyl)-6-(methylthio)pyrimido[5,4-d]pyrimidin-4-amine

8-Chloro-2-(methylthio)pyrimido[5,4-d]pyrimidine (1.0 eq) obtained in step 1 and 4-([1,2,4]triazolo[1,5-*a*]pyridin-7-yloxy)-2-methoxy-5-methylaniline (1.0 eq) obtained in step 2 of Preparation Example 1 were dissolved in isopropyl alcohol (0.5 M), and then the reaction mixture was stirred at 45 °C for 4 hours. The reaction mixture was concentrated and purified using MPLC to obtain the desired compound as a yellow solid (yield: 16%, MS (ESI): m/z 447 [M+H]⁺).

### [Step 3] Preparation of N-(4-([1,2,4]triazolo[1,5-a]pyridin-7-yloxy)-2-methoxy-5-methylphenyl)-6-(methylsulfonyl)pyrimido[5,4-d]pyrimidin-4-amine

*N*-(4-([1,2,4]Triazolo[1,5-*a*]pyridin-7-yloxy)-2-methoxy-5-methylphenyl)-6-(methylthio)pyrimido[5,4-d]pyrimidin-4-amine (1.0 eq) obtained in step 2 was dissolved in dichloromethane (0.06 M), and then meta-chloroperoxybenzoic acid (m-CPBA, 2.3 eq) was slowly added thereto at 0 °C. The reaction mixture was stirred at room temperature for 2 hours. To the reaction mixture, was added a saturated aqueous NaHCO₃ solution, and organic materials were extracted with DCM, and then after remaining water in collected organic layer was removed using MgSO₄, the collected organic layer was filtered and concentrated under reduced pressure. The obtained desired compound as a white solid was used in the following reaction without further purification (yield: 83%, MS (ESI): m/z 479 [M+H]⁺).

### [Step 4] Preparation of tert-butyl 4-(8-((4-([1,2,4]triazolo[1,5-a]pyridin-7-yloxy)-2-methoxy-5-methylphenyl)amino)pyrimido[5,4-d]pyrimidin-2-yl)piperazine-1-carboxylate

*N*-(4-([1,2,4]Triazolo[1,5-*a*]pyridin-7-yloxy)-2-methoxy-5-methylphenyl)-6-(methylsulfonyl)pyrimido[5,4-d]pyrimidin-4-amine (1.0 eq) obtained in step 3 was dissolved in 1,4-dioxane (0.1 M), and then *tert*-butyl piperazine-1-carboxylate (1.7 eq) and DIPEA (1.3 eq) were added thereto. The reaction mixture was stirred at 70 °C for 2 hours. To the reaction mixture, was added a saturated aqueous NaHCO₃ solution, and organic materials were extracted with DCM, and then after remaining water in collected organic layer was removed using MgSO₄, the collected organic layer was filtered and concentrated under reduced pressure. The obtained desired compound as a brown liquid was used in the following reaction without further purification (yield: 100%, MS (ESI): m/z 585 [M+H]⁺).

### [Step 5] Preparation of N-(4-([1,2,4]triazolo[1,5-a]pyridin-7-yloxy)-2-methoxy-5-methylphenyl)-6-(piperazin-1-yl)pyrimido[5,4-d]pyrimidin-4-amine

*tert-*Butyl 4-(8-((4-([ 1,2,4]triazolo1,5-*a*]pyridin-7-yloxy)-2-methoxy-5-methylphenyl)amino)pyrimido[5,4-d]pyrimidin-2-yl)piperazine-1-carboxylate (1.0 eq) obtained in step 4 was dissolved in dichloromethane (0.06 M), trifluoroacetic acid (40.0 eq) was added thereto, and the mixture was stirred at room temperature for 2 hours. The reaction mixture was concentrated and then purified using MPLC to obtain the desired compound as an orange solid (yield: 82%, MS (ESI): m/z 485 [M+H]⁺).

### [Step 6] Preparation of 1-(4-(8-((4-([1,2,4]triazolo[1,5-a]pyridin-7-yloxy)-2-methoxy-5-methylphenyl)amino)pyrimido[5,4-d]pyrimidin-2-yl)piperazin-1-yl)prop-2-en-1-one

*N*-(4-([1,2,4]Triazolo[1,5-*a*]pyridin-7-yloxy)-2-methoxy-5-methylphenyl)-6-(piperazin-1-yl)pyrimido[5,4-*d*]pyrimidin-4-amine (1.0 eq) obtained in step 5 was dissolved in DCM (0.04 M), triethylamine (8.0 eq) was added thereto, and then acryloyl chloride (1.5 eq) was added thereto at 0 °C. The reaction mixture was stirred at room temperature for 1 hour. The reaction mixture was concentrated and then purified using prep-HPLC to obtain the desired compound as a yellow solid (yield: 29%, MS (ESI): m/z 539 [M+H]⁺).

**[Table 8]**

| Ex. | Structure | Compound name | ¹H NMR, MS | Yield (%) | HPLC (min), Purity (%) |
|---|---|---|---|---|---|
| 27 | 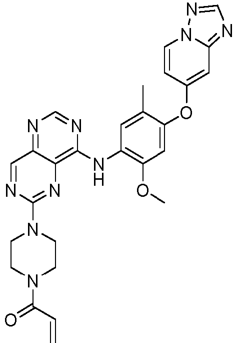 | 1-(4-(8-((4-([1,2,4]triazolo[1,5 -*a*]pyridin-7-yloxy)-2-methoxy-5-methylphenyl)amin o)pyrimido[5,4-*d*]pyrimidin-2-yl)piperazin-1-yl)prop-2-en-1-one | ¹H NMR (400 MHz, FA salt, DMSO-d₆) δ9.47 (s, 1H), 9.17 (s, 1H), 8.94 (d, J = 7.6 Hz, 1H), 8.52 (s, 2H), 8.37 (s, 1H), 7.05-7.02 (m, 2H), 6.93-6.86 (m, 1H), 6.81 (d, J = 2.4 Hz, 1H), 6.20-6.15 (m, 1H), 5.76-5.73 (m, 1H), 3.96-3.93 (m, 7H), 3.77-3.73 (m, 4H) ; MS (ESI): m/z 539 [M+H]⁺ | 29 | 11.19, 97 |

### Preparation Example 9: Preparation of N-(4-((4-([1,2,4]triazolo[1,5-a]pyridin-7-yloxy)-2-methoxy-5-methylphenyl)amino)-7-methoxyquinazolin-6-yl)but-2-ynamide

### (Example 28)

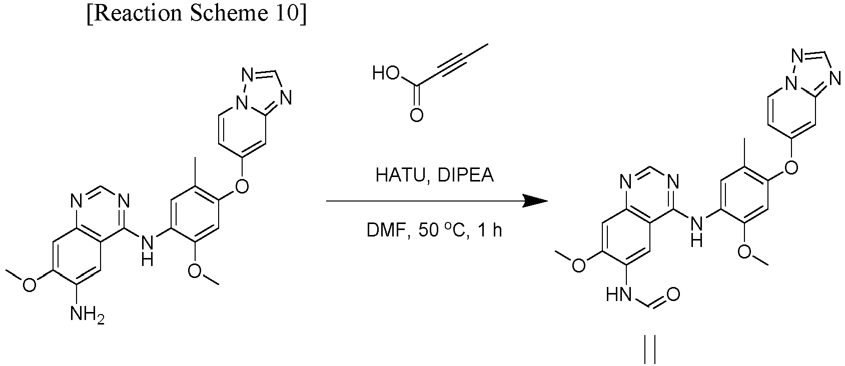

### [Step 1] Preparation of N-(4-((4-([1,2,4]triazolo[1,5-a]pyridin-7-yloxy)-2-methoxy-5-methylphenyl)amino)-7-methoxyquinazolin-6-yl)but-2-ynamide

Tetrolic acid (3.0 eq) and 1-[bis(dimethylamino)methylene]-1H-1,2,3-triazolo[4,5-b]pyridinium 3-oxide hexafluorophosphate (HATU, 3.0 eq) were dissolved in DMF (0.5 M) and stirred at 50 °C for 30 minutes. *N*⁴-(4-([1,2,4]triazolo[1,5-*a*]pyridin-7-yloxy)-2-methoxy-5-methylphenyl)-7-methoxyquinazoline-4,6-diamine (1.0 eq) obtained in step 4 of Preparation Example 1 and DIPEA (3.0 eq) were added to the reaction mixture and then stirred at 50 °C for 1 hour. The reaction mixture was purified using prep-HPLC to obtain the desired compound (yield: 7%, MS (ESI): m/z 510 [M+H]⁺).

**[Table 9]**

| Ex. | Structure | Compound name | ¹H NMR, MS | Yield (%) | HPLC (min), Purity (%) |
|---|---|---|---|---|---|
| 28 | 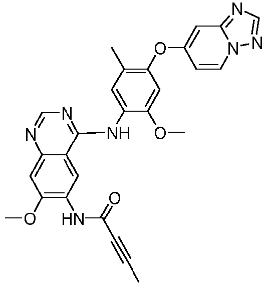 | N-(4-((4-([1,2,4]triazolo[1, 5-*a*]pyridin-7-yloxy)-2-methoxy-5-methylphenyl)am ino)-7-methoxyquinazoli n-6-yl)but-2-ynamide | ¹H NMR (400 MHz, FA salt, Methanol-d₄) δ8.86 (s, 1H), 8.77 (d, J = 7.6 Hz, 1H), 8.45 (s, 1H), 8.31 (s, 1H), 7.94 (s, 1H), 7.24 (s, 1H), 7.12-7.09 (m, 1H), 6.94 (s, 2H), 4.90 (s, 3H), 3.87 (s, 3H), 2.18 (s, 3H), 2.10 (s, 3H); MS (ESI): m/z 510 [M+H]⁺ | 7 | 9.07, 97 |

### Preparation Example 10: (R,E)-N-(4-((4-([1,2,4]triazolo[1,5-a]pyridin-7-yloxy)-2-methoxy-5-methylphenyl)amino)-7-methoxyquinazolin-6-yl)-3-(1-methylpyrrolidin-2-yl)acrylamide (Example 29)

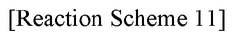

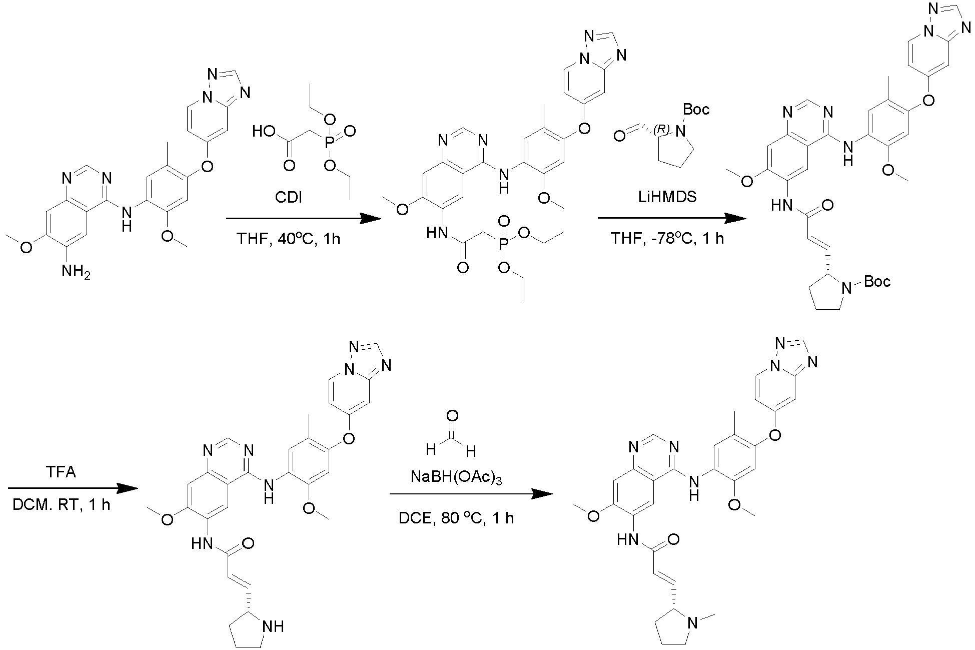

### [Step 1] Preparation of diethyl (2-((4-((4-([1,2,4]triazolo[1,5-a]pyridin-7-yloxy)-2-methoxy-5-methylphenyl)amino)-7-methoxyquinazolin-6-yl)amino)-2-oxoethyl)phosphonate

2-(Diethoxyphosphoryl)acetic acid (3.0 eq) and 1,1'-carbonyldiimidazole (CDI, 3.0 eq) were dissolved in tetrahydrofuran (0.5 M), followed by stirring at 40 °C for 30 minutes. *N*⁴-(4-([1,2,4]triazolo[1,5-*a*]pyridin-7-yloxy)-2-methoxy-5-methylphenyl)-7-methoxyquinazoline-4,6-diamine (1.0 eq) prepared in step 4 of Preparation Example 1 was added to the mixture and then stirred at 40 °C for 1 hour. Water was added and organic materials were extracted with dichloromethane, and then after remaining water in collected organic layer was removed using MgSO₄, the collected organic layer was concentrated. The obtained yellow desired compound was used in the following reaction without further purification (yield: 100%, MS (ESI): m/z 622 [M+H]⁺).

### [Step 2] Preparation of tert-butyl (R,E)-2-(3-((4-((4-([1,2,4]triazolo[1,5-a]pyridin-7-yloxy)-2-methoxy-5-methylphenyl)amino)-7-methoxyquinazolin-6-yl)amino)-3-oxoprop-1-en-1-yl)pyrrolidine-1-carboxylate

Diethyl (2-((4-((4-([1,2,4]triazolo[1,5-*a*]pyridin-7-yloxy)-2-methoxy-5-methylphenyl)amino)-7-methoxyquinazolin-6-yl)amino)-2-oxoethyl)phosphonate (1.0 eq) obtained in step 1 was dissolved in tetrahydrofuran (0.05 M), and then lithium bis(trimethylsilyl)amide (LiHMDS, 1.0 eq) was added thereto at -78 °C. The mixture was stirred at -78 °C for 1 hour, then tert-butyl (*R*)-2-formylpyrrolidine-1-carboxylate (3.3 eq) was added thereto and stirred at room temperature for 1 hour. Water was added and organic materials were extracted with dichloromethane, and then after remaining water in collected organic layer was removed using MgSO₄, the collected organic layer was concentrated. The obtained desired compound as a yellow solid was used in the following reaction without further purification (yield: 100%, MS (ESI): m/z 667 [M+H]⁺).

### [Step 3] Preparation of (R,E)-N-(4-((4-([1,2,4]triazolo[1,5-a]pyridin-7-yloxy)-2-methoxy-5-methylphenyl)amino)-7-methoxyquinazolin-6-yl)-3-(pyrrolidin-2-yl)acrylamide

The compound obtained in step 2, *tert-*butyl (*R,E*)-2-(3-((4-((4-([1,2,4]triazolo[1,5-*a*]pyridin-7-yloxy)-2-methoxy-5-methylphenyl)amino)-7-methoxyquinazolin-6-yl)amino)-3-oxoprop-1-en-1-yl)pyrrolidine-1-carboxylate (1.0 eq) was dissolved in dichloromethane (0.1 M), and then TFA (60.0 eq) was added thereto and stirred at room temperature for 1 hour. The reaction mixture was concentrated and purified through MPLC (dichloromethane:methanol) to obtain the desired compound as a yellow solid (yield: 59%, MS (ESI): m/z 567 [M+H]⁺).

### [Step 4] Preparation of (R,E)-N-(4-((4-([1,2,4]triazolo[1,5-a]pyridin-7-yloxy)-2-methoxy-5-methylphenyl)amino)-7-methoxyquinazolin-6-yl)-3-(1-methylpyrrolidin-2-yl)acrylamide

(*R*,*E*)*-N-*(4-((4-([1,2,4]triazolo[1,5-*a*]pyridin-7-yloxy)-2-methoxy-5-methylphenyl)amino)-7-methoxyquinazolin-6-yl)-3-(pyrrolidin-2-yl)acrylamide (1.0 eq) obtained in step 3 and 37% formaldehyde (5.0 eq) were dissolved in 1,2-dichloroethane (DCE, 0.1 M), and then stirred at room temperature for 1 hour. Sodium triacetoxyborohydride (4.0 eq) was added to the mixture and then stirred at 80 °C for 1 hour. A saturated aqueous NaHCO₃ solution and dichloromethane were added to extract organic materials, and then remaining water in collected organic layer was removed using MgSO₄ and the collected organic layer was concentrated. The reaction mixture was purified through MPLC (dichloromethane:methanol) to obtain the desired compound as a white solid (yield: 33%, MS (ESI): m/z 581 [M+H]⁺).

### Preparation of Compounds of Examples 30 to 37

The compounds of Examples 30 to 37 according to the present invention were prepared in a similar manner to Preparation Example 10.

The chemical structural formulas, compound names, ¹H NMR, MS, yields, HPLC data, and purities of the compounds of Examples 29 to 37 are summarized in Table 10 below.

**[Table 10]**

| Ex. | Structure | Compound name | ¹H NMR, MS | Yield (%) | HPLC (min), Purity (%) |
|---|---|---|---|---|---|
| 29 | 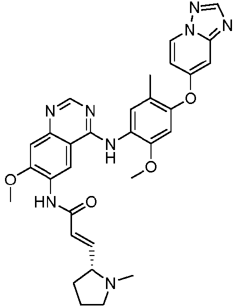 | (*R*,*E*)-*N-*(4-((4-([1,2,4]triazolo[1, 5-*a*]pyridin-7-yloxy)-2-methoxy-5-methylphenyl)am ino)-7-methoxyquinazoli n-6-yl)-3-(1-methylpyrrolidin-2-yl)acrylamide | ¹H NMR (400 MHz, TFA salt, Methanol-d₄) δ 9.16 (s, 1H), 8.79 (d, J = 7.6 Hz, 1H), 8.53 (s, 1H), 8.33 (s, 1H), 7.84 (s, 1H), 7.31 (s, 1H), 7.15-7.12 (m, 1H), 7.00 (s, 1H), 6.99-6.87 (m, 3H), 4.15 (s, 3H), 4.14-4.11 (m, 1H), 3.86 (s, 3H), 3.84-3.80 (m, 1H), 3.15-3.14 (m, 1H), 2.96 (s, 3H), 2.50-2.47 (m, 1H), 2.28-2.01 (m, 6H); MS (ESI): m/z 581 [M+H]⁺ | 17 | |
| 30 | 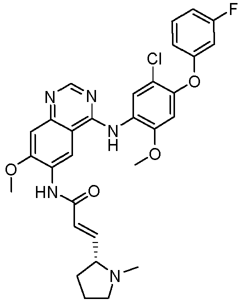 | (*R,E*)-*N*-(4-((5-chloro-4-(3-fluorophenoxy)-2-methoxyphenyl)a mino)-7-methoxyquinazoli n-6-yl)-3-(1-methylpyrrolidin-2-yl)acrylamide | ¹H NMR (400 MHz, HCl salt, Methanol-d₄) δ 9.29 (s, 1H), 8.70 (s, 1H), 7.81 (s, 1H), 7.42-7.36 (m, 1H), 7.33 (s, 1H), 7.00 (s, 1H), 6.96-6.92 (m, 1H), 6.91-6.86 (m, 2H), 6.83-6.81 (m, 1H), 6.75-6.72 (m, 1H), 4.19-4.09 (m, 4H), 3.84-3.78 (m, 4H), 3.26-3.24 (m, 1H), 2.96 (s, 3H), 2.51-2.46 (m, 1H), 2.31-2.03 (m, 3H); MS (ESI): m/z 578 [M+H]⁺ | 80 | 9.27, 99 |
| 31 | 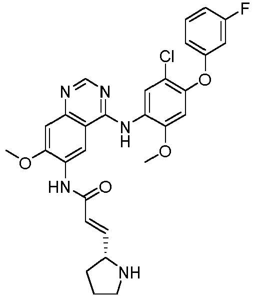 | (*R*,*E*)-*N*-(4-((5-chloro-4-(3-fluorophenoxy)-2-methoxyphenyl)a mino)-7-methoxyquinazoli n-6-yl)-3-(pyrrolidin-2-yl)acrylamide | ¹H NMR (400 MHz, TFA salt, Methanol-d₄) δ 9.19 (s, 1H), 8.62 (s, 1H), 7.96 (s, 1H), 7.40-7.34 (m, 1H), 7.31 (s, 1H), 7.07-7.01 (m, 1H), 6.99 (s, 1H), 6.89-6.76 (m, 3H), 6.75-6.71 (m, 1H), 4.38-4.31 (m, 1H), 4.16 (s, 3H), 3.84 (s, 3H), 3.44-3.38 (m, 2H), 2.43-2.34 (m, 1H), 2.25-2.10 (m, 2H), 2.03-1.93 (m, 1H); MS (ESI): m/z 564 [M+H]⁺ | 18 | 9.24, 99 |
| 32 | 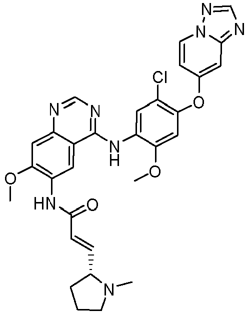 | (*R*,*E*)-*N*-(4-((4-([1,2,4]triazolo[1, 5-*a*]pyridin-7-yloxy)-5-chloro-2-methoxyphenyl)a mino)-7-methoxyquinazoli n-6-yl)-3-(1-methylpyrrolidin-2-yl)acrylamide | ¹H NMR (400 MHz, TFA salt, Methanol-d₄) δ 9.21 (s, 1H), 8.80 (d, J = 7.5 Hz, 1H), 8.63 (d, J = 6.9 Hz, 1H), 8.33 (s, 1H), 8.12 (d, J = 36.0 Hz, 1H), 7.32 (s, 1H), 7.24 (d, J = 2.8 Hz, 1H), 7.13 (dd, J = 7.5, 2.6 Hz, 1H), 7.00-6.84 (m, 3H), 4.19-4.07 (m, 4H), 3.91 (d, J = 5.8 Hz, 3H), 3.84-3.74 (m, 1H), 3.21 (dd, J = 14.7, 7.5 Hz, 1H), 2.96 (d, J = 10.9 Hz, 3H), 2.53-2.43 (m, 1H), 2.31-2.04 (m, 3H); MS (ESI): m/z 601 [M+H]⁺ | 37 | 7.46, 98 |
| 33 | 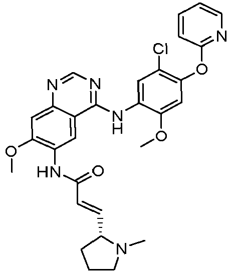 | (*R,E)*-*N*-(4-((5-chloro-2-methoxy-4-(pyridin-2-yloxy)phenyl)ami no)-7-methoxyquinazoli n-6-yl)-3-(1-methylpyrrolidin-2-yl)acrylamide | ¹H NMR (400 MHz, TFA salt, Methanol-d₄) δ 9.33 (s, 1H), 8.72 (s, 1H), 8.15-8.13 (m, 1H), 7.93-7.89 (m, 1H), 7.82 (s, 1H), 7.36 (s, 1H), 7.19-7.16 (m, 2H), 7.15 (d, J = 15.3 Hz, 1H), 7.09-6.78 (m, 1H), 6.92 (d, J = 15.2 Hz, 1H), 4.19 (s, 3H), 4.14-4.12 (m, 2H), 3.86 (s, 3H), 3.84-3.80 (m, 1H), 3.37-3.27 (m, 1H), 2.99 (s, 3H), 2.52-2.49 (m, 1H), 2.33-2.12 (m, 2H); MS (ESI): m/z 561 [M+H]⁺ | 15 | 8.10, 100 |
| 34 | 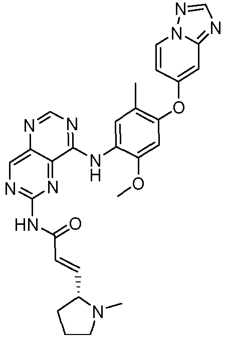 | (*R*,*E*)-*N*-(8-( (4-([1,2,4]triazolo[1, 5-*a*]pyridin-7-yloxy)-2-methoxy-5-methylphenyl)am ino)pyrimido[5,4-d]pyrimidin-2-yl)-3-(1-methylpyrrolidin-2-yl)acrylamide | MS (ESI): m/z 553 [M+H]⁺ | | |
| 35 | 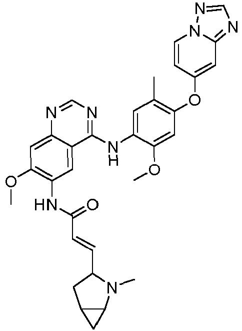 | (*E*)-*N*-(4-((4-([1,2,4]triazolo[1, 5-*a*]pyridin-7-yloxy)-2-methoxy-5-methylphenyl)am ino)-7-methoxyquinazoli n-6-yl)-3-(2-methyl-2-azabicyclo[3.1.0] hexan-3-yl)acrylamide | MS (ESI): m/z 593 [M+H]⁺ | | |
| 36 | 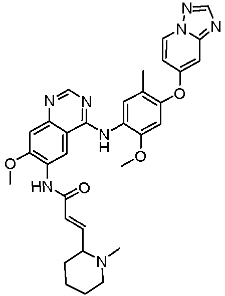 | (*E*)-N-(4-((4-([1,2,4]triazolo[1, 5-*a*]pyridin-7-yloxy)-2-methoxy-5-methylphenyl)am ino)-7-methoxyquinazoli n-6-yl)-3-(1-methylpiperidin-2-yl)acrylamide | MS (ESI): m/z 595 [M+H]⁺ | | |
| 37 | 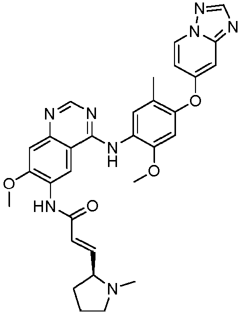 | (*S,E*)*-N-*(4-((4-([1,2,4]triazolo[1, 5-*a*]pyridin-7-yloxy)-2-methoxy-5-methylphenyl)am ino)-7-methoxyquinazoli n-6-yl)-3-(1-methylpyrrolidin-2-yl)acrylamide | MS (ESI): m/z 581 [M+H]⁺ | | |

### Preparation Example 11: (E)-N-(4-((4-([1,2,4]triazolo[1,5-a]pyridin-7-yloxy)-2-methoxy-5-methylphenyl)amino)-7-methoxyquinazolin-6-yl)-4-((1,1-dioxidotetrahydro-2H-thiopyran-4-yl)amino)but-2-enamide (Example 38)

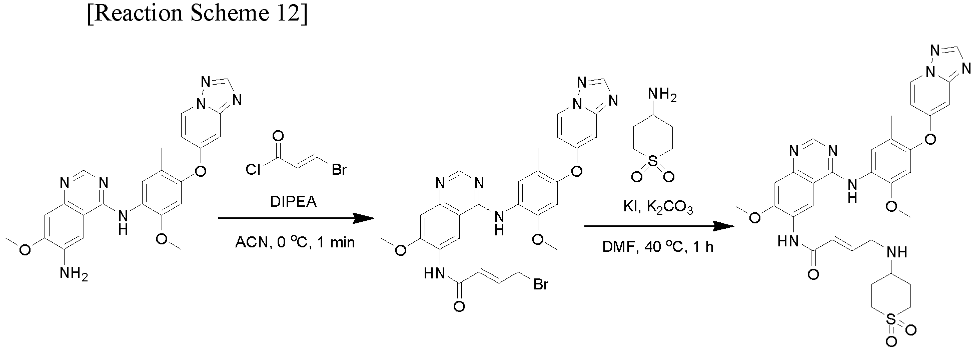

### [Step 1] Preparation of (E)-N-(4-((4-([1,2,4]triazolo[1,5-a]pyridin-7-yloxy)-2-methoxy-5-methylphenyl)amino)-7-methoxyquinazolin-6-yl)-4-bromobut-2-enamide

*N*⁴-(4-([1,2,4]Triazolo[1,5-*a*]pyridin-7-yloxy)-2-methoxy-5-methylphenyl)-7-methoxyquinazoline-4,6-diamine (1.0 eq) prepared in step 4 of Preparation Example 1 was dissolved in acetonitrile (0.2 M), and then (E)-3-bromoacryloyl chloride (3.0 eq) and DIPEA (4.0 eq) were added thereto at 0 °C. The reaction mixture was reacted at 0 °C for 1 minute, then water was added thereto, and organic materials were extracted with ethyl acetate (EA). Remaining water in collected organic layer was removed using Na₂SO₄, and the collected organic layer was concentrated. The obtained desired compound as a brown oil was used in the following reaction without further purification (yield: 100%, MS (ESI): m/z 590 [M+H]⁺).

### [Step 2] Preparation of (E)-N-(4-((4-([1,2,4]triazolo[1,5-a]pyridin-7-yloxy)-2-methoxy-5-methylphenyl)amino)-7-methoxyquinazolin-6-yl)-4-((1,1-dioxidotetrahydro-2H-thiopyran-4-yl)amino)but-2-enamide

(*E*)-*N*-(4-4-([1,2,4]Triazolo[1,5*-a*]pyridin-7-yloxy)-2-methoxy-5-methylphenyl)amino)-7-methoxyquinazolin-6-yl)-4-bromobut-2-enamide (1.0 eq) obtained in step 1, KI (1.8 eq), and K₂CO₃ (2.3 eq) were dissolved in DMF (0.2 M) and then reacted at 40 °C for 1 hour. The reaction mixture was purified using prep-HPLC to obtain the desired compound as a white solid (yield: 8%, MS (ESI): m/z 659 [M+H]⁺).

### Preparation of Compounds of Examples 39 to 40

The compounds of Examples 39 to 40 according to the present invention were prepared in a similar manner to Preparation Example 11.

The chemical structural formulas, compound names, ¹H NMR, MS, yields, HPLC data, and purities of the compounds of Examples 38 to 40 are summarized in Table 11 below.

**[Table 11]**

| Ex. | Structure | Compound name | ¹H NMR, MS | Yield (%) | HPLC (min), Purity (%) |
|---|---|---|---|---|---|
| 38 | 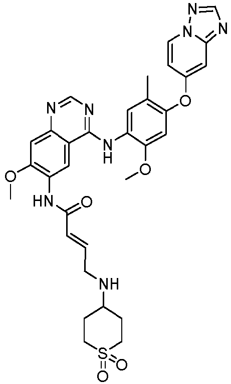 | (*E*)-*N*-(4-((4-([1,2,4]triazolo[1, 5-*a*]pyridin-7-yloxy)-2-methoxy-5-methylphenyl)ami no)-7-methoxyquinazoli n-6-yl)-4-((1,1-dioxidotetrahydro -2*H*-thiopyran-4-yl)amino)but-2-enamide | ¹H NMR (400 MHz, TFA salt, Methanol-d₄) δ 9.18 (s, 1H), 8.70 (d, J = 7.6 Hz, 1H), 8.57 (s, 1H), 8.25 (s, 1H), 7.49 (s, 1H), 7.23 (s, 1H), 7.05-7.02 (m, 1H), 6.93-6.89 (m, 2H), 6.77-6.71 (m, 2H), 4.08 (s, 3H), 3.93 (d, J = 6.4 Hz, 2H), 3.72 (s, 3H), 3.45-3.42 (m, 1H), 3.18-3.09 (m, 4H), 2.46-2.42 (m, 2H), 2.15-2.12 (m, 2H), 2.09 (s, 3H); MS (ESI): m/z 659 [M+H]⁺ | 8 | 7.29, 100 |
| 39 | 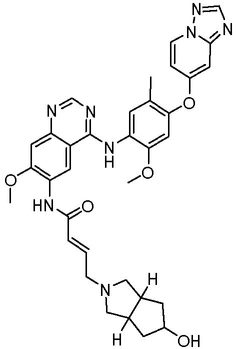 | (*E*)-*N*-(4-((4-([1,2,4]triazolo[1, 5-*a*]pyridin-7-yloxy)-2-methoxy-5-methylphenyl)ami no)-7-methoxyquinazoli n-6-yl)-4-(5-hydroxyhexahydr ocyclopenta[c]pyr rol-2(1*H*)-yl)but-2-enamide | MS (ESI): m/z 637 [M+H]⁺ | | |
| 40 | 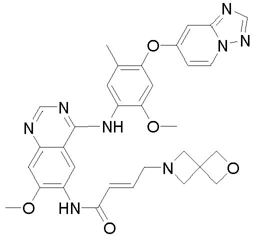 | (*E*)-*N*-(4-((4-([1,2,4]triazolo[1, 5-*a*]pyridin-7-yloxy)-2-methoxy-5-methylphenyl)ami no)-7-methoxyquinazoli n-6-yl)-4-(2-oxa-6-azaspiro[3.3]hepta n-6-yl)but-2-enamide | ¹H NMR (400 MHz, TFA salt, Methanol-d4) δ9.18 (s, 1H), 8.71 (d, J = 7.2 Hz, 1H), 8.57 (s, 1H), 8.26 (s, 1H), 7.49 (s, 1H), 7.23 (s, 1H), 7.05-7.03 (m, 1H), 6.93 (s, 1H), 6.81-6.66 (m, 3H), 4.73-4.69 (m, 4H), 4.45-4.16 (m, 4H), 4.06 (s, 3H), 3.97 (d, J = 6.0 Hz, 2H), 3.88 (s, 3H), 2.76 (s, 3H); MS (ESI): m/z 609 [M+H]⁺ | 6 | 7.28, 100 |

### Preparation Example 12: (R,E)-N-(4-((4-([1,2,4]triazolo[1,5-a]pyridin-7-yloxy)-2-methoxy-5-methylphenyl)amino)-7-methoxyquinazolin-6-yl)-2-fluoro-3-(1-methylpyrrolidin-2-yl)acrylamide (Example 41) and (R,Z)-N-(4-((4-([1,2,4]triazolo[1,5-a]pyridin-7-yloxy)-2-methoxy-5-methylphenyl)amino)-7-methoxyquinazolin-6-yl)-2-fluoro-3-(1-methylpyrrolidin-2-yl)acrylamide (Example 111)

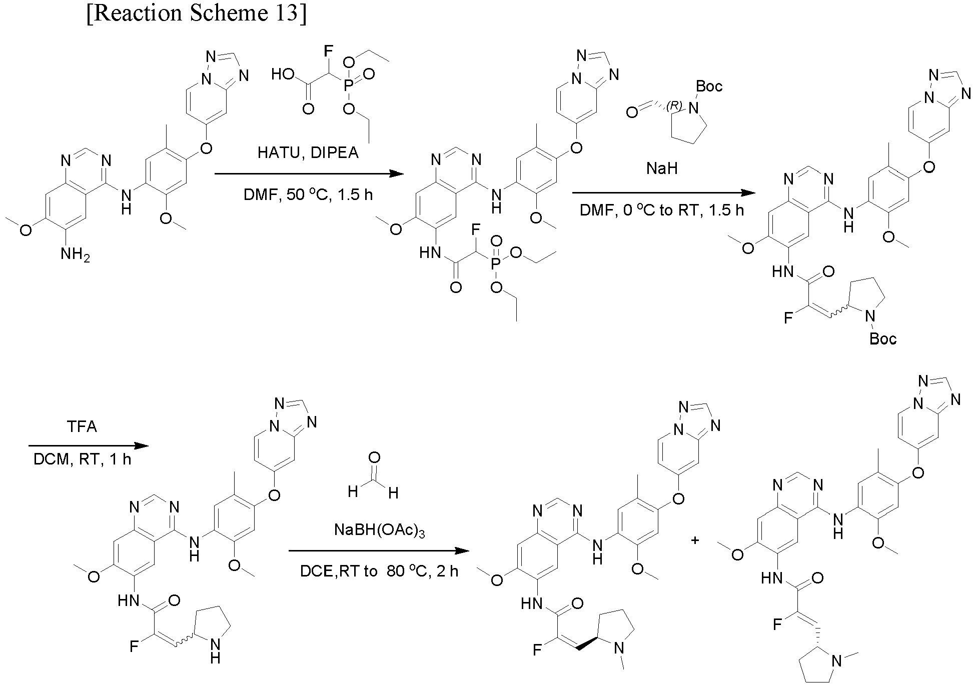

### [Step 1] Preparation of diethyl (2-((4-((4-([1,2,4]triazolo[1,5-a]pyridin-7-yloxy)-2-methoxy-5-methylphenyl)amino)-7-methoxyquinazolin-6-yl)amino)- 1-fluoro-2-oxoethyl)phosphonate

2-(Diethoxyphosphoryl)-2-fluoroacetic acid (2.0 eq) and HATU (3.0 eq) were dissolved in DMF (0.5 M), followed by stirring at 50 °C for 30 minutes. *N*⁴-(4-([1,2,4]Triazolo[1,5-*a*]pyridin-7-yloxy)-2-methoxy-5-methylphenyl)-7-methoxyquinazoline-4,6-diamine (1.0 eq) prepared in step 4 of Preparation Example 1 and DIPEA (3.0 eq) were added to the mixture and then stirred at 50 °C for 1 hour. Ice water was poured into the reaction mixture to form a solid, and then the solid was filtered to obtain the desired compound as a yellow solid (yield: 95%, MS (ESI): m/z 640 [M+H]⁺).

### [Step 2] Preparation of tert-butyl (R,E/Z)-2-(3-((4-((4-([1,2,4]triazolo[1,5-a]pyridin-7-yloxy)-2-methoxy-5-methylphenyl)amino)-7-methoxyquinazolin-6-yl)amino)-2-fluoro-3-oxoprop-1-en-1-yl)pyrrolidine-1-carboxylate

Diethyl (2-((4-((4-([1,2,4]triazolo[1,5-*a*]pyridin-7-yloxy)-2-methoxy-5-methylphenyl)amino)-7-methoxyquinazolin-6-yl)amino)-1-fluoro-2-oxoethyl)phosphonate (1.0 eq) obtained in step 1 was dissolved in DMF (0.5 M), and then NaH (1.5 eq) was added at 0 °C. The mixture was stirred at 0 °C for 30 minutes, and then *tert*-butyl (*R*)-2-formylpyrrolidine-1-carboxylate (2.0 eq) was added thereto and stirred at room temperature for 1 hour. Ice water was poured into the reaction mixture to form a solid, and then the solid was filtered to obtain the desired compound as a yellow solid (yield: 85%, MS (ESI): m/z 685 [M+H]⁺).

### [Step 3] Preparation of (R,E/Z)-N-(4-((4-([1,2,4]triazolo[1,5-a]pyridin-7-yloxy)-2-methoxy-5-methylphenyl)amino)-7-methoxyquinazolin-6-yl)-2-fluoro-3-(pyrrolidin-2-yl)acrylamide

*tert-*Butyl (*R*,*E*/*Z*)-2-(3-((4-((4-([1,2,4]triazolo[1,5-*a*]pyridin-7-yloxy)-2-methoxy-5-methylphenyl)amino)-7-methoxyquinazolin-6-yl)amino)-2-fluoro-3-oxoprop-1-en-1-yl)pyrrolidine-1-carboxylate (1.0 eq) obtained in step 2 was dissolved in dichloromethane (0.1 M), and then TFA (60.0 eq) was added thereto and concentrated after being stirred at room temperature for 1 hour. The concentrated mixture was purified using MPLC (dichloromethane:methanol) to obtain the desired compound as a yellow solid (yield: 57%, MS (ESI): m/z 585 [M+H]⁺).

### [Step 4] Preparation of (R,E)-N-(4-((4-([1,2,4]triazolo[1,5-a]pyridin-7-yloxy)-2-methoxy-5-methylphenyl)amino)-7-methoxyquinazolin-6-yl)-2-fluoro-3-(1-methylpyrrolidin-2-yl)acrylamide and (R,Z)-N-(4-((4-([1,2,4]triazolo[1,5-a]pyridin-7-yloxy)-2-methoxy-5-methylphenyl)amino)-7-methoxyquinazolin-6-yl)-2-fluoro-3-(1-methylpyrrolidin-2-yl)acrylamide

(*R*,*E*/*Z*)-*N*-(4-((4-([1,2,4]Triazolo[1,5-*a*]pyridin-7-yloxy)-2-methoxy-5-methylphenyl)amino)-7-methoxyquinazolin-6-yl)-2-fluoro-3-(pyrrolidin-2-yl)acrylamide (1.0 eq) obtained in step 3 and 37% formaldehyde (5.0 eq) were dissolved in DCE (0.1 M) and stirred at room temperature for 1 hour. Sodium triacetoxyborohydride (4.0 eq) was added to the mixture and then stirred at 80 °C for 1 hour. To the reaction mixture, a saturated aqueous NaHCO₃ solution and dichloromethane were added to extract organic materials, and remaining water was removed using MgSO₄, and the organic layer was concentrated. The reaction mixture was purified using MPLC (dichloromethane:methanol) to obtain the desired compound as a yellow solid.

((*R*,*E*)-*N*-(4-((4-([1,2,4]Triazolo[1,5-*a*]pyridin-7-yloxy)-2-methoxy-5-methylphenyl)amino)-7-methoxyquinazolin-6-yl)-2-fluoro-3-(1-methylpyrrolidin-2-yl)acrylamide: yield: 39%, MS (ESI): m/z 599 [M+H]⁺, ((*R*,*Z*)-*N*-(4-((4-([1,2,4]triazolo[1,5-*a*]pyridin-7-yloxy)-2-methoxy-5-methylphenyl)amino)-7-methoxyquinazolin-6-yl)-2-fluoro-3-(1-methylpyrrolidin-2-yl)acrylamide: yield: 9%, MS (ESI): m/z 599 [M+H]⁺).

### Preparation of Compounds of Examples 42 to 110 and 112 to 143

The compounds of Examples 42 to 110 and 112 to 143 according to the present invention were prepared in a similar manner to Preparation Example 12.

The chemical structural formulas, compound names, ¹H NMR, MS, yields, HPLC data, and purities of the compounds of Examples 41 to 143 are summarized in Table 12 below.

**[Table 12]**

| Ex. | Structure | Compound name | ¹H NMR, MS | Yield (%) | HPLC (min), Purity (%) |
|---|---|---|---|---|---|
| 41 | 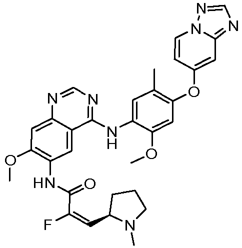 | (*R*,*E*)-*N*-(4-((4-([1,2,4]triazolo[1, 5-*a*]pyridin-7-yloxy)-2-methoxy-5-methylphenyl)am ino)-7-methoxyquinazoli n-6-yl)-2-fluoro-3-(1-methylpyrrolidin-2-yl)acrylamide | ¹H NMR (400 MHz, TFA salt, Methanol-d₄) δ 9.00 (s, 1H), 8.77 (d, J = 7.6 Hz, 1H), 8.45 (s, 1H), 8.31 (s, 1H), 7.86 (s, 1H), 7.29 (s, 1H), 7.13-7.10 (m, 1H), 6.96 (s, 2H), 5.97-5.89 (m, 1H), 4.13 (s, 3H), 4.12-4.04 (m, 1H), 3.88 (s, 3H), 3.19-3.14 (m, 1H), 2.49-2.42 (m, 4H), 2.37-2.31 (m, 1H), 2.16 (s, 3H), 1.99-1.87 (m, 2H), 1.77-1.72 (m, 1H); MS (ESI): m/z 599 [M+H]⁺ | 39 | 7.34, 100 |
| 42 | 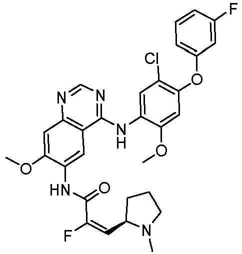 | (*R,E*)-*N*-(4-((5-chloro-4-(3-fluorophenoxy)-2-methoxyphenyl)a mino)-7-methoxyquinazoli n-6-yl)-2-fluoro-3-(1-methylpyrrolidin-2-yl)acrylamide | ¹H NMR (400 MHz, FA slat, Methanol-d₄) δ 8.94 (s, 1H), 8.50-8.47 (m, 1H), 8.37 (s, 1H), 8.08 (s, 1H), 7.39-7.32 (m, 1H), 7.30 (s, 1H), 6.97 (d, J = 3.4 Hz, 1H), 6.86-6.77 (m, 2H), 6.76-6.70 (m, 1H), 6.08-5.99 (m, 1H), 5.03-4.96 (m, 1H), 4.11 (s, 3H), 3.85 (s, 3H), 3.66-3.57 (m, 1H), 3.15-3.06 (m, 1H), 2.86 (s, 3H), 2.54-2.44 (m, 1H), 2.21-2.05 (m, 2H), 1.98-1.87 (m, 1H); MS (ESI): m/z 596 [M+H]⁺ | 6 | 9.41, 98 |
| 43 | 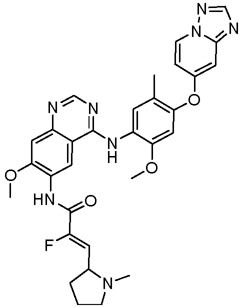 | (*Z*)-*N*-(4-((4-([1,2,4]triazolo[1, 5-*a*]pyridin-7-yloxy)-2-methoxy-5-methylphenyl)am ino)-7-methoxyquinazoli n-6-yl)-2-fluoro-3-(1-methylpyrrolidin-2-yl)acrylamide | ¹H NMR (400 MHz, free base, Methanol-d₄) δ9.20 (s, 1H), 8.92 (d, J = 7.6 Hz, 1H), 8.72 (s, 1H), 8.59 (s, 1H), 7.60 (s, 1H), 7.38 (s, 1H), 7.27-7.25 (m, 1H), 7.08 (s, 1H), 7.00 (s, 1H), 6.45-6.35 (m, 1H), 4.53-4.51 (m, 1H), 4.22 (s, 3H), 3.87-3.84 (m, 4H), 3.37-3.33 (m, 1H), 3.16 (s, 3H), 2.62-2.60 (m, 1H), 2.39-2.21 (m, 5H), 2.11-2.05 (m, 1H); MS (ESI): m/z 599 [M+H]⁺ | 59 | 7.28, 100 |
| 44 | 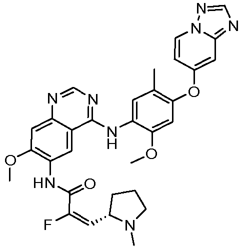 | *(S,E)-N-(4-((4-*([1,2,4]triazolo[1, 5-*a*]pyridin-7-yloxy)-2-methoxy-5-methylphenyl)am ino)-7-methoxyquinazoli n-6-yl)-2-fluoro-3-(1-methylpyrrolidin-2-yl)acrylamide | ¹H NMR (400 MHz, free base, DMSO-d₆) δ10.17 (s, 1H), 9.33 (s, 1H), 8.97 (d, J = 7.6 Hz, 1H), 8.75 (s, 1H), 8.43 (s, 1H), 8.40 (s, 1H), 7.56 (s, 1H), 7.29 (s, 1H), 7.07-7.04 (m, 1H), 6.99 (s, 1H), 6.81 (d, J = 2.4 Hz, 1H), 5.96-5.88 (m, 1H), 4.11-4.01 (m, 1H), 4.00 (s, 3H), 3.74 (s, 3H), 3.02-3.01 (m, 1H), 2.34-2.33 (m, 4H), 2.25-2.23 (m, 4H), 1.75-1.73 (m, 2H), 1.62-1.60 (m, 1H); MS (ESI): m/z 599 [M+H]⁺ | 23 | 7.42, 99 |
| 45 | 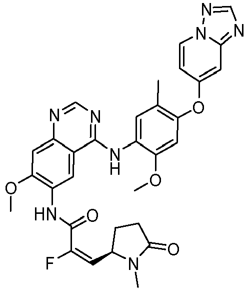 | (*R*,*E*)-N-(4-((4-([1,2,4]triazolo[1, 5-*a*]pyridin-7-yloxy)-2-methoxy-5-methylphenyl)am ino)-7-methoxyquinazoli n-6-yl)-2-fluoro-3-(1-methyl-5-oxopyrrolidin-2-yl)acrylamide | MS (ESI): m/z 613 [M+H]⁺ | | |
| 46 | 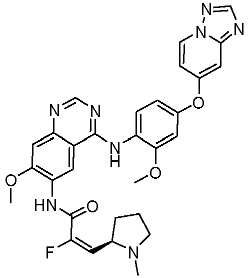 | (*R*,*E*)-*N*-(4-((4-([1,2,4]triazolo[1, 5-*a*]pyridin-7-yloxy)-2-methoxyphenyl)a mino)-7-methoxyquinazoli n-6-yl)-2-fluoro-3-(1-methylpyrrolidin-2-yl)acrylamide | ¹H NMR (400 MHz, free, Methanol-d₄) δ 8.98 (s, 1H), 8.75 (d, J = 8.4 Hz, 1H), 8.43 (s, 1H), 8.31 (s, 1H), 7.92 (d, J = 8.6 Hz, 1H), 7.30 (s, 1H), 7.14-7.07 (m, 2H), 7.03 (d, J = 2.4 Hz, 1H), 5.95 (dd, J = 22.7, 9.4 Hz, 1H), 4.43-4.33 (m, 1H), 4.12 (s, 3H), 3.89 (s, 3H), 2.73-2.49 (m, 4H), 2.40 - 2.30 (m, 1H), 2.08-1.92 (m, 2H), 1.82-1.68 (m, 1H) ; MS (ESI): m/z 585 [M+H]⁺ | 12 | 7.32, 96 |
| 47 | 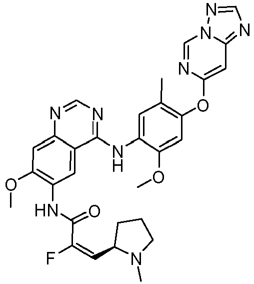 | (*R*,*E*)-*N*-(4-((4-([1,2,4]triazolo[1, 5-c]pyrimidin-7-yloxy)-2-methoxy-5-methylphenyl)am ino)-7-methoxyquinazoli n-6-yl)-2-fluoro-3-(1-methylpyrrolidin-2-yl)acrylamide | ¹H NMR (400 MHz, FA salt, Methanol-d₄) δ 9.46 (s, 1H), 8.96 (s, 1H), 8.43 (s, 2H), 7.82 (s, 1H), 7.27 (s, 1H), 6.99 (s, 1H), 6.96 (s, 1H), 5.98-5.90 (m, 1H), 4.29-4.23 (m, 1H), 4.11 (s, 3H), 3.84 (s, 3H), 3.27-3.22 (m, 1H), 2.56-2.49 (m, 4H), 2.40-2.22 (m, 1H), 2.18 (s, 3H), 2.03-1.92 (m, 2H), 1.77-1.68 (m, 1H) ; MS (ESI): m/z 600 [M+H]⁺ | 4 | 7.11, 98 |
| 48 | 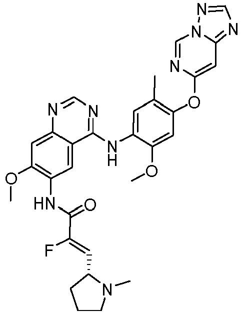 | (*R*,*Z*)-*N*-(4-((4-([1,2,4]triazolo[1, 5-c]pyrimidin-7-yloxy)-2-methoxy-5-methylphenyl)am ino)-7-methoxyquinazoli n-6-yl)-2-fluoro-3-(1-methylpyrrolidin-2-yl)acrylamide | ¹H NMR (400 MHz, FA salt, Methanol-d₄) δ 9.46 (d, J = 1.2 Hz, 1H), 8.96 (s, 1H), 8.43 (d, J = 5.6 Hz, 2H), 7.88 (s, 1H), 7.29 (s, 1H), 7.00 (d, J= 1.2 Hz, 1H), 6.97 (s, 1H), 6.26-6.15 (m, 1H), 4.12 (s, 3H), 3.85 (s, 3H) ; MS (ESI): m/z 600 [M+H]⁺ | 2 | 6.98, 99 |
| 49 | 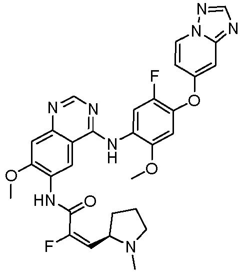 | (*R*,*E*)-*N*-(4-((4-([1,2,4]triazolo[1, 5-*a*]pyridin-7-yloxy)-5-fluoro-2-methoxyphenyl)a mino)-7-methoxyquinazoli n-6-yl)-2-fluoro-3-(1-methylpyrrolidin-2-yl)acrylamide | ¹H NMR (400 MHz, free, Methanol-d₄) δ 8.99 (s, 1H), 8.76 (d, J = 7.5 Hz, 1H), 8.54 (s, 1H), 8.31 (s, 1H), 8.26 (d, J = 12.0 Hz, 1H), 7.30 (s, 1H), 7.19-7.09 (m, 2H), 7.05 (d, J = 2.6 Hz, 1H), 5.92 (dd, J = 23.7, 9.2 Hz, 1H), 4.12 (s, 3H), 4.04 (q, J = 8.1 Hz, 1H), 3.94 (s, 3H), 3.19-3.09 (m, 1H), 2.41-2.30 (m, 4H), 2.30-2.20 (m, 1H), 1.97-1.84 (m, 2H), 1.73-1.61 (m, 1H) ; MS (ESI): m/z 603 [M+H]⁺ | 17 | 7.43, 99 |
| 50 | 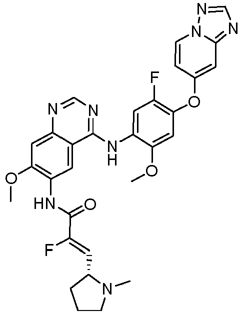 | (*R,Z*)-*N-*(4-((4-([1,2,4]triazolo[1, 5-*a*]pyridin-7-yloxy)-5-fluoro-2-methoxyphenyl)a mino)-7-methoxyquinazoli n-6-yl)-2-fluoro-3-(1-methylpyrrolidin-2-yl)acrylamide | ¹H NMR (400 MHz, free, Methanol-d₄) δ 9.00 (s, 1H), 8.77 (d, J = 7.5 Hz, 1H), 8.56 (s, 1H), 8.41-8.28 (m, 2H), 7.32 (s, 1H), 7.18-7.09 (m, 2H), 7.04 (d, J = 2.1 Hz, 1H), 6.21 (dd, J = 35.5, 9.4 Hz, 1H), 4.63-4.53 (m, 1H), 4.13 (s, 3H), 3.96 (s, 3H), 2.42-2.30 (m, 4H), 2.24-2.12 (m, 1H), 2.00-1.87 (m, 2H), 1.79-1.66 (m, 1H) ; MS (ESI): m/z 603 [M+H]⁺ | 6 | 7.36, 99 |
| 51 | 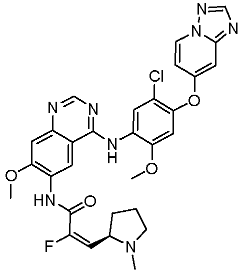 | (*R*,*E*)-*N*-(4-((4-([1,2,4]triazolo[1, 5-*a*]pyridin-7-yloxy)-5-chloro-2-methoxyphenyl)a mino)-7-methoxyquinazoli n-6-yl)-2-fluoro-3-(1-methylpyrrolidin-2-yl)acrylamide | ¹H NMR (400 MHz, TFA salt, Methanol-d₄) δ9.01 (s, 1H), 8.80 (d, J = 7.6 Hz, 1H), 8.53 (s, 1H), 8.33 (s, 1H), 8.32 (s, 1H), 7.32 (s, 1H), 7.19 (s, 1H), 7.14-7.12 (m, 1H), 6.99 (d, J = 2.4 Hz, 1H), 5.98-5.89 (m, 1H), 4.41 (s, 3H), 4.08-4.06 (m, 1H), 3.94 (s, 3H), 3.17-3.15 (m, 1H), 2.40 (s, 3H), 2.38-2.35 (m, 2H), 1.94-1.90 (m, 2H), 1.75-1.73 (m, 1H); MS (ESI): m/z 619 [M+H]⁺ | 30 | 7.67, 100 |
| 52 | 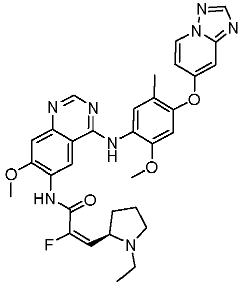 | (*R*,*E*)-*N*-(4-((4-([1,2,4]triazolo[1, 5-*a*]pyridin-7-yloxy)-2-methoxy-5-methylphenyl)am ino)-7-methoxyquinazoli n-6-yl)-3-(1-ethylpyrrolidin-2-yl)-2-fluoroacrylamide | ¹H NMR (400MHz, TFA salt, Methanol-d₄) δ9.20 (s, 1H), 8.83-8.81 (m, 1H), 8.71 (s, 1H), 8..8 (s, 1H), 7.56 (s, 1H), 7.39 (s, 1H), 7.18-7.15 (m, 1H), 7.06 (s, 1H), 6.88-6.87 (m, 1H), 6.15-6.10 (m, 1H), 5.30-5.20 (m, 1H), 4.88-4.84 (m, 1H), 4.21 (s, 3H), 3.83 (s, 3H), 3.65 (s, 3H), 3.53-3.52 (m, 1H), 3.28-3.20 (m, 2H), 2.01-1.98 (m, 1H), 1.42-1.38 (m, 3H); MS (ESI): m/z 613 [M+H]⁺ | 25 | 7.63, 95 |
| 53 | 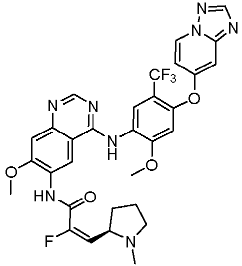 | (*R*,*E*)-*N*-(4-((4-([1,2,4]triazolo[1, 5-*a*]pyridin-7-yloxy)-2-methoxy-5-(trifluoromethyl) phenyl)amino)-7-methoxyquinazoli n-6-yl)-2-fluoro-3-(1-methylpyrrolidin-2-yl)acrylamide | MS (ESI): m/z 653 [M+H]⁺ | | |
| 54 | 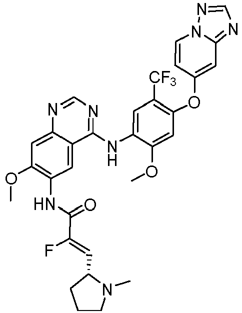 | (*R*,*Z*)-*N*-(4-((4-([1,2,4]triazolo[1, 5-*a*]pyridin-7-yloxy)-2-methoxy-5-(trifluoromethyl) phenyl)amino)-7-methoxyquinazoli n-6-yl)-2-fluoro-3-(1-methylpyrrolidin-2-yl)acrylamide | ¹H NMR (400 MHz, HCl salt, Methanol-d₄) δ 9.00 (s, 1H), 8.80-8.78 (m, 1H), 8.52 (s, 1H), 8.48 (s, 1H), 8.33 (s, 1H), 7.32 (s, 1H), 7.15-7.11 (m, 3H), 6.26-6.15 (m, 1H), 4.58 (s, 1H), 4.13 (s, 3H), 3.93 (s, 3H), 3.35 (s, 3H), 3.16-3.11 (m, 2H), 2.40-2.20 (m, 4H), 2.20-2.13 (m, 1H), 1.97-1.89 (m, 2H), 1.77-1.70 (m, 1H), 1.29 (brs, 3H); MS (ESI): m/z 653 [M+H]⁺ | 78 | 8.00, 99 |
| 55 | 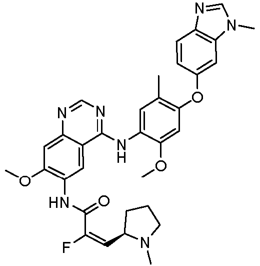 | (*R,E*)-2-fluoro-*N-*(7-methoxy-4-((2-methoxy-5-methyl-4-((1-methyl-1*H-*benzo[*d*]imidazol -6-yl)oxy)phenyl)am ino)quinazolin-6-yl)-3-(1-methylpyrrolidin-2-yl)acrylamide | ¹H NMR (400 MHz, free, Methanol-d₄) δ 8.96 (s, 1H), 8.39 (s, 1H), 8.07 (s, 1H), 7.68-7.60 (m, 2H), 7.26 (s, 1H), 7.11 (d, J = 2.2 Hz, 1H), 7.04 (dd, J = 8.8, 2.3 Hz, 1H), 6.71 (s, 1H), 5.91 (dd, J = 23.5, 9.3 Hz, 1H), 4.13-4.03 (m, 4H), 3.83 (s, 3H), 3.72 (s, 3H), 3.20-3.11 (m, 1H), 2.43-2.33 (m, 4H), 2.32-2.20 (m, 4H), 1.97-1.82 (m, 2H), 1.72-1.61 (m, 1H) ; MS (ESI): m/z 612 [M+H]⁺ | 19 | 6.89, 99 |
| 56 | 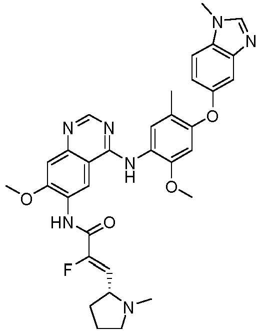 | (*R*,*Z*)-*N*-(4-((4-(7-methoxy-4-((2-methoxy-5-methyl-4-((1-methyl-1*H-*benzo[*d*]imidazol -5-yl)oxy)phenyl)am ino)quinazolin-6-yl)-3-(1-methylpyrrolidin-2-yl)acrylamide | ¹H NMR (400 MHz, TFA salt. Methanol-d₄) δ 9.23 (s, 1H), 9.15 (s, 1H), 8.66 (s, 1H), 7.91 (d, J = 9.1 Hz, 1H), 7.53 (s, 1H), 7.44-7.35 (m, 2H), 7.30 (d, J = 2.1 Hz, 1H), 6.84 (s, 1H), 6.37 (dd, J= 32.0, 10.2 Hz, 1H), 4.53-4.43 (m, 1H), 4.19 (s, 3H), 4.13 (s, 3H), 3.90-3.79 (m, 1H), 3.74 (s, 3H), 3.28-3.23 (m, 1H), 2.97 (s, 3H), 2.58-2.46 (m, 1H), 2.35-2.18 (m, 5H), 2.13-1.99 (m, 1H) ; MS (ESI): m/z 612 [M+H]⁺ | 11 | 6.88, 98 |
| 57 | 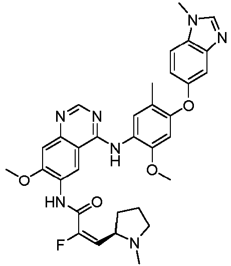 | (*R,E*)-2-fluoro-*N-*(7-methoxy-4-((2-methoxy-5-methyl-4-((1-methyl-1*H-*benzo[d]imidazol -5-yl)oxy)phenyl)am ino)quinazolin-6-yl)-3-(1-methylpyrrolidin-2-yl)acrylamide | ¹H NMR (400 MHz, TFA salt, Methanol-d₄) δ 9.27 (s, 1H), 9.18 (s, 1H), 8.66 (s, 1H), 7.92 (d, J = 9.1 Hz, 1H), 7.49 (s, 1H), 7.43-7.36 (m, 2H), 7.31 (d, J = 2.1 Hz, 1H), 6.85 (s, 1H), 6.09 (dd, J = 20.5, 9.8 Hz, 1H), 5.25-5.14 (m, 1H), 4.18 (s, 3H), 4.14 (s, 3H), 3.84-3.76 (m, 1H), 3.74 (s, 3H), 2.99 (s, 3H), 2.61-2.49 (m, 1H), 2.31-2.15 (m, 5H), 2.08-1.94 (m, 1H) ; MS (ESI): m/z 612 [M+H]⁺ | 16 | 6.91, 97 |
| 58 | 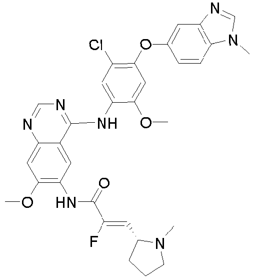 | (*R,Z*)*-N*-(4-((5-chloro-2-methoxy-4-((1-methyl-1*H-*benzo[*d*]imidazol -5-yl)oxy)phenyl)am ino)-7-methoxyquinazoli n-6-yl)-2-fluoro-3-(1-methylpyrrolidin-2-yl)acrylamide | ¹H NMR (400 MHz, TFA salt, Methanol-d₄) δ 9.23-9.16 (m, 2H), 8.71 (s, 1H), 7.92-7.90 (m, 1H), 7.85 (s, 1H), 7.40-7.36 (m, 3H), 7.03 (s, 1H), 6.42-6.32 (m, 1H), 4.49-4.47 (m, 1H), 4.19 (s, 3H), 4.12 (s, 3H), 3.79 (s, 3H), 2.97 (s, 3H), 2.52-2.50 (m, 1H), 2.29-2.20 (m, 2H), 2.09-1.99 (m, 1H); MS (ESI): m/z 632 [M+H]⁺ | 6 | 7.18, 99 |
| 59 | 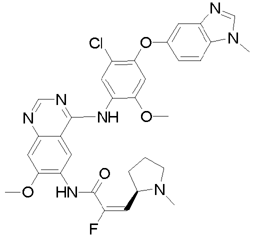 | (*R*,*E*)-*N*-(4-((4-chloro-2-methoxy-4-((1-methyl-1*H-*benzo[*d*]imidazol -5-yl)oxy)phenyl)am ino)-7-methoxyquinazoli n-6-yl)-2-fluoro-3-(1-methylpyrrolidin-2-yl)acrylamide | ¹H NMR (400 MHz, TFA salt, Methanol-d₄) δ8.61 (s, 1H), 8.34 (s, 1H), 8.27 (s, 1H), 8.10 (s, 1H), 7.51 (d, J = 8.4 Hz, 1H), 7.21 (s, 1H), 7.07 (d, J = 7.6 Hz, 1H), 7.01 (s, 1H), 6.63 (s, 1H), 5.97-5.89 (m, 1H), 4.22 (brs, 1H), 4.00 (s, 3H), 3.88 (s, 3H), 3.71 (s, 3H), 3.22 (s, 1H), 2.46 (s, 3H), 2.29 (s, 1H), 1.94 (s, 3H), 1.71 (s, 1H); MS (ESI): m/z 632 [M+H]⁺ | 3 | 7.01, 97 |
| 60 | 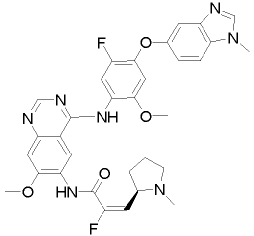 | (*R,E*)-2-fluoro-N-(4-((5-fluoro-2-methoxy-4-((1-methyl-1*H-*benzo[*d*]imidazol -5-yl)oxy)phenyl)am ino)-7-methoxyquinazoli n-6-yl)-3-(1-methylpyrrolidin-2-yl)acrylamide | ¹H NMR (400 MHz, TFA salt, Methanol-d₄) δ9.06 (s, 1H), 8.62 (s, 2H), 7.82-7.72 (m, 2H), 7.35 (s, 1H), 7.31-7.28 (m, 2H), 6.98 (d, J = 7.2 Hz, 1H), 6.12-6.05 (m, 1H), 5.22 (s, 1H), 4.15 (s, 3H), 4.01 (s, 3H), 3.80 (s, 3H), 2.98 (s, 3H), 2.56-2.54 (m, 1H), 2.22-2.15 (m, 2H), 2.02-2.00 (m, 1H) ; MS (ESI): m/z 616 [M+H]⁺ | 19 | 6.95, 100 |
| 61 | 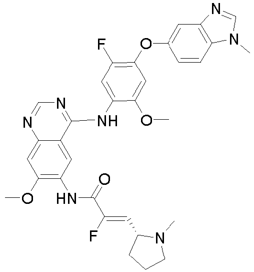 | (*R*,*Z*)-2-fluoro-*N-*(4-((5-fluoro-2-methoxy-4-((1-methyl-1H-benzo[*d*]imidazol -5-yl)oxy)phenyl)am ino)-7-methoxyquinazoli n-6-yl)-3-(1-methylpyrrolidin-2-yl)acrylamide | ¹H NMR (400 MHz, TFA salt, Methanol-d₄) δ8.93 (s, 1H), 8.55 (s, 1H), 8.29 (s, 1H), 8.01 (d, J = 12.0 Hz, 1H), 7.62 (d, J = 8.8 Hz, 1H), 7.28-7.26 (m, 2H), 7.21-7.18 (m, 1H), 6.88 (d, J = 2.8 Hz, 1H), 6.41-6.05 (m, 1H), 4.48-4.46 (m, 1H), 4.11 (s, 3H), 3.94 (s, 3H), 3.81-3.73 (m, 4H), 2.94 (s, 3H), 2.50-2.46 (m, 1H), 2.27-2.22 (m, 2H), 2.21-2.15 (m, 1H) ; MS (ESI): m/z 616 [M+H]⁺ | 3 | 6.81, 91 |
| 62 | 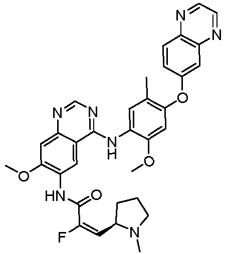 | (*R,E*)-2-fluoro-*N-*(7-methoxy-4-((2-methoxy-5-methyl-4-(quinoxalin-6-yloxy)phenyl)ami no)quinazolin-6-yl)-3-(1-methylpyrrolidin-2-yl)acrylamide | MS (ESI): m/z 610 [M+H]⁺ | | |
| 63 | 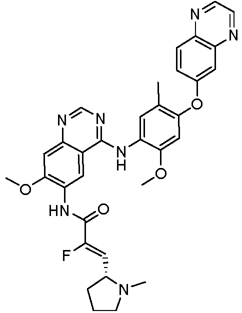 | (*R*,*Z*)-2-fluoro-*N-*(7-methoxy-4-((2-methoxy-5-methyl-4-(quinoxalin-6-yloxy)phenyl)ami no)quinazolin-6-yl)-3-(1-methylpyrrolidin-2-yl)acrylamide | ¹H NMR (400 MHz, FA salt, Methanol-d₄) δ8.99 (s, 1H), 8.82-8.78 (m, 2H), 8.48 (s, 1H), 8.15 (d, J = 8.8 Hz, 1H), 7.93 (s, 1H), 7.75-7.72 (m, 1H), 7.33-7.31 (m, 2H), 6.95 (s, 1H), 6.37-6.26 (m, 1H), 4.14 (s, 3H), 4.09-4.01 (m, 1H), 3.86 (s, 3H), 3.55-3.54 (m, 1H), 2.97-2.95 (m, 1H), 2.74 (s, 3H), 2.45-2.42 (m, 1H), 2.20 (s, 3H), 2.12-2.10 (m, 2H), 1.99-1.97 (m, 1H); MS (ESI): m/z 610 [M+H]⁺ | 12 | 8.17, 100 |
| 64 | 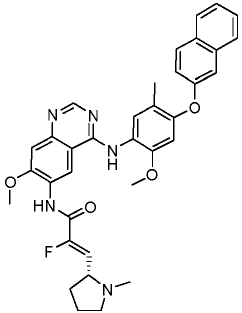 | (*R*,*Z*)-2-fluoro-*N-*(7-methoxy-4-((2-methoxy-5-methyl-4-(naphthalen-2-yloxy)phenyl)ami no)quinazolin-6-yl)-3-(1-methylpyrrolidin-2-yl)acrylamide | ¹H NMR (400MHz, TFA salt, Methanol-d₄) δ 9.14 (s, 1H), 8.66 (s, 1H), 7.91 (d, J = 8.8 Hz, 1H), 7.85 (d, J = 7.6 Hz, 1H), 7.71 (d, J = 8.4 Hz, 1H), 7.49 (s, 1H), 7.48-7.38 (m, 2H), 7.34 (s, 1H), 7.30 (d, J = 2.8 Hz, 1H), 7.21 (s, 1H), 6.80 (s, 1H), 6.40-6.29 (m, 1H), 4.48-4.47 (m, 1H), 4.18 (s, 3H), 3.81 (s, 1H), 3.75 (s, 3H), 2.97 (s, 3H), 2.51 (s, 1H), 2.89 (s, 1H). 2.26 (s, 3H), 2.03 (s, 1H); MS (ESI): m/z 608 [M+H]⁺ | 12 | 10.3, 94 |
| 65 | 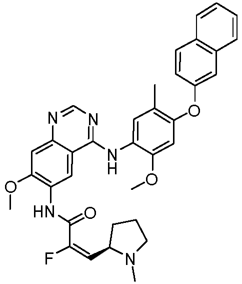 | (*R,E)*-2-fluoro-N-(7-methoxy-4-((2-methoxy-5-methyl-4-(naphthalen-2-yloxy)phenyl)ami no)quinazolin-6-yl)-3-(1-methylpyrrolidin-2-yl)acrylamide | ¹H NMR (400MHz, TFA salt, Methanol-d₄) δ 8.96 (s, 1H), 8.42 (s, 1H), 7.89 (d, J = 8.8 Hz, 1H), 7.84 (d, J = 8.0 Hz, 1H), 7.70 (s, 2H), 7.46-7.36 (m, 2H), 7.32-7.29 (m, 2H), 7.21 (s, 1H), 6.81 (s, 1H), 7.21 (s, 1H), 6.00-5.93 (m, 1H), 4.58-4.54 (m, 1H), 4.11 (s, 3H), 3.76 (s, 1H), 3.39 (s, 3H), 2.75 (s, 1H), 2.63 (s, 3H), 2.41-2.36 (m, 1H), 2.21 (s, 3H). 2.05-2.01 (m, 2H), 1.83 (s, 1H); MS (ESI): m/z 608 [M+H]⁺ | 17 | 9.9, 97 |
| 66 | 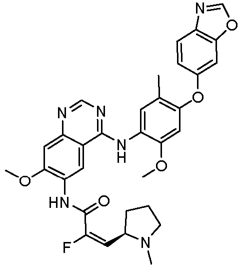 | (*R*,*E*)-*N*-(4-((4-(benzo[d]oxazol-6-yloxy)-2-methoxy-5-methylphenyl)am ino)-7-methoxyquinazoli n-6-yl)-2-fluoro-3-(1-methylpyrrolidin-2-yl)acrylamide | MS (ESI): m/z 599 [M+H]⁺ | | |
| 67 | 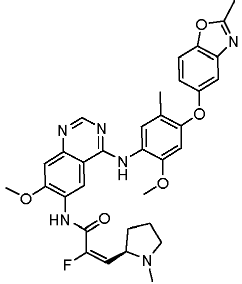 | (*R,E*)-2-fluoro*-N-*(7-methoxy-4-((2-methoxy-5-methyl-4-((2-methylbenzo[*d*]o xazol-5-yl)oxy)phenyl)am ino)quinazolin-6-yl)-3-(1-methylpyrrolidin-2-yl)acrylamide | ¹H NMR (400 MHz, free base, Methanol-d₄) δ 8.95 (s, 1H), 8.40 (s, 1H), 7.68 (s, 1H), 7.55 (d, J = 8.8 Hz, 1H), 7.25 (s, 1H), 7.14 (s, 1H), 7.07-7.04 (m, 1H), 6.76 (s, 1H), 6.25-6.14 (m, 1H), 4.10 (s, 3H), 3.73 (s, 3H), 3.40 (m, 1H), 2.63 (s, 3H), 2.45 (s, 3H), 2.31-2.21 (m, 2H), 2.17 (s, 3H), 1.93-1.71 (m, 4H); MS (ESI): m/z 613 [M+H]⁺ | 3 | |
| 68 | 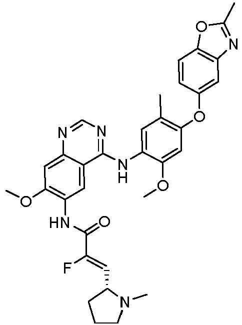 | (*R*,*Z*)-2-fluoro-*N-*(7-methoxy-4-((2-methoxy-5-methyl-4-((2-methylbenzo[*d*]o xazol-5-yl)oxy)phenyl)am ino)quinazolin-6-yl)-3-(1-methylpyrrolidin-2-yl)acrylamide | ¹H NMR (400 MHz, free base, Methanol-d₄) δ 8.93 (s, 1H), 8.40 (s, 1H), 7.75 (s, 1H), 7.55 (d, J = 8.8 Hz, 1H), 7.27 (s, 1H), 7.07-7.04 (m, 1H), 6.70 (s, 1H),6.25-6.14 (m, 1H), 4.11 (s, 3H), 3.75 (s, 3H), 3.40 (t, J = 8.4 Hz, 1H), 2.63 (s, 3H), 2.42-2.16 (m, 2H), 2.38 (s, 3H), 2.20 (s, 3H), 1.97-1.73 (m, 4H); MS (ESI): m/z 613 [M+H]⁺ | 4 | 8.56, 89 |
| 69 | 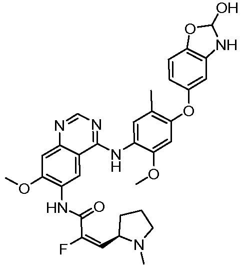 | (*E*)-2-fluoro-*N-*(4-((4-((2-hydroxy-2,3-dihydrobenzo[d]o xazol-5-yl)oxy)-2-methoxy-5-methylphenyl)am ino)-7-methoxyquinazoli n-6-yl)-3-((*R*)-1-methylpyrrolidin-2-yl)acrylamide | ¹H NMR (400 MHz, TFA salt, Methanol-d₄) δ 9.02 (s, 1H), 8.53 (s, 1H), 8.31 (s, 1H), 7.87 (d, J = 2.9 Hz, 1H), 7.45 (s, 1H), 7.31 (s, 1H), 6.86 (d, J = 8.7 Hz, 1H), 6.70-6.61 (m, 1H), 6.12-6.02 (m, 1H), 5.24-5.15 (m, 1H), 4.14 (s, 3H), 3.86-3.74 (m, 1H), 3.69 (s, 3H), 3.28-3.21 (m, 1H), 2.99 (s, 3H), 2.61-2.49 (m, 1H), 2.31-2.14 (m, 5H), 2.05-1.92 (m, 1H) ; MS (ESI): m/z 617 [M+H]⁺ | 5 | 7.93, 90 |
| 70 | 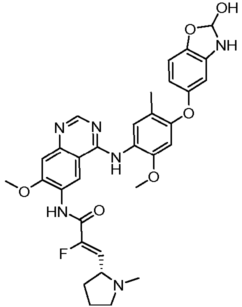 | (*Z*)-2-fluoro-*N-*(4-((4-((2-hydroxy-2,3-dihydrobenzo[*d*]o xazol-5-yl)oxy)-2-methoxy-5-methylphenyl)am ino)-7-methoxyquinazoli n-6-yl)-3-((*R*)-1-methylpyrrolidin-2-yl)acrylamide | ¹H NMR (400 MHz, FA salt, Methanol-d₄) δ 8.91 (s, 1H), 8.38 (d, J = 7.2 Hz, 2H), 8.30 (s, 1H), 7.83 (d, J = 2.9 Hz, 1H), 7.65 (s, 1H), 7.27 (s, 1H), 6.84 (d, J = 8.7 Hz, 1H), 6.68-6.59 (m, 2H), 6.23 (dd, J = 34.3, 9.6 Hz, 1H), 4.58 (s, 1H), 4.11 (s, 3H), 3.80-3.70 (m, 4H), 2.78-2.68 (m, 1H), 2.66 (s, 1H), 2.57 (s, 3H), 2.32-2.24 (m, 1H), 2.23 (s, 3H), 2.08-1.98 (m, 2H), 1.89-1.79 (m, 1H) ; MS (ESI): m/z 617 [M+H]⁺ | 1 | 7.85, 90 |
| 71 | 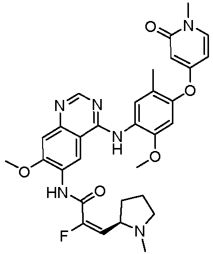 | (*R,E*)-2-fluoro-*N-*(7-methoxy-4-((2-methoxy-5-methyl-4-((1-methyl-2-oxo-1,2-dihydropyridin-4-yl)oxy)phenyl)am ino)quinazolin-6-yl)-3-(1-methylpyrrolidin-2-yl)acrylamide | ¹H NMR (400 MHz, TFA salt, Methanol-d₄) δ 9.17 (s, 1H), 8.67 (s, 1H), 7.70 (d, J = 7.5 Hz, 1H), 7.48 (s, 1H), 7.36 (s, 1H), 6.96 (s, 1H), 6.32 (dd, J = 7.5, 2.7 Hz, 1H), 6.09 (dd, J = 20.1, 9.8 Hz, 1H), 5.64 (d, J = 2.7 Hz, 1H), 5.24 - 5.15 (m, 1H), 4.18 (s, 3H), 3.85 - 3.78 (m, 4H), 3.27 - 3.22 (m, 1H), 3.00 (s, 3H), 2.60 - 2.51 (m, 1H), 2.31 - 2.18 (m, 2H), 2.15 (s, 3H), 2.04 - 1.94 (m, 1H); MS (ESI): m/z 589 [M+H]⁺ | 9 | 7.27, 100 |
| 72 | 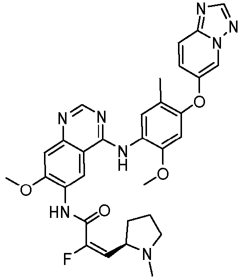 | (*R*,*E*)-*N*-(4-((4-([1,2,4]triazolo[1, 5-*a*]pyridin-6-yloxy)-2-methoxy-5-methylphenyl)am ino)-7-methoxyquinazoli n-6-yl)-2-fluoro-3-(1-methylpyrrolidin-2-yl)acrylamide | ¹H NMR (400MHz, TFA salt, Methanol-d₄) δ 8.97 (s, 1H), 8.44 (s, 1H), 8.39 (s, 1H), 8.35 (s, 1H), 7.80 (d, J = 9.6 Hz, 1H), 7.67 (s, 1H), 7.65 (s, 1H), 7.27 (s, 1H), 6.87 (s, 1H), 5.94-5.86 (m, 1H), 4.10 (m, 4H), 3.77 (s, 3H), 3.14-3.12 (m, 1H), 2.38 (s, 3H), 2.30 (s, 4H), 1.90 (s, 2H). 1.31 (s, 1H); MS (ESI): m/z 599 [M+H]⁺ | 15 | 7.51, 99 |
| 73 | 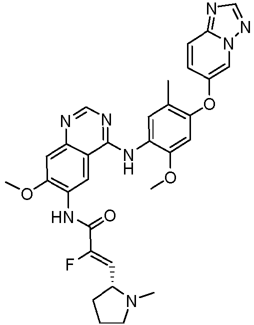 | (*R*,*E*)-*N*-(4-((4-([1,2,4]triazolo[1, 5-*a*]pyridin-6-yloxy)-2-methoxy-5-methylphenyl)am ino)-7-methoxyquinazoli n-6-yl)-2-fluoro-3-(1-methylpyrrolidin-2-yl)acrylamide | ¹H NMR (400MHz, TFA salt, Methanol-d₄) δ 9.15 (s, 1H), 8.67 (s, 1H), 8.46 (d, J = 2.1 Hz, 1H), 8.41 (s, 1H), 7.85 (d, J = 9.6 Hz, 1H), 7.67 (d, J = 9.7 Hz, 1H), 7.52 (s, 1H), 7.36 (s, 1H), 6.90 (s, 1H), 6.36 (m, 1H), 4.48 (s, 1H), 4.19 (s, 3H), 3.83 (s, 1H), 3.76 (s, 3H), 2.98 (s, 3H), 2.50 (s, 1H), 2.27 (s, 5H), 2.20 (s, 1H), 2.04 (s, 1H); MS (ESI): m/z 599 [M+H]⁺ | 10 | 7.60, 100 |
| 74 | 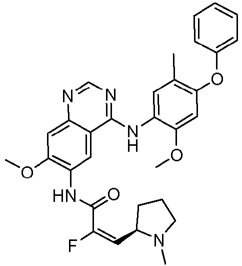 | (*R,E*)-2-fluoro-*N-*(7-methoxy-4-((2-methoxy-5-methyl-4-phenoxyphenyl)a mino)quinazolin-6-yl)-3-(1-methylpyrrolidin-2-yl)acrylamide | ¹H NMR (400 MHz, FA salt, Methanol-d₄) δ 8.94 (s, 1H), 8.39 (s, 1H), 7.65 (s, 1H), 7.36-7.31 (m, 2H), 7.28 (s, 1H), 7.07-7.04 (m, 1H), 6.98-6.95 (m, 2H), 6.71 (s, 1H), 5.99-5.91 (m, 1H), 4.49-4.47 (m, 1H), 4.11 (s, 3H), 3.75 (s, 3H), 3.38-3.35 (m, 1H), 2.74-2.67 (m, 1H), 2.59 (s, 3H), 2.41-2.32 (m, 1H), 2.17 (s, 3H), 2.05-1.97 (m, 2H), 1.81-1.75 (m, 1H); MS (ESI): 558 m/z [M+H]⁺ | 23 | 9.23, 99 |
| 75 | 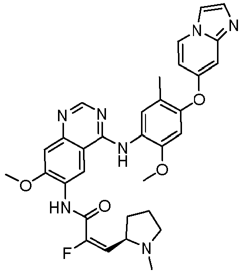 | (*R,E*)-2-fluoro-*N-*(4-((4-(imidazo[1,2-*a*]pyridin-7-yloxy)-2-methoxy-5-methylphenyl)am ino)-7-methoxyquinazoli n-6-yl)-3-(1-methylpyrrolidin-2-yl)acrylamide | MS (ESI): m/z 598 [M+H]⁺ | | |
| 76 | 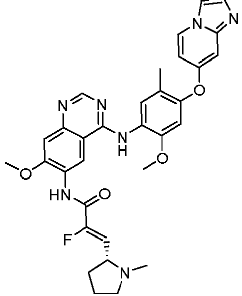 | (*R*,*Z*)-2-fluoro-*N-*(4-((4-(imidazo[1,2-*a*]pyridin-7-yloxy)-2-methoxy-5-methylphenyl)am ino)-7-methoxyquinazoli n-6-yl)-3-(1-methylpyrrolidin-2-yl)acrylamide | ¹H NMR (400 MHz, TFA salt, Methanol-d₄) δ8.98 (s, 1H), 8.46-8.43 (m, 2H), 7.91 (s, 1H), 7.75 (s, 1H), 7.46 (s, 1H), 7.31 (s, 1H), 6.91-6.86 (m, 2H), 6.73 (d, J = 2.4 Hz, 1H), 6.29-6.26 (m, 1H), 4.14 (s, 3H), 3.86 (s, 3H), 3.35-3.32 (m, 1H), 3.19-3.17 (m, 1H), 2.40-2.38 (m, 4H), 2.21-2.18 (m, 4H), 1.92-1.90 (m, 2H), 1.75-1.73 (m, 1H); MS (ESI): m/z 598 [M+H]⁺ | 4 | 6.39, 100 |
| 77 | 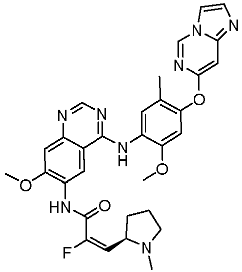 | (*R,E*)-2-fluoro-*N-*(4-((4-(imidazo[1,2-c]pyrimidin-7-yloxy)-2-methoxy-5-methylphenyl)am ino)-7-methoxyquinazoli n-6-yl)-3-(1-methylpyrrolidin-2-yl)acrylamide | MS (ESI): m/z 599 [M+H]⁺ | | |
| 78 | 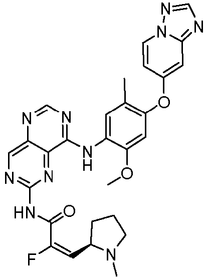 | (*R,E*)-*N*-(8-((4-([1,2,4]triazolo[1, 5-*a*]pyridin-7-yloxy)-2-methoxy-5-methylphenyl)am ino)pyrimido[5,4-*d*]pyrimidin-2-yl)-2-fluoro-3-(1-methylpyrrolidin-2-yl)acrylamide | MS (ESI): m/z 571 [M+H]⁺ | | |
| 79 | 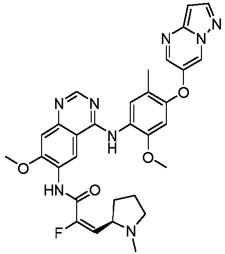 | (*R,E*)-2-fluoro-*N-*(7-methoxy-4-((2-methoxy-5-methyl-4-(pyrazolo[1,5-*a*]pyrimidin-6-yloxy)phenyl)ami no)quinazolin-6-yl)-3-(1-methylpyrrolidin-2-yl)acrylamide | ¹H NMR (400 MHz, TFA salt, Methanol-d₄) δ9.13 (s, 1H), 8.61-8.58 (m, 3H), 8.11 (d, J = 2.4 Hz, 1H), 7.52 (s, 1H), 7.36 (s, 1H), 6.90 (s, 1H), 6.75-6.74 (m, 1H), 6.14-6.07 (m, 1H), 5.20 (s, 1H), 4.13 (s, 3H), 3.78 (s, 3H), 2.98 (s, 3H), 2.57-2.52 (m, 1H), 2.27-2.13 (m, 8H), 2.03-1.97 (m, 1H) ; MS (ESI): m/z 599 [M+H]⁺ | 20 | 7.91, 100 |
| 80 | 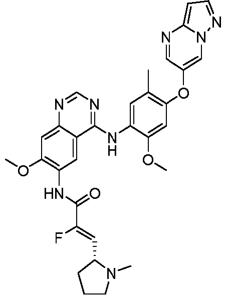 | (*R*,*Z*)-2-fluoro-*N-*(7-methoxy-4-((2-methoxy-5-methyl-4-(pyrazolo[1,5-*a*]pyrimidin-6-yloxy)phenyl)ami no)quinazolin-6-yl)-3-(1-methylpyrrolidin-2-yl)acrylamide | ¹H NMR (400 MHz, TFA salt, Methanol-d₄) δ9.07 (s, 1H), 8.60-8.58 (m, 3H), 8.11 (d, J = 2.8 Hz, 1H), 7.57 (s, 1H), 7.34 (s, 1H), 6.90 (s, 1H), 6.75-6.74 (m, 1H), 6.41-6.31 (m, 1H), 4.48 (s, 1H), 4.16 (s, 3H), 3.75 (s, 3H), 2.96 (s, 3H), 2.52-2.47 (m, 1H), 2.29-2.18 (m, 10H), 2.07-2.02 (m, 1H) ; MS (ESI): m/z 599 [M+H]⁺ | 6 | 7.82, 100 |
| 81 | 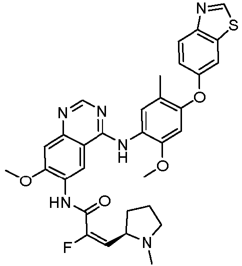 | (*R*,*E*)-*N*-(4-((4-(benzo[*d*]thiazol-6-yloxy)-2-methoxy-5-methylphenyl)am ino)-7-methoxyquinazoli n-6-yl)-2-fluoro-3-(1-methylpyrrolidin-2-yl)acrylamide | ¹H NMR (400 MHz, TFA salt, Methanol-d₆) δ 9.19 (s, 1H), 9.14 (s, 1H), 8.65 (s, 1H), 8.05 (d, J = 8.92 Hz, 1H), 7.57 (d, J = 2.43 Hz, 1H), 7.45 (s, 1H), 7.39 (s, 1H), 7.28 (dd, J = 8.92, 2.48 Hz 1H), 6.80 (s, 1H), 6.12 (dd, J = 20.2, 9.79 Hz 1H), 5.23-5.16 (m, 1H), 4.18 (s, 3H), 3.86-3.78 (m, 1H), 3.72 (s, 3H), 3.30-3.23 (m, 1H), 2.99 (s, 3H), 2.60-2.52 (m, 1H), 2.30-2.15 (m, 5H), 2.06-1.95 (m, 1H); MS (ESI): m/z 615 [M+H]⁺ | 5 | 8.59, 99 |
| 82 | 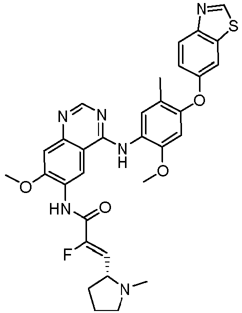 | *(R,Z)-N-(4-((4-*(benzo[*d*]thiazol-6-yloxy)-2-methoxy-5-methylphenyl)am ino)-7-methoxyquinazoli n-6-yl)-2-fluoro-3-(1-methylpyrrolidin-2-yl)acrylamide | ¹H NMR (400 MHz, TFA salt, Methanol-d₆) δ 9.15 (d, J = 3.55 Hz, 2H), 8.67 (s, 1H), 8.06 (d, J = 8.91 Hz, 1H), 7.59 (d, J = 2.43 Hz, 1H), 7.49 (s, 1H), 7.35 (s, 1H), 7.28 (dd, J = 8.94, 2.44 Hz 1H), 6.81 (s, 1H), 6.36 (dd, J = 35, 9.47 Hz 1H), 4.53-4.45 (m, 1H), 4.19 (s, 3H), 3.86-3.78 (m, 1H), 3.73 (s, 3H), 3.29-3.22 (m, 1H), 2.98 (s, 3H), 2.56-2.45 (m, 1H), 2.35-2.14 (m, 5H), 2.10-1.98 (m, 1H); MS (ESI): m/z 615 [M+H]⁺ | 5 | 8.57, 99 |
| 83 | 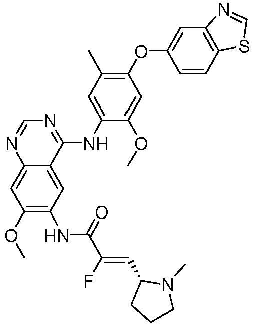 | (*R,Z*)-*N-*(4-((4-(benzo[*d*]thiazol-5-yloxy)-2-methoxy-5-methylphenyl)am ino)-7-methoxyquinazoli n-6-yl)-2-fluoro-3-(1-methylpyrrolidin-2-yl)acrylamide | ¹H NMR (400 MHz, free base, Methanol-d₄) δ 9.20 (s, 1H), 8.90 (s, 1H), 8.37 (s, 1H), 8.01 (d, J = 8.8 Hz, 1H), 7.70 (s, 1H), 7.49 (s, 1H), 7.23 (s, 2H), 6.76 (s, 1H), 6.20-6.11 (m, 1H), 4.07 (s, 3H), 3.73 (s, 3H), 3.48 (s, 1H), 2.43-2.35 (m, 2H), 2.16 (s, 3H), 1.91 (s, 3H), 1.67-1.25 (m, 4H); MS (ESI): m/z 615 [M+H]⁺ | 1 | 8.75, 78 |
| 84 | 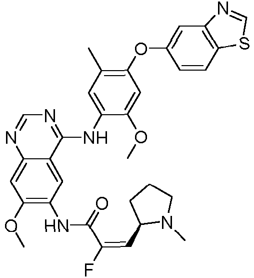 | (*R*,*E*)-*N*-(4-((4-(benzo[*d*]thiazol-5-yloxy)-2-methoxy-5-methylphenyl)am ino)-7-methoxyquinazoli n-6-yl)-2-fluoro-3-(1-methylpyrrolidin-2-yl)acrylamide | ¹H NMR (400 MHz, free base, Methanol-d₄) δ 9.23 (s, 1H), 8.93 (s, 1H), 8.39 (s, 1H), 8.03 (d, J = 8.8 Hz, 1H), 7.76 (s, 1H), 7.53 (d, J = 2.4 Hz, 1H), 7.26-7.24 (m, 1H), 7.21 (s, 1H), 6.77 (s, 1H), 5.95-5.87 (m, 1H), 4.08 (s, 3H), 3.76 (s, 3H), 3.18 (t, J = 2.4 Hz, 1H), 2.41-2.26 (m, 2H), 2.37 (s, 3H), 2.18 (s, 3H), 1.93-1.68 (m, 4H); MS (ESI): m/z 615 [M+H]⁺ | 11 | 8.55, 89 |
| 85 | 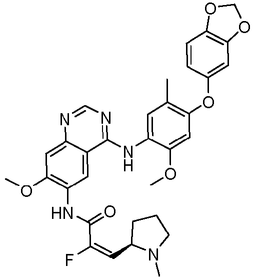 | (*R*,*E*)-*N*-(4-((4-(benzo[d][1,3]dio xol-5-yloxy)-2-methoxy-5-methylphenyl)am ino)-7-methoxyquinazoli n-6-yl)-2-fluoro-3-(1-methylpyrrolidin-2-yl)acrylamide | ¹H NMR (400 MHz, TFA salt, Methanol-d₆) δ 9.14 (s, 1H), 8.63 (s, 1H), 7.37 (s, 1H), 7.34 (s, 1H), 6.81 (d, J = 8.44 Hz, 1H), 6.64 (s, 1H), 6.57 (d, J = 2.40 Hz, 1H), 6.45 (dd, J = 8.42, 2.40 Hz, 1H), 6.09 (dd, J = 20.2, 9.87 Hz, 1H), 5.97 (s, 2H), 5.22-5.15 (m, 1H), 4.17 (s, 3H), 3.84-3.78 (m, 1H), 3.69 (s, 3H), 3.28-3.22 (m, 1H), 2.99 (s, 3H), 2.60-2.52 (m, 1H), 2.31-2.16 (m, 5H), 2.09-1.94 (m, 1H); MS (ESI): m/z 602 [M+H]⁺ | 13 | 8.95, 96 |
| 86 | 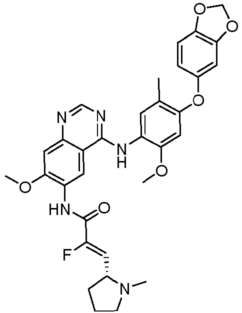 | (*R*,*E*)-*N*-(4-((4-(benzo[*d*][1,3]dio xol-5-yloxy)-2-methoxy-5-methylphenyl)am ino)-7-methoxyquinazoli n-6-yl)-2-fluoro-3-(1-methylpyrrolidin-2-yl)acrylamide | ¹H NMR (400 MHz, TFA salt, Methanol-d₆) δ 9.12 (s, 1H), 8.63 (s, 1H), 7.40 (s, 1H), 7.34 (s, 1H), 6.81 (d, J = 8.43 Hz, 1H), 6.63 (s, 1H), 6.58 (d, J = 2.35 Hz, 1H), 6.45 (dd, J = 8.42, 2.44 Hz, 1H), 6.35 (dd, J = 32.4, 9.67 Hz, 1H), 5.97 (s, 2H), 4.52-4.45 (m, 1H), 4.18 (s, 3H), 3.85-3.78 (m, 1H), 3.70 (s, 3H), 3.29-3.24 (m, 1H), 2.97 (s, 3H), 2.56-2.46 (m, 1H), 2.33-2.13 (m, 5H), 2.09-1.98 (m, 1H); MS (ESI): m/z 602 [M+H]⁺ | 10 | 8.91, 97 |
| 87 | 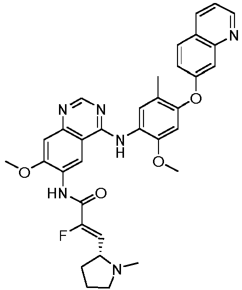 | (*R*,*Z*)-2-fluoro-*N-*(7-methoxy-4-((2-methoxy-5-methyl-4-(quinolin-7-yloxy)phenyl)ami no)quinazolin-6-yl)-3-(1-methylpyrrolidin-2-yl)acrylamide | ¹H NMR (400 MHz, free, Methanol-d₄) δ 8.97 (s, 1H), 8.76 (dd, J = 4.4, 1.6 Hz, 1H), 8.45 (s, 1H), 8.34 (d, J = 7.4 Hz, 1H), 7.98 (d, J = 9.0 Hz, 1H), 7.91 (s, 1H), 7.50-7.40 (m, 2H), 7.29 (d, J = 3.0 Hz, 2H), 6.90 (s, 1H), 6.20 (dd, J = 35.4, 9.3 Hz, 1H), 4.58 (s, 1H), 4.12 (s, 3H), 3.83 (s, 3H), 3.19-3.11 (m, 1H), 2.42-2.31 (m, 4H), 2.22-2.14 (m, 4H), 1.97-1.89 (m, 2H), 1.77-1.66 (m, 1H) ; MS (ESI): m/z 609 [M+H]⁺ | 10 | 7.15, 97 |
| 88 | 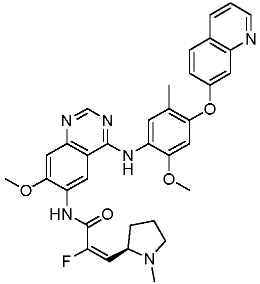 | (R,E)-2-fluoro-N-(7-methoxy-4-((2-methoxy-5-methyl-4-(quinolin-7-yloxy)phenyl)ami no)quinazolin-6-yl)-3-(1-methylpyrrolidin-2-yl)acrylamide | ¹H NMR (400 MHz, free, Methanol-d₄) δ 8.99 (s, 1H), 8.76 (dd, J = 4.4, 1.6 Hz, 1H), 8.44 (s, 1H), 8.35 (d, J = 8.1 Hz, 1H), 7.99 (d, J = 9.1 Hz, 1H), 7.85 (s, 1H), 7.51-7.39 (m, 2H), 7.29 (s, 2H), 6.90 (s, 1H), 5.91 (dd, J = 23.7, 9.2 Hz, 1H), 4.64-4.54 (m, 2H), 4.12 (s, 3H), 4.08-4.00 (m, 1H), 3.82 (s, 3H), 2.40-2.31 (m, 4H), 2.31-2.21 (m, 1H), 2.19 (s, 3H), 1.95-1.84 (m, 2H), 1.72-1.59 (m, 1H) ; MS (ESI): m/z 609 [M+H]⁺ | 10 | 7.22, 99 |
| 89 | 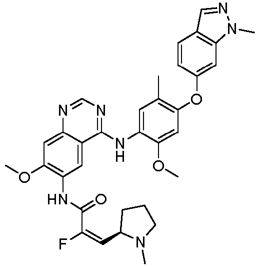 | (*R,E*)-2-fluoro-*N-*(7-methoxy-4-((2-methoxy-5-methyl-4-((1-methyl-1*H-*indazol-6-yl)oxy)phenyl)am ino)quinazolin-6-yl)-3-(1-methylpyrrolidin-2-yl)acrylamide | ¹H NMR (400 MHz, TFA salt, DMSO-d₆) δ10.43 (s, 1H), 10.17-10.02 (m, 2H), 8.73 (s, 1H), 8.60 (s, 1H), 8.00 (s, 1H), 7.77 (d, J = 8.8 Hz, 1H), 7.44 (s, 1H), 7.36 (s, 1H), 7.04 (s, 1H), 6.87-6.79 (m, 2H), 6.19-6.12 (m, 1H), 4.93-4.91 (m, 1H), 4.01 (s, 3H), 3.95 (s, 3H), 3.66 (s, 3H), 2.83 (s, 3H), 2.38-2.32 (m, 1H), 2.15-2.00 (m, 10H), 1.95-1.83 (m, 1H) ; MS (ESI): m/z 612 [M+H]⁺ | 27 | 8.60, 100 |
| 90 | 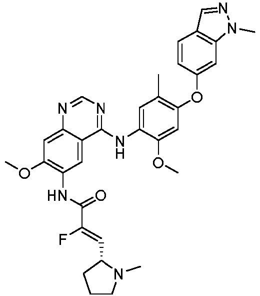 | (*R*,*Z*)-2-fluoro-*N-*(7-methoxy-4-((2-methoxy-5-methyl-4-((1-methyl-1*H-*indazol-6-yl)oxy)phenyl)am ino)quinazolin-6-yl)-3-(1-methylpyrrolidin-2-yl)acrylamide | ¹H NMR (400 MHz, TFA salt, DMSO-d₆) δ10.58 (s, 1H), 10.14 (s, 1H), 8.83 (s, 1H), 8.69 (s, 1H), 8.01 (s, 1H), 7.78 (d, J = 8.8 Hz, 1H), 7.43 (d, J = 6.0 Hz, 1H), 7.07 (s, 1H), 6.87-6.79 (m, 2H), 6.37-6.26 (m, 1H), 4.41-4.39 (m, 1H), 4.03 (s, 3H), 3.95 (s, 3H), 3.66 (s, 3H), 2.83 (s, 3H), 2.37-2.32 (m, 1H), 2.15-2.00 (m, 10H), 1.95-1.86 (m, 1H) ; MS (ESI): m/z 612 [M+H]⁺ | 23 | 8.62, 100 |
| 91 | 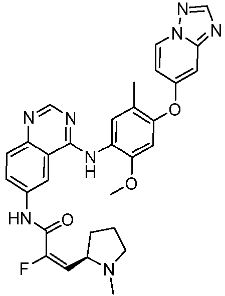 | (*R*,*E*)-*N*-(4-((4-([1,2,4]triazolo[1, 5-*a*]pyridin-7-yloxy)-2-methoxy-5-methylphenyl)am ino)quinazolin-6-yl)-2-fluoro-3-(1-methylpyrrolidin-2-yl)acrylamide | ¹H NMR (400 MHz, TFA salt, Methanol-d₄) δ8.77 (d, J = 7.6 Hz, 1H), 8.72 (s, 1H), 8.51 (s, 1H), 8.31 (s, 1H), 8.06-8.03 (m, 1H), 7.97 (s, 1H), 7.84 (d, J = 9.2 Hz, 1H), 7.13-7.10 (m, 1H), 6.97-6.94 (m, 2H), 5.93-5.84 (m, 1H), 4.07-4.05 (m, 1H), 3.89 (s, 3H), 3.17-3.15 (m, 1H), 2.40-2.37 (m, 5H), 2.20 (s, 3H), 1.94-1.90 (m, 2H), 1.73-1.70 (m, 1H); MS (ESI): m/z 569 [M+H]⁺ | 25 | 7.19, 100 |
| 92 | 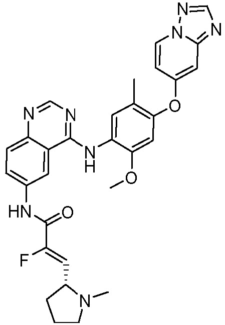 | (*R*,*E*)-*N*-(4-((4-([1,2,4]triazolo[1, 5-*a*]pyridin-7-yloxy)-2-methoxy-5-methylphenyl)am ino)quinazolin-6-yl)-2-fluoro-3-(1-methylpyrrolidin-2-yl)acrylamide | ¹H NMR (400 MHz, TFA salt, Methanol-d₆) δ 9.03 (d, J = 2.09 Hz, 1H), 8.82 (d, J = 7.48 Hz, 1H), 8.74 (s, 1H), 8.39 (s, 1H), 8.27 (dd, J = 9.06, 2.14 Hz 1H), 7.90 (d, J = 9.04 Hz, 1H), 7.65 (s, 1H), 7.15 (dd, J = 7.44, 1.92 Hz, 1H), 7.05 (s, 1H), 6.89 (s, 1H), 6.38 (dd, J = 32, 9.96 Hz 1H), 4.52-4.44 (m, 1H), 3.87-3.78 (m, 4H), 2.97 (s, 3H), 2.55-2.48 (m, 1H), 2.33-2.14 (m, 5H), 2.10-1.99 (m, 1H); M S(ESI): m/z 569 [M+H]⁺ | 2 | 7.35, 97 |
| 93 | 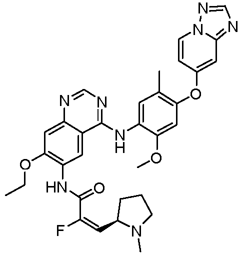 | (*R*,*E*)-*N*-(4-((4-([1,2,4]triazolo[1, 5-*a*]pyridin-7-yloxy)-2-methoxy-5-methylphenyl)am ino)-7-ethoxyquinazolin -6-yl)-2-fluoro-3-(1-methylpyrrolidin-2-yl)acrylamide | ¹H NMR (400 MHz, Methanol-d₄) δ 8.99 (s, 1H), 8.75 (d, J = 7.5 Hz, 1H), 8.42 (s, 1H), 8.29 (s, 1H), 7.83 (s, 1H), 7.26 (s, 1H), 7.10 (dd, J = 7.5, 2.6 Hz, 1H), 6.95 (s, 2H), 5.91 (dd, J = 23.6, 9.3 Hz, 1H), 4.36 (q, J = 7.0 Hz, 2H), 4.09 (dd, J = 16.9, 7.9 Hz, 1H), 3.85 (s, 3H), 3.17-3.12 (m, 1H), 2.43-2.32 (m, 4H), 2.30-2.22 (m, 1H), 2.18 (s, 3H), 1.97-1.86 (m, 2H), 1.74-1.63 (m, 1H), 1.59-1.53 (m, 2H) ; MS (ESI) : m/z 613 [M+H]⁺ | 36 | 7.61, 94 |
| 94 | 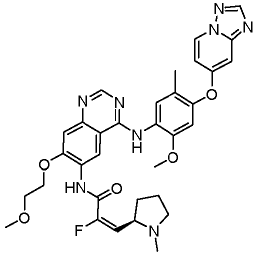 | (*R*,*E*)-*N*-(4-((4-([1,2,4]triazolo[1, 5-*a*]pyridin-7-yloxy)-2-methoxy-5-methylphenyl)am ino)-7-(2-methoxyethoxy)q uinazolin-6-yl)-2-fluoro-3-(1-methylpyrrolidin-2-yl)acrylamide | ¹H NMR (400 MHz, free, Methanol-d₄) δ 9.00 (s, 1H), 8.75 (d, J = 7.5 Hz, 1H), 8.43 (s, 1H), 8.29 (s, 1H), 7.82 (s, 1H), 7.30 (s, 1H), 7.10 (dd, J = 7.5, 2.6 Hz, 1H), 6.95 (s, 2H), 5.90 (dd, J = 23.5, 9.3 Hz, 1H), 4.46-4.39 (m, 2H), 4.08 (dd, J = 17.7, 8.8 Hz, 1H), 3.92-3.87 (m, 2H), 3.84 (s, 3H), 3.49 (s, 3H), 3.18-3.09 (m, 1H), 2.40-2.32 (m, 4H), 2.31-2.21 (m, 1H), 2.18 (s, 3H), 1.98-1.85 (m, 2H), 1.74-1.60 (m, 1H) ; MS (ESI) : m/z 643 [M+H]⁺ | 19 | 7.52, 96 |
| 95 | 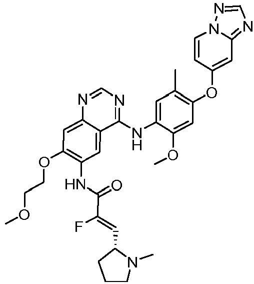 | (*R*,*E*)-*N*-(4-((4-([1,2,4]triazolo[1, 5-*a*]pyridin-7-yloxy)-2-methoxy-5-methylphenyl)am ino)-7-(2-methoxyethoxy)q uinazolin-6-yl)-2-fluoro-3-(1-methylpyrrolidin-2-yl)acrylamide | ¹H NMR (400 MHz, FA salt, Methanol-d₄) δ 9.00 (s, 1H), 8.74 (d, J = 7.4 Hz, 1H), 8.55 (s, 1H), 8.44 (s, 1H), 8.29 (s, 1H), 7.89 (s, 1H), 7.31 (s, 1H), 7.10 (dd, J = 7.5, 2.5 Hz, 1H), 6.94 (d, J = 3.3 Hz, 2H), 6.21 (dd, J = 35.4, 9.4 Hz, 1H), 4.66-4.54 (m, 1H), 4.48-4.39 (m, 2H), 3.93-3.88 (m, 2H), 3.86 (s, 3H), 3.49 (s, 3H), 3.20-3.10 (m, 1H), 2.41-2.33 (m, 4H), 2.22-2.13 (m, 4H), 1.97-1.87 (m, 2H), 1.80-1.67 (m, 1H) ; MS (ESI) : m/z 643 [M+H]⁺ | 3 | 7.51, 98 |
| 96 | 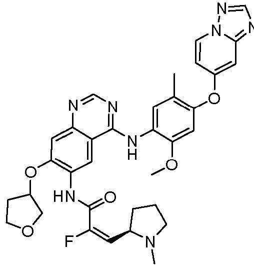 | (*E*)-*N*-(4-((4-([1,2,4]triazolo[1, 5-*a*]pyridin-7-yloxy)-2-methoxy-5-methylphenyl)am ino)-7-((tetrahydrofuran-3-yl)oxy)quinazolin -6-yl)-2-fluoro-3-((*R*)-1-methylpyrrolidin-2-yl)acrylamide | ¹H NMR (400 MHz, FA salt, Methanol-d₆) δ 8.90 (d, J = 4.79 Hz, 1H), 8.74 (d, J = 7.50 Hz, 1H), 8.45 (s, 1H), 8.31 (s, 1H), 8.29 (s, 1H), 7.82 (s, 1H), 7.24 (s, 1H), 7.10 (dd, J = 13.5, 2.59 Hz 1H), 6.95 (s, 1H), 6.91 (d, J = 2.44 Hz, 1H), 6.10 (dd, J = 20.8, 9.78 Hz, 1H), 5.34-5.32 (m, 1H), 5.15-5.08 (m, 1H), 4.10-4.01 (m, 3H), 3.95-3.90 (m, 1H), 3.84 (s, 3H), 3.72-3.66 (m, 1H), 3.24-3.16 (m, 1H), 2.91 (sm 3H), 2.56-2.39 (m, 2H), 2.28-2.11 (m, 6H), 2.03-1.93 (m, 1H); MS (ESI): m/z 655 [M+H]⁺ | 7 | 7.84, 99 |
| 97 | 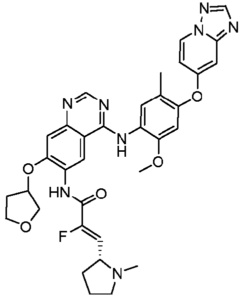 | (*Z*)-*N*-(4-((4-([1,2,4]triazolo[1, 5-*a*]pyridin-7-yloxy)-2-methoxy-5-methylphenyl)am ino)-7-((tetrahydrofuran-3-yl)oxy)quinazolin -6-yl)-2-fluoro-3-((*R*)-1-methylpyrrolidin-2-yl)acrylamide | ¹H NMR (400 MHz, FA salt, Methanol-d₆) δ 8.96 (s, 1H), 8.74 (d, J = 7.48 Hz, 1H), 8.45 (s, 1H), 8.29 (s, 1H), 7.89 (s, 1H), 7.25 (s, 1H), 7.10 (dd, J = 7.50, 2.60 Hz 1H), 6.95 (s, 1H), 6.93 (d, J = 2.48 Hz, 1H), 6.23 (dd, J = 34.8, 9.51 Hz, 1H), 5.36-5.34 (m, 1H), 4.61-4.55 (m, 1H), 4.12-4.02 (m, 1H), 3.97-3.91 (m, 1H), 3.85 (s, 3H), 3.64-3.57 (m, 1H), 3.29-3.26 (m, 1H), 2.62-2.55 (m, 1H), 2.50 (s, 1H), 2.48-2.42 (m, 1H), 2.29-2.21 (m, 1H), 2.18 (s, 3H), 2.03-1.96 (m, 2H), 1.84-1.75 (m, 1H); MS (ESI): m/z 655 [M+H]⁺ | 2 | 7.82, 98 |
| 98 | 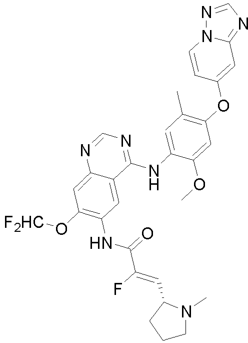 | (*R*,*Z*)-*N*-(4-((4-([1,2,4]triazolo[1, 5-*a*]pyridin-7-yloxy)-2-methoxy-5-methylphenyl)am ino)-7-(difluoromethoxy )quinazolin-6-yl)-2-fluoro-3-(1-methylpyrrolidin-2-yl)acrylamide | ¹H NMR (400 MHz, free, Methanol-d₄) δ 8.88 (s, 1H), 8.74 (d, J = 7.5 Hz, 1H), 8.48 (s, 1H), 8.29 (s, 1H), 7.83 (s, 1H), 7.56 (s, 1H), 7.18 (t, J = 72.4 Hz, 1H), 7.09 (dd, J = 7.5, 2.6 Hz, 1H), 6.97-6.89 (m, 2H), 6.20 (dd, J = 35.2, 9.4 Hz, 1H), 3.84 (s, 3H), 3.40-3.28 (m, 1H), 3.20-3.07 (m, 1H), 2.41-2.33 (m, 4H), 2.24-2.13 (m, 4H), 1.98-1.88 (m, 2H), 1.77-1.66 (m, 1H) ; MS (ESI) : m/z 635 [M+H]⁺ | 2 | 7.80, 96 |
| 99 | 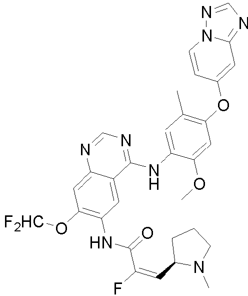 | (*R*,*E*)-*N*-(4-((4-([1,2,4]triazolo[1, 5-*a*]pyridin-7-yloxy)-2-methoxy-5-methylphenyl)am ino)-7-(difluoromethoxy )quinazolin-6-yl)-2-fluoro-3-(1-methylpyrrolidin-2-yl)acrylamide | ¹H NMR (400 MHz, free, Methanol-d₄) δ 8.87 (s, 1H), 8.75 (d, J = 7.5 Hz, 1H), 8.48 (s, 1H), 8.29 (s, 1H), 7.77 (s, 1H), 7.57 (s, 1H), 7.18 (t, J = 72.5 Hz, 1H), 7.10 (dd, J = 7.5, 2.6 Hz, 1H), 6.98-6.91 (m, 2H), 5.92 (dd, J = 23.4, 9.3 Hz, 1H), 4.58 (s, 1H), 4.00 (dd, J = 16.1, 7.6 Hz, 1H), 3.83 (s, 3H), 3.16-3.09 (m, 1H), 2.43-2.31 (m, 4H), 2.29-2.17 (m, 4H), 1.97-1.85 (m, 2H), 1.74-1.62 (m, 1H) ; MS (ESI) : m/z 635 [M+H]⁺ | 12 | 7.86, 95 |
| 100 | 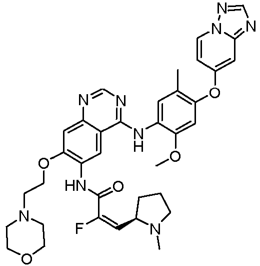 | (*R*,*E*)-*N*-(4-((4-([1,2,4]triazolo[1, 5-*a*]pyridin-7-yloxy)-2-methoxy-5-methylphenyl)am ino)-7-(2-morpholinoethox y)quinazolin-6-yl)-2-fluoro-3-(1-methylpyrrolidin-2-yl)acrylamide | ¹H NMR (400 MHz, free base, Methanol-d₄) δ 8.96 (s, 1H), 8.73 (d, J = 7.6 Hz, 1H), 8.44 (s, 1H), 8.27 (s, 1H), 7.89 (s, 1H), 7.28 (s, 1H), 7.08-7.06 (m, 1H), 6.93-6.92 (m, 2H), 4.43 (t, J = 4.8 Hz, 2H), 3.85 (s, 3H), 3.74 (t, J = 4.4 Hz, 4H), 3.24-3.14 (m, 1H), 2.95-2.93 (m, 2H), 2.65-2.63 (m, 4H), 2.46 (s, 3H), 2.34-2.20 (m, 2H), 2.17 (s, 3H), 1.99-1.69 (m, 4H); MS (ESI): m/z 698 [M+H]⁺ | 23 | 70(NMR ) |
| 101 | 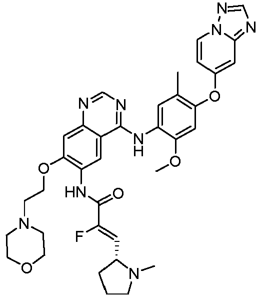 | (*R*,*Z*)-*N*-(4-((4-([1,2,4]triazolo[1, 5-*a*]pyridin-7-yloxy)-2-methoxy-5-methylphenyl)am ino)-7-(2-morpholinoethox y)quinazolin-6-yl)-2-fluoro-3-(1-methylpyrrolidin-2-yl)acrylamide | ¹H NMR (400 MHz, free base, Methanol-d₄) δ 8.95 (s, 1H), 8.75 (d, J = 7.6 Hz, 1H), 8.44 (s, 1H), 8.29 (s, 1H), 7.89 (s, 1H), 7.30 (s, 1H), 7.11-7.08 (m, 1H), 6.95-6.93 (m, 2H), 6.27-6.15 (m, 1H), 4.45 (t, J = 4.8 Hz, 2H), 3.85 (s, 3H), 3.75 (t, J = 4.4 Hz, 4H), 3.40-3.35 (m, 1H), 2.97 (t, J = 5.2 Hz, 2H), 2.67 (t, J = 9.2 Hz, 4H), 2.42-2.40 (m, 2H), 2.39 (s, 3H), 2.18 (s, 3H), 1.94-1.92 (m, 4H); MS (ESI): m/z 698 [M+H]⁺ | 21 | 7.02, 98 |
| 102 | 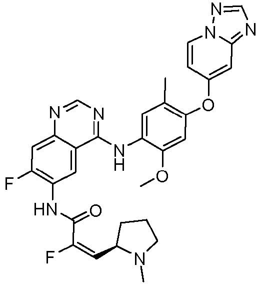 | (*R*,*E*)-*N*-(4-((4-([1,2,4]triazolo[1, 5-*a*]pyridin-7-yloxy)-2-methoxy-5-methylphenyl)am ino)-7-fluoroquinazolin-6-yl)-2-fluoro-3-(1-methylpyrrolidin-2-yl)acrylamide | ¹H NMR (400 MHz, TFA salt, Methanol-d₄) δ8.85 (d, J = 7.6 Hz, 1H), 8.77 (d, J = 7.6 Hz, 1H), 8.56 (s, 1H), 8.30 (s, 1H), 7.67 (s, 1H), 7.64 (d, J = 10.8 Hz, 1H), 7.12-7.09 (m, 1H), 6.98 (s, 1H), 6.89 (d, J = 4.8 Hz, 1H), 6.14-6.07 (m, 1H), 5.19 (s, 1H), 3.81-3.79 (m, 4H), 2.97 (s, 3H), 2.56-2.51 (m, 1H), 2.27-2.15 (m, 8H), 2.03-1.97 (m, 1H) ; MS (ESI): m/z 587 [M+H]⁺ | 22 | 7.43, 100 |
| 103 | 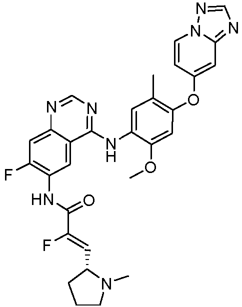 | (*R*,*Z*)-*N*-(4-((4-([1,2,4]triazolo[1, 5-*a*]pyridin-7-yloxy)-2-methoxy-5-methylphenyl)am ino)-7-fluoroquinazolin-6-yl)-2-fluoro-3-(1-methylpyrrolidin-2-yl)acrylamide | ¹H NMR (400 MHz, TFA salt, Methanol-d₄) δ8.78-8.73 (m, 2H), 8.47 (s, 1H), 8.29 (s, 1H), 7.78 (s, 1H), 7.57 (d, J = 11.2 Hz, 1H), 7.11-7.08 (m, 1H), 6.95-6.92 (m, 2H), 6.25-6.14 (m, 1H), 5.35-5.33 (m, 1H), 3.82 (s, 3H), 3.47-3.45 (m, 1H), 3.21-3.18 (m, 1H), 2.47-2.42 (m, 4H), 2.23-2.17 (m, 5H), 2.03-1.97 (m, 1H) ; MS (ESI): m/z 587 [M+H]⁺ | 8 | 7.23, 100 |
| 104 | 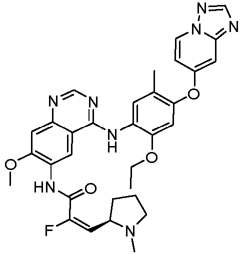 | (*R*,*E*)-*N*-(4-((4-([1,2,4]triazolo[1, 5-*a*]pyridin-7-yloxy)-2-ethoxy-5-methylphenyl)am ino)-7-methoxyquinazoli n-6-yl)-2-fluoro-3-(1-methylpyrrolidin-2-yl)acrylamide | ¹H NMR (400 MHz, free base, Methanol-d₄) δ 9.22 (s, 1H), 8.86 (d, J = 7.4 Hz, 1H), 8.73 (s, 1H), 8.51 (s, 1H), 7.66 (s, 1H), 7.37 (s, 1H), 7.21 (dd, J = 7.5, 2.4 Hz, 1H), 7.04 (s, 1H), 6.93 (d, J = 2.4 Hz, 1H), 6.15 - 6.05 (m, 1H), 5.23 - 5.14 (m, 1H), 4.20 (s, 3H), 4.10 - 4.06 (m, 2H), 3.85 - 3.78 (m, 1H), 3.00 (s, 3H), 2.60 - 2.53 (m, 1H), 2.30 - 2.21 (m, 2H), 2.20 (s, 3H), 2.05 - 2.00 (m, 1H), 1.33 - 1.29 (m, 3H); MS (ESI): m/z 613 [M+H]⁺ | 8 | 7.84, 99 |
| 105 | 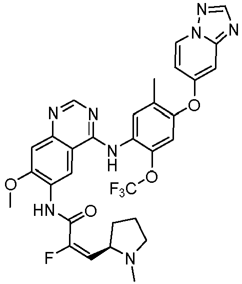 | (*R*,*E*)-*N*-(4-((4-([1,2,4]triazolo[1, 5-*a*]pyridin-7-yloxy)-5-methyl-2-(trifluoromethoxy )phenyl)amino)-7-methoxyquinazoli n-6-yl)-2-fluoro-3-(1-methylpyrrolidin-2-yl)acrylamide | ¹H NMR (400 MHz, free, Methanol-d₄) δ 9.04 (s, 1H), 8.75 (d, J = 7.5 Hz, 1H), 8.49 (s, 1H), 8.29 (s, 1H), 7.92 (s, 1H), 7.29 (s, 1H), 7.08 (dd, J = 7.5, 2.6 Hz, 1H), 6.81 (d, J = 2.5 Hz, 1H), 5.96 (dd, J = 22.4, 9.4 Hz, 1H), 4.59 (s, 1H), 4.47 (dd, J = 16.6, 8.1 Hz, 1H), 4.11 (s, 3H), 3.38-3.32 (m, 1H), 2.74-2.65 (m, 1H), 2.60 (s, 3H), 2.41-2.30 (m, 1H), 2.10 (s, 3H), 2.06-1.96 (m, 2H), 1.85-1.71 (m, 1H) ; MS (ESI) : m/z 653 [M+H]⁺ | 10 | 8.09, 90 |
| 106 | 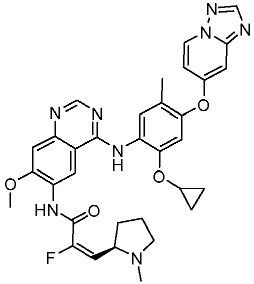 | (*R*,*E*)-*N*-(4-((4-([1,2,4]triazolo[1, 5-*a*]pyridin-7-yloxy)-2-cyclopropoxy-5-methylphenyl)am ino)-7-methoxyquinazoli n-6-yl)-2-fluoro-3-(1-methylpyrrolidin-2-yl)acrylamide | ¹H NMR (400 MHz, TFA salt, Methanol-d₆) δ 9.21 (s, 1H), 8.92 (d, J = 7.52 Hz, 1H), 8.69 (s, 1H), 8.63 (s, 1H), 7.57 (s, 1H), 7.41 (s, 1H), 7.32 (s, 1H), 7.29 (dd, J = 7.52, 2.50 Hz 1H), 7.05 (d, J = 2.39 Hz, 1H), 6.12 (dd, J = 20.16, 9.88 Hz, 1H), 5.23-5.17 (m, 1H), 4.19 (s, 3H), 3.88-3.79 (m, 2H), 3.30-3.23 (m, 1H), 2.99 (s, 3H), 2.61-2.52 (m, 1H), 2.29-2.16 (m, 5H), 2.05-1.95 (m, 1H), 0.77-0.64 (m, 4H); MS (ESI): m/z 625 [M+H]⁺ | 9 | 7.91, 99 |
| 107 | 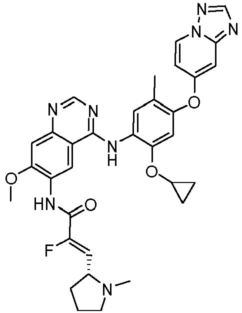 | (*R*,*Z*)-*N*-(4-((4-([1,2,4]triazolo[1, 5-*a*]pyridin-7-yloxy)-2-cyclopropoxy-5-methylphenyl)am ino)-7-methoxyquinazoli n-6-yl)-2-fluoro-3-(1-methylpyrrolidin-2-yl)acrylamide | ¹H NMR (400 MHz, TFA salt, Methanol-d₆) δ 9.16 (s, 1H), 8.80 (d, J = 7.48 Hz, 1H), 8.70 (s, 1H), 8.36 (s, 1H), 7.58 (s, 1H), 7.36 (s, 1H), 7.29 (s, 1H), 7.15 (dd, J = 7.46, 2.60 Hz 1H), 6.89 (d, J = 1.64 Hz, 1H), 6.37 (dd, J = 32.0, 9.50 Hz, 1H), 4.52-4.44 (m, 1H), 4.19 (s, 3H), 3.88-3.78 (m, 2H), 3.30-3.27 (m, 1H), 2.98 (s, 3H), 2.56-2.46 (m, 1H), 2.33-2.25 (m, 1H), 2.24-2.11 (m, 4H), 2.10-1.99 (m, 1H), 0.77-0.64 (m, 4H); MS (ESI): m/z 625 [M+H]⁺ | 2 | 7.82, 99 |
| 108 | 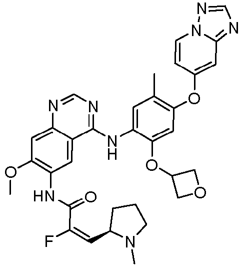 | (*R*,*E*)-*N*-(4-((4-([1,2,4]triazolo[1, 5-*a*]pyridin-7-yloxy)-5-methyl-2-(oxetan-3-yloxy)phenyl)ami no)-7-methoxyquinazoli n-6-yl)-2-fluoro-3-(1-methylpyrrolidin-2-yl)acrylamide | MS (ESI): m/z 641 [M+H]⁺ | | |
| 109 | 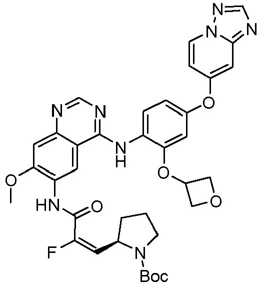 | *tert-*butyl (*R,E*)-2-(3-((4-((4-([1,2,4]triazolo[1, 5-*a*]pyridin-7-yloxy)-2-(oxetan-3-yloxy)phenyl)ami no)-7-methoxyquinazoli n-6-yl)amino)-2-fluoro-3-oxoprop-1-en-1-yl)pyrrolidine-1-carboxylate | ¹H NMR (400 MHz, free base, Methanol-d₄) δ 8.87 (s, 1H), 8.51-8.40 (m, 2H), 7.50-7.32 (m, 3H), 7.20-7.14 (m, 2H), 6.95 (s, 1H), 6.89 (s, 1H), 5.95-5.91 (m, 1H), 4.61 (s, 1H), 4.11 (s, 3H), 3.98 (s, 1H), 3.86-3.60 (m, 4H), 2.10-2.08 (m, 2H), 1.89-1.86 (m, 2H), 1.67-1.63 (m, 2H), 1.31 (s, 9H); MS (ESI): m/z 713 [M+H]⁺ | 6 | 11.43, 85 |
| 110 | 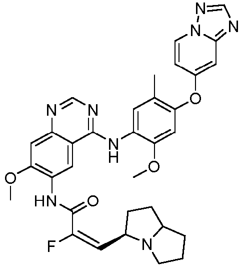 | (*E*)-*N-*(4-((4-([1,2,4]triazolo[1, 5-*a*]pyridin-7-yloxy)-2-methoxy-5-methylphenyl)am ino)-7-methoxyquinazoli n-6-yl)-2-fluoro-3-((3*R*)-hexahydro-1*H-*pyrrolizin-3-yl)acrylamide | MS (ESI): m/z 625 [M+H]⁺ | | |
| 111 | 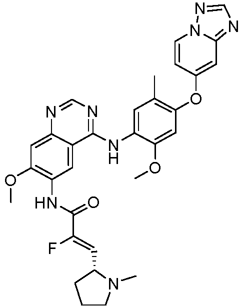 | (*R*,*Z*)-*N*-(4-((4-([1,2,4]triazolo[1, 5-*a*]pyridin-7-yloxy)-2-methoxy-5-methylphenyl)am ino)-7-methoxyquinazoli n-6-yl)-2-fluoro-3-(1-methylpyrrolidin-2-yl)acrylamide | ¹H NMR (400 MHz, TFA, DMSO-d₆) δ11.23 (s, 1H), 10.23 (s, 1H), 8.99-8.96 (m, 2H), 8.86 (s, 1H), 8.43 (s, 1H), 7.45 (s, 1H), 7.43 (s, 1H), 7.10-7.07 (m, 2H), 6.83 (s, 1H), 6.53-6.45 (m, 1H), 4.41-4.37 (m, 1H), 3.75 (s, 3H), 3.67 (s, 3H), 3.66-3.63 (m, 1H), 3.17-3.12 (m, 1H), 2.79 (s, 3H), 2.37-2.32 (m, 1H), 2.12-2.04 (m, 5H), 1.95-1.91 (m, 1H); MS (ESI): m/z 599 [M+H]⁺ | 9 | 7.26, 99 |
| 112 | 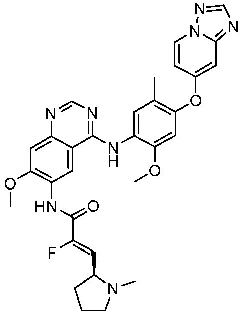 | (*S*,*Z*)-*N*-(4-((4-([1,2,4]triazolo[1, 5-*a*]pyridin-7-yloxy)-2-methoxy-5-methylphenyl)am ino)-7-methoxyquinazoli n-6-yl)-2-fluoro-3-(1-methylpyrrolidin-2-yl)acrylamide | ¹H NMR (400 MHz, free base, Methanol-d₄) δ9.72 (s, 1H), 9.30 (s, 1H), 8.97 (d, J = 7.2 Hz, 1H), 8.67 (s, 1H), 8.44 (s, 1H), 8.40 (s, 1H), 7.59 (s, 1H), 7.29 (s, 1H), 7.07-7.04 (m, 1H), 6.99 (s, 1H), 6.82 (d, J = 2.4 Hz, 1H), 6.09-5.97 (m, 1H), 4.00 (s, 3H), 3.75 (s, 3H), 3.27-3.13 (m, 1H), 3.01-2.99 (m, 1H), 2.23-2.19 (m, 4H), 2.12-2.09 (m, 4H), 1.79-1.76 (m, 2H), 1.62-1.60 (m, 1H); MS (ESI): m/z 599 [M+H]⁺ | 9 | 7.36, 100 |
| 113 | 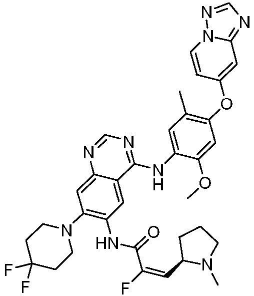 | (*R*,*E*)-*N*-(4-((4-([1,2,4]triazolo[1, 5-*a*]pyridin-7-yloxy)-2-methoxy-5-methylphenyl)am ino)-7-(4,4-difluoropiperidin-1-yl)quinazolin-6-yl)-2-fluoro-3-(1-methylpyrrolidin-2-yl)acrylamide | MS (ESI): m/z 688 [M+H]⁺ | | |
| 114 | 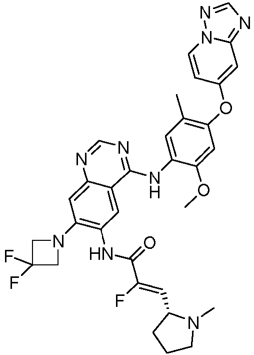 | (*R*,*Z*)-*N*-(4-((4-([1,2,4]triazolo[1, 5-*a*]pyridin-7-yloxy)-2-methoxy-5-methylphenyl)am ino)-7-(3,3-difluoroazetidin-1-yl)quinazolin-6-yl)-2-fluoro-3-(1-methylpyrrolidin-2-yl)acrylamide | ¹H NMR (400 MHz, FA salt, Methanol-d₄) δ 8.75 (d, J = 7.5 Hz, 1H), 8.39 (s, 1H), 8.29 (s, 1H), 8.27 - 8.22 (m, 4H), 7.70 (s, 1H), 7.10 (dd, J = 7.5, 2.5 Hz, 1H), 6.94 (s, 1H), 6.91 (d, J = 2.5 Hz, 1H), 6.82 (s, 1H), 6.29 (dd, J = 33.1, 9.7 Hz, 1H), 4.49 (t, J = 12.0 Hz, 4H), 4.15 (dd, J = 17.3, 9.2 Hz, 1H), 3.81 (s, 3H), 3.60 - 3.54 (m, 1H), 3.06 - 2.97 (m, 1H), 2.78 (s, 3H), 2.45 - 2.36 (m, 1H), 2.19 - 2.09 (m, 5H), 2.01 - 1.90 (m, 1H); MS (ESI): m/z 660 [M+H]⁺ | 1.35 | 7.05, 98 |
| 115 | 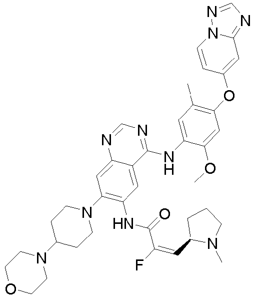 | (*R*,*E*)-*N*-(4-((4-([1,2,4]triazolo[1, 5-*a*]pyridin-7-yloxy)-2-methoxy-5-methylphenyl)am ino)-7-(4-morpholinopiperi din-1-yl)quinazolin-6-yl)-2-fluoro-3-(1-methylpyrrolidin-2-yl)acrylamide | MS (ESI): m/z 737 [M+H]⁺ | | |
| 116 | 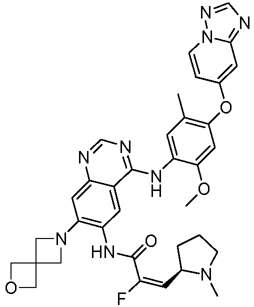 | (*R*,*E*)-*N*-(4-((4-([1,2,4]triazolo[1, 5-*a*]pyridin-7-yloxy)-2-methoxy-5-methylphenyl)am ino)-7-(2-oxa-6-azaspiro[3.3]hept an-6-yl)quinazolin-6-yl)-2-fluoro-3-(1-methylpyrrolidin-2-yl)acrylamide | MS (ESI): m/z 666 [M+H]⁺ | | |
| 117 | 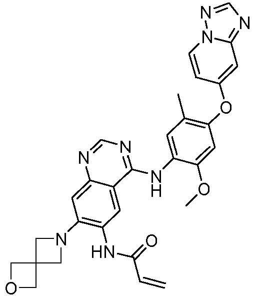 | *N*-(4-((4-([1,2,4]triazolo[1, 5-*a*]pyridin-7-yloxy)-2-methoxy-5-methylphenyl)am ino)-7-(2-oxa-6-azaspiro[3.3]hept an-6-yl)quinazolin-6-yl)acrylamide | ¹H NMR (400MHz, MeOH-d₄) δ 8.74 (d, J = 7.4 Hz, 1H), 8.32 (s, 1H), 8.29 (s, 1H), 8.15 (s, 1H), 7.73 (s, 1H), 7.09 (d, J = 2.6 Hz, 1H), 6.94 (d, J = 2.3 Hz, 1H), 6.92 (s, 1H), 6.73 (s, 1H), 6.62 - 6.44 (m, 2H), 5.90 (d, J = 9.9 Hz, 1H), 4.58 (s, 3H), 4.29 - 4.25 (m, 4H), 3.94 - 3.89 (m, 2H), 3.81 (s, 2H), 2.16 (s, 3H) ; MS (ESI): m/z 565 [M+H]⁺ | 1 | |
| 118 | 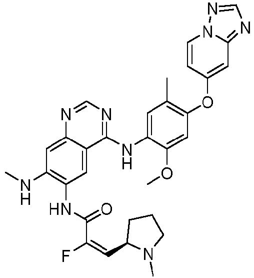 | (*R*,*E*)-*N*-(4-((4-([1,2,4]triazolo[1, 5-*a*]pyridin-7-yloxy)-2-methoxy-5-methylphenyl)am ino)-7-(methylamino)qui nazolin-6-yl)-2-fluoro-3-(1-methylpyrrolidin-2-yl)acrylamide | MS (ESI): m/z 598 [M+H]⁺ | | |
| 119 | 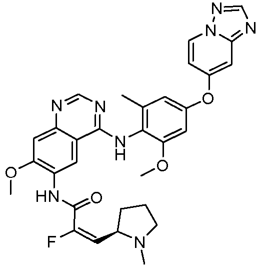 | (*R*,*E*)-*N*-(4-((4-([1,2,4]triazolo[1, 5-*a*]pyridin-7-yloxy)-2-methoxy-6-methylphenyl)am ino)-7-methoxyquinazoli n-6-yl)-2-fluoro-3-(1-methylpyrrolidin-2-yl)acrylamide | MS (ESI): m/z 599 [M+H]⁺ | | |
| 120 | 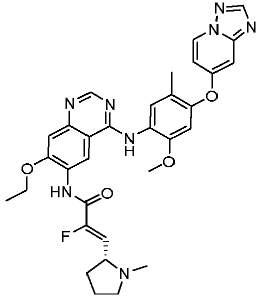 | (*R*,*Z*)-*N*-(4-((4-([1,2,4]triazolo[1, 5-*a*]pyridin-7-yloxy)-2-methoxy-5-methylphenyl)am ino)-7-ethoxyquinazolin -6-yl)-2-fluoro-3-(1-methylpyrrolidin-2-yl)acrylamide | ¹H NMR (400 MHz, TFA salt, Methanol-d₄) δ 9.19 (s, 1H), 8.81 (d, J = 7.5 Hz, 1H), 8.68 (s, 1H), 8.38 (s, 1H), 7.57 (s, 1H), 7.35 (s, 1H), 7.15 (dd, J = 7.5, 2.5 Hz, 1H), 7.04 (s, 1H), 6.90 - 6.86 (m, 1H), 6.38 (dd, J = 33.0, 9.4 Hz, 1H), 4.54 - 4.40 (m, 3H), 3.86 - 3.78 (m, 4H), 3.26 - 3.23 (m, 1H), 2.97 (s, 3H), 2.55 - 2.45 (m, 1H), 2.35 - 2.20 (m, 2H), 2.19 (s, 3H), 2.10 - 1.98 (m, 1H), 1.60 (t, J = 7.0 Hz, 3H); MS (ESI): m/z 613 [M+H]⁺ | 2 | 7.78, 99 |
| 121 | 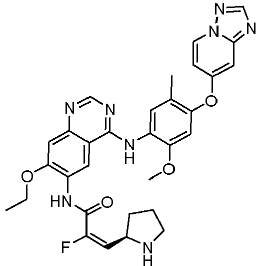 | (*R*,*E*)-*N*-(4-((4-([1,2,4]triazolo[1, 5-*a*]pyridin-7-yloxy)-2-methoxy-5-methylphenyl)am ino)-7-ethoxyquinazolin -6-yl)-2-fluoro-3-(pyrrolidin-2-yl)acrylamide | MS (ESI): m/z 599 [M+H]⁺ | | |
| 122 | 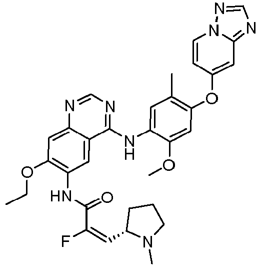 | (*S,E*)-*N*-(4-((4-([1,2,4]triazolo[1, 5-*a*]pyridin-7-yloxy)-2-methoxy-5-methylphenyl)am ino)-7-ethoxyquinazolin -6-yl)-2-fluoro-3-(1-methylpyrrolidin-2-yl)acrylamide | MS (ESI): m/z 613 [M+H]⁺ | | |
| 123 | 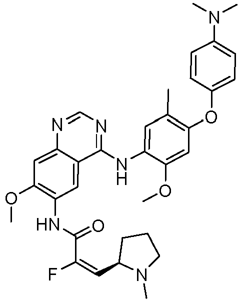 | (*R*,*E*)-*N*-(4-((4-(4-(dimethylamino)p henoxy)-2-methoxy-5-methylphenyl)am ino)-7-methoxyquinazoli n-6-yl)-2-fluoro-3-(1-methylpyrrolidin-2-yl)acrylamide | ¹H NMR (400 MHz, TFA salt, Methanol-d₄) δ 9.15 (s, 1H), 8.64 (s, 1H), 7.47-7.37 (m, 3H), 7.36 (s, 1H), 7.09 (d, J = 9.1 Hz, 2H), 6.76 (s, 1H), 6.09 (dd, J= 20.0, 10.1 Hz, 1H), 5.23-5.13 (m, 1H), 4.18 (s, 3H), 3.85-3.77 (m, 1H), 3.73 (s, 3H), 3.28-3.23 (m, 1H), 3.20 (s, 6H), 2.99 (s, 3H), 2.61-2.51 (m, 1H), 2.31 - 2.16 (m, 5H), 2.05-1.92 (m, 1H) ; MS (ESI) : m/z 601 [M+H]⁺ | 24 | 7.23, 98 |
| 124 | 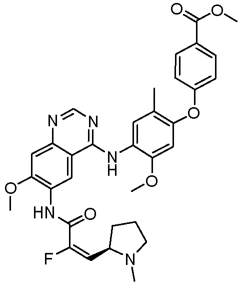 | methyl (*R,E*)-4-(4-((6-(2-fluoro-3-(1-methylpyrrolidin-2-yl)acrylamido)-7-methoxyquinazoli n-4-yl)amino)-5-methoxy-2-methylphenoxy)b enzoate | MS (ESI): m/z 616 [M+H]⁺ | | |
| 125 | 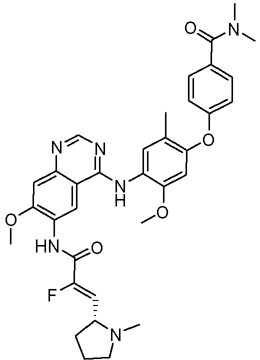 | (*R,Z*)-4-(4-((6-(2-fluoro-3-(1-methylpyrrolidin-2-yl)acrylamido)-7-methoxyquinazoli n-4-yl)amino)-5-methoxy-2-methylphenoxy)-*N,N-*dimethylbenzami de | ¹H NMR (400 MHz, TFA salt, Methanol-d₄) δ 9.15 (s, 1H), 8.66 (s, 1H), 7.48-7.45 (m, 3H), 7.39 (s, 1H), 7.03 (s, 1H), 7.01 (s, 1H), 6.82 (s, 1H), 6.44-6.33 (m, 1H), 4.49-4.47 (m, 1H), 4.18 (s, 3H), 3.82 (brs, 1H), 3.75 (s, 3H), 3.28 (brs, 1H), 3.10 (s, 3H), 3.06 (s, 3H), 2.96 (s, 3H), 2.54-2.46 (m, 1H), 2.33-2.17 (m, 5H), 2.10-2.01 (m, 1H); MS (ESI): m/z 629 [M+H]⁺ | 5 | 8.05, 98 |
| 126 | 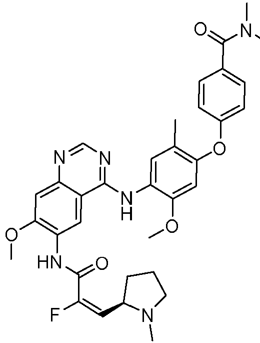 | (R,E)-4-(4-((6-(2-fluoro-3-(1-methylpyrrolidin-2-yl)acrylamido)-7-methoxyquinazoli n-4-yl)amino)-5-methoxy-2-methylphenoxy)-*N,N-*dimethylbenzami de | ¹H NMR (400 MHz, Free, Methanol-d₄) δ 8.75 (s, 1H), 8.31 (s, 1H), 7.82 (s, 1H), 7.44-7.41 (m, 2H), 7.04 (s, 1H), 6.99-6.95 (m, 2H), 6.66 (s, 1H), 5.96-5.88 (m, 1H), 4.17-4.10 (m, 1H), 4.00 (s, 3H), 3.75 (s, 3H), 3.21-3.16 (m, 1H), 3.08 (s, 3H), 3.04 (s, 3H), 2.44-2.37 (m, 4H), 2.29-2.24 (m, 1H), 2.07 (s, 3H), 1.95-1.85 (m, 2H), 1.71-1.69 (m, 1H); MS (ESI): m/z 629 [M+H]⁺ | 5 | 8.03, 87 |
| 127 | 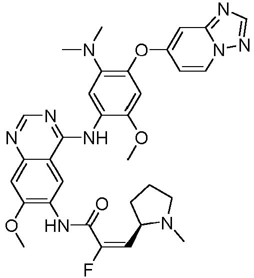 | (*R*,*E*)-*N-*(4-((4-([1,2,4]triazolo[1, 5-*a*]pyridin-7-yloxy)-5-(dimethylamino)-2-methoxyphenyl)a mino)-7-methoxyquinazoli n-6-yl)-2-fluoro-3-(1-methylpyrrolidin-2-yl)acrylamide | ¹H NMR (400 MHz, FA salt, Methanol-d₄) δ 8.98 (s, 1H), 8.70 (d, J = 7.4 Hz, 1H), 8.48 (s, 1H), 8.46 (s, 1H), 8.28 (s, 1H), 7.77 (s, 1H), 7.30 (s, 1H), 7.08 (dd, J = 7.5, 2.6 Hz, 1H), 7.00 (s, 1H), 6.98 (d, J = 2.4 Hz, 1H), 5.96 (dd, J = 22.6, 9.4 Hz, 1H), 4.45 - 4.36 (m, 1H), 4.12 (s, 3H), 3.85 (s, 3H), 2.72 (s, 6H), 2.67 - 2.61 (m, 2H), 2.56 (s, 3H), 2.38 - 2.31 (m, 1H), 2.04 - 1.95 (m, 2H), 1.82 - 1.71 (m, 1H); MS (ESI): m/z 628 [M+H]⁺ | 10 | 6.56, 100 |
| 128 | 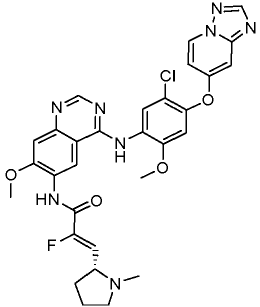 | (*R*,*Z*)*-N*-(4-((4-([1,2,4]triazolo[1, 5-*a*]pyridin-7-yloxy)-5-chloro-2-methoxyphenyl)a mino)-7-methoxyquinazoli n-6-yl)-2-fluoro-3-(1-methylpyrrolidin-2-yl)acrylamide | MS (ESI): m/z 619 [M+H]⁺ | | |
| 129 | 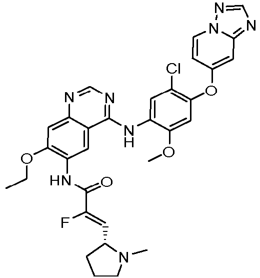 | (*R,Z*)-*N*-(4-((4-([1,2,4]triazolo[1, 5-*a*]pyridin-7-yloxy)-5-chloro-2-methoxyphenyl)a mino)-7-ethoxyquinazolin -6-yl)-2-fluoro-3-(1-methylpyrrolidin-2-yl)acrylamide | MS (ESI): m/z 633 [M+H]⁺ | | |
| 130 | 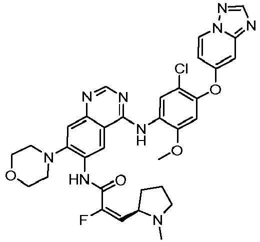 | (*R*,*E*)*-N-*(4-((4-([1,2,4]triazolo[1, 5-*a*]pyridin-7-yloxy)-5-chloro-2-methoxyphenyl)a mino)-7-morpholinoquina zolin-6-yl)-2-fluoro-3-(1-methylpyrrolidin-2-yl)acrylamide | MS (ESI): m/z 674 [M+H]⁺ | | |
| 131 | 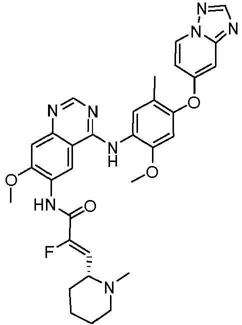 | (*R*,*Z*)*-N-*(4-((4-([1,2,4]triazolo[1, 5-*a*]pyridin-7-yloxy)-2-methoxy-5-methylphenyl)am ino)-7-methoxyquinazoli n-6-yl)-2-fluoro-3-(1-methylpiperidin-2-yl)acrylamide | ¹H NMR (400 MHz, free base, Methanol-d₄) δ 8.97 (s, 1H), 8.75 (d, J = 7.2 Hz, 1H), 8.45 (s, 1H), 8.29 (s, 1H), 7.90 (s, 1H), 7.30 (s, 1H), 7.11-7.09 (m, 1H), 6.95-6.94 (m, 2H), 6.30-6.18 (m, 1H), 4.12 (s, 3H), 3.86 (s, 3H), 2.96-2.94 (m, 1H), 2.27 (s, 3H), 2.18 (s, 3H), 1.82-1.41 (m, 8H); MS (ESI): m/z 613 [M+H]⁺ | 3 | 7.56, 90 |
| 132 | 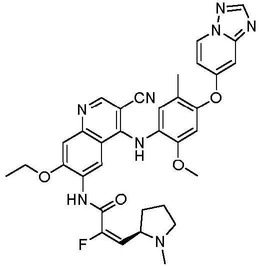 | (*R*,*E*)*-N-*(4-((4-([1,2,4]triazolo[1, 5-*a*]pyridin-7-yloxy)-2-methoxy-5-methylphenyl)am ino)-3-cyano-7-ethoxyquinolin-6-yl)-2-fluoro-3-(1-methylpyrrolidin-2-yl)acrylamide | ¹H NMR (400 MHz, TFA salt, Methanol-d₄) δ 9.17 (s, 1H), 8.75 (d, J = 7.1 Hz, 2H), 8.30 (s, 1H), 7.44 (s, 1H), 7.41 (s, 1H), 7.13 (dd, J = 7.5, 2.5 Hz, 1H), 7.04 (d, J = 2.5 Hz, 1H), 7.01 (s, 1H), 6.09 (dd, J = 20.3, 10.1 Hz, 1H), 5.21 (dd, J = 18.5, 8.8 Hz, 1H), 4.46-4.36 (m, 2H), 3.85-3.77 (m, 4H), 3.28-3.25 (m, 1H), 2.99 (s, 3H), 2.61-2.48 (m, 1H), 2.32-2.15 (m, 5H), 2.05-1.93 (m, 1H), 1.59 (t, J = 7.0 Hz, 3H) ; MS (ESI) : m/z 637 [M+H]⁺ | 18 | 8.17, 99 |
| 133 | 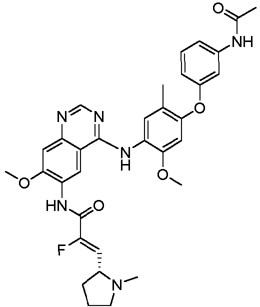 | (*R,Z*)*-N-*(4-((4-(3-acetamidophenox y)-2-methoxy-5-methylphenyl)am ino)-7-methoxyquinazoli n-6-yl)-2-fluoro-3-(1-methylpyrrolidin-2-yl)acrylamide | ¹H NMR (400 MHz, FA salt, Methanol-d₄) δ 8.80 (s, 1H), 8.38 (s, 1H), 7.84 (s, 1H), 7.31-7.30 (m, 1H), 7.26-7.22 (m, 1H), 7.18-7.16 (m, 2H), 6.67-6.64 (m, 2H), 6.39-6.28 (m, 1H), 4.28-4.21 (m, 1H), 4.06 (s, 3H), 3.78 (s, 3H), 3.67-3.62 (m, 1H), 3.14-3.07 (m, 1H), 2.82 (s, 3H), 2.46-2.38 (m, 1H), 2.22-2.09 (m, 8H), 2.04-1.88 (m, 1H); MS (ESI): m/z 615 [M+H]⁺ | 1 | 8.10, 99 |
| 134 | 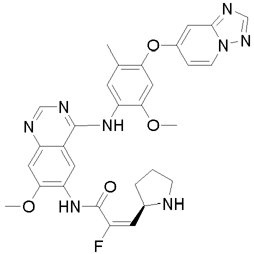 | (*R*,*E*)*-N-*(4-((4-([1,2,4]triazolo[1, 5-*a*]pyridin-7-yloxy)-2-methoxy-5-methylphenyl)am ino)-7-methoxyquinazoli n-6-yl)-2-fluoro-3-(pyrrolidin-2-yl)acrylamide | ¹H NMR (400 MHz, FA, Methanol-d₄) δ8.96 (s, 1H), 8.78 8.79 (d, J = 7.6 Hz, 1H), 8.47 (s, 1H), 8.32 (s, 1H), 7.87 (s, 1H), 7.32 (s, 1H), 7.14-7.11 (m, 1H), 6.98-6.92 (m, 2H), 6.21-6.14 (m, 1H), 5.27-5.25 (m, 1H), 4.13 (s, 3H), 3.87 (s, 3H), 3.15-3.35 (m, 2H), 2.49-2.43 (m, 1H), 2.24-2.11 (m, 5H), 1.98-1.93 (m, 1H); MS (ESI): m/z 585 [M+H]⁺ | 92 | 7.19, 100 |
| 135 | 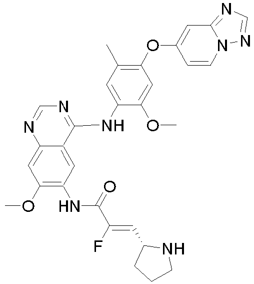 | (*R*,*Z*)*-N-*(4-((4-([1,2,4]triazolo[1, 5-*a*]pyridin-7-yloxy)-2-methoxy-5-methylphenyl)am ino)-7-methoxyquinazoli n-6-yl)-2-fluoro-3-(pyrrolidin-2-yl)acrylamide | ¹H NMR (400 MHz, free base, Methanol-d₄) δ9.09 (s, 1H), 8.79 (d, J = 7.6 Hz, 1H), 8.12 (s, 1H), 8.33 (s, 1H), 7.71 (s, 1H), 7.36 (s, 1H), 7.15-7.12 (m, 1H), 7.02 (s, 1H), 6.90-6.88 (m, 1H), 6.49-6.41 (m, 1H), 4.67-4.65 (m, 1H), 4.18 (s, 3H), 3.18 (s, 3H), 3.50-3.42 (m, 2H), 2.52-2.49 (m, 1H), 2.23-2.19 (m, 5H), 2.02-1.92 (m, 1H); MS (ESI): m/z 585 [M+H]⁺ | 21 | 7.37, 100 |
| 136 | 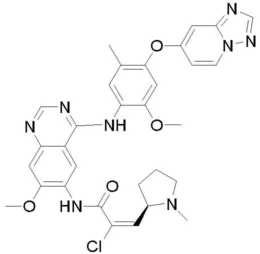 | (*R*,*E*)*-N*-(4-((4-([1,2,4]triazolo[1, 5-*a*]pyridin-7-yloxy)-2-methoxy-5-methylphenyl)am ino)-7-methoxyquinazoli n-6-yl)-2-chloro-3-(1-methylpyrrolidin-2-yl)acrylamide | ¹H NMR (400 MHz, TFA salt, Methanol-d₆) δ 9.23 (s, 1H), 8.80 (d, J = 7.47 Hz, 1H), 8.69 (s, 1H), 8.36 (s, 1H), 7.58 (s, 1H), 7.38 (s, 1H), 7.28 (d, J = 9.11 Hz, 1H), 7.14 (dd, J = 7.47, 2.56 Hz, 1H), 7.04 (s, 1H), 6.87 (d, J = 2.42 Hz, 1H), 4.60-4.56 (m, 1H), 4.21 (s, 3H), 3.98 (s, 3H), 3..87-3.79 (m, 4H), 2.98 (s, 3H), 2.62-2.53 (m, 1H), 2.36-2.28 (m, 1H), 2.25-2.14 (m, 1H), 2.11-1.99 (m, 1H); MS (ESI): m/z 615 [M+H]⁺ | 21 | 7.49, 96 |
| 137 | 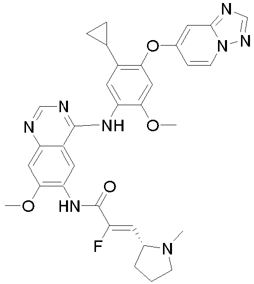 | (*R*,*Z*)*-N-*(4-((4-([1,2,4]triazolo[1, 5-*a*]pyridin-7-yloxy)-5-cyclopropyl-2-methoxyphenyl)a mino)-7-methoxyquinazoli n-6-yl)-2-fluoro-3-(1-methylpyrrolidin-2-yl)acrylamide | ¹H NMR (400 MHz, TFA salt, Methanol-d₄) δ 9.16 (s, 1H), 8.81 (d, J = 7.2 Hz, 1H), 8.69 (s, 1H), 8.38 (s, 1H), 7.37 (s, 1H), 7.29 (s, 1H), 7.19-7.16 (m, 1H), 7.04 (s, 1H), 6.92 (d, J = 2 Hz, 1H), 6.43-6.32 (m, 1H), 4.49-4.47 (m, 1H), 4.19 (s, 3H), 3.81 (brs, 4H), 2.97 (s, 3H), 2.52-2.50 (m, 1H), 2.29-2.20 (m, 2H), 2.09-2.03 (m, 1H), 1.93-1.87 (m, 1H), 0.87-0.82 (m, 2H), 0.67-0.65 (m, 2H); MS (ESI): m/z 625 [M+H]⁺ | 1 | 7.70, 98 |
| 138 | 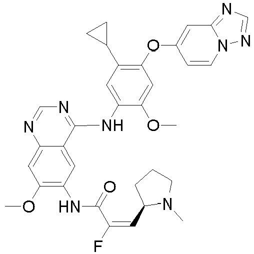 | (*R*,*E*)*-N-*(4-((4-([1,2,4]triazolo[1, 5-*a*]pyridin-7-yloxy)-5-cyclopropyl-2-methoxyphenyl)a mino)-7-methoxyquinazoli n-6-yl)-2-fluoro-3-(1-methylpyrrolidin-2-yl)acrylamide | ¹H NMR (400 MHz, Free, Methanol-d₄) δ 8.92 (s, 1H), 8.73 (d, J = 7.6 Hz, 1H), 8.42 (s, 1H), 8.28 (s, 1H), 7.59 (s, 1H), 7.23 (s, 1H), 7.12-7.09 (m, 1H), 6.95-6.93 (m, 2H), 6.03-5.95 (m, 1H), 4.50-4.48 (m, 1H), 4.09 (s, 3H), 3.84 (s, 3H), 3.39-3.34 (m, 1H), 2.73-2.66 (m, 1H), 2.60 (s, 3H), 2.39-2.34 (m, 1H), 2.05-1.96 (m, 6H), 1.89-1.77 (m, 2H), 0.84-0.79 (m, 2H), 0.68-0.64 (m, 2H); MS (ESI): m/z 625 [M+H]⁺ | 2 | 8.05, 91 |
| 139 | 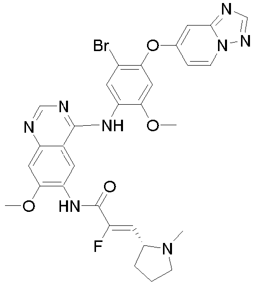 | (*R*,*Z*)-*N-*(4-((4-([1,2,4]triazolo[1, 5-*a*]pyridin-7-yloxy)-5-bromo-2-methoxyphenyl)a mino)-7-methoxyquinazoli n-6-yl)-2-fluoro-3-(1-methylpyrrolidin-2-yl)acrylamide | ¹H NMR (400 MHz, FA salt, Methanol-d₆) δ 8.96 (s, 1H), 8.77 (d, J = 7.37 Hz, 1H), 8.53 (s, 1H), 8.50 (s, 1H), 8.32 (s, 1H), 8.31 (s, 1H), 7.31 (s, 1H), 7.17 (s, 1H), 7.11 (dd, J = 7.52, 2.58 Hz, 1H), 6.94 (d, J = 2.42 Hz, 1H), 6.27 (dd, J = 34.1, 9.66 Hz, 1H), 4.12 (s, 3H), 3.93 (s, 3H), 3.92-3.84 (m, 1H), 3.46-3.39 (m, 1H), 2.84-2.77 (m, 1H), 2.64 (s, 3H), 2.37-2.29 (m, 1H), 2.11-2.04 (m, 2H), 1.92-1.83 (m, 1H), 2.25-2.14 (m, 1H), 2.11-1.99 (m, 1H); MS (ESI): m/z 663 [M+H]⁺ | 7 | 7.73, 98 |
| 140 | 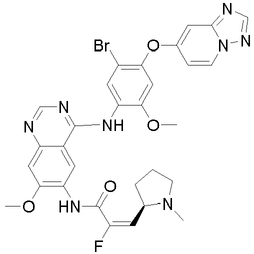 | (*R*,*E*)*-N*-(4-((4-([1,2,4]triazolo[1, 5-*a*]pyridin-7-yloxy)-5-bromo-2-methoxyphenyl)a mino)-7-methoxyquinazoli n-6-yl)-2-fluoro-3-(1-methylpyrrolidin-2-yl)acrylamide | ¹H NMR (400 MHz, FA salt, Methanol-d₆) δ 8.89 (s, 1H), 8.77 (d, J = 7.49 Hz, 1H), 8.51 (s, 1H), 8.45 (s, 1H), 8.31 (s, 1H), 7.26 (s, 1H), 7.16 (s, 1H), 7.10 (dd, J = 7.52, 2.57 Hz, 1H), 6.92 (d, J = 2.50 Hz, 1H), 6.11 (dd, J = 20.9, 9.74 Hz, 1H), 5.13-5.07 (m, 1H), 4.10 (s, 3H), 3.91 (s, 3H), 3.71-3.65 (m, 1H), 3.23-3.16 (m, 1H), 2.91 (s, 3H), 2.57-2.49 (m, 1H), 2.23-2.14 (m, 2H), 2.03-1.94 (m, 1H); MS (ESI): m/z 663 [M+H]⁺ | 5 | 7.97, 99 |
| 141 | 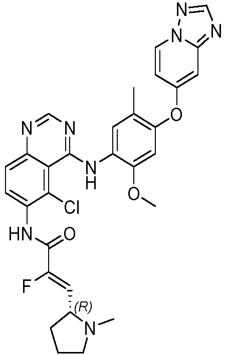 | (*R*,*Z*)*-N-*(4-((4-([1,2,4]triazolo[1, 5-*a*]pyridin-7-yloxy)-2-methoxy-5-methylphenyl)am ino)-5-chloroquinazolin-6-yl)-2-fluoro-3-(1-methylpyrrolidin-2-yl)acrylamide | ¹H NMR (400 MHz, TFA salt, Methanol-d₄) δ 8.81 (s, 1H), 8.78 (d, J = 7.5 Hz, 1H), 8.54 (s, 1H), 8.34 (s, 1H), 8.25 (d, J = 9.0 Hz, 1H), 7.88 (d, J = 9.0 Hz, 1H), 7.12 (dd, J = 7.5, 2.6 Hz, 1H), 7.04 (s, 1H), 6.82 (d, J = 2.5 Hz, 1H), 6.34 (dd, J = 32.1, 9.9 Hz, 1H), 4.81-4.74 (m, 1H), 4.55-4.45 (m, 1H), 3.95 (s, 3H), 3.88-3.78 (m, 1H), 2.99 (s, 3H), 2.60-2.48 (m, 1H), 2.34-2.14 (m, 5H), 2.10-2.00 (m, 1H) ; MS (ESI): m/z 603 [M+H]⁺ | 13 | 8.19, 98 |
| 142 | 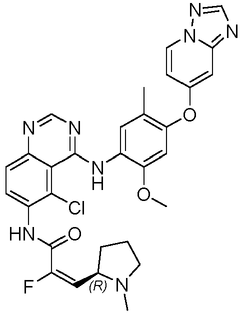 | (*R*,*E*)-*N*-(4-((4-([1,2,4]triazolo[1, 5-*a*]pyridin-7-yloxy)-2-methoxy-5-methylphenyl)am ino)-5-chloroquinazolin-6-yl)-2-fluoro-3-(1-methylpyrrolidin-2-yl)acrylamide | ¹H NMR (400 MHz, TFA salt Methanol-d₄) δ 8.82 (s, 1H), 8.79 (d, J = 7.5 Hz, 1H), 8.51 (s, 1H), 8.36 (s, 1H), 8.29 (d, J = 9.0 Hz, 1H), 7.88 (d, J = 9.0 Hz, 1H), 7.13 (dd, J = 7.5, 2.6 Hz, 1H), 7.05 (s, 1H), 6.83 (d, J = 2.4 Hz, 1H), 6.10 (dd, J = 19.9, 9.9 Hz, 1H), 5.16 (dd, J = 18.1, 9.2 Hz, 1H), 4.80-4.77 (m, 1H), 3.95 (s, 3H), 3.79 (s, 1H), 2.99 (s, 3H), 2.61-2.49 (m, 1H), 2.31-2.13 (m, 5H), 2.06-1.92 (m, 1H) ; MS (ESI): m/z 603 [M+H]⁺ | 5 | 8.38, 96 |
| 143 | 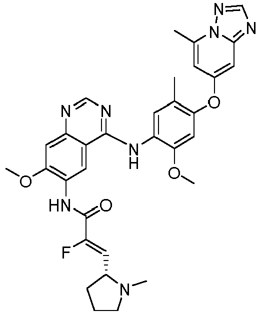 | (*R*,*Z*)-2-fluoro-N-(7-methoxy-4-((2-methoxy-5-methyl-4-((5-methyl-[1,2,4]triazolo[1, 5-*a*]pyridin-7-yl)oxy)phenyl)am ino)quinazolin-6-yl)-3-(1-methylpyrrolidin-2-yl)acrylamide | MS (ESI): m/z 613 [M+H]⁺ | | |

### Preparation Example 13: Preparation of (Z)-N-(4-((4-([1,2,4]triazolo[1,5-a]pyridin-7-yloxy)-2-methoxy-5-methylphenyl)amino)-7-methoxyquinazolin-6-yl)-2-cyano-4-methyl-4-(4-(oxetan-3-yl)piperazin-1-yl)pent-2-enamide (Example 144)

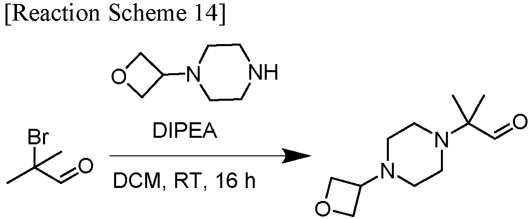

### [Step A1] Preparation of 2-methyl-2-(4-(oxetan-3-yl)piperazin-1-yl)propanal

After 2-bromo-2-methylpropanal (2.0 eq) was dissolved in dichloromethane (0.15 M), 1-(oxetan-3-yl)piperazine (1.0 eq) was slowly added thereto at 0 °C. DIPEA (1.0 eq) was added to the reaction mixture and stirred at room temperature for 16 hours. Organic materials were extracted by adding water and dichloromethane to the reaction mixture, and then remaining water in collected organic layer was removed using Na₂SO₄, and the collected organic layer was concentrated to obtain the desired compound as a yellow liquid (yield: 100%, MS (ESI): m/z 213 [M+H]⁺).

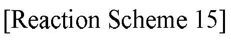

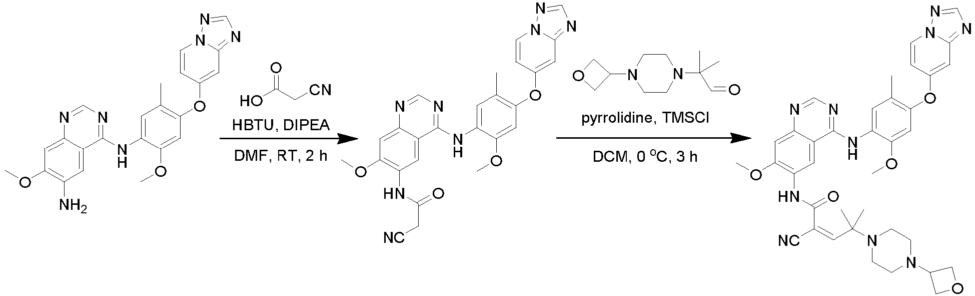

### [Step B1] Preparation of N-(4-((4-([1,2,4]triazolo[1,5-a]pyridin-7-yloxy)-2-methoxy-5-methylphenyl)amino)-7-methoxyquinazolin-6-yl)-2-cyanoacetamide

Cyanoacetic acid (2.0 eq), 2-(1H-benzotriazol-1-yl)-1,1,3,3-tetramethyluronium hexafluorophosphate (HBTU, 2.0 eq), and DIPEA (3.0 eq) were dissolved in DMF (0.1 M), and then *N*⁴-(4-([1,2,4]triazolo[1,5-*a*]pyridin-7-yloxy)-2-methoxy-5-methylphenyl)-7-methoxyquinazoline-4,6-diamine (1.0 eq) obtained in step 4 of Preparation Example 1 was added thereto and stirred at room temperature for 2 hours. A solid produced by adding a reaction mixture to cold water was filtered and then dried in vacuum to obtain the desired compound as a yellow solid. The desired compound was used in the following reaction without further purification (yield: 100%, MS (ESI): m/z 511 [M+H]⁺).

### [Step B2] Preparation of (Z)-N-(4-((4-([1,2,4]triazolo[1,5-a]pyridin-7-yloxy)-2-methoxy-5-methylphenyl)amino)-7-methoxyquinazolin-6-yl)-2-cyano-4-methyl-4-(4-(oxetan-3-yl)piperazin-1-yl)pent-2-enamide

*N*-(4-((4-([1,2,4]Triazolo[1,5-*a*]pyridin-7-yloxy)-2-methoxy-5-methylphenyl)amino)-7-methoxyquinazolin-6-yl)-2-cyanoacetamide (1.0 eq) obtained in step B1 and 2-methyl-2-(4-(oxetan-3-yl)piperazin-1-yl)propanal (2.0 eq) obtained in step A1 of Reaction Scheme 14 were dissolved in dichloromethane (0.1 M), and then pyrrolidine (6.0 eq) and chlorotrimethylsilane (4.0 eq) were added thereto at 0 °C, followed by stirring for 3 hours. Water and brine were added to the reaction mixture, and organic materials were extracted with dichloromethane, and then remaining water in collected organic layer was removed using Na₂SO₄ and the collected organic layer was concentrated. The reaction mixture was purified through prep-HPLC to obtain the desired compound as a yellow solid (yield: 68%, MS (ESI): m/z 705 [M+H]⁺).

### Preparation of Compounds of Examples 145 and 146

The compounds of Examples 145 and 146 according to the present invention were prepared in a similar manner to Preparation Example 13.

The chemical structural formulas, compound names, ¹H NMR, MS, yields, HPLC data, and purities of the compounds of Examples 144 to 146 are summarized in Table 13 below.

**[Table 13]**

| Ex. | Structure | Compound name | ¹H NMR, MS | Yield (%) | HPLC (min), Purity (%) |
|---|---|---|---|---|---|
| 144 | 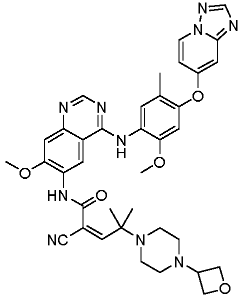 | (*Z*)-*N*-(4-((4-([1,2,4]triazolo[1,5 -*a*]pyridin-7-yloxy)-2-methoxy-5-methylphenyl)amin o)-7-methoxyquinazolin -6-yl)-2-cyano-4-methyl-4-(4-(oxetan-3-yl)piperazin-1-yl)pent-2-enamide | ¹H NMR (400 MHz, TFA salt, Methanol-d₄) δ 9.23 (s, 1H), 8.78 (d, J = .2 Hz, 1H), 8.65 (s, 1H), 8.32 (s, 1H), 7.72 (s, 1H), 7.64 (s, 1H), 7.35 (s, 1H), 7.14-7.11 (m, 1H), 7.03 (s, 1H), 6.86 (d, J = 2.0 Hz, 1H), 4.80-4.77 (m, 4H), 4.38-4.37 (m, 1H), 4.20 (s, 3H), 3.82 (s, 3H), 2.19 (s, 3H), 2.16 (s, 8H), 1.30 (s, 6H); MS (ESI): m/z 705 [M+H]⁺ | 68 | 8.16, 96 |
| 145 | 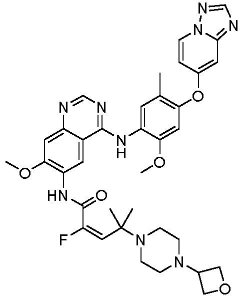 | *(E)-N-(4-((4-*([1,2,4]triazolo[1,5 -*a*]pyridin-7-yloxy)-2-methoxy-5-methylphenyl)amin o)-7-methoxyquinazolin -6-yl)-2-fluoro-4-methyl-4-(4-(oxetan-3-yl)piperazin-1-yl)pent-2-enamide | MS (ESI): m/z 698 [M+H]⁺ | | |
| 146 | 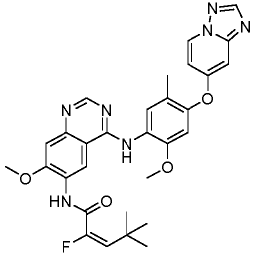 | (*E*)*-N-*(4-((4-([1,2,4]triazolo[1,5 -*a*]pyridin-7-yloxy)-2-methoxy-5-methylphenyl)amin o)-7-methoxyquinazolin -6-yl)-2-fluoro-4,4-dimethylpent-2-enamide | MS (ESI): m/z 572 [M+H]⁺ | | |

### Preparation Example 14: Preparation of N-(4-((4-([1,2,4]triazolo[1,5-a]pyridin-7-yloxy)-2-methoxy-5-methylphenyl)amino)-7-methoxyquinazolin-6-yl)-2-(1H-pyrazol-1-yl)acrylamide (Example 147)

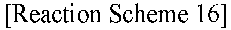

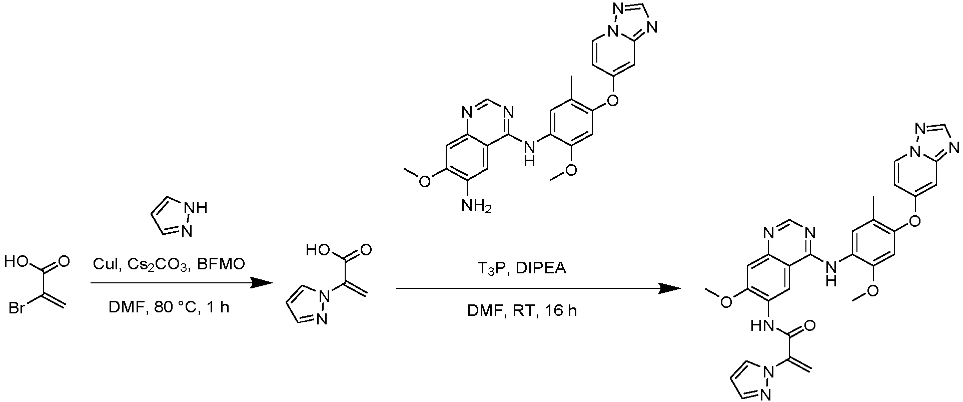

### [Step 1] Preparation of 2-(1H-pyrazol-1-yl)acrylic acid

2-Bromoacrylic acid (1.0 eq), 1H-pyrazole (1.5 eq), CuI (0.2 eq), Cs₂CO₃ (2.5 eq), and *N*¹,*N*²-bis(furan-2-ylmethyl)oxalamide (BFMO, 0.2 eq) were dissolved in DMSO (0.5 M), followed by stirring at 80 °C for 1 hour. Water and brine were added to the reaction mixture, and organic materials were extracted with ethyl acetate, and then remaining water in collected organic layer was removed using Na₂SO₄ and the collected organic layer was concentrated. The reaction mixture was purified through MPLC (dichloromethane:methanol) to obtain the desired compound as a yellow solid (yield: 6%, MS (ESI): m/z 139 [M+H]⁺).

### [Step 2] Preparation of N-(4-((4-([1,2,4]triazolo[1,5-a]pyridin-7-yloxy)-2-methoxy-5-methylphenyl)amino)-7-methoxyquinazolin-6-yl)-2-(1H-pyrazol-1-yl)acrylamide

After dissolving 2-(1*H*-Pyrazol-1-yl)acrylic acid (2.0 eq) prepared in step 1 of Preparation Example 14 and propylphosphonic acid (5.0 eq) in DMF (0.01 M) and DIPEA (1.0 eq) was added to *N*⁴-(4-([1,2,4]triazolo[1,5-*a*]pyridin-7-yloxy)-2-methoxy-5-methylphenyl)-7-methoxyquinazoline-4,6-diamine (1.0 eq) obtained in step 4 of Preparation Example 1. The mixture was stirred at room temperature for 2 hours. The reaction mixture was added to cold water, the resulting solid was filtered and then purified through prep-HPLC to obtain the desired compound as a yellow solid (yield: 4%, MS (ESI): m/z 564 [M+H]⁺).

**[Table 14]**

| Ex. | Structure | Compound name | ¹H NMR, MS | Yield (%) | HPLC (min), Purity (%) |
|---|---|---|---|---|---|
| 147 | 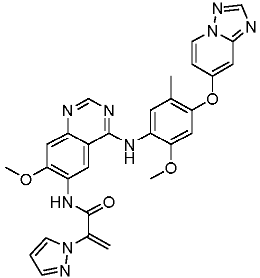 | *N*-(4-((4-([1,2,4]triazolo[1, 5-*a*]pyridin-7-yloxy)-2-methoxy-5-methylphenyl)am ino)-7-methoxyquinazoli n-6-yl)-2-(1*H-*pyrazol-1-yl)acrylamide | ¹H NMR (400 MHz, FA salt, Methanol-d₄) δ 9.14 (s, 1H), 8.76 (d, J = 8.0 Hz, 1H), 8.44 (s, 1H), 8.30 (s, 1H), 8.11 (d, J = 2.4 Hz, 1H), 7.92 (s, 1H), 7.88 (d, J = 1.6 Hz, 1H), 7.27 (s, 1H), 7.12-7.09 (m, 1H), 6.96-6.92 (m, 2H), 6.58-6.57 (m, 1H), 6.34 (s, 1H), 5.60 (s, 1H), 4.10 (s, 3H), 3.87 (s, 3H), 2.18 (s, 3H); MS (ESI): m/z 564 [M+H]⁺ | 4 | 9.71, 88 |

### Preparation Example 15: Preparation of (R,Z)-N-(4-((4-([1,2,4]triazolo[1,5-a]pyridin-7-yloxy)-2-(methoxy-d₃)-5-methylphenyl)amino)-7-methoxyquinazolin-6-yl)-2-fluoro-3-(1-methylpyrrolidin-2-yl)acrylamide (Example 149) and (R,E)-N-(4-((4-([1,2,4]triazolo[1,5-a]pyridin-7-yloxy)-2-(methoxy-d₃)-5-methylphenyl)amino)-7-methoxyquinazolin-6-yl)-2-fluoro-3-(1-methylpyrrolidin-2-yl)acrylamide (Example 150)

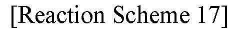

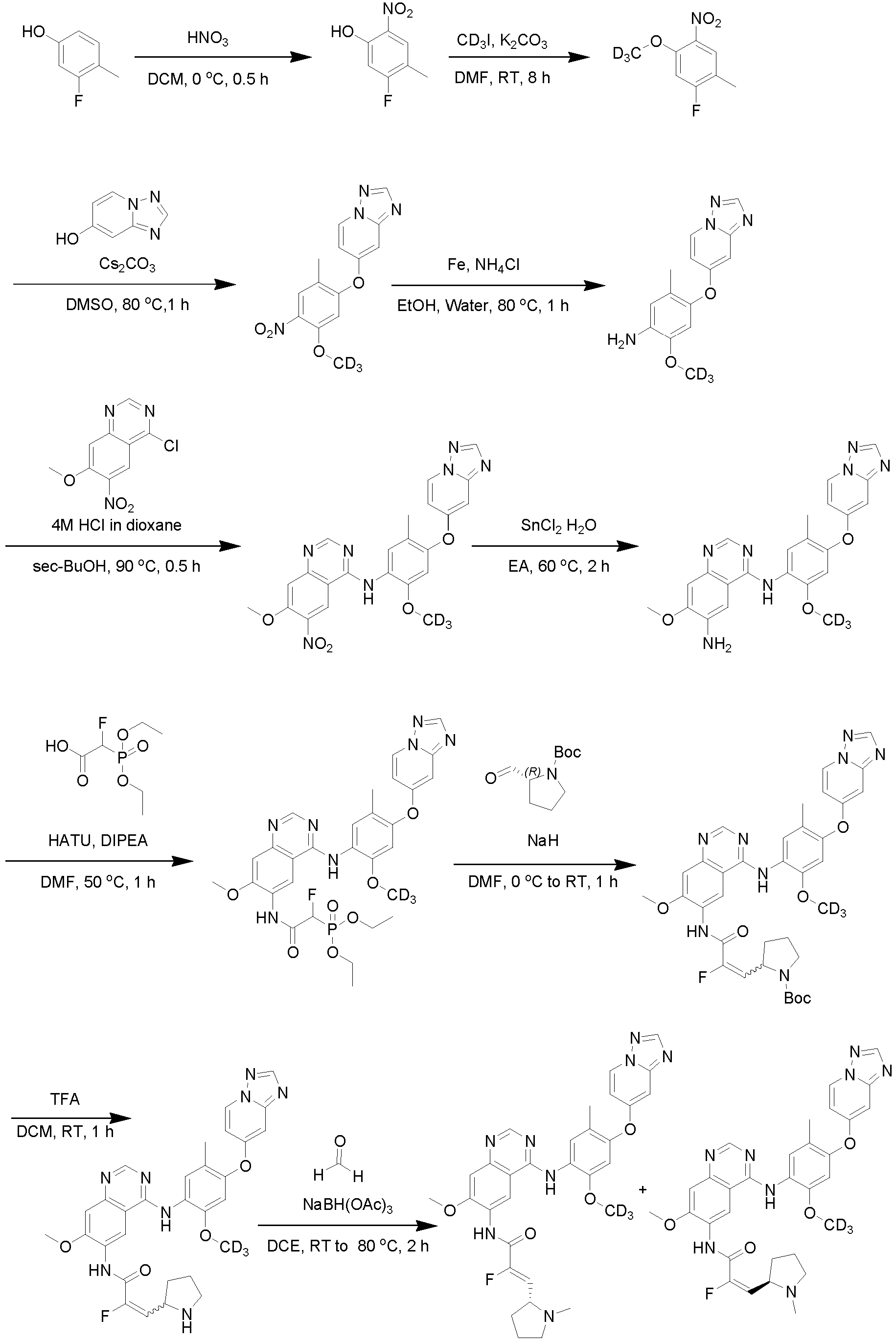

### [Step 1] Preparation of 5-fluoro-4-methyl-2-nitrophenol

3-Fluoro-4-methylphenol (1.0 eq) was dissolved in dichloromethane (0.4 M), and then 70% nitric acid (1.5 eq) was added at 0 °C and stirred at 0 °C for 30 minutes. After water was added to the reaction mixture and organic materials were extracted with dichloromethane, remaining water in collected organic layer was removed using Na₂SO₄, and the collected organic layer was concentrated. The reaction mixture was purified through MPLC (hexane:ethyl acetate) to obtain the desired compound as a yellow solid (yield: 63%).

### [Step 2] Preparation of 1-fluoro-5-(methoxy-d₃)-2-methyl-4-nitrobenzene

5-Fluoro-4-methyl-2-nitrophenol (1.0 eq) prepared in step 1 was dissolved in DMF (0.5 M), iodomethane-*d*₃ (3.0 eq) and K₂CO₃ (3.0 eq) were then added thereto, and the mixture was stirred at room temperature for 8 hours. A saturated aqueous NaHCO₃ solution was added to the reaction mixture, and organic materials were extracted with ethyl acetate. Then, remaining water in collected organic layer was removed using Na₂SO₄, and the collected organic layer was concentrated. The reaction mixture was purified through MPLC (hexane:ethyl acetate) to obtain the desired compound as a yellow solid (yield: 80%).

### [Step 3] Preparation of 7-(5-(methoxy-d₃)-2-methyl-4-nitrophenoxy)-[1,2,4]triazolo[1,5-a]pyridine

1-Fluoro-5-(methoxy-*d*₃)-2-methyl-4-nitrobenzene (1.0 eq) prepared in step 2 was dissolved in DMSO (0.25 M), [1,2,4]triazolo[1,5-*a*]pyridin-7-ol (1.2 eq) and Cs₂CO₃ (2.4 eq) were then added thereto, and the mixture was stirred at 80 °C for 1 hour. Ice water was added to the reaction mixture to form a solid, and the resulting solid was filtered through a filter to obtain the desired compound as a brown solid. The obtained desired compound was used in the following reaction without further purification (yield: 100%, MS (ESI): m/z 304 [M+H]⁺).

### [Step 4] Preparation of 4-([1,2,4]triazolo[1,5-a]pyridin-7-yloxy)-2-(methoxy-d₃)-5-methylaniline

7-(5-(Methoxy-*d*₃)-2-methyl-4-nitrophenoxy)-[1,2,4]triazolo[1,5-*a*]pyridine (1.0 eq) prepared in step 3, ammonium chloride (0.45 eq), and iron (4.5 eq) were dissolved in an ethanol-water solution (3: 1), followed by stirring at 80 °C for 1 hour. The mixture was filtered through a celite filter and then concentrated to obtain the desired compound as a brown solid. The obtained desired compound was used in the following reaction without further purification (yield: 92%, MS (ESI): m/z 274 [M+H]⁺).

### [Step 51 Preparation of N-(4-([1,2,4]triazolo[1,5-a]pyridin-7-yloxy)-2-(methoxy-d₃)-5-methylphenyl)-7-methoxy-6-nitroquinazolin-4-amine

4-([1,2,4]Triazolo[1,5-*a*]pyridin-7-yloxy)-2-(methoxy-*d*₃)-5-methylaniline (1.0 eq) prepared in step 4 and 4-chloro-7-methoxy-6-nitroquinazoline (1.0 eq) were dissolved in *sec-*BuOH (0.25 M), and then 4 M HCl (0.2 eq) dissolved in 1,4-dioxane was added thereto, followed by stirring at 90 °C for 30 minutes. After methanol and ether were added to the reaction mixture, the desired compound as a yellow solid was obtained through a filter (yield: 65%, MS (ESI): m/z 477 [M+H]⁺).

### [Step 6] Preparation of N⁴-(4-([1,2,4]triazolo[1,5-a]pyridin-7-yloxy)-2-(methoxy-d₃)-5-methylphenyl)-7-methoxyquinazoline-4,6-diamine

*N*-(4-([1,2,4]Triazolo[1,5-*a*]pyridin-7-yloxy)-2-(methoxy-*d*₃)-5-methylphenyl)-7-methoxy-6-nitroquinazolin-4-amine (1.0 eq) prepared in step 5 and SnCl₂.2H₂O (6.0 eq) were dissolved in ethyl acetate (0.1 M), and then the mixture was stirred at 60 °C for 2 hours. An aqueous ammonia solution was added to the reaction mixture and then concentrated. The reaction mixture was purified using MPLC (dichloromethane:methanol) to obtain the desired compound as a yellow solid (yield: 64%, MS (ESI): m/z 447 [M+H]⁺).

### [Step 7] Preparation of diethyl(2-((4-((4-([1,2,4]triazolo[1,5-a]pyridin-7-yloxy)-2-(methoxy-d₃)-5-methylphenyl)amino)-7-methoxyquinazolin-6-yl)amino)-1-fluoro-2-oxoethyl)phosphonate

2-(Diethoxyphosphoryl)-2-fluoroacetic acid (2.0 eq) and HATU (3.0 eq) were dissolved in DMF (0.5 M), followed by stirring at 50 °C for 30 minutes. *N*⁴-(4-([1,2,4]Triazolo[1,5-*a*]pyridin-7-yloxy)-2-(methoxy-*d*₃)-5-methylphenyl)-7-methoxyquinazoline-4,6-diamine (1.0 eq) prepared in step 6 and DIPEA (3.0 eq) were added to the mixture, followed by stirring at 50 °C for 1 hour. Ice water was added into the reaction mixture to form a solid, and then the solid was filtered to obtain the desired compound as a yellow solid (yield: 100%, MS (ESI): m/z 643 [M+H]⁺).

### [Step 8] Preparation of tert-butyl (R,E/Z)-2-(3-((4-((4-([1,2,4]triazolo[1,5-a]pyridin-7-yloxy)-2-(methoxy-d₃)-5-methylphenyl)amino)-7-methoxyquinazolin-6-yl)amino)-2-fluoro-3-oxoprop-1-en-1-yl)pyrrolidine-1-carboxylate

Diethyl(2-((4-((4-([1,2,4]triazolo[1,5-*a*]pyridin-7-yloxy)-2-(methoxy-(*d*₃)-5-methylphenyl)amino)-7-methoxyquinazolin-6-yl)amino)-1-fluoro-2-oxoethyl)phosphonate (1.0 eq) obtained in step 7 was dissolved in DMF (0.5 M), and then tert-butyl (*R*)*-2-*formylpyrrolidine-1-carboxylate (1.5 eq) and MgSO₄ (1.5 eq) were added thereto at 0 °C, followed by stirring at room temperature for 1 hour. After water was added to the reaction mixture and organic materials were extracted with dichloromethane, remaining water in collected organic layer was removed using Na₂SO₄, and the collected organic layer was concentrated. The obtained desired compound as a brown solid was used in the following reaction without further purification (yield: 100%, MS (ESI): m/z 688 [M+H]⁺).

### [Step 9] Preparation of (R,E/Z)-N-(4-((4-([1,2,4]triazolo[1,5-a]pyridin-7-yloxy)-2-(methoxy-d₃)-5-methylphenyl)amino)-7-methoxyquinazolin-6-yl)-2-fluoro-3-(pyrrolidin-2-yl)acrylamide

tert-Butyl (*R,E*/*Z*)-2-(3-((4-((4-([1,2,4]triazolo[1,5-*a*]pyridin-7-yloxy)-2-(methoxy-*d*₃)-5-methylphenyl)amino)-7-methoxyquinazolin-6-yl)amino)-2-fluoro-3-oxoprop-1-en-1-yl)pyrrolidine-1-carboxylate (1.0 eq) obtained in step 8 was dissolved in dichloromethane (0.1 M), TFA (20.0 eq) was then added thereto, and the mixture was stirred at room temperature for 1 hour and then concentrated. The concentrated mixture was purified using MPLC (dichloromethane:methanol) to obtain the desired compound as a brown liquid (yield: 100%, MS (ESI): m/z 588 [M+H]⁺).

### [Step 10] Preparation of (R,Z)-N-(4-((4-([1,2,4]triazolo[1,5-a]pyridin-7-yloxy)-2-(methoxy-d₃)-5-methylphenyl)amino)-7-methoxyquinazolin-6-yl)-2-fluoro-3-(1-methylpyrrolidin-2-yl)acrylamide and (R,E)-N-(4-((4-([1,2,4]triazolo[1,5-a]pyridin-7-yloxy)-2-(methoxy-d₃)-5-methylphenyl)amino)-7-methoxyquinazolin-6-yl)-2-fluoro-3-(1-methylpyrrolidin-2-yl)acrylamide

(*R,E*/*Z*)-*N*-(4-((4-([1,2,4]Triazolo[1,5-*a*]pyridin-7-yloxy)-2-(methoxy-*d*₃)-5-methylphenyl)amino)-7-methoxyquinazolin-6-yl)-2-fluoro-3-(pyrrolidin-2-yl)acrylamide (1.0 eq) obtained in step 9 and 37% formaldehyde (5.0 eq) were dissolved in DCE (0.2 M), followed by stirring at room temperature for 1 hour. Sodium triacetoxyborohydride (4.0 eq) was added to the mixture and then stirred at 80 °C for 1 hour. To the reaction mixture, a saturated aqueous NaHCO₃ solution and dichloromethane were added to extract organic materials, and remaining water was removed using MgSO₄, and the organic layer was concentrated. The reaction mixture was purified using MPLC (dichloromethane:methanol) to obtain the desired compound as a yellow solid. ((*R,E*)-*N*-(4-((4-([1,2,4]Triazolo[1,5-*a*]pyridin-7-yloxy)-2-(methoxy-*d*₃)-5-methylphenyl)amino)-7-methoxyquinazolin-6-yl)-2-fluoro-3-(1-methylpyrrolidin-2-yl)acrylamide - yield: 11%, MS (ESI): m/z 602 [M+H]⁺, ((R,Z)-N-(4-((4-([1,2,4]triazolo[1,5-*a*]pyridin-7-yloxy)-2-(methoxy-*d*₃)-5-methylphenyl)amino)-7-methoxyquinazolin-6-yl)-2-fluoro-3-(1-methylpyrrolidin-2-yl)acrylamide - yield: 4%, MS (ESI): m/z 602 [M+H]⁺).

### Preparation of Compounds of Examples 148, 151, and 152

The compounds of Examples 148, 151, and 152 according to the present invention were prepared in a similar manner to Preparation Example 15.

The chemical structural formulas, compound names, ¹H NMR, MS, yields, HPLC data, and purities of the compounds of Examples 148 to 152 are summarized in Table 15 below.

**[Table 15]**

| Ex. | Structure | Compound name | ¹H NMR, MS | Yield (%) | HPLC (min), Purity (%) |
|---|---|---|---|---|---|
| 148 | 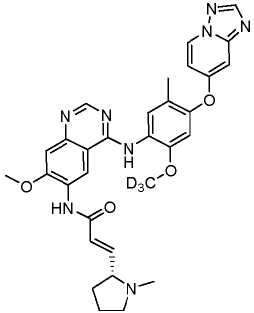 | (*R*,*E*)-*N*-(4-((4-([1,2,4]triazolo[1, 5-*a*]pyridin-7-yloxy)-2-(methoxy-*d*₃)-5-methylphenyl)am ino)-7-methoxyquinazoli n-6-yl)-3-(1-methylpyrrolidin-2-yl)acrylamide | ¹H NMR (400 MHz,FA salt, Methanol-d₄) δ 9.03 (s, 1H), 8.74 (d, J = 7.6 Hz, 1H), 8.43 (s, 1H), 8.34 (s, 1H), 8.28 (s, 1H), 7.93 (s, 1H), 7.21 (s, 1H), 7.08 (d, J = 7.6 Hz, 1H), 6.97 - 6.89 (m, 3H), 6.81 - 6.77 (m, 1H), 4.08 (s, 3H), 3.97 - 3.90 (m, 1H), 3.70 - 3.64 (m, 1H), 3.17 - 3.10 (m, 1H), 2.83 (m , 3H), 2.42 - 2.38 (m, 1H), 2.21 - 2.01 (m, 6H); MS (ESI): m/z 584 [M+H]⁺ | 4 | 9.74, 100 |
| 149 | 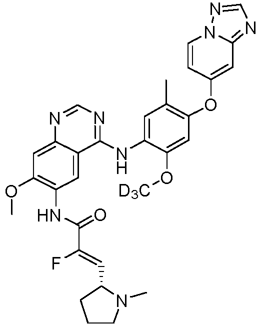 | (*R*,*Z*)*-N-*(4-((4-([1,2,4]triazolo[1, 5-*a*]pyridin-7-yloxy)-2-(methoxy-*d*₃)-5-methylphenyl)am ino)-7-methoxyquinazoli n-6-yl)-2-fluoro-3-(1-methylpyrrolidin-2-yl)acrylamide | ¹H NMR (400 MHz, Free, DMSO-d₆) δ 9.71 (s, 1H), 9.28 (s, 1H), 8.95 (d, J = 7.5 Hz, 1H), 8.67 (s, 1H), 8.43 (s, 1H), 8.39 (s, 1H), 7.58 (s, 1H), 7.28 (s, 1H), 7.09 - 7.01 (m, 1H), 6.98 (s, 1H), 6.81 (d, J = 2.6 Hz, 1H), 6.16 - 5.90 (m, 1H), 3.99 (s, 3H), 3.04 - 2.97 (m, 1H), 2.24 - 2.15 (m, 5H), 2.10 (s, 3H), 1.83 - 1.70 (m, 2H), 1.65 - 1.50 (m, 2H); MS (ESI): m/z 602 [M+H]⁺ | 4 | 93* |
| 150 | 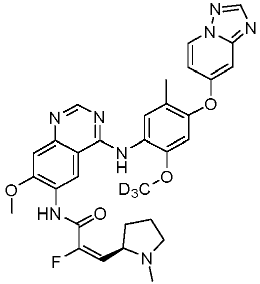 | (*R*,*E*)*-N-*(4-((4-([1,2,4]triazolo[1, 5-*a*]pyridin-7-yloxy)-2-(methoxy-*d*₃)-5-methylphenyl)am ino)-7-methoxyquinazoli n-6-yl)-2-fluoro-3-(1-methylpyrrolidin-2-yl)acrylamide | ¹H NMR (400 MHz, Free, DMSO-d₆) δ 10.15 (s, 1H), 9.31 (s, 1H), 8.95 (d, J = 7.4 Hz, 1H), 8.72 (s, 1H), 8.43 (d, J = 4.0 Hz, 1H), 8.39 (s, 1H), 7.57 (d, J = 10.0 Hz, 1H), 7.28 (s, 1H), 7.12 - 7.00 (m, 1H), 6.98 (s, 1H), 6.80 (d, J = 2.7 Hz, 1H), 6.04 - 5.84 (m, 1H), 4.00 (s, 3H), 3.07 (s, 1H), 2.30 - 2.20 (m, 3H), 2.10 (s, 5H), 1.78 (s, 2H), 1.60 (s, 2H); MS (ESI): m/z 602 [M+H]⁺ | 11 | 89* |
| 151 | 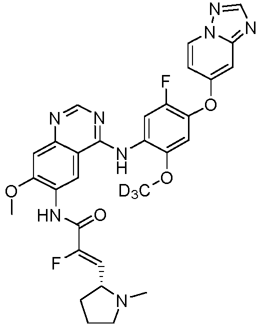 | *(R,Z)-N-(4-((4-*([1,2,4]triazolo[1, 5-*a*]pyridin-7-yloxy)-5-fluoro-2-(methoxy-*d*₃)phenyl)amino) -7-methoxyquinazoli n-6-yl)-2-fluoro-3-(1-methylpyrrolidin-2-yl)acrylamide | ¹H NMR (400 MHz, Free, CDCl₃) δ 9.09 (s, 1H), 8.90-8.84 (m, 2H), 8.71 (s, 1H), 8.48 (d, J = 7.2 Hz, 1H), 8.22 (s, 1H), 6.98 (s, 1H), 6.90-6.87 (m, 1H), 6.76 (d, J = 6.8 Hz, 1H), 6.32-6.20 (m, 1H), 4.05 (s, 3H), 3.26-3.20 (m, 1H), 3.13-3.09 (m, 1H), 2.32-2.24 (m, 4H), 2.10-2.03 (m, 1H), 1.95-1.80 (m, 2H), 1.75-1.72 (m, 1H); MS (ESI): m/z 606 [M+H]⁺ | 5 | 7.55, 97 |
| 152 | 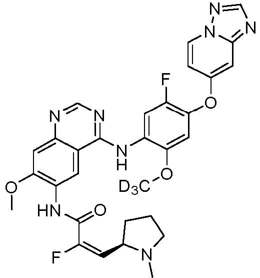 | (*R*,*E*)*-N-*(4-((4-([1,2,4]triazolo[1, 5-*a*]pyridin-7-yloxy)-5-fluoro-2-(methoxy-*d*₃)phenyl)amino) -7-methoxyquinazoli n-6-yl)-2-fluoro-3-(1-methylpyrrolidin-2-yl)acrylamide | ¹H NMR (400 MHz, Free, CDCl₃) δ 9.09 (s, 1H), 8.90-8.84 (m, 2H), 8.71 (s, 1H), 8.48 (d, J = 7.2 Hz, 1H), 8.22 (s, 1H), 6.98 (s, 1H), 6.90-6.87 (m, 1H), 6.76 (d, J = 6.8 Hz, 1H), 6.32-6.20 (m, 1H), 4.05 (s, 3H), 3.26-3.20 (m, 1H), 3.13-3.09 (m, 1H), 2.32-2.24 (m, 4H), 2.10-2.03 (m, 1H), 1.95-1.80 (m, 2H), 1.75-1.72 (m, 1H); MS (ESI): m/z 606 [M+H]⁺ | 5 | 7.55, 97 |

| | | | | | |
|---|---|---|---|---|---|
| (*: When "*" is indicated to the right of the number listed in the last column, the number means NMR purity.) | | | | | |

### Preparation Example 16: Preparation of (R,Z)-N-(4-((4-([1,2,4]triazolo[1,5-a]pyridin-7-yloxy)-5-fluoro-2-methoxyphenyl)amino)-7-methoxyquinazolin-6-yl)-2-fluoro-3-(1-(methyl-d₃)pyrrolidin-2-yl)acrylamide (Example 155) and (R,E)-N-(4-((4-([1,2,4]triazolo[1,5-a]pyridin-7-yloxy)-5-fluoro-2-methoxyphenyl)amino)-7-methoxyquinazolin-6-yl)-2-fluoro-3-(1-(methyl-d₃)pyrrolidin-2-yl)acrylamide (Example 157)

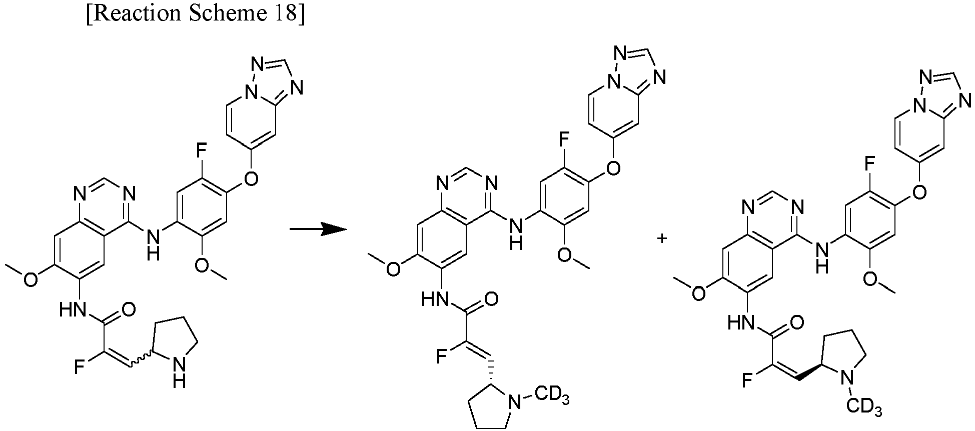

(*R,E*/*Z*)-*N*-(4-((4-([1,2,4]Triazolo[1,5-*a*]pyridin-7-yloxy)-5-fluoro-2-methoxyphenyl)amino)-7-methoxyquinazolin-6-yl)-2-fluoro-3-(pyrrolidin-2-yl)acrylamide (1.0 eq) prepared in a similar manner to Preparation Example 12 and K₂CO₃ (1.0 eq) were dissolved in ACN (0.13 M), then iodomethane-*d*₃ (1.0 eq) dissolved in ACN (0.5 M) was added thereto at 0 °C, and the mixture was stirred at room temperature for 16 hours. Organic materials were extracted by adding water and ethyl acetate to the reaction mixture, and remaining water was removed using MgSO₄ and the organic layer was concentrated. The reaction mixture was purified using prep-HPLC to obtain the desired compound as a yellow solid. *((R,Z)-N-(4-((4-*([1,2,4]triazolo[1,5-*a*]pyridin-7-yloxy)-5-fluoro-2-methoxyphenyl)amino)-7-methoxyquinazolin-6-yl)-2-fluoro-3-(1-(methyl-*d*₃)pyrrolidin-2-yl)acrylamide - yield: 1%, MS (ESI): m/z 606 [M+H]⁺, (*R*,*E*)-*N*-(4-((4-([1,2,4]triazolo[1,5-*a*]pyridin-7-yloxy)-5-fluoro-2-methoxyphenyl)amino)-7-methoxyquinazolin-6-yl)-2-fluoro-3-(1-(methyl-*d*₃)pyrrolidin-2-yl)acrylamide - yield: 1%, MS (ESI): m/z 606 [M+H]⁺).

### Preparation of Compounds of Examples 153, 154, and 156

The compounds of Examples 153, 154 and 156 according to the present invention were prepared in a similar manner to Preparation Example 16.

The chemical structural formulas, compound names, ¹H NMR, MS, yields, HPLC data, and purities of the compounds of Examples 153 to 157 are summarized in Table 16 below.

**[Table 16]**

| Ex. | Structure | Compound name | ¹H NMR, MS | Yield (%) | HPLC (min), Purity (%) |
|---|---|---|---|---|---|
| 153 | 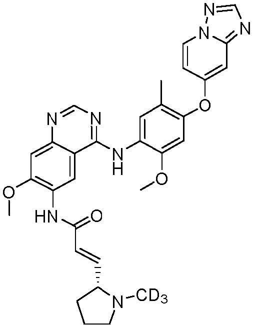 | (*R*,*E*)*-N*-(4-((4-([1,2,4]triazolo[1, 5-*a*]pyridin-7-yloxy)-2-methoxy-5-methylphenyl)am ino)-7-methoxyquinazoli n-6-yl)-3-(1-(methyl-*d*₃)pyrrolidin-2-yl)acrylamide | ¹H NMR (400MHz, Free form, MeOH-d₄) δ 9.03 (s, 1H), 8.75 (d, J = 7.6 Hz, 1H), 8.44 (d, J = 2.4 Hz, 1H), 8.29 (s, 1H), 7.92 (s, 1H), 7.26 (s, 1H), 7.10 (dd, J = 7.5, 2.7 Hz, 1H), 6.95 (s, 2H), 6.94 - 6.84 (m, 1H), 6.62 - 6.53 (m, 1H), 4.10 (s, 3H), 3.87 (s, 3H), 3.25 - 3.18 (m, 1H), 3.06 - 3.04 (m, 1H), 2.45 (q, J = 9.0 Hz, 1H), 2.19 (s, 4H), 1.95 - 1.90 (m, 2H), 1.85 - 1.75 (m, 1H); MS (ESI) : m/z 584 [M+1]⁺ | 10 | 93* |
| 154 | 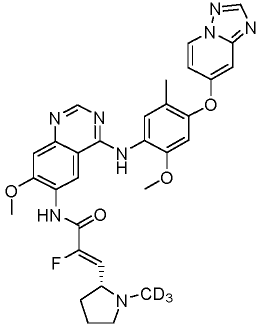 | (*R,Z*)*-N*-(4-((4-([1,2,4]triazolo[1, 5-*a*]pyridin-7-yloxy)-2-methoxy-5-methylphenyl)am ino)-7-methoxyquinazoli n-6-yl)-2-fluoro-3-(1-(methyl-*d*₃)pyrrolidin-2-yl)acrylamide | ¹H NMR (400 MHz, MeOD) δ 8.96 (s, 1H), 8.75 (d, J = 7.5 Hz, 1H), 8.45 (s, 1H), 8.40 (s, 1H), 8.29 (s, 1H), 7.90 (s, 1H), 7.29 (s, 1H), 7.10 (dd, J = 7.5, 2.6 Hz, 1H), 6.95 (s, 1H), 6.93 (d, J = 2.3 Hz, 1H), 6.24 (dd, J = 34.6, 9.5 Hz, 1H), 4.62 - 4.54 (m, 1H), 4.12 (s, 3H), 3.85 (s, 3H), 3.74 - 3.66 (m, 1H), 2.70 - 2.62 (m, 1H), 2.33 - 2.23 (m, 1H), 2.18 (s, 3H), 2.07 - 1.97 (m, 2H), 1.88 - 1.77 (m, 1H); MS (ESI): m/z 602 [M+H]⁺ | 10 | 7.65, 90 |
| 155 | 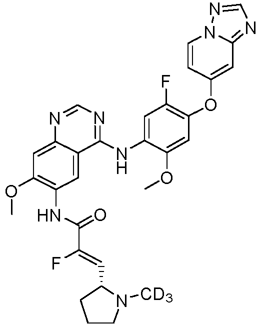 | (*R,Z*)-*N-*(4-((4-([1,2,4]triazolo[1, 5-*a*]pyridin-7-yloxy)-5-fluoro-2-methoxyphenyl)a mino)-7-methoxyquinazoli n-6-yl)-2-fluoro-3-(1-(methyl-*d*₃)pyrrolidin-2-yl)acrylamide | ¹H NMR (400 MHz, FA salt, Methanol-d₆) δ 8.98 (s, 1H), 8.78 (d, J = 6.91 Hz, 1H), 8.56 (s, 1H), 8.34-8.31 (m, 2H), 7.33 (s, 1H), 7.17-7.12 (m, 2H), 7.03 (s, 1H), 6.29 (dd, J = 33.9, 9.58 Hz, 1H), 4.13 (s, 3H), 3.99-3.91 (m, 4H), 3.49-3.43 (m, 1H), 2.89-2.82 (m, 1H), 2.37-2.30 (m, 1H), 2.14-2.07 (m, 1H), 1.95-1.87 (m, 1H); MS (ESI): m/z 606 [M+H]⁺ | 1 | 7.70, 99 |
| 156 | 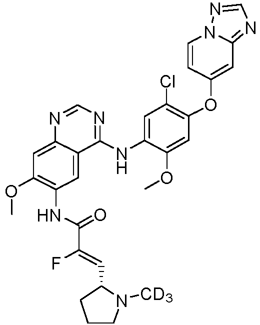 | (*R*,*Z*)-*N-*(4-((4-([1,2,4]triazolo[1, 5-*a*]pyridin-7-yloxy)-5-chloro-2-methoxyphenyl)a mino)-7-methoxyquinazoli n-6-yl)-2-fluoro-3-(1-(methyl-*d*₃)pyrrolidin-2-yl)acrylamide | ¹H NMR (400MHz, TFA salt, Methanol-d₄) δ 9.21 (s, 1H), 8.85 (d, J = 7.6 Hz, 1H), 8.77 (s, 1H), 8.40 (s, 1H), 7.91 (s, 1H), 7.41 (s, 1H), 7.30 (s, 1H), 7.19-7.16 (m, 1H), 6.95 (s, 1H), 6.45-6.37 (m, 1H), 4.51-4.49 (m, 1H), 4.22 (s, 3H), 3.89-3.84 (m, 4H), 3.19-3.15 (m, 1H), 2.53-2.52 (m, 1H), 2.29-2.22 (m, 2H), 2.19-2.01 (m, 1H); MS (ESI): m/z 622 [M+H]⁺ | 11 | 100* |
| 157 | 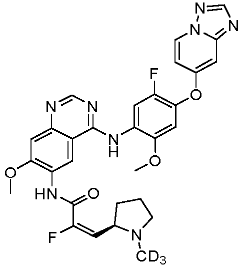 | (*R*,*E*)*-N*-(4-((4-([1,2,4]triazolo[1, 5-*a*]pyridin-7-yloxy)-5-fluoro - 2-methoxyphenyl)a mino)-7-methoxyquinazoli n-6-yl)-2-fluoro-3-(1-(methyl-*d*₃)pyrrolidin-2-yl)acrylamide | ¹H NMR (400 MHz, FA salt, Methanol-d₄) δ 8.98 (s, 1H), 8.77 (d, J = 7.44 Hz, 1H), 8.55 (s, 1H), 8.43 (s, 2H), 8.32 (s, 2H), 8.23 (d, J = 12 Hz, 1H), 7.33 (s, 1H), 7.17-7.12 (m, 2H), 7.04 (d, J = 2.08 Hz, 1H), 6.00 (dd, J = 9.68, 22 Hz, 1H), 4.73-4.67 (m, 1H), 4.12 (s, 3H), 3.94 (s, 3H), 3.49-3.43 (m, 1H), 2.92-2.83 (m, 1H), 2.48-2.39 (m, 1H), 2.12-2.04 (m, 2H), 1.90-1.80 (m, 1H); MS (ESI): m/z 606 [M+H]⁺ | 1 | 7.76, 98 |

| | | | | | |
|---|---|---|---|---|---|
| (*: When "*" is indicated to the right of the number listed in the last column, the number means NMR purity.) | | | | | |

### <Experimental Examples>

Example Compounds prepared above were evaluated through the following experiments.

### Experimental Example 1. Evaluation of Activity of Inhibiting HER2 Enzyme

A biochemical kinase assay for HER2 was conducted through Reaction Biology Corp. (San Diego, USA). Example Compounds were diluted by 3-fold from 10 µM under ATP (10 µM) conditions for HER2 enzyme to measure IC₅₀ with 10-dose, and the results are shown in Table 17 below.

**[Table 17]**

| Example | HER2 | Example | HER2 |
|---|---|---|---|
| 2 | A | 42 | A |
| 8 | A | 50 | A |
| 25 | A | 54 | A |
| 29 | A | 92 | A |
| 30 | A | 111 | A |
| 33 | A | 128 | A |

| | | | |
|---|---|---|---|
| (A: IC₅₀ ≤ 50 nM, B: 50 nM < IC₅₀ ≤100 nM, C: 100 nM < IC₅₀ ≤ 500 nM, D: 500 nM < IC₅₀) | | | |

### Experimental Example 2. Evaluation of Activity of Inhibiting BT-474 and N-87 Cell Proliferation

BT-474 cancer cells were cultured by using RPMI-1640 medium containing 20% FBS. The BT-474 cancer cells were seeded to a white clear bottom 96 well plate (Coming) at 5,000 cells per well 48 hours before the BT-474 cancer cells were treated with Example Compounds. Example Compounds were diluted in dimethylsulfoxide (3-fold diluted, total 11 concentrations) and 1 µL each was injected so that the final concentration was 0.2 nM to 10 µM. For the measurement of living cells, the cells were stored at room temperature for 10 minutes using the CellTiter-Glo cell viability reagent (Promega) after a predetermined time (120 hours) after the treatment of Example Compounds, and then the luminescence intensity was measured using a reader (SynergyNeo, Biotek). Each test was repeated three times.

N-87 cancer cells were cultured by using RPMI-1640 medium with 10% FBS. The N-87 cancer cells were seeded to a white clear bottom 96 well plate (Coming) at 5,000 cells per well 24 hours before the BT-474 cancer cells were treated with Example Compounds. Example Compounds were diluted in dimethylsulfoxide (5-fold diluted, total 11 concentrations) and 0.5 µL each was injected so that the final concentration was 0.5 µM to 5 µM. For the measurement of living cells, the cells were stored at room temperature for 10 minutes using the CellTiter-Glo cell viability reagent (Promega) after 72 hours after the treatment of Example Compounds, and then the luminescence intensity was measured using the reader (SynergyNeo, Biotek).

The result value was calculated as a cell growth rate (%) compared to a control group. Graphs were plotted and GI₅₀ values were calculated using the GraphPad Prism version 8.0 program, and the results are shown in Table 18 below.

**[Table 18]**

| Example | GI₅₀ | | Example | GI₅₀ | | Example | GI₅₀ | |
|---|---|---|---|---|---|---|---|---|
| | BT-474 | N-87 | | BT-474 | N-87 | | BT-474 | N-87 |
| 1 | A | A | 51 | A | A | 102 | A | - |
| 2 | A | A | 52 | A | A | 103 | A | - |
| 3 | A | A | 54 | A | - | 104 | A | A |
| 4 | C | - | 55 | A | A | 105 | D | - |
| 5 | A | A | 56 | A | - | 106 | A | - |
| 6 | A | A | 57 | A | - | 107 | A | - |
| 7 | A | A | 58 | A | - | 109 | D | - |
| 8 | A | A | 59 | A | - | 111 | A | A |
| 9 | B | A | 60 | A | - | 112 | A | - |
| 10 | C | - | 61 | A | - | 114 | A | - |
| 11 | A | - | 63 | A | - | 120 | A | - |
| 14 | A | - | 64 | A | - | 123 | B | - |
| 15 | A | A | 65 | A | - | 125 | B | - |
| 16 | A | A | 68 | A | - | 126 | C | - |
| 18 | A | - | 70 | A | - | 127 | C | - |
| 19 | A | - | 71 | A | - | 128 | A | A |
| 21 | C | - | 72 | A | - | 131 | A | - |
| 22 | B | - | 73 | A | - | 132 | C | - |
| 23 | D | - | 74 | A | A | 133 | A | - |
| 24 | A | A | 76 | A | A | 134 | A | A |
| 25 | A | A | 79 | A | - | 135 | A | - |
| 26 | A | - | 80 | A | - | 136 | A | A |
| 27 | A | - | 81 | A | - | 137 | B | - |
| 28 | A | - | 82 | A | - | 139 | A | - |
| 29 | A | A | 84 | A | - | 140 | A | - |
| 30 | A | - | 85 | A | - | 141 | A | - |
| 31 | A | A | 86 | A | - | 144 | D | - |
| 32 | A | A | 87 | A | - | 147 | C | - |
| 33 | A | A | 88 | A | - | 148 | A | - |
| 38 | A | - | 89 | A | - | 149 | A | - |
| 40 | A | - | 90 | A | - | 150 | A | - |
| 41 | A | A | 91 | A | A | 151 | A | - |
| 42 | A | A | 92 | A | - | 152 | A | - |
| 43 | A | - | 93 | A | A | 153 | A | - |
| 44 | A | - | 94 | A | - | 154 | A | - |
| 46 | A | A | 95 | A | A | 155 | A | - |
| 47 | A | - | 97 | A | - | 156 | A | - |
| 48 | A | - | 98 | A | - | 157 | A | - |
| 49 | A | - | 99 | A | - | | | |
| 50 | A | A | 101 | A | - | | | |

| | | | | | | | | |
|---|---|---|---|---|---|---|---|---|
| (A: GI₅₀ ≤ 50 nM, B: 50 nM < GI₅₀ ≤ 100 nM, C: 100 nM < GI₅₀ ≤ 500 nM, D: 500 nM < GI₅₀) | | | | | | | | |

### Experimental Example 3. Evaluation of Activity of Inhibiting Transduced Ba/F3 Cell Proliferation

Transduced Ba/F3 cells were cultured by adding 1 µg/mL of puromycin (Invitrogen) to a medium (RPMI-1640) containing 10% fetal bovine serum (FBS). 3,000 Ba/F3 HER2 WT cells were seeded to a white clear bottom 96 well plate (Coming) 24 hours before the Ba/F3 HER2 WT cells were treated with Example compounds. For Ba/F3 HER2 L755S, Ba/F3 HER2 L755P, Ba/F3 HER2 T862A, and Ba/F3 HER2 L869R/T798I cells, 5,000 cells each were seeded to a white clear bottom 96 well plate (Coming) 24 hours before the cells were treated with the compounds. Example Compounds were diluted in dimethylsulfoxide (3-fold diluted, total 11 concentrations) and 0.5 µL each was injected so that the final concentration was 0.2 nM to 10 µM. For the measurement of living cells, the cells were stored at room temperature for 10 minutes using the CellTiter-Glo cell viability reagent (Promega) after 72 hours after the treatment of Example Compounds, and then the luminescence intensity was measured using the reader (SynergyNeo, Biotek). Each test was repeated twice.

The result value was calculated as a cell growth rate (%) compared to a control group, and the GI₅₀ values were calculated using the software (GraphPad Prism version 8.0), and the results are shown in Table 19 below.

**[Table 19]**

| Example | Ba/F3 (GI₅₀) | | | | |
|---|---|---|---|---|---|
| | HER2 WT | HER2 L755S | HER2 L755P | HER2 T862A | HER2 L869R/T798I |
| 42 | A | A | A | A | A |
| 50 | A | A | A | A | A |
| 128 | A | A | A | A | A |

| | | | | | |
|---|---|---|---|---|---|
| (A: GI₅₀ ≤ 50 nM, B: 50 nM < GI₅₀ ≤100 nM, C: 100 nM < GI₅₀ ≤ 500 nM, D: 500 < GI₅₀ nM) | | | | | |

Through the results of the Experimental Examples, it was confirmed that Example Compounds of the present invention exhibited high inhibitory activity against HER2 and inhibited the proliferation of BER2-activated cells.

Although the present invention has been described in detail with reference to the preferred embodiments and experimental examples, the scope of the present invention is not limited to the specific example compounds, and should be construed by the appended claims. Also, those skilled in the art will understand that many modifications and variations can be made without departing from the scope of the present invention.

## Claims

1. A compound represented by Formula (I), or a pharmaceutically acceptable salt or stereoisomer thereof: 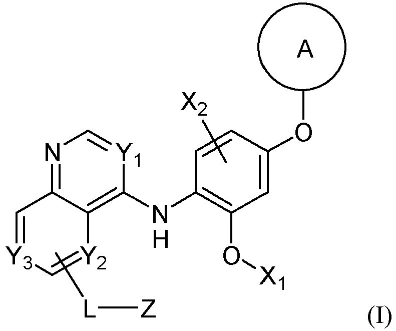 wherein:
ring A is an aromatic or non-aromatic C₆₋₁₂ carbocycle, or 5-12 membered heterocycle, wherein ring A has or does not have one or more substituents selected from the group consisting of C₁₋₆ alkyl, C₁₋₆ hydroxyalkyl, C₁₋₆ haloalkyl, -CN, -NR_{A1}R_{A2}, -NO₂, -OR_{A3}, -C(=O)-NR_{A1}R_{A2}, -C(=O)-OR_{A3}, -NH-C(=O)-(C₁₋₆ alkyl), =O, halo, and 5-6 membered heteroaryl, wherein the heteroaryl is unsubstituted or substituted with C₁₋₆ alkyl, C₁₋₆ haloalkyl, C₁₋₆ alkoxy, or halo;
R_{A1} to R_{A3} are each independently H or C₁₋₆ alkyl;
X₁ is C₁₋₆ alkyl, deuterated C₁₋₆ alkyl, deuterated C₁₋₆ haloalkyl, C₁₋₆ haloalkyl, C₃₋₁₂ cycloalkyl, or 3-12 membered heterocycloalkyl, wherein the cycloalkyl and heterocycloalkyl are unsubstituted or substituted with C₁₋₆ alkyl or C₁₋₆ haloalkyl;
X₂ is H, C₁₋₆ alkyl, C₃₋₆ cycloalkyl, C₁₋₆ hydroxyalkyl, C₁₋₆ haloalkyl, -CN, -NR_{B1}R_{B2}, - NO₂, -OR_{B3}, or halo;
R_{B1} to R_{B3} are each independently H or C₁₋₆ alkyl;
Y₁ is CR₁ or N;
Y₂ is CR₂, C(-L-Z), or N;
Y₃ is CR₃ or N;
R₁ is H, C₁₋₆ alkyl, C₁₋₆ alkylamino, or -CN;
R₂ is H, C₁₋₆ alkyl, C₁₋₆ hydroxyalkyl, C₁₋₆ alkylamino, C₁₋₆ haloalkyl, -CN, -NH₂, or halo;
R₃ is H, halo, -OR₄, or -NR₅R₆, or R₃ is linked to L to form a 5-6 membered heterocycle;
R₄ is H, C₁₋₆ alkyl, -(CH₂)n-O-(C₁₋₆ alkyl), C₁₋₆ hydroxyalkyl, C₁₋₆ haloalkyl, -(CH₂)n-(C₃₋₆ cycloalkyl), 3-6 membered heterocycloalkyl, or -(CH₂)n-(3-6 membered heterocycloalkyl), wherein the cycloalkyl and heterocycloalkyl have or do not have one or more substituents selected from the group consisting of C₁₋₆ alkyl, C₁₋₆ hydroxyalkyl, C₁₋₆ haloalkyl, -CN, - NR_{C1}R_{C2}, -NO₂, -OR_{C3}, and halo;
n is each independently an integer of 1 to 4;
R_{C1} to R_{C3} are each independently H or C₁₋₆ alkyl;
R₅ and R₆ are each independently H or C₁₋₆ alkyl, or R₅ and R₆ are linked to each other to form a 3-12 membered heterocycle, wherein the heterocycle is unsubstituted or substituted with C₁₋₆ alkyl, C₁₋₆ hydroxyalkyl, C₁₋₆ haloalkyl, -CN, -NR_{D1}R_{D2}, -NO₂, -OR_{D3}, halo, or 3-6 membered heterocycloalkyl;
R_{D1} to R_{D3} are each independently H or C₁₋₆ alkyl;
L is -NH-, 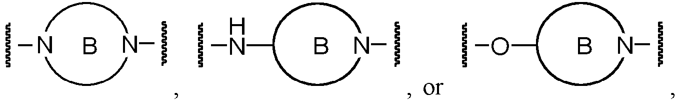 or L is linked to R₃ to form a 5-6 membered heterocycle, wherein ring B is a 3-8 membered heterocycle containing N atom in the ring, and ring B has or does not have one or more substituents selected from the group consisting of C₁₋₆ alkyl, C₁₋₆ hydroxyalkyl, C₁₋₆ alkylamino, C₁₋₆ haloalkyl, -OH, and halo;
Z is 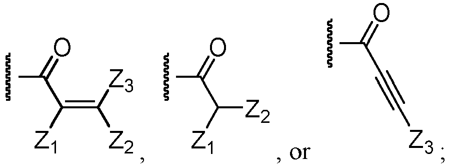
Z₁ is H, C₁₋₆ alkyl, C₁₋₆ haloalkyl, -CN, halo, or 5-6 membered heteroaryl;
Z₂ and Z₃ are each independently H, C₁₋₆ alkyl, C₁₋₆ hydroxyalkyl, C₁₋₆ alkylamino, C₁₋₆ haloalkyl, halo, C₃₋₁₂ cycloalkyl, 3-12 membered heterocycloalkyl, -(CH₂)m-(C₁₋₆ alkylamino), - (CH₂)m-(C₃₋₁₂ cycloalkyl), -(CH₂)m-(3-12 membered heterocycloalkyl), or -(CH₂)m-NH-(3-12 membered heterocycloalkyl), wherein -CH₂-, the cycloalkyl, and the heterocycloalkyl have or do not have one or more substituents selected from the group consisting of C₁₋₆ alkyl, deuterated C₁₋₆ alkyl, deuterated C₁₋₆ haloalkyl, -OH, =O, -C(=O)-(C₁₋₆ alkoxy), and 3-6 membered heterocycloalkyl;
m is each independently an integer of 1 to 4; and
the heterocycle, the heteroaryl, and the heterocycloalkyl each independently contain at least one heteroatom selected from the group consisting of N, S, and O.

2. The compound, or a pharmaceutically acceptable salt or stereoisomer thereof according to claim 1, wherein:
ring A is an aromatic C₆₋₁₂ carbocycle, or an aromatic or non-aromatic 5-12 membered heterocycle, wherein ring A has or does not have one or more substituents selected from the group consisting of C₁₋₆ alkyl, -NR_{A1}R_{A2}, -OR_{A3}, -C(=O)-NR_{A1}R_{A2}, -C(=O)-OR_{A3}, -NH-C(=O)-(C₁₋₆ alkyl), =O, halo, and 5-6 membered heteroaryl, wherein the heteroaryl is unsubstituted or substituted with C₁₋₆ alkyl, C₁₋₆ alkoxy, or halo;
R_{A1} to R_{A3} are each independently H or C₁₋₆ alkyl;
X₁ is C₁₋₆ alkyl, deuterated C₁₋₆ alkyl, C₁₋₆ haloalkyl, C₃₋₁₂ cycloalkyl, or 3-12 membered heterocycloalkyl, wherein the cycloalkyl and heterocycloalkyl are unsubstituted or substituted with C₁₋₆ alkyl or C₁₋₆ haloalkyl;
X₂ is H, C₁₋₆ alkyl, C₃₋₆ cycloalkyl, C₁₋₆ haloalkyl, -NR_{B1}R_{B2}, or halo;
R_{B1} and R_{B2} are each independently C₁₋₆ alkyl; and
the heterocycle, the heteroaryl, and the heterocycloalkyl each independently contain at least one heteroatom selected from the group consisting of N, S, and O.

3. The compound, or a pharmaceutically acceptable salt or stereoisomer thereof according to claim 1, wherein:
Y₁ is CR₁ or N;
Y₂ is CR₂, C(-L-Z), or N;
Y₃ is CR₃ or N;
R₁ is H or -CN;
R₂ is H or halo;
R₃ is H, halo, -OR₄, or -NR₅R₆, or R₃ is linked to L to form a 5-6 membered heterocycle;
R₄ is C₁₋₆ alkyl, -(CH₂)n-O-(C₁₋₆ alkyl), C₁₋₆ haloalkyl, 3-6 membered heterocycloalkyl, or -(CH₂)n-(3-6 membered heterocycloalkyl), wherein the cycloalkyl and heterocycloalkyl have or do not have one or more substituents selected from the group consisting of C₁₋₆ alkyl, C₁₋₆ hydroxyalkyl, C₁₋₆ haloalkyl, -CN, -NR_{C1}R_{C2}, -NO₂, -OR_{C3}, and halo;
n is each independently an integer of 1 to 4;
R_{C1} to R_{C3} are each independently H or C₁₋₆ alkyl;
R₅ and R₆ are each independently H or C₁₋₆ alkyl, or R₅ and R₆ are linked to each other to form a 3-12 membered heterocycle, wherein the heterocycle is a 4-6 membered monocyclic ring or a 5-12 membered spiro ring, and the heterocycle is unsubstituted or substituted with halo, or 3-6 membered heterocycloalkyl; and
the heterocycle and heterocycloalkyl each independently contain at least one heteroatom selected from the group consisting of N, S, and O.

4. The compound, or a pharmaceutically acceptable salt or stereoisomer thereof according to claim 1, wherein:
L is -NH-, 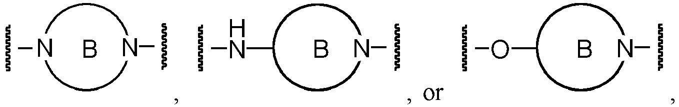
or L is linked to R₃ to form a 5-6 membered heterocycle, wherein ring B is a 3-8 membered heterocycle containing N atom in the ring, and ring B has or does not have one or more substituents selected from the group consisting of C₁₋₆ alkyl, C₁₋₆ haloalkyl, and halo;
Z is 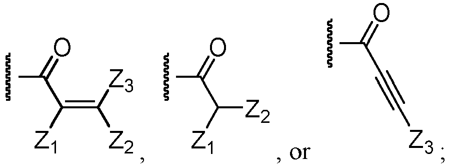
Z₁ is H, -CN, halo, or 5-6 membered heteroaryl;
Z₂ and Z₃ are each independently H, C₁₋₆ alkyl, C₁₋₆ alkylamino, halo, C₃₋₁₂ cycloalkyl, 3-12 membered heterocycloalkyl, -(CH₂)m-(C₁₋₆ alkylamino), -(CH₂)m-(C₃₋₁₂ cycloalkyl), - (CH₂)m-(3-12 membered heterocycloalkyl), or -(CH₂)m-NH-(3-12 membered heterocycloalkyl), wherein -CH₂-, the cycloalkyl, and the heterocycloalkyl have or do not have one or more substituents selected from the group consisting of C₁₋₆ alkyl, deuterated C₁₋₆ alkyl, -OH, =O, - C(=O)-(C₁₋₆ alkoxy), and 3-6 membered heterocycloalkyl;
m is each independently an integer of 1 to 4; and
the heterocycle, the heteroaryl, and the heterocycloalkyl each independently contain at least one heteroatom selected from the group consisting of N, S, and O.

5. The compound, or a pharmaceutically acceptable salt or stereoisomer thereof according to claim 1, wherein:
ring A is 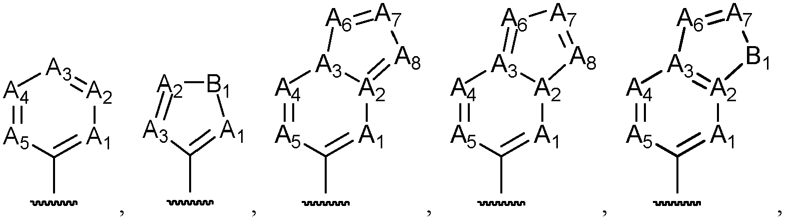 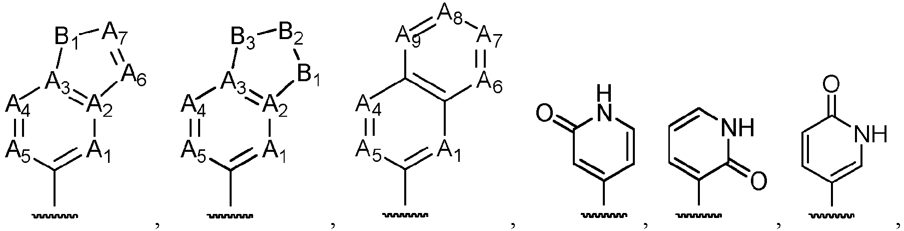 or 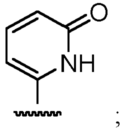
A₁ to A₉ are each independently CH or N;
B₁ to B₃ are each independently CH₂, NH, O, or S;
wherein ring A has or does not have one or more substituents selected from the group consisting of C₁₋₆ alkyl, -NR_{A1}R_{A2}, -OR_{A3}, -C(=O)-NR_{A1}R_{A2}, -C(=O)-OR_{A3}, -NH-C(=O)-(C₁₋₆ alkyl), =O, halo, and 5-6 membered heteroaryl, wherein the heteroaryl is unsubstituted or substituted with C₁₋₆ alkyl, C₁₋₆ alkoxy, or halo;
R_{A1} to R_{A3} are each independently H or C₁₋₆ alkyl; and
the heteroaryl contains at least one heteroatom selected from the group consisting of N, S, and O.

6. The compound, or a pharmaceutically acceptable salt or stereoisomer thereof according to claim 1, wherein:
L is -NH-, 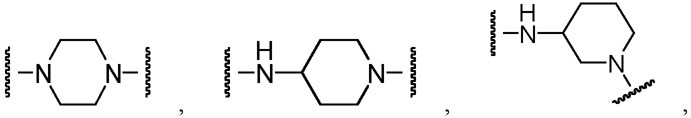 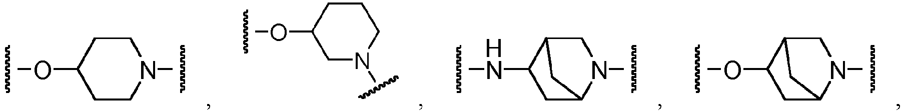 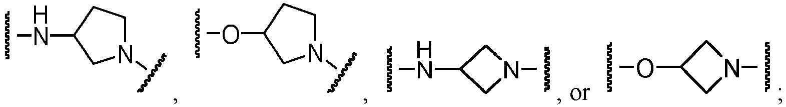
wherein the heterocycle containing N has or does not have one or more substituents selected from the group consisting of C₁₋₆ alkyl, C₁₋₆ haloalkyl, and halo, or
L is linked to R₃ to form a 5-6 membered heterocycle containing N; and
the heterocycle containing N further contains or does not contain at least one heteroatom among S and O in addition to N.

7. The compound, or a pharmaceutically acceptable salt or stereoisomer thereof according to claim 1, wherein the compound is selected from the following group:
1> 1-(4-((4-((4-([1,2,4]triazolo[1,5-*a*]pyridin-7-yloxy)-2-methoxy-5-methylphenyl)amino)-7-methoxyquinazolin-6-yl)amino)piperidin-1-yl)prop-2-en-1-one;
2> 1-(4-((4-((5-chloro-4-(3-fluorophenoxy)-2-methoxyphenyl)amino)-7-methoxyquinazolin-6-yl)amino)piperidin-1-yl)prop-2-en-1-one;
3> 1-(4-((4-((5-chloro-2-methoxy-4-(pyridin-2-yloxy)phenyl)amino)-7-methoxyquinazolin-6-yl)amino)piperidin-1-yl)prop-2-en-1-one;
4> 1-(4-((4-((4-([1,2,4]triazolo[1,5-a]pyridin-7-yloxy)-5-methyl-2-((1-methylazetidin-3-yl)oxy)phenyl)amino)-7-methoxyquinazolin-6-yl)amino)piperidin-1-yl)prop-2-en-1-one;
5> 1-(4-((4-((4-([1,2,4]triazolo[1,5-*a*]pyridin-7-yloxy)-5-chloro-2-methoxyphenyl)amino)-7-methoxyquinazolin-6-yl)oxy)piperidin-1-yl)prop-2-en-1-one;
6> 1-(4-((4-((5-chloro-4-(3-fluorophenoxy)-2-methoxyphenyl)amino)-7-methoxyquinazolin-6-yl)oxy)-3-fluoropiperidin-1-yl)prop-2-en-1-one;
7> 1-(4-((4-((4-(3-fluorophenoxy)-2-methoxyphenyl)amino)-7-methoxyquinazolin-6-yl)oxy)piperidin-1-yl)prop-2-en-1-one;
8> 1-(4-((4-((5-chloro-4-(3-fluorophenoxy)-2-methoxyphenyl)amino)-7-methoxyquinazolin-6-yl)oxy)piperidin-1-yl)prop-2-en-1-one;
9> 1-(4-((4-((4-((1-(5-fluoro-6-methylpyridin-3-yl)-1H-pyrazol-3-yl)oxy)-2-methoxyphenyl)amino)-7-methoxyquinazolin-6-yl)oxy)piperidin-1-yl)prop-2-en-1-one;
10> 1-(4-((7-methoxy-4-((2-methoxy-4-((1-(2-methoxypyrimidin-5-yl)-1H-pyrazol-3-yl)oxy)phenyl)amino)quinazolin-6-yl)oxy)piperidin-1-yl)prop-2-en-1-one;
11> 1-(4-((4-((5-chloro-2-methoxy-4-(3-methoxyphenoxy)phenyl)amino)-7-methoxyquinazolin-6-yl)oxy)piperidin-1-yl)prop-2-en-1-one;
12> 4-((4-([1,2,4]triazolo[1,5-a]pyridin-7-yloxy)-2-methoxy-5-methylphenyl)amino)-6-((1-acryloylpiperidin-4-yl)oxy)-7-methoxyquinoline-3-carbonitrile;
13> methyl 4-(4-((6-((1-acryloylpiperidin-4-yl)oxy)-7-methoxyquinazolin-4-yl)amino)-5-methoxy-2-methylphenoxy)benzoate;
14> 1-(4-((4-((4-([1,2,4]triazolo[1,5-*a*]pyridin-7-yloxy)-2-methoxy-5-methylphenyl)amino)-7-methoxyquinazolin-6-yl)oxy)piperidin-1-yl)-2-chloro-2-fluoroethan-1-one;
15> 1-(4-((4-((4-([1,2,4]triazolo[1,5-*a*]pyridin-7-yloxy)-2-methoxy-5-methylphenyl)amino)-7-methoxyquinazolin-5-yl)oxy)-3,3-difluoropiperidin-1-yl)prop-2-en-1-one;
16> 1-(3-((4-((4-([1,2,4]triazolo[1,5-*a*]pyridin-7-yloxy)-2-methoxy-5-methylphenyl)amino)-7-methoxyquinazolin-5-yl)oxy)-4,4-difluoropiperidin-1-yl)prop-2-en-1-one;
17> 1-(4-((4-((4-([1,2,4]triazolo[1,5-*a*]pyridin-7-yloxy)-2-methoxy-5-methylphenyl)amino)pyrido[3,4-*d*]pyrimidin-6-yl)oxy)piperidin-1-yl)prop-2-en-1-one;
18> 1-(5-((4-((4-([1,2,4]triazolo[1,5-*a*]pyridin-7-yloxy)-2-methoxy-5-methylphenyl)amino)-7-methoxyquinazolin-6-yl)oxy)-2-azabicyclo[2.2.1]heptan-2-yl)prop-2-en-1-one;
19> 1-(3-((4-((4-([1,2,4]triazolo[1,5-*a*]pyridin-7-yloxy)-2-methoxy-5-methylphenyl)amino)-7-methoxyquinazolin-6-yl)oxy)azetidin-1-yl)prop-2-en-1-one;
20> 1-(3-((4-((4-([1,2,4]triazolo[1,5-*a*]pyridin-7-yloxy)-2-methoxy-5-methylphenyl)amino)-7-methoxyquinazolin-6-yl)oxy)pyrrolidin-1-yl)prop-2-en-1-one;
21> 1-(4-((4-([1,2,4]triazolo[1,5-*a*]pyridin-7-yloxy)-2-methoxy-5-methylphenyl)amino)-7,8-dihydro-6*H*-[1,4]oxazino[3,2-*g*]quinazolin-6-yl)prop-2-en-1-one;
22> *N*-(4-((4-([1,2,4]triazolo[1,5-*a*]pyridin-7-yloxy)-2-methoxy-5-methylphenyl)amino)-7-(4-morpholinopiperidin-1-yl)quinazolin-6-yl)acrylamide;
23> *N*-(4-((4-([1,2,4]triazolo[1,5-*a*]pyridin-7-yloxy)-2-methoxy-5-methylphenyl)amino)-7-morpholinoquinazolin-6-yl)acrylamide;
24> N-(4-((5-chloro-2-methoxy-4-(pyridin-2-yloxy)phenyl)amino)-7-methoxyquinazolin-6-yl)acrylamide;
25> (*E*)*-N-(4-((5-chloro-4-(3-fluorophenoxy)-2-methoxyphenyl)amino)-7-*methoxyquinazolin-6-yl)-4-(dimethylamino)but-2-enamide;
*26>* (*E*)-*N*-(4-((4-([1,2,4]triazolo[1,5-*a*]pyridin-7-yloxy)-2-methoxy-5-methylphenyl)amino)-5-chloroquinazolin-6-yl)-4-(dimethylamino)but-2-enamide;
27> 1-(4-(8-((4-([1,2,4]triazolo[1,5-*a*]pyridin-7-yloxy)-2-methoxy-5-methylphenyl)amino)pyrimido[5,4-*d*]pyrimidin-2-yl)piperazin-1-yl)prop-2-en-1-one;
28> *N*-(4-((4-([1,2,4]triazolo[1,5-*a*]pyridin-7-yloxy)-2-methoxy-5-methylphenyl)amino)-7-methoxyquinazolin-6-yl)but-2-ynamide;
*29>* (*R*,*E*)-*N*-(4-((4-([1,2,4]triazolo[1,5-*a*]pyridin-7-yloxy)-2-methoxy-5-methylphenyl)amino)-7-methoxyquinazolin-6-yl)-3-(1-methylpyrrolidin-2-yl)acrylamide;
30> *(R,E)-N-(4-((5-chloro-4-(3-fluorophenoxy)-2-methoxyphenyl)amino)-7-*methoxyquinazolin-6-yl)-3-(1-methylpyrrolidin-2-yl)acrylamide;
31> *(R,E)-N-(4-((5-chloro-4-(3-fluorophenoxy)-2-methoxyphenyl)amino)-7-*methoxyquinazolin-6-yl)-3-(pyrrolidin-2-yl)acrylamide;
*32> (R,E)-N-(*4-((4-([1,2,4]triazolo[1,5-*a*]pyridin-7-yloxy)-5-chloro-2-methoxyphenyl)amino)-7-methoxyquinazolin-6-yl)-3-(1-methylpyrrolidin-2-yl)acrylamide;
33> *(R,E)-N-(4-((5-chloro-2-methoxy-4-(pyridin-2-yloxy)phenyl)amino)-7-*methoxyquinazolin-6-yl)-3-(1-methylpyrrolidin-2-yl)acrylamide;
34> *(R,E)-N-(8-(*(4-([1,2,4]triazolo[1,5-*a*]pyridin-7-yloxy)-2-methoxy-5-methylphenyl)amino)pyrimido[5,4-*d*]pyrimidin-2-yl)-3-(1-methylpyrrolidin-2-yl)acrylamide;
35> (E)-N-(4-((4-([1,2,4]triazolo[1,5-*a*]pyridin-7-yloxy)-2-methoxy-5-methylphenyl)amino)-7-methoxyquinazolin-6-yl)-3-(2-methyl-2-azabicyclo[3.1.0]hexan-3-yl)acrylamide;
36> (*E*)-*N*-(4-((4-([1,2,4]triazolo[1,5-*a*]pyridin-7-yloxy)-2-methoxy-5-methylphenyl)amino)-7-methoxyquinazolin-6-yl)-3-(1-methylpiperidin-2-yl)acrylamide;
*37>* (*S*,*E*)-*N*-(4-((4-([1,2,4]triazolo[1,5-*a*]pyridin-7-yloxy)-2-methoxy-5-methylphenyl)amino)-7-methoxyquinazolin-6-yl)-3-(1-methylpyrrolidin-2-yl)acrylamide;
38> (*E*)-*N*-(4-((4-([1,2,4]triazolo[1,5-*a*]pyridin-7-yloxy)-2-methoxy-5-methylphenyl)amino)-7-methoxyquinazolin-6-yl)-4-((1,1-dioxidotetrahydro-2H-thiopyran-4-yl)amino)but-2-enamide;
39> (*E*)-*N*-(4-((4-([1,2,4]triazolo[1,5-*a*]pyridin-7-yloxy)-2-methoxy-5-methylphenyl)amino)-7-methoxyquinazolin-6-yl)-4-(5-hydroxyhexahydrocyclopenta[c]pyrrol-2(1H)-yl)but-2-enamide;
40> (*E*)-*N*-(4-((4-([1,2,4]triazolo[1,5-*a*]pyridin-7-yloxy)-2-methoxy-5-methylphenyl)amino)-7-methoxyquinazolin-6-yl)-4-(2-oxa-6-azaspiro[3.3]heptan-6-yl)but-2-enamide;
41> (*R,E*)-*N*-(4-((4-([1,2,4]triazolo[1,5-*a*]pyridin-7-yloxy)-2-methoxy-5-methylphenyl)amino)-7-methoxyquinazolin-6-yl)-2-fluoro-3-(1-methylpyrrolidin-2-yl)acrylamide;
*42>* (*R*,*E*)-*N*-(4-((5-chloro-4-(3-fluorophenoxy)-2-methoxyphenyl)amino)-7-methoxyquinazolin-6-yl)-2-fluoro-3-(1-methylpyrrolidin-2-yl)acrylamide;
43> (*Z*)-*N*-(4-((4-([1,2,4]triazolo[1,5-*a*]pyridin-7-yloxy)-2-methoxy-5-methylphenyl)amino)-7-methoxyquinazolin-6-yl)-2-fluoro-3-(1-methylpyrrolidin-2-yl)acrylamide;
44> (*S,E*)-*N*-(4-((4-([1,2,4]triazolo[1,5-*a*]pyridin-7-yloxy)-2-methoxy-5-methylphenyl)amino)-7-methoxyquinazolin-6-yl)-2-fluoro-3-(1-methylpyrrolidin-2-yl)acrylamide;
45> (*R,E*)-N-(4-((4-([1,2,4]triazolo[1,5-*a*]pyridin-7-yloxy)-2-methoxy-5-methylphenyl)amino)-7-methoxyquinazolin-6-yl)-2-fluoro-3-(1-methyl-5-oxopyrrolidin-2-yl)acrylamide;
46> (*R*,*E*)-*N*-(4-((4-([1,2,4]triazolo[1,5-*a*]pyridin-7-yloxy)-2-methoxyphenyl)amino)-7-methoxyquinazolin-6-yl)-2-fluoro-3-(1-methylpyrrolidin-2-yl)acrylamide;
*47>* (*R*,*E*)-*N*-(4-((4-([1,2,4]triazolo[1,5-*c*]pyrimidin-7-yloxy)-2-methoxy-5-methylphenyl)amino)-7-methoxyquinazolin-6-yl)-2-fluoro-3-(1-methylpyrrolidin-2-yl)acrylamide;
48> (*R,Z*)-N-(4-((4-([1,2,4]triazolo[1,5-c]pyrimidin-7-yloxy)-2-methoxy-5-methylphenyl)amino)-7-methoxyquinazolin-6-yl)-2-fluoro-3-(1-methylpyrrolidin-2-yl)acrylamide;
49> (*R*,*E*)-*N*-(4-((4-([1,2,4]triazolo[1,5-*a*]pyridin-7-yloxy)-5-fluoro-2-methoxyphenyl)amino)-7-methoxyquinazolin-6-yl)-2-fluoro-3-(1-methylpyrrolidin-2-yl)acrylamide;
50> (*R*,*Z*)-*N*-(4-((4-([1,2,4]triazolo[1,5-*a*]pyridin-7-yloxy)-5-fluoro-2-methoxyphenyl)amino)-7-methoxyquinazolin-6-yl)-2-fluoro-3-(1-methylpyrrolidin-2-yl)acrylamide;
51> (*R*,*E*)-*N*-(4-((4-([1,2,4]triazolo[1,5-*a*]pyridin-7-yloxy)-5-chloro-2-methoxyphenyl)amino)-7-methoxyquinazolin-6-yl)-2-fluoro-3-(1-methylpyrrolidin-2-yl)acrylamide;
52> (*R*,*E*)-*N*-(4-((4-([1,2,4]triazolo[1,5-*a*]pyridin-7-yloxy)-2-methoxy-5-methylphenyl)amino)-7-methoxyquinazolin-6-yl)-3-(1-ethylpyrrolidin-2-yl)-2-fluoroacrylamide;
53> (*R*,*E*)-*N*-(4-((4-([1,2,4]triazolo[1,5-*a*]pyridin-7-yloxy)-2-methoxy-5-(trifluoromethyl)phenyl)amino)-7-methoxyquinazolin-6-yl)-2-fluoro-3-(1-methylpyrrolidin-2-yl)acrylamide;
54> (*R*,*Z*)-*N*-(4-((4-([1,2,4]triazolo[1,5-*a*]pyridin-7-yloxy)-2-methoxy-5-(trifluoromethyl)phenyl)amino)-7-methoxyquinazolin-6-yl)-2-fluoro-3-(1-methylpyrrolidin-2-yl)acrylamide;
55> (*R*,*E*)-2-fluoro-*N*-(7-methoxy-4-((2-methoxy-5-methyl-4-((1-methyl-1*H-*benzo[*d*]imidazol-6-yl)oxy)phenyl)amino)quinazolin-6-yl)-3-(1-methylpyrrolidin-2-yl)acrylamide;
56> (*R,Z*)-2-fluoro-*N*-(7-methoxy-4-((2-methoxy-5-methyl-4-((1-methyl-1*H-*benzo[*d*]imidazol-5-yl)oxy)phenyl)amino)quinazolin-6-yl)-3-(1-methylpyrrolidin-2-yl)acrylamide;
57> (*R*,*E*)-2-fluoro-*N*-(7-methoxy-4-((2-methoxy-5-methyl-4-((1-methyl-1*H-*benzo[*d*]imidazol-5-yl)oxy)phenyl)amino)quinazolin-6-yl)-3-(1-methylpyrrolidin-2-yl)acrylamide;
58> (*R,Z*)-*N*-(4-((5-chloro-2-methoxy-4-((1-methyl-1H-benzo[*d*]imidazol-5-yl)oxy)phenyl)amino)-7-methoxyquinazolin-6-yl)-2-fluoro-3-(1-methylpyrrolidin-2-yl)acrylamide;
59> (*R,E*)-N-(4-((5-chloro-2-methoxy-4-((1-methyl-1*H*-benzo[*d*]imidazol-5-yl)oxy)phenyl)amino)-7-methoxyquinazolin-6-yl)-2-fluoro-3-(1-methylpyrrolidin-2-yl)acrylamide;
60> (*R,E*)-2-fluoro-*N*-(4-((5-fluoro-2-methoxy-4-((1-methyl-1*H*-benzo[*d*]imidazol-5-yl)oxy)phenyl)amino)-7-methoxyquinazolin-6-yl)-3-(1-methylpyrrolidin-2-yl)acrylamide;
61> (*R,Z*)-2-fluoro-*N*-(4-((5-fluoro-2-methoxy-4-((1-methyl-1*H*-benzo[*d*]imidazol-5-yl)oxy)phenyl)amino)-7-methoxyquinazolin-6-yl)-3-(1-methylpyrrolidin-2-yl)acrylamide;
62> (*R,E*)-2-fluoro-*N*-(7-methoxy-4-((2-methoxy-5-methyl-4-(quinoxalin-6-yloxy)phenyl)amino)quinazolin-6-yl)-3-(1-methylpyrrolidin-2-yl)acrylamide;
63> (*R,Z*)-2-fluoro-*N*-(7-methoxy-4-((2-methoxy-5-methyl-4-(quinoxalin-6-yloxy)phenyl)amino)quinazolin-6-yl)-3-(1-methylpyrrolidin-2-yl)acrylamide;
64> (*R,Z*)-2-fluoro-*N*-(7-methoxy-4-((2-methoxy-5-methyl-4-(naphthalen-2-yloxy)phenyl)amino)quinazolin-6-yl)-3-(1-methylpyrrolidin-2-yl)acrylamide;
65> (*R,E*)-2-fluoro-*N*-(7-methoxy-4-((2-methoxy-5-methyl-4-(naphthalen-2-yloxy)phenyl)amino)quinazolin-6-yl)-3-(1-methylpyrrolidin-2-yl)acrylamide;
66> (*R,E*)-*N*-(4-((4-(benzo[*d*]oxazol-6-yloxy)-2-methoxy-5-methylphenyl)amino)-7-methoxyquinazolin-6-yl)-2-fluoro-3-(1-methylpyrrolidin-2-yl)acrylamide;
67> (*R,E*)-2-fluoro-*N*-(7-methoxy-4-((2-methoxy-5-methyl-4-((2-methylbenzo[*d*]oxazol-5-yl)oxy)phenyl)amino)quinazolin-6-yl)-3-(1-methylpyrrolidin-2-yl)acrylamide;
68> (*R,Z*)-2-fluoro-*N*-(7-methoxy-4-((2-methoxy-5-methyl-4-((2-methylbenzo[*d*]oxazol-5-yl)oxy)phenyl)amino)quinazolin-6-yl)-3-(1-methylpyrrolidin-2-yl)acrylamide;
69> (*E*)-2-fluoro-*N*-(4-((4-((2-hydroxy-2,3-dihydrobenzo[d]oxazol-5-yl)oxy)-2-methoxy-5-methylphenyl)amino)-7-methoxyquinazolin-6-yl)-3-((*R*)-1-methylpyrrolidin-2-yl)acrylamide;
70> (*Z*)-2-fluoro-*N*-(4-((4-((2-hydroxy-2,3-dihydrobenzo[d]oxazol-5-yl)oxy)-2-methoxy-5-methylphenyl)amino)-7-methoxyquinazolin-6-yl)-3-((*R*)-1-methylpyrrolidin-2-yl)acrylamide;
71> (*R,E*)-2-fluoro-*N*-(7-methoxy-4-((2-methoxy-5-methyl-4-((1-methyl-2-oxo-1,2-dihydropyridin-4-yl)oxy)phenyl)amino)quinazolin-6-yl)-3-(1-methylpyrrolidin-2-yl)acrylamide;
*72> (R,E)-N-(*4-((4-([1,2,4]triazolo[1,5-*a*]pyridin-6-yloxy)-2-methoxy-5-methylphenyl)amino)-7-methoxyquinazolin-6-yl)-2-fluoro-3-(1-methylpyrrolidin-2-yl)acrylamide;
73> (*R,Z*)-N-(4-((4-([1,2,4]triazolo[1,5-*a*]pyridin-6-yloxy)-2-methoxy-5-methylphenyl)amino)-7-methoxyquinazolin-6-yl)-2-fluoro-3-(1-methylpyrrolidin-2-yl)acrylamide;
74> (*R,E*)-2-fluoro-*N*-(7-methoxy-4-((2-methoxy-5-methyl-4-phenoxyphenyl)amino)quinazolin-6-yl)-3-(1-methylpyrrolidin-2-yl)acrylamide;
75> (R,E)-2-fluoro-*N*-(4-((4-(imidazo[1,2-*a*]pyridin-7-yloxy)-2-methoxy-5-methylphenyl)amino)-7-methoxyquinazolin-6-yl)-3-(1-methylpyrrolidin-2-yl)acrylamide;
76> (*R,Z*)-2-fluoro-*N*-(4-((4-(imidazo[1,2-*a*]pyridin-7-yloxy)-2-methoxy-5-methylphenyl)amino)-7-methoxyquinazolin-6-yl)-3-(1-methylpyrrolidin-2-yl)acrylamide;
77> (*R*,*E*)-2-fluoro-*N*-(4-((4-(imidazo[1,2-*c*]pyrimidin-7-yloxy)-2-methoxy-5-methylphenyl)amino)-7-methoxyquinazolin-6-yl)-3-(1-methylpyrrolidin-2-yl)acrylamide;
78> (*R,E*)-N-(8-((4-([1,2,4]triazolo[1,5-*a*]pyridin-7-yloxy)-2-methoxy-5-methylphenyl)amino)pyrimido[5,4-*d*]pyrimidin-2-yl)-2-fluoro-3-(1-methylpyrrolidin-2-yl)acrylamide;
79> (*R*,*E*)-2-fluoro-*N*-(7-methoxy-4-((2-methoxy-5-methyl-4-(pyrazolo[1,5-*a*]pyrimidin-6-yloxy)phenyl)amino)quinazolin-6-yl)-3-(1-methylpyrrolidin-2-yl)acrylamide;
80> (*R*,*Z*)-2-fluoro-*N*-(7-methoxy-4-((2-methoxy-5-methyl-4-(pyrazolo[1,5-*a*]pyrimidin-6-yloxy)phenyl)amino)quinazolin-6-yl)-3-(1-methylpyrrolidin-2-yl)acrylamide;
81> (*R,E*)-N-(4-((4-(benzo[*d*]thiazol-6-yloxy)-2-methoxy-5-methylphenyl)amino)-7-methoxyquinazolin-6-yl)-2-fluoro-3-(1-methylpyrrolidin-2-yl)acrylamide;
82> (*R,Z*)-*N*-(4-((4-(benzo[*d*]thiazol-6-yloxy)-2-methoxy-5-methylphenyl)amino)-7-methoxyquinazolin-6-yl)-2-fluoro-3-(1-methylpyrrolidin-2-yl)acrylamide;
83> (*R,Z*)-*N*-(4-((4-(benzo[d]thiazol-5-yloxy)-2-methoxy-5-methylphenyl)amino)-7-methoxyquinazolin-6-yl)-2-fluoro-3-(1-methylpyrrolidin-2-yl)acrylamide;
84> (*R,E*)-*N*-(4-((4-(benzo[d]thiazol-5-yloxy)-2-methoxy-5-methylphenyl)amino)-7-methoxyquinazolin-6-yl)-2-fluoro-3-(1-methylpyrrolidin-2-yl)acrylamide;
85> (*R,E*)-N-(4-((4-(benzo[*d*][1,3]dioxol-5-yloxy)-2-methoxy-5-methylphenyl)amino)-7-methoxyquinazolin-6-yl)-2-fluoro-3-(1-methylpyrrolidin-2-yl)acrylamide;
86> (*R,Z*)-N-(4-((4-(benzo[*d*][1,3]dioxol-5-yloxy)-2-methoxy-5-methylphenyl)amino)-7-methoxyquinazolin-6-yl)-2-fluoro-3-(1-methylpyrrolidin-2-yl)acrylamide;
87> (*R,Z*)-2-fluoro-*N*-(7-methoxy-4-((2-methoxy-5-methyl-4-(quinolin-7-yloxy)phenyl)amino)quinazolin-6-yl)-3-(1-methylpyrrolidin-2-yl)acrylamide;
88> (*R,E*)-2-fluoro-*N*-(7-methoxy-4-((2-methoxy-5-methyl-4-(quinolin-7-yloxy)phenyl)amino)quinazolin-6-yl)-3-(1-methylpyrrolidin-2-yl)acrylamide;
89> (*R,E*)-2-fluoro-*N*-(7-methoxy-4-((2-methoxy-5-methyl-4-((1-methyl-1*H*-indazol-6-yl)oxy)phenyl)amino)quinazolin-6-yl)-3-(1-methylpyrrolidin-2-yl)acrylamide;
90> (R,Z)-2-fluoro-*N*-(7-methoxy-4-((2-methoxy-5-methyl-4-((1-methyl-1*H*-indazol-6-yl)oxy)phenyl)amino)quinazolin-6-yl)-3-(1-methylpyrrolidin-2-yl)acrylamide;
91> (*R*,*E*)-*N*-(4-((4-([1,2,4]triazolo[1,5-*a*]pyridin-7-yloxy)-2-methoxy-5-methylphenyl)amino)quinazolin-6-yl)-2-fluoro-3-(1-methylpyrrolidin-2-yl)acrylamide;
92> (*R*,*Z*)-*N*-(4-((4-([1,2,4]triazolo[1,5-*a*]pyridin-7-yloxy)-2-methoxy-5-methylphenyl)amino)quinazolin-6-yl)-2-fluoro-3-(1-methylpyrrolidin-2-yl)acrylamide;
93> (*R*,*E*)-*N*-(4-((4-([1,2,4]triazolo[1,5-*a*]pyridin-7-yloxy)-2-methoxy-5-methylphenyl)amino)-7-ethoxyquinazolin-6-yl)-2-fluoro-3-(1-methylpyrrolidin-2-yl)acrylamide;
94> (*R*,*E*)-*N*-(4-((4-([1,2,4]triazolo[1,5-*a*]pyridin-7-yloxy)-2-methoxy-5-methylphenyl)amino)-7-(2-methoxyethoxy)quinazolin-6-yl)-2-fluoro-3-(1-methylpyrrolidin-2-yl)acrylamide;
95> (*R*,*Z*)-*N*-(4-((4-([1,2,4]triazolo[1,5-*a*]pyridin-7-yloxy)-2-methoxy-5-methylphenyl)amino)-7-(2-methoxyethoxy)quinazolin-6-yl)-2-fluoro-3-(1-methylpyrrolidin-2-yl)acrylamide;
96> (*E*)-*N*-(4-((4-([1,2,4]triazolo[1,5-*a*]pyridin-7-yloxy)-2-methoxy-5-methylphenyl)amino)-7-((tetrahydrofuran-3-yl)oxy)quinazolin-6-yl)-2-fluoro-3-((*R*)-1-methylpyrrolidin-2-yl)acrylamide;
97> (*Z*)-*N*-(4-((4-([1,2,4]triazolo[1,5-*a*]pyridin-7-yloxy)-2-methoxy-5-methylphenyl)amino)-7-((tetrahydrofuran-3-yl)oxy)quinazolin-6-yl)-2-fluoro-3-((*R*)-1-methylpyrrolidin-2-yl)acrylamide;
98> (*R*,*Z*)-*N*-(4-((4-([1,2,4]triazolo[1,5-*a*]pyridin-7-yloxy)-2-methoxy-5-methylphenyl)amino)-7-(difluoromethoxy)quinazolin-6-yl)-2-fluoro-3-(1-methylpyrrolidin-2-yl)acrylamide;
99> (*R*,*E*)-*N*-(4-((4-([1,2,4]triazolo[1,5-*a*]pyridin-7-yloxy)-2-methoxy-5-methylphenyl)amino)-7-(difluoromethoxy)quinazolin-6-yl)-2-fluoro-3-(1-methylpyrrolidin-2-yl)acrylamide;
100> (*R*,*E*)-*N*-(4-((4-([1,2,4]triazolo[1,5-*a*]pyridin-7-yloxy)-2-methoxy-5-methylphenyl)amino)-7-(2-morpholinoethoxy)quinazolin-6-yl)-2-fluoro-3-(1-methylpyrrolidin-2-yl)acrylamide;
101> (*R*,*Z*)-*N*-(4-((4-([1,2,4]triazolo[1,5-*a*]pyridin-7-yloxy)-2-methoxy-5-methylphenyl)amino)-7-(2-morpholinoethoxy)quinazolin-6-yl)-2-fluoro-3-(1-methylpyrrolidin-2-yl)acrylamide;
102> (*R*,*E*)-*N*-(4-((4-([1,2,4]triazolo[1,5-*a*]pyridin-7-yloxy)-2-methoxy-5-methylphenyl)amino)-7-fluoroquinazolin-6-yl)-2-fluoro-3-(1-methylpyrrolidin-2-yl)acrylamide;
103> (*R,Z*)-N-(4-((4-([1,2,4]triazolo[1,5-*a*]pyridin-7-yloxy)-2-methoxy-5-methylphenyl)amino)-7-fluoroquinazolin-6-yl)-2-fluoro-3-(1-methylpyrrolidin-2-yl)acrylamide;
104> (*R,E*)*-N-*(4-((4-([1,2,4]triazolo[1,5-*a*]pyridin-7-yloxy)-2-ethoxy-5-methylphenyl)amino)-7-methoxyquinazolin-6-yl)-2-fluoro-3-(1-methylpyrrolidin-2-yl)acrylamide;
105> (*R,E*)*-N-*(4-((4-([1,2,4]triazolo[1,5-*a*]pyridin-7-yloxy)-5-methyl-2-(trifluoromethoxy)phenyl)amino)-7-methoxyquinazolin-6-yl)-2-fluoro-3-(1-methylpyrrolidin-2-yl)acrylamide;
106> (*R,E*)*-N-(*4-((4-([1,2,4]triazolo[1,5-*a*]pyridin-7-yloxy)-2-cyclopropoxy-5-methylphenyl)amino)-7-methoxyquinazolin-6-yl)-2-fluoro-3-(1-methylpyrrolidin-2-yl)acrylamide;
107> (*R,Z*)-N-(4-((4-([1,2,4]triazolo[1,5-*a*]pyridin-7-yloxy)-2-cyclopropoxy-5-methylphenyl)amino)-7-methoxyquinazolin-6-yl)-2-fluoro-3-(1-methylpyrrolidin-2-yl)acrylamide;
108> (*R*,*E*)-*N*-(4-((4-([1,2,4]triazolo[1,5-*a*]pyridin-7-yloxy)-5-methyl-2-(oxetan-3-yloxy)phenyl)amino)-7-methoxyquinazolin-6-yl)-2-fluoro-3-(1-methylpyrrolidin-2-yl)acrylamide;
109> *tert*-butyl (*R*,*E*)-2-(3-((4-((4-([1,2,4]triazolo[1,5-*a*]pyridin-7-yloxy)-2-(oxetan-3-yloxy)phenyl)amino)-7-methoxyquinazolin-6-yl)amino)-2-fluoro-3-oxoprop-1-en-1-yl)pyrrolidine-1-carboxylate;
110> (*E*)-*N*-(4-((4-([1,2,4]triazolo[1,5-*a*]pyridin-7-yloxy)-2-methoxy-5-methylphenyl)amino)-7-methoxyquinazolin-6-yl)-2-fluoro-3-((3*R*)-hexahydro-1*H*-pyrrolizin-3-yl)acrylamide;
111> (*R*,*Z*)-*N*-(4-((4-([1,2,4]triazolo[1,5-*a*]pyridin-7-yloxy)-2-methoxy-5-methylphenyl)amino)-7-methoxyquinazolin-6-yl)-2-fluoro-3-(1-methylpyrrolidin-2-yl)acrylamide;
112> (*S*,*Z*)-*N*-(4-((4-([1,2,4]triazolo[1,5-*a*]pyridin-7-yloxy)-2-methoxy-5-methylphenyl)amino)-7-methoxyquinazolin-6-yl)-2-fluoro-3-(1-methylpyrrolidin-2-yl)acrylamide;
113> (*R*,*E*)-*N*-(4-((4-([1,2,4]triazolo[1,5-*a*]pyridin-7-yloxy)-2-methoxy-5-methylphenyl)amino)-7-(4,4-difluoropiperidin-1-yl)quinazolin-6-yl)-2-fluoro-3-(1-methylpyrrolidin-2-yl)acrylamide;
114> (*R*,*Z*)-*N*-(4-((4-([1,2,4]triazolo[1,5-*a*]pyridin-7-yloxy)-2-methoxy-5-methylphenyl)amino)-7-(3,3-difluoroazetidin-1-yl)quinazolin-6-yl)-2-fluoro-3-(1-methylpyrrolidin-2-yl)acrylamide;
115> (*R*,*E*)-*N*-(4-((4-([1,2,4]triazolo[1,5-*a*]pyridin-7-yloxy)-2-methoxy-5-methylphenyl)amino)-7-(4-morpholinopiperidin-1-yl)quinazolin-6-yl)-2-fluoro-3-(1-methylpyrrolidin-2-yl)acrylamide;
116> (*R*,*E*)-*N*-(4-((4-([1,2,4]triazolo[1,5-*a*]pyridin-7-yloxy)-2-methoxy-5-methylphenyl)amino)-7-(2-oxa-6-azaspiro[3.3]heptan-6-yl)quinazolin-6-yl)-2-fluoro-3-(1-methylpyrrolidin-2-yl)acrylamide;
117> *N*-(4-((4-([1,2,4]triazolo[1,5-*a*]pyridin-7-yloxy)-2-methoxy-5-methylphenyl)amino)-7-(2-oxa-6-azaspiro[3.3]heptan-6-yl)quinazolin-6-yl)acrylamide;
118> (*R*,*E*)-*N*-(4-((4-([1,2,4]triazolo[1,5-*a*]pyridin-7-yloxy)-2-methoxy-5-methylphenyl)amino)-7-(methylamino)quinazolin-6-yl)-2-fluoro-3-(1-methylpyrrolidin-2-yl)acrylamide;
119> *(R,E)-N-(*4-((4-([1,2,4]triazolo[1,5-*a*]pyridin-7-yloxy)-2-methoxy-6-methylphenyl)amino)-7-methoxyquinazolin-6-yl)-2-fluoro-3-(1-methylpyrrolidin-2-yl)acrylamide;
120> (*R*,*Z*)-*N*-(4-((4-([1,2,4]triazolo[1,5-*a*]pyridin-7-yloxy)-2-methoxy-5-methylphenyl)amino)-7-ethoxyquinazolin-6-yl)-2-fluoro-3-(1-methylpyrrolidin-2-yl)acrylamide;
121> (*R*,*E*)-*N*-(4-((4-([1,2,4]triazolo[1,5-*a*]pyridin-7-yloxy)-2-methoxy-5-methylphenyl)amino)-7-ethoxyquinazolin-6-yl)-2-fluoro-3-(pyrrolidin-2-yl)acrylamide;
*122>* (*S,E*)-*N*-(4-((4-([1,2,4]triazolo[1,5-*a*]pyridin-7-yloxy)-2-methoxy-5-methylphenyl)amino)-7-ethoxyquinazolin-6-yl)-2-fluoro-3-(1-methylpyrrolidin-2-yl)acrylamide;
123> (*R,E*)*-N-*(4-((4-(4-(dimethylamino)phenoxy)-2-methoxy-5-methylphenyl)amino)-7-methoxyquinazolin-6-yl)-2-fluoro-3-(1-methylpyrrolidin-2-yl)acrylamide;
124> methyl (*R,E*)-4-(4-((6-(2-fluoro-3-(1-methylpyrrolidin-2-yl)acrylamido)-7-methoxyquinazolin-4-yl)amino)-5-methoxy-2-methylphenoxy)benzoate;
125> (*R,Z*)-4-(4-((6-(2-fluoro-3-(1-methylpyrrolidin-2-yl)acrylamido)-7-methoxyquinazolin-4-yl)amino)-5-methoxy-2-methylphenoxy)-*N*,*N*-dimethylbenzamide;
126> (*R,E*)-4-(4-((6-(2-fluoro-3-(1-methylpyrrolidin-2-yl)acrylamido)-7-methoxyquinazolin-4-yl)amino)-5-methoxy-2-methylphenoxy)-*N*,*N*-dimethylbenzamide;
*127>* (*R*,*E*)-*N*-(4-((4-([1,2,4]triazolo[1,5-*a*]pyridin-7-yloxy)-5-(dimethylamino)-2-methoxyphenyl)amino)-7-methoxyquinazolin-6-yl)-2-fluoro-3-(1-methylpyrrolidin-2-yl)acrylamide;
128> (*R*,*Z*)-*N*-(4-((4-([1,2,4]triazolo[1,5-*a*]pyridin-7-yloxy)-5-chloro-2-methoxyphenyl)amino)-7-methoxyquinazolin-6-yl)-2-fluoro-3-(1-methylpyrrolidin-2-yl)acrylamide;
129> (*R*,*Z*)-*N*-(4-((4-([1,2,4]triazolo[1,5-*a*]pyridin-7-yloxy)-5-chloro-2-methoxyphenyl)amino)-7-ethoxyquinazolin-6-yl)-2-fluoro-3-(1-methylpyrrolidin-2-yl)acrylamide;
130> (*R*,*E*)-*N*-(4-((4-([1,2,4]triazolo[1,5-*a*]pyridin-7-yloxy)-5-chloro-2-methoxyphenyl)amino)-7-morpholinoquinazolin-6-yl)-2-fluoro-3-(1-methylpyrrolidin-2-yl)acrylamide;
131> (*R*,*Z*)-*N*-(4-((4-([1,2,4]triazolo[1,5-*a*]pyridin-7-yloxy)-2-methoxy-5-methylphenyl)amino)-7-methoxyquinazolin-6-yl)-2-fluoro-3-(1-methylpiperidin-2-yl)acrylamide;
132> (*R*,*E*)-*N*-(4-((4-([1,2,4]triazolo[1,5-*a*]pyridin-7-yloxy)-2-methoxy-5-methylphenyl)amino)-3-cyano-7-ethoxyquinolin-6-yl)-2-fluoro-3-(1-methylpyrrolidin-2-yl)acrylamide;
133> (*R,Z*)-*N*-(4-((4-(3-acetamidophenoxy)-2-methoxy-5-methylphenyl)amino)-7-methoxyquinazolin-6-yl)-2-fluoro-3-(1-methylpyrrolidin-2-yl)acrylamide;
134> (*R*,*E*)-*N*-(4-((4-([1,2,4]triazolo[1,5-*a*]pyridin-7-yloxy)-2-methoxy-5-methylphenyl)amino)-7-methoxyquinazolin-6-yl)-2-fluoro-3-(pyrrolidin-2-yl)acrylamide;
135> (*R*,*Z*)-*N*-(4-((4-([1,2,4]triazolo[1,5-*a*]pyridin-7-yloxy)-2-methoxy-5-methylphenyl)amino)-7-methoxyquinazolin-6-yl)-2-fluoro-3-(pyrrolidin-2-yl)acrylamide;
136> (*R*,*E*)-*N*-(4-((4-([1,2,4]triazolo[1,5-*a*]pyridin-7-yloxy)-2-methoxy-5-methylphenyl)amino)-7-methoxyquinazolin-6-yl)-2-chloro-3-(1-methylpyrrolidin-2-yl)acrylamide;
137> (*R,Z*)-N-(4-((4-([1,2,4]triazolo[1,5-*a*]pyridin-7-yloxy)-5-cyclopropyl-2-methoxyphenyl)amino)-7-methoxyquinazolin-6-yl)-2-fluoro-3-(1-methylpyrrolidin-2-yl)acrylamide;
138> (*R,E*)*-N-(*4-((4-([1,2,4]triazolo[1,5-*a*]pyridin-7-yloxy)-5-cyclopropyl-2-methoxyphenyl)amino)-7-methoxyquinazolin-6-yl)-2-fluoro-3-(1-methylpyrrolidin-2-yl)acrylamide;
139> (*R,Z*)-*N*-(4-((4-([1,2,4]triazolo[1,5-*a*]pyridin-7-yloxy)-5-bromo-2-methoxyphenyl)amino)-7-methoxyquinazolin-6-yl)-2-fluoro-3-(1-methylpyrrolidin-2-yl)acrylamide;
140> (*R,E*)*-N-*(4-((4-([1,2,4]triazolo[1,5-*a*]pyridin-7-yloxy)-5-bromo-2-methoxyphenyl)amino)-7-methoxyquinazolin-6-yl)-2-fluoro-3-(1-methylpyrrolidin-2-yl)acrylamide;
141> (*R*,*Z*)-*N*-(4-((4-1,5-*a*]pyridin-7-yloxy)-2-methoxy-5-methylphenyl)amino)-5-chloroquinazolin-6-yl)-2-fluoro-3-(1-methylpyrrolidin-2-yl)acrylamide;
142> (*R*,*E*)-*N*-(4-((4-([1,2,4]triazolo[1,5-*a*]pyridin-7-yloxy)-2-methoxy-5-methylphenyl)amino)-5-chloroquinazolin-6-yl)-2-fluoro-3-(1-methylpyrrolidin-2-yl)acrylamide;
143> (*R,Z*)-2-fluoro-N-(7-methoxy-4-((2-methoxy-5-methyl-4-((5-methyl-[1,2,4]triazolo[1,5-*a*]pyridin-7-yl)oxy)phenyl)amino)quinazolin-6-yl)-3-(1-methylpyrrolidin-2-yl)acrylamide;
144> (*Z*)-*N*-(4-((4-([1,2,4]triazolo[1,5-*a*]pyridin-7-yloxy)-2-methoxy-5-methylphenyl)amino)-7-methoxyquinazolin-6-yl)-2-cyano-4-methyl-4-(4-(oxetan-3-yl)piperazin-1-yl)pent-2-enamide;
145> (*E*)-*N*-(4-((4-([1,2,4]triazolo[1,5-*a*]pyridin-7-yloxy)-2-methoxy-5-methylphenyl)amino)-7-methoxyquinazolin-6-yl)-2-fluoro-4-methyl-4-(4-(oxetan-3-yl)piperazin-1-yl)pent-2-enamide;
146> (*E*)-*N*-(4-((4-([1,2,4]triazolo[1,5-*a*]pyridin-7-yloxy)-2-methoxy-5-methylphenyl)amino)-7-methoxyquinazolin-6-yl)-2-fluoro-4,4-dimethylpent-2-enamide;
147> *N*-(4-((4-([1,2,4]triazolo[1,5-*a*]pyridin-7-yloxy)-2-methoxy-5-methylphenyl)amino)-7-methoxyquinazolin-6-yl)-2-(1*H*-pyrazol-1-yl)acrylamide;
148> (*R,E*)*-N-*(4-((4-([1,2,4]triazolo[1,5-*a*]pyridin-7-yloxy)-2-(methoxy-*d*₃)-5-methylphenyl)amino)-7-methoxyquinazolin-6-yl)-3-(1-methylpyrrolidin-2-yl)acrylamide;
149> (*R,Z*)-*N*-(4-((4-([1,2,4]triazolo[1,5-*a*]pyridin-7-yloxy)-2-(methoxy-*d*₃)-5-methylphenyl)amino)-7-methoxyquinazolin-6-yl)-2-fluoro-3-(1-methylpyrrolidin-2-yl)acrylamide;
150> (*R,E*)*-N-*(4-((4-([1,2,4]triazolo[1,5-*a*]pyridin-7-yloxy)-2-(methoxy-*d*₃)-5-methylphenyl)amino)-7-methoxyquinazolin-6-yl)-2-fluoro-3-(1-methylpyrrolidin-2-yl)acrylamide;
151> (*R,Z*)-*N*-(4-((4-([1,2,4]triazolo[1,5-*a*]pyridin-7-yloxy)-5-fluoro-2-(methoxy-*d*₃)phenyl)amino)-7-methoxyquinazolin-6-yl)-2-fluoro-3-(1-methylpyrrolidin-2-yl)acrylamide;
152> (*R,E*)-N-(4-((4-([1,2,4]triazolo[1,5-*a*]pyridin-7-yloxy)-5-fluoro-2-(methoxy-*d*₃)phenyl)amino)-7-methoxyquinazolin-6-yl)-2-fluoro-3-(1-methylpyrrolidin-2-yl)acrylamide;
153> (*R,E*)-N-(4-((4-([1,2,4]triazolo[1,5-*a*]pyridin-7-yloxy)-2-methoxy-5-methylphenyl)amino)-7-methoxyquinazolin-6-yl)-3-(1-(methyl-*d*₃)pyrrolidin-2-yl)acrylamide;
154> (*R,Z*)-*N*-(4-((4-([1,2,4]triazolo[1,5-*a*]pyridin-7-yloxy)-2-methoxy-5-methylphenyl)amino)-7-methoxyquinazolin-6-yl)-2-fluoro-3-(1-(methyl-*d*₃)pyrrolidin-2-yl)acrylamide;
155> (*R,Z*)-*N*-(4-(((4-([1,2,4]triazolo[1,5-*a*]pyridin-7-yloxy)-5-fluoro-2-methoxyphenyl)amino)-7-methoxyquinazolin-6-yl)-2-fluoro-3-(1-(methyl-*d*₃)pyrrolidin-2-yl)acrylamide;
156> (*R,Z*)-*N*-(4-((4-([1,2,4]triazolo[1,5-*a*]pyridin-7-yloxy)-5-chloro-2-methoxyphenyl)amino)-7-methoxyquinazolin-6-yl)-2-fluoro-3-(1-(methyl-*d*₅)pyrrolidin-2-yl)acrylamide; and
157> (*R*,*E*)-*N*-(4-((4-([1,2,4]triazolo[1,5-*a*]pyridin-7-yloxy)-5-fluoro-2-methoxyphenyl)amino)-7-methoxyquinazolin-6-yl)-2-fluoro-3-(1-(methyl-*d*₃)pyrrolidin-2-yl)acrylamide.

8. A pharmaceutical composition for treating or preventing human epidermal growth factor receptor 2 (HER2)-related diseases, the pharmaceutical composition comprising the compound of Formula (I) according to any one of claims 1 to 7, or a pharmaceutically acceptable salt or stereoisomer thereof as an active ingredient.

9. The pharmaceutical composition of claim 8, wherein the HER2-related diseases comprise cancer.

10. The pharmaceutical composition of claim 8, wherein the HER2-related diseases are selected from the group consisting of pseudomyxoma, intrahepatic cholangiocarcinoma, hepatoblastoma, liver cancer, thyroid cancer, colon cancer, testicular cancer, myelodysplastic syndrome, glioblastoma, oral cancer, lip cancer, mycosis fungoides, acute myeloid leukemia, acute lymphoblastic leukemia, basal cell carcinoma, ovarian epithelial cancer, ovarian germ cell carcinoma, male breast cancer, brain cancer, pituitary adenoma, multiple myeloma, gallbladder cancer, biliary tract cancer, colorectal cancer, chronic myelogenous leukemia, chronic lymphocytic leukemia, retinoblastoma, choroidal melanoma, ampulla of Vater cancer, bladder cancer, peritoneal cancer, parathyroid cancer, adrenal cancer, sinonasal cancer, non-small cell lung cancer, tongue cancer, astrocytoma, small cell lung cancer, pediatric brain cancer, pediatric lymphoma, pediatric leukemia, small bowel cancer, meningioma, esophageal cancer, glioma, renal pelvis cancer, renal cancer, heart cancer, duodenal cancer, malignant soft tissue tumor, malignant bone cancer, malignant lymphoma, malignant mesothelioma, malignant melanoma, eye cancer, vulvar cancer, ureteral cancer, urethral cancer, cancer of unknown primary site, gastric lymphoma, gastric cancer, gastric carcinoid, gastrointestinal stromal cancer, Wilms' tumor, breast cancer, sarcoma, penile cancer, pharyngeal cancer, gestational choriocarcinoma, cervical cancer, endometrial cancer, uterine sarcoma, prostate cancer, metastatic bone cancer, metastatic brain cancer, mediastinal cancer, rectal cancer, rectal carcinoid, vaginal cancer, spinal cord cancer, vestibular schwannoma, pancreatic cancer, salivary gland cancer, Kaposi's sarcoma, Paget's disease, tonsil cancer, squamous cell carcinoma, lung adenocarcinoma, lung cancer, squamous cell carcinoma of lung, skin cancer, anal cancer, rhabdomyosarcoma, laryngeal cancer, pleura cancer, hematological cancer, and thymus cancer.

11. A pharmaceutical composition comprising the compound of Formula (I) according to any one of claims 1 to 7, or a pharmaceutically acceptable salt or stereoisomer thereof, and a pharmaceutically acceptable additive.

12. Use of the compound of Formula (I) according to any one of claims 1 to 7, or a pharmaceutically acceptable salt or stereoisomer thereof for treating or preventing HER2-related diseases.

13. Use of the compound of Formula (I) according to any one of claims 1 to 7, or a pharmaceutically acceptable salt or stereoisomer thereof for preparing a medicament for treating or preventing HER2-related diseases.

14. A method for treating or preventing HER2-related diseases, comprising administering to a subject in need thereof, a therapeutically effective amount of the compound of Formula (I) according to any one of claims 1 to 7 or a pharmaceutically acceptable salt or stereoisomer thereof.

15. A method for treating or preventing at least one disease selected from the group consisting of pseudomyxoma, intrahepatic cholangiocarcinoma, hepatoblastoma, liver cancer, thyroid cancer, colon cancer, testicular cancer, myelodysplastic syndrome, glioblastoma, oral cancer, lip cancer, mycosis fungoides, acute myeloid leukemia, acute lymphoblastic leukemia, basal cell carcinoma, ovarian epithelial cancer, ovarian germ cell carcinoma, male breast cancer, brain cancer, pituitary adenoma, multiple myeloma, gallbladder cancer, biliary tract cancer, colorectal cancer, chronic myelogenous leukemia, chronic lymphocytic leukemia, retinoblastoma, choroidal melanoma, ampulla of Vater cancer, bladder cancer, peritoneal cancer, parathyroid cancer, adrenal cancer, sinonasal cancer, non-small cell lung cancer, tongue cancer, astrocytoma, small cell lung cancer, pediatric brain cancer, pediatric lymphoma, pediatric leukemia, small bowel cancer, meningioma, esophageal cancer, glioma, renal pelvis cancer, renal cancer, heart cancer, duodenal cancer, malignant soft tissue tumor, malignant bone cancer, malignant lymphoma, malignant mesothelioma, malignant melanoma, eye cancer, vulvar cancer, ureteral cancer, urethral cancer, cancer of unknown primary site, gastric lymphoma, gastric cancer, gastric carcinoid, gastrointestinal stromal cancer, Wilms' tumor, breast cancer, sarcoma, penile cancer, pharyngeal cancer, gestational choriocarcinoma, cervical cancer, endometrial cancer, uterine sarcoma, prostate cancer, metastatic bone cancer, metastatic brain cancer, mediastinal cancer, rectal cancer, rectal carcinoid, vaginal cancer, spinal cord cancer, vestibular schwannoma, pancreatic cancer, salivary gland cancer, Kaposi's sarcoma, Paget's disease, tonsil cancer, squamous cell carcinoma, lung adenocarcinoma, lung cancer, squamous cell carcinoma of lung, skin cancer, anal cancer, rhabdomyosarcoma, laryngeal cancer, pleura cancer, hematological cancer, and thymus cancer, the method comprising administering to a subject in need thereof, a therapeutically effective amount of the compound of Formula (I) according to any one of claims 1 to 7 or a pharmaceutically acceptable salt or stereoisomer thereof.
